# EUROPEAN PATENT SPECIFICATION

(11) **EP 3 840 739 B1**
(45) Date of publication and mention of the grant of the patent: **12.11.2025**
(21) Application number: 19851394.7
(22) Date of filing: 23.08.2019
(51) Int. Cl.: A61K 31/167, A61K 31/165, A61P 19/02, A61P 29/02, A61F 7/10, A61F 7/00, A61K 9/00, A61K 9/08, A61K 47/26, A61K 47/32, A61K 47/40, A61K 47/44, A61P 23/02, A61P 29/00

(54) **CAPSAICIN SEQUENTIAL DOSING METHOD FOR TREATMENT OF KNEE JOINT PAIN**
VERFAHREN ZUR SEQUENZIELLEN DOSIERUNG VON CAPSAICIN ZUR BEHANDLUNG VON KNIEGELENKSCHMERZEN
MÉTHODE DE DOSAGE SÉQUENTIEL DE LA CAPSAÏCINE POUR LE TRAITEMENT D'UNE DOULEUR AU NIVEAU DE L'ARTICULATION DU GENOU

(30) Priority: 24.08.2018 US 201862722396 P
(43) Date of publication of application: 30.06.2021
(73) Proprietor: Centrexion Therapeutics Corporation, Boston, MA 02109 (US)
(72) Inventor: CAMPBELL, James N., Baltimore, Maryland 21230 (US); HANSON, Peter D., Prides Crossing, Massachusetts 01965 (US); STEVENS, Randall, Rockport, Massachusetts 01966 (US)
(74) Representative: Graham Watt & Co
(86) International application number: PCT/US2019/047823
(87) International publication number: WO 2020/041658

(56) References cited:
- WO-A1-2017/127628
- WO-A1-2018/085476
- WO-A2-2014/075084
- US-A1- 2015 133 561
- ANONYMOUS: "Study to Evaluate the Safety and Efficacy of CNTX-4975 in Subjects With Chronic, Moderate to Severe Osteoarthritis Knee Pain", CLINICALTRIALS.GOV, 24 September 2015 (2015-09-24), Internet, pages 1 - 7, XP055910981, Retrieved from the Internet <URL:https://clinicaltrials.gov/ct2/show/record/NCT02558439?view=record> [retrieved on 20220408]
- STEVENS R ET AL: "(382) Safety and Tolerability of CNTX-4975 in Subjects with Chronic, Moderate to Severe Knee Pain Associated With Osteoarthritis (OA): A Pilot Study", JOURNAL OF PAIN, vol. 18, no. 4, 1 June 2020 (2020-06-01), XP029960246, ISSN: 1526-5900, DOI: 10.1016/J.JPAIN.2017.02.356

## Description

### FIELD OF THE INVENTION

The invention provides compositions for use in treating knee joint pain in a patient, such as knee joint pain due to osteoarthritis, while minimizing transient burning sensation experienced by patients due to capsaicin administration, the use comprising sequential dosing of capsaicin.

### BACKGROUND

Pain can function as a protective mechanism that allows healthy human beings and animals to avoid tissue damage and/or prevent further damage to injured tissue. However, there are many instances in which pain persists beyond its usefulness. Such unnecessary suffering from pain can impair a subject's physical mobility, mental performance, and even contribute to depression.

Substantial resources have been devoted over the years to researching the causes of various types of pain and to the development of medicine to attenuate pain experienced by a patient. Exemplary classes of common pain-relief medications include opioids, non-steroidal anti-inflammatory agents, corticosteroids, and centrally acting agents such as anti-depressants, anti-epileptics, pregabalin, and gabapentin. Capsaicin has been described for use in treating pain. See, for example, U.S. Patent Nos. 5,962,532; 8,420,600; 8,367,733; and 8,158,682. Certain commercial products containing capsaicin for pain relief formulate the capsaicin as a cream (e.g., Capzasin) or in a patch (e.g., a capsaicin-containing transdermal patch marketed under the trade name QUTENZA^{®}) for topical application to the skin of a patient.

One type of pain that affects a substantial number of patients is osteoarthritic knee joint pain. Osteoarthritic knee joint pain can be debilitating. Patients suffering from osteoarthritic knee joint pain are often impaired in their ability to perform simple daily tasks such as walking or climbing stairs. The pain may be felt even while sitting in a chair or lying in bed and may interfere with ability to sleep.

The following documents relate to the use of capsaicin for use in treating knee pain: WO2018/085476; "Study to Evaluate the Safety and Efficacy of CNTX-4975 in Subjects with Chronic, Moderate to Severe Osteoartritis Knee Pain" - ClinicalTrials.gov, 24 September 2015, pages 1-7; "Safety and Tolerability of CNTX-4975 in Subjects with Chronic, Moderate to Severe Knee Pain Associated with Osteoartritis: a Pilot Study", Journal of Pain, vol. 18, no.4. Long duration relief from osteoarthritic knee j oint pain would provide a substantial benefit to patients suffering from osteoarthritic knee joint pain.

Accordingly, the need exists for long duration relief from knee joint pain. The present invention addresses this need and provides other related advantages.

### SUMMARY

The invention is defined by the appended claims.

The invention provides compositions for use in treating knee joint pain in a patient, such as knee joint pain due to osteoarthritis, while minimizing transient burning sensation experienced by patients due to capsaicin administration, the use comprising sequential dosing of capsaicin. The uses desirably provide relief from joint pain, such as osteoarthritic knee joint pain, for an extended duration, such as at least about 8 months, 10 months, 12 months, 18 months, or 24 months. Because administration of capsaicin causes initial neuronal excitation resulting in the adverse side effect of a transient burning sensation, the usesdesirably use a cooling article, such as a material wrap cooled via a circulating fluid, to reduce the temperature of tissue to be exposed to capsaicin for certain durations of time, optionally in combination with administering a local anesthetic agent, in order to attenuate the transient burning sensation experienced by patients, resulting in the substantial reduction or even elimination of transient burning sensation caused by capsaicin. The cooling article desirably has an exterior surface temperature in the range of from about 5°C to about 15°C, and more desirably from about 5°C to about 10°C, for application to the exterior surface of the patient's knee.

Because overcooling of skin tissue can cause the adverse effect of skin necrosis, while insufficient cooling can be inadequate to sufficiently reduce the transient burning sensation experienced by patients due to capsaicin administration, the uses desirably apply a cooling article having a particular temperature range (e.g., from about 5°C to about 15°C, and more desirably from about 5°C to about 10°C) for particular durations of time both before and after administration of capsaicin. The therapeutic usess can be further characterized according to the temperature of tissue and/or fluid in the joint into which capsaicin is administered, and in certain embodiments, fluid in the intra-articular space of the knee joint, is cooled to a temperature in the range from about 26°C to about 30°C prior to administration of capsaicin, and then maintained at a temperature in the range from about 26°C to about 33°C for a duration of at least 30 minutes after administration of capsaicin.

Various aspects and embodiments of the invention are described in further detail below. Accordingly, one aspect of the invention provides capsaicin for use in ameliorating osteoarthritic knee joint pain in a human patient for a duration of at least 8 months. The use comprises administering by injection into the intra-articular space of the joint of said knee at least a first dose of capsaicin and a second dose of capsaicin to thereby ameliorate osteoarthritic knee joint pain in the human patient for a duration of at least 8 months, wherein the use is characterized by:
(i) the first dose of capsaicin is an amount of 1 mg capsaicin;
(ii) the second dose of capsaicin is an amount of 1 mg capsaicin;
(iii) the second dose of capsaicin is administered no sooner than 5 months after administration of the first dose of capsaicin;
(iv) if any additional dose of capsaicin is administered by injection into the intra-articular space of the joint of said knee, any such additional dose is in an amount of 1 mg of capsaicin and any said additional dose is administered no sooner than 3 months after administration of the prior dose of capsaicin administered by injection into the intra-articular space of the joint of said knee; and
(v) when administering the first dose of capsaicin:
   (a) a cooling article is applied for a duration of 15 minutes to an exterior surface of the human patient's knee presenting with osteoarthritic knee joint pain, wherein the cooling article has an exterior surface temperature in the range of from 3°C to 15°C for application to the exterior surface of said knee; then
   (b) a pharmaceutical composition comprising a single pain-relief agent selected from the group consisting of lidocaine and a pharmaceutically acceptable salt thereof is administered by injection into the intra-articular space of the joint of said knee in order to deliver a dose of lidocaine in an amount ranging from 0.1 g to 0.5 g; then
   (c) a cooling article is applied for a duration of about 30 minutes to an exterior surface of said knee, wherein the cooling article has an exterior surface temperature in the range of from 3°C to 15°C for application to the exterior surface of said knee; then
   (d) the first dose of capsaicin is administered by injecting into the intra-articular space of the joint of said knee a pharmaceutical composition comprising capsaicin; and then
   (e) a cooling article is applied to an exterior surface of said knee for a duration of at least about 30 minutes, wherein the cooling article has an exterior surface temperature in the range of from 3°C to 15°C for application to the exterior surface of said knee.

Another aspect of the invention provides capsaicin for use in ameliorating knee joint pain in a human patient for a duration of at least 8 months. The use comprises administering by injection into the intra-articular space of the joint of said knee at least a first dose of capsaicin and a second dose of capsaicin to thereby ameliorate knee joint pain in the human patient for a duration of at least 8 months, wherein the use is characterized by:
(i) the first dose of capsaicin is an amount of 1 mg capsaicin;
(ii) the second dose of capsaicin is an amount of 1 mg capsaicin;
(iii) the second dose of capsaicin is administered no sooner than 5 months after administration of the first dose of capsaicin; and
(iv) if any additional dose of capsaicin is administered by injection into the intra-articular space of the joint of said knee, any such additional dose is in an amount of 1 mg of capsaicin and any said additional dose is administered no sooner than 3 months after administration of the prior dose of capsaicin administered by injection into the intra-articular space of the joint of said knee.

In certain embodiments, the second dose of capsaicin is administered no sooner than 4, 6, or 8 months after administration of the first dose of capsaicin. In certain embodiments, the second dose of capsaicin is administered about 6 months after administration of the first dose of capsaicin. In certain embodiments, the method is further characterized by the duration over which pain is ameliorated, e.g., where the pain is ameliorated for a duration of at least 10, 12, 14, 16, or 18 months.

The foregoing therapeutic uses may be further characterized according to various features, such as the time at which the second dose of capsaicin is administered, the time at which any additional dose of capsaicin is administered, the dose of capsaicin, the duration of reduction in pain, and features of the cooling article when used. These and other features are more fully described in the detailed description below.

### BRIEF DESCRIPTION OF THE DRAWINGS

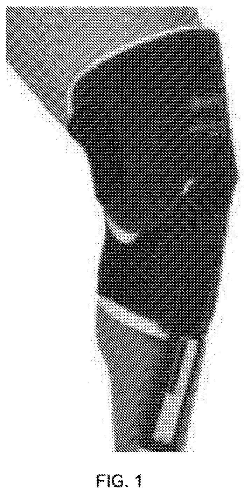

**FIGURE 1** is an illustration of a cooling article, which is a wrap-on pad, applied to a human knee.

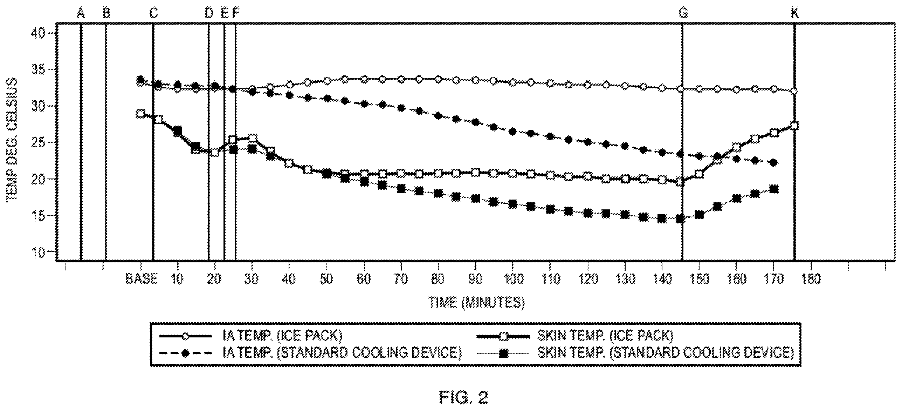

**FIGURE 2** is a graph showing mean intraarticular (IA) temperature and mean skin temperature over time using the following cooling devices: (i) a Breg Knee WrapOn Polar Pad or (ii) Ice Pack, as further described in Example 6. The "Standard Cooling Device" was a Breg Knee WrapOn Polar Pad. The following designations apply to the graph: A is the time at which any temperature reading of the temperature probe was made prior to insertion into the intraarticular space of the patient's knee; B is the time which the temperature probe was inserted into the intraarticular space of the patient's knee; C is time at which the cooling device was applied to the patient's knee; D is the time at which the cooling device was removed from the patient's knee; E is the time at which a solution of 2% w/w lidocaine was administered to the patient's knee by intraarticular injection; F is the time at which the cooling device was reapplied to the patient's knee; G is the time at which the cooling device was removed from the patient's knee; K is the time at which the temperature probe was removed from the patient's knee.

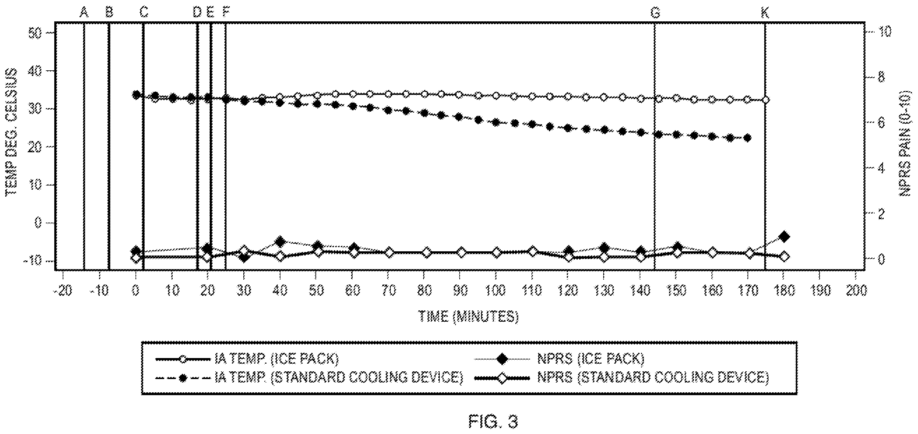

**FIGURE 3** is a graph showing mean intraarticular (IA) temperature and mean NPRS Pain scores over time using the following cooling devices: (i) a Breg Knee WrapOn Polar Pad or (ii) Ice Pack, as further described in Example 6. The "Standard Cooling Device" was a Breg Knee WrapOn Polar Pad. The following designations apply to the graph: A is the time at which any temperature reading of the temperature probe was made prior to insertion into the intraarticular space of the patient's knee; B is the time which the temperature probe was inserted into the intraarticular space of the patient's knee; C is time at which the cooling device was applied to the patient's knee; D is the time at which the cooling device was removed from the patient's knee; E is the time at which a solution of 2% w/w lidocaine was administered to the patient's knee by intraarticular injection; F is the time at which the cooling device was reapplied to the patient's knee; G is the time at which the cooling device was removed from the patient's knee; K is the time at which the temperature probe was removed from the patient's knee.

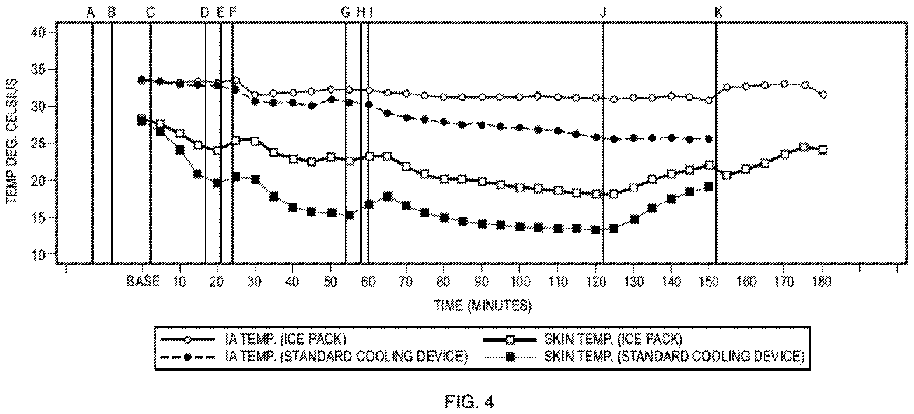

**FIGURE 4** is a graph showing mean intraarticular (IA) temperature and mean skin temperature over time using the following cooling devices: (i) a Breg Knee WrapOn Polar Pad or (ii) Ice Pack, as further described in Example 7. The "Standard Cooling Device" was a Breg Knee WrapOn Polar Pad. The following designations apply to the graph: A is the time at which any temperature reading of the temperature probe was made prior to insertion into the intraarticular space of the patient's knee; B is the time which the temperature probe was inserted into the intraarticular space of the patient's knee; C is time at which the cooling device was applied to the patient's knee; D is the time at which the cooling device was removed from the patient's knee; E is the time at which a solution of 2% w/w lidocaine was administered to the patient's knee by intraarticular injection; F is the time at which the cooling device was reapplied to the patient's knee; G is the time at which the cooling device was removed from the patient's knee; H is the time that *trans*-capsaicin was administered by intraarticular injection; I is the time at which the cooling device was reapplied to the patient's knee; J is the time at which the cooling device was removed from the patient's knee; K is the time at which the temperature probe was removed from the patient's knee.

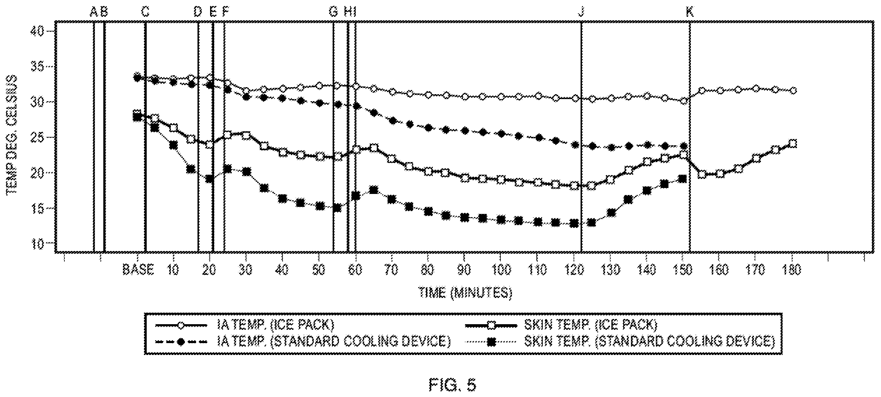

**FIGURE 5** is a graph showing mean intraarticular (IA) temperature and mean skin temperature over time using the following cooling devices: (i) a Breg Knee WrapOn Polar Pad or (ii) Ice Pack, as further described in Example 7. The "Standard Cooling Device" was a Breg Knee WrapOn Polar Pad. The following designations apply to the graph: A is the time at which any temperature reading of the temperature probe was made prior to insertion into the intraarticular space of the patient's knee; B is the time which the temperature probe was inserted into the intraarticular space of the patient's knee; C is time at which the cooling device was applied to the patient's knee; D is the time at which the cooling device was removed from the patient's knee; E is the time at which a solution of 2% w/w lidocaine was administered to the patient's knee by intraarticular injection; F is the time at which the cooling device was reapplied to the patient's knee; G is the time at which the cooling device was removed from the patient's knee; H is the time that *trans*-capsaicin was administered by intraarticular injection; I is the time at which the cooling device was reapplied to the patient's knee; J is the time at which the cooling device was removed from the patient's knee; K is the time at which the temperature probe was removed from the patient's knee.

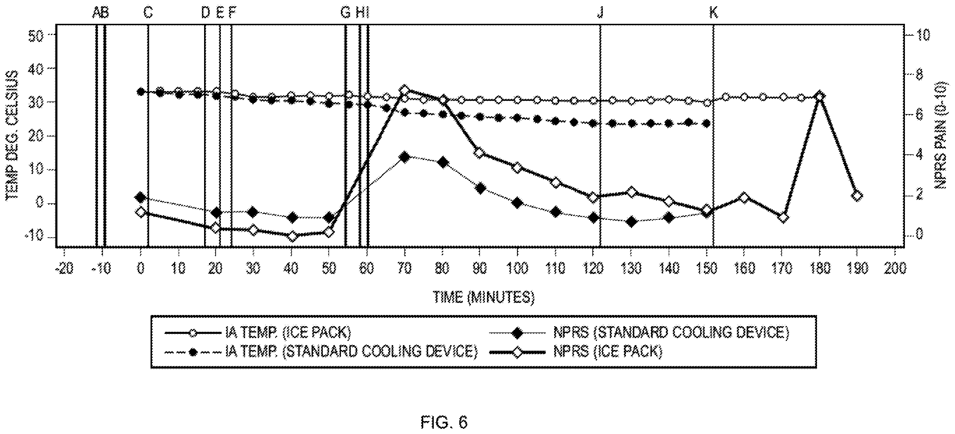

**FIGURE 6** is a graph showing mean intraarticular (IA) temperature and mean NPRS Pain scores over time using the following cooling devices: (i) a Breg Knee WrapOn Polar Pad or (ii) Ice Pack, as further described in Example 7. The "Standard Cooling Device" was a Breg Knee WrapOn Polar Pad. The following designations apply to the graph: A is the time at which any temperature reading of the temperature probe was made prior to insertion into the intraarticular space of the patient's knee; B is the time which the temperature probe was inserted into the intraarticular space of the patient's knee; C is time at which the cooling device was applied to the patient's knee; D is the time at which the cooling device was removed from the patient's knee; E is the time at which a solution of 2% w/w lidocaine was administered to the patient's knee by intraarticular injection; F is the time at which the cooling device was reapplied to the patient's knee; G is the time at which the cooling device was removed from the patient's knee; H is the time that *trans*-capsaicin was administered by intraarticular injection; I is the time at which the cooling device was reapplied to the patient's knee; J is the time at which the cooling device was removed from the patient's knee; K is the time at which the temperature probe was removed from the patient's knee.

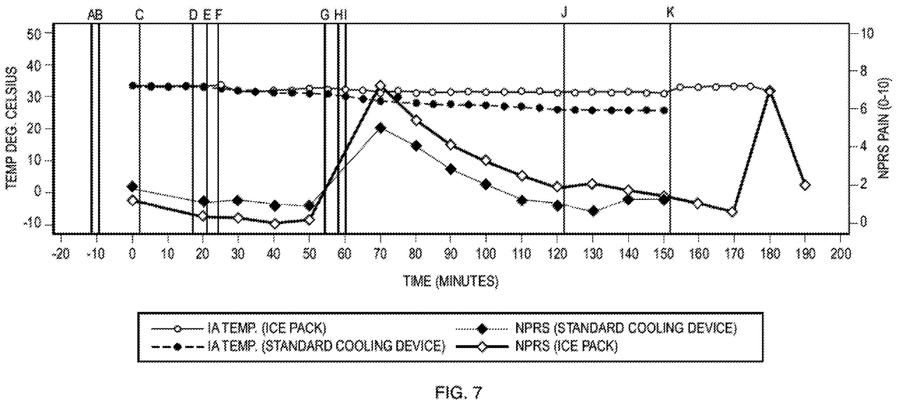

**FIGURE 7** is a graph showing mean intraarticular (IA) temperature and mean NPRS Pain scores over time using the following cooling devices: (i) a Breg Knee WrapOn Polar Pad or (ii) Ice Pack, as further described in Example 7. The "Standard Cooling Device" was a Breg Knee WrapOn Polar Pad. The following designations apply to the graph: A is the time at which any temperature reading of the temperature probe was made prior to insertion into the intraarticular space of the patient's knee; B is the time which the temperature probe was inserted into the intraarticular space of the patient's knee; C is time at which the cooling device was applied to the patient's knee; D is the time at which the cooling device was removed from the patient's knee; E is the time at which a solution of 2% w/w lidocaine was administered to the patient's knee by intraarticular injection; F is the time at which the cooling device was reapplied to the patient's knee; G is the time at which the cooling device was removed from the patient's knee; H is the time that *trans*-capsaicin was administered by intraarticular injection; I is the time at which the cooling device was reapplied to the patient's knee; J is the time at which the cooling device was removed from the patient's knee; K is the time at which the temperature probe was removed from the patient's knee.

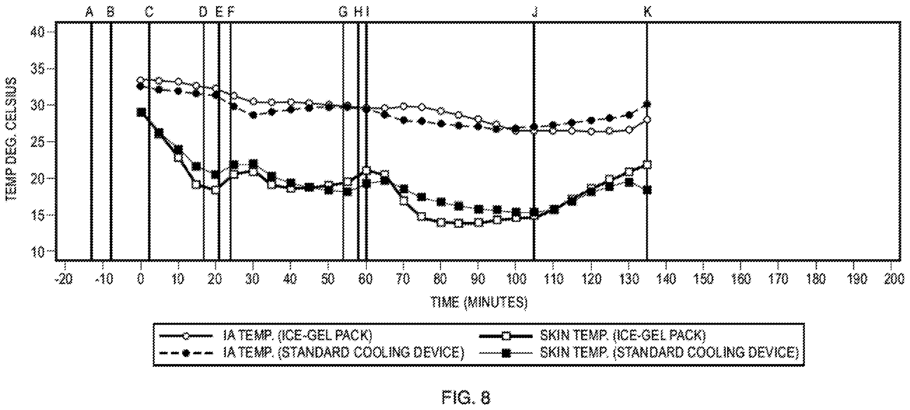

**FIGURE 8** is a graph showing mean intraarticular (IA) temperature and mean skin temperature over time using the following cooling devices: (i) a Breg Knee WrapOn Polar Pad or (ii) Elasto-Gel All Purpose Therapy Wrap, as further described in Example 8. The "Standard Cooling Device" was a Breg Knee WrapOn Polar Pad. The "Ice-Gel Pack" was an Elasto-Gel All Purpose Therapy Wrap. The following designations apply to the graph: A is the time at which any temperature reading of the temperature probe was made prior to insertion into the intraarticular space of the patient's knee; B is the time which the temperature probe was inserted into the intraarticular space of the patient's knee; C is time at which the cooling device was applied to the patient's knee; D is the time at which the cooling device was removed from the patient's knee; E is the time at which a solution of 2% w/w lidocaine was administered to the patient's knee by intraarticular injection; F is the time at which the cooling device was reapplied to the patient's knee; G is the time at which the cooling device was removed from the patient's knee; H is the time that *trans*-capsaicin was administered by intraarticular injection; I is the time at which the cooling device was reapplied to the patient's knee; J is the time at which the cooling device was removed from the patient's knee; K is the time at which the temperature probe was removed from the patient's knee.

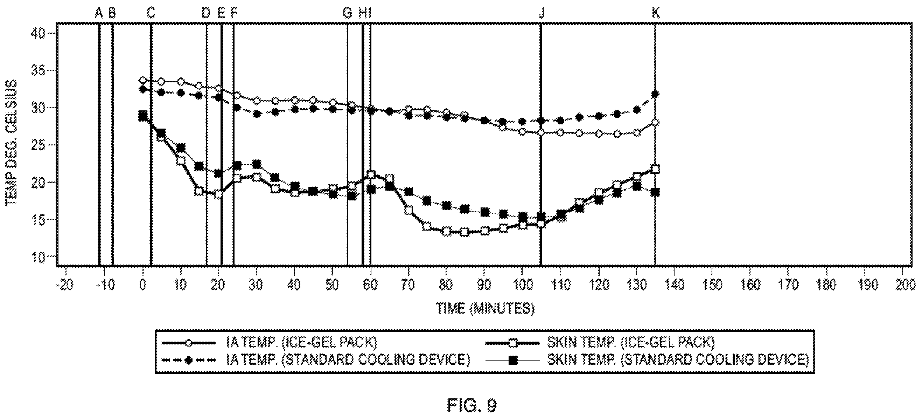

**FIGURE 9** is a graph showing mean intraarticular (IA) temperature and mean skin temperature over time using the following cooling devices: (i) a Breg Knee WrapOn Polar Pad or (ii) Elasto-Gel All Purpose Therapy Wrap, as further described in Example 8. The "Standard Cooling Device" was a Breg Knee WrapOn Polar Pad. The "Ice-Gel Pack" was an Elasto-Gel All Purpose Therapy Wrap. The following designations apply to the graph: A is the time at which any temperature reading of the temperature probe was made prior to insertion into the intraarticular space of the patient's knee; B is the time which the temperature probe was inserted into the intraarticular space of the patient's knee; C is time at which the cooling device was applied to the patient's knee; D is the time at which the cooling device was removed from the patient's knee; E is the time at which a solution of 2% w/w lidocaine was administered to the patient's knee by intraarticular injection; F is the time at which the cooling device was reapplied to the patient's knee; G is the time at which the cooling device was removed from the patient's knee; H is the time that *trans*-capsaicin was administered by intraarticular injection; I is the time at which the cooling device was reapplied to the patient's knee; J is the time at which the cooling device was removed from the patient's knee; K is the time at which the temperature probe was removed from the patient's knee.

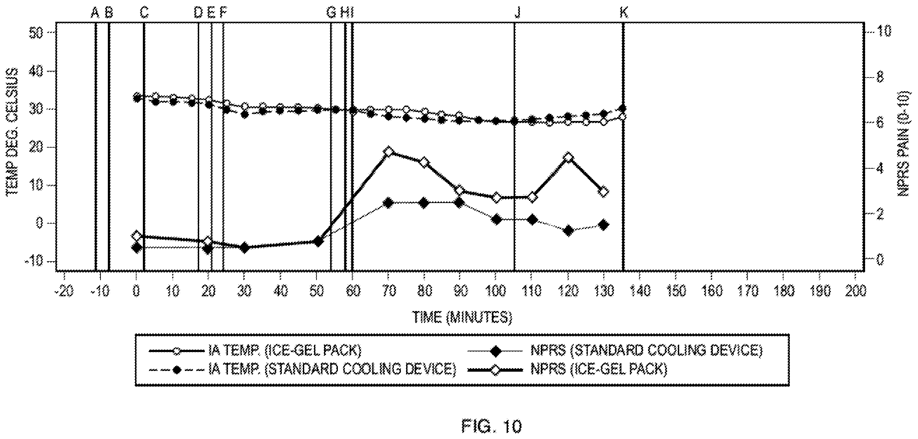

**FIGURE 10** is a graph showing mean intraarticular (IA) temperature and mean NPRS Pain scores over time using the following cooling devices: (i) a Breg Knee WrapOn Polar Pad or (ii) Elasto-Gel All Purpose Therapy Wrap, as further described in Example 8. The "Standard Cooling Device" was a Breg Knee WrapOn Polar Pad. The "Ice-Gel Pack" was an Elasto-Gel All Purpose Therapy Wrap. The following designations apply to the graph: A is the time at which any temperature reading of the temperature probe was made prior to insertion into the intraarticular space of the patient's knee; B is the time which the temperature probe was inserted into the intraarticular space of the patient's knee; C is time at which the cooling device was applied to the patient's knee; D is the time at which the cooling device was removed from the patient's knee; E is the time at which a solution of 2% w/w lidocaine was administered to the patient's knee by intraarticular injection; F is the time at which the cooling device was reapplied to the patient's knee; G is the time at which the cooling device was removed from the patient's knee; H is the time that *trans*-capsaicin was administered by intraarticular injection; I is the time at which the cooling device was reapplied to the patient's knee; J is the time at which the cooling device was removed from the patient's knee; K is the time at which the temperature probe was removed from the patient's knee.

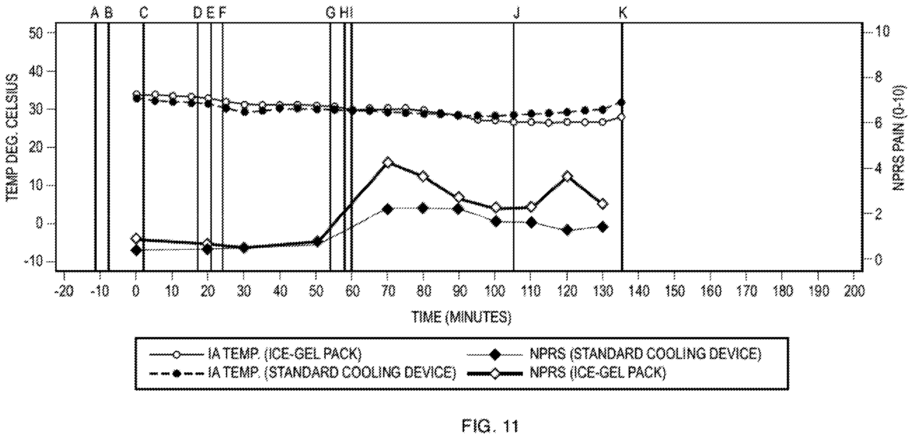

**FIGURE 11** is a graph showing mean intraarticular (IA) temperature and mean NPRS Pain scores over time using the following cooling devices: (i) a Breg Knee WrapOn Polar Pad or (ii) Elasto-Gel All Purpose Therapy Wrap, as further described in Example 8. The "Standard Cooling Device" was a Breg Knee WrapOn Polar Pad. The "Ice-Gel Pack" was an Elasto-Gel All Purpose Therapy Wrap. The following designations apply to the graph: A is the time at which any temperature reading of the temperature probe was made prior to insertion into the intraarticular space of the patient's knee; B is the time which the temperature probe was inserted into the intraarticular space of the patient's knee; C is time at which the cooling device was applied to the patient's knee; D is the time at which the cooling device was removed from the patient's knee; E is the time at which a solution of 2% w/w lidocaine was administered to the patient's knee by intraarticular injection; F is the time at which the cooling device was reapplied to the patient's knee; G is the time at which the cooling device was removed from the patient's knee; H is the time that *trans*-capsaicin was administered by intraarticular injection; I is the time at which the cooling device was reapplied to the patient's knee; J is the time at which the cooling device was removed from the patient's knee; K is the time at which the temperature probe was removed from the patient's knee.

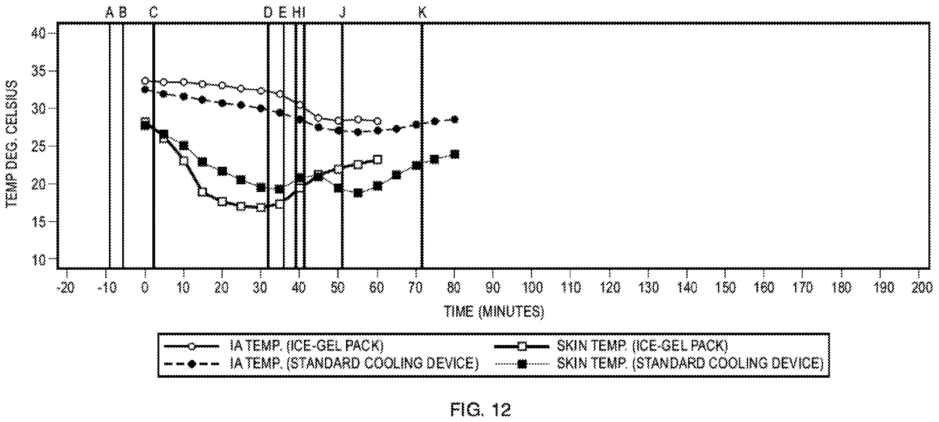

**FIGURE 12** is a graph showing mean intraarticular (IA) temperature and mean skin temperature over time using the following cooling devices: (i) a Breg Knee WrapOn Polar Pad or (ii) Elasto-Gel All Purpose Therapy Wrap, as further described in Example 9. The "Standard Cooling Device" was a Breg Knee WrapOn Polar Pad. The "Ice-Gel Pack" was an Elasto-Gel All Purpose Therapy Wrap. The following designations apply to the graph: A is the time at which any temperature reading of the temperature probe was made prior to insertion into the intraarticular space of the patient's knee; B is the time which the temperature probe was inserted into the intraarticular space of the patient's knee; C is time at which the cooling device was applied to the patient's knee; D is the time at which the cooling device was removed from the patient's knee; E is the time at which a solution of 2% w/w lidocaine was administered to the patient's knee by intraarticular injection; F is the time at which the cooling device was reapplied to the patient's knee; G is the time at which the cooling device was removed from the patient's knee; H is the time that *trans*-capsaicin was administered by intraarticular injection; I is the time at which the cooling device was reapplied to the patient's knee; J is the time at which the cooling device was removed from the patient's knee; K is the time at which the temperature probe was removed from the patient's knee.

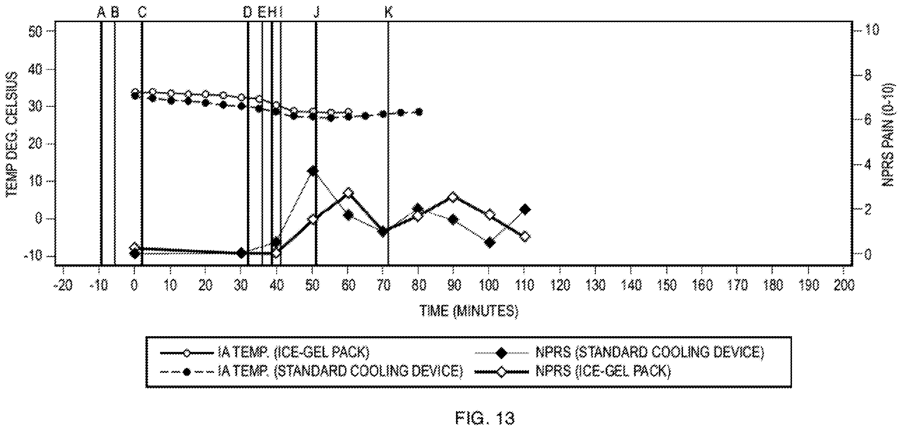

**FIGURE 13** is a graph showing mean intraarticular (IA) temperature and mean NPRS Pain scores over time using the following cooling devices: (i) a Breg Knee WrapOn Polar Pad or (ii) Elasto-Gel All Purpose Therapy Wrap, as further described in Example 9. The "Standard Cooling Device" was a Breg Knee WrapOn Polar Pad. The "Ice-Gel Pack" was an Elasto-Gel All Purpose Therapy Wrap. The following designations apply to the graph: A is the time at which any temperature reading of the temperature probe was made prior to insertion into the intraarticular space of the patient's knee; B is the time which the temperature probe was inserted into the intraarticular space of the patient's knee; C is time at which the cooling device was applied to the patient's knee; D is the time at which the cooling device was removed from the patient's knee; E is the time at which a solution of 2% w/w lidocaine was administered to the patient's knee by intraarticular injection; F is the time at which the cooling device was reapplied to the patient's knee; G is the time at which the cooling device was removed from the patient's knee; H is the time that *trans*-capsaicin was administered by intraarticular injection; I is the time at which the cooling device was reapplied to the patient's knee; J is the time at which the cooling device was removed from the patient's knee; K is the time at which the temperature probe was removed from the patient's knee.

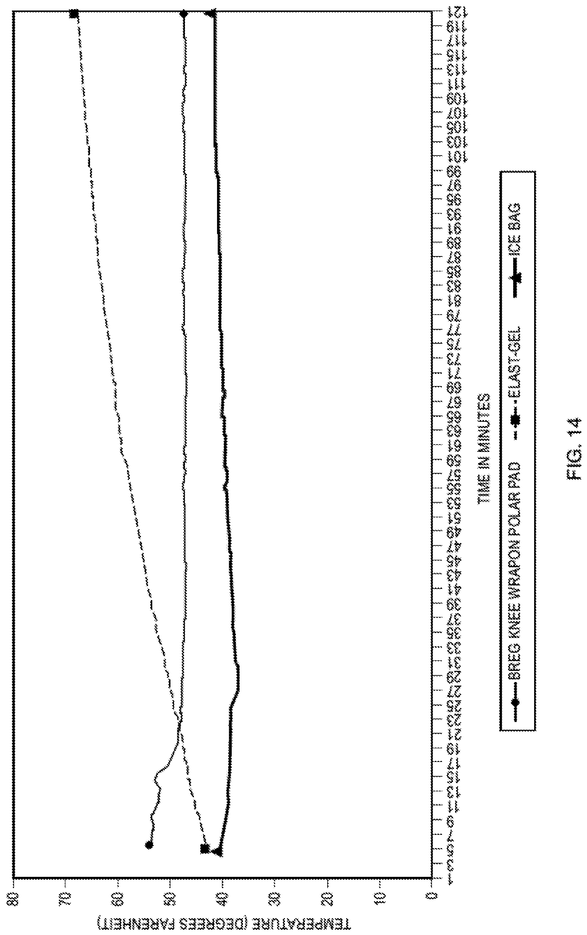

**FIGURE 14** is a graph showing temperature profiles recorded for Breg Knee WrapOn Polar Pad, Elasto-gel cooling device, and ice-pack, as further described in Example 10.

### DETAILED DESCRIPTION

The invention provides compositions for use in treating knee joint pain in a patient, such as knee joint pain due to osteoarthritis, while minimizing transient burning sensation experienced by patients due to capsaicin administration, the use comprising sequential dosing of capsaicin. The uses desirably provide relief from joint pain, such as osteoarthritic knee joint pain, for an extended duration, such as at least about 8 months, 10 months, 12 months, 18 months, or 24 months. Because administration of capsaicin causes initial neuronal excitation resulting in the adverse side effect of a transient burning sensation, the uses desirably use a cooling article, such as a material wrap cooled via a circulating fluid, to reduce the temperature of tissue to be exposed to capsaicin for certain durations of time, optionally in combination with administering a local anesthetic agent, in order to attenuate the transient burning sensation experienced by patients, resulting in the substantial reduction or even elimination of transient burning sensation caused by capsaicin. The cooling article desirably has an exterior surface temperature in the range of from about 5°C to about 15°C, and more desirably from about 5°C to about 10°C, for application to the exterior surface of the patient's knee.

Because overcooling of skin tissue can cause the adverse effect of skin necrosis, while insufficient cooling can be inadequate to sufficiently reduce the transient burning sensation experienced by patients due to capsaicin administration, the uses desirably apply a cooling article having a particular temperature range (e.g., from about 5°C to about 15°C, and more desirably from about 5°C to about 10°C) for particular durations of time both before and after administration of capsaicin. The therapeutic uses can be further characterized according to the temperature of tissue and/or fluid in the joint into which capsaicin is administered, and in certain embodiments, fluid in the intra-articular space of the knee joint, is cooled to a temperature in the range from about 26°C to about 30°C prior to administration of capsaicin, and then maintained at a temperature in the range from about 26°C to about 33°C for a duration of at least 30 minutes after administration of capsaicin.

Transient burning sensation due to capsaicin administration may manifest in patients in the form of a burning sensation, pain, and/or ache in the area in which capsaicin was administered. Techniques described herein are designed to reduce the magnitude of such transient burning sensation experienced by the patient.

The practice of the present invention employs, unless otherwise indicated, conventional techniques of organic chemistry, pharmacology, cell biology, and biochemistry. Such techniques are explained in the literature, such as in "Comprehensive Organic Synthesis" (B.M. Trost & I. Fleming, eds., 1991-1992); "Current protocols in molecular biology" (F.M. Ausubel et al., eds., 1987, and periodic updates); and "Current protocols in immunology" (J.E. Coligan et al., eds., 1991). Various aspects of the invention are set forth below in sections; however, aspects of the invention described in one particular section are not to be limited to any particular section.

### I. DEFINITIONS

To facilitate an understanding of the present invention, a number of terms and phrases are defined below.

The terms "a" and "an" as used herein mean "one or more" and include the plural unless the context is inappropriate.

The phrase "Injection Pain Scale" refers to a measure of pain experienced by a patient upon administration of capsaicin by injection, where the extent of pain experienced by the patient is rated by the patient as one of the following: (i) none, (ii) mild pain, (iii) moderate pain, or (iv) intense pain.

The abbreviation "NPRS" refers to Numerical Pain Rating Scale, as further described herein.

As used herein, the terms "subject" and "patient" refer to organisms to be treated by the uses of the present invention. Such organisms are preferably mammals (*e*.*g*., murines, simians, equines, bovines, porcines, canines, felines, and the like), and more preferably humans.

As used herein, the term "effective amount" refers to the amount of a compound (*e.g.*, a compound of the present invention) sufficient to effect beneficial or desired results. An effective amount can be administered in one or more administrations, applications or dosages and is not intended to be limited to a particular formulation or administration route. As used herein, the term "treating" includes any effect (*e*.*g*., lessening, reducing, modulating, or eliminating) that results in the improvement of the condition, disease, disorder, and the like. The terms "ameliorate" and "ameliorating" refer to lessening, reducing, and/or eliminating the stated condition, such as pain. The terms "attenuate" and "attenuating" refer to lessening, reducing, and/or eliminating the stated condition, such as pain.

Compounds of the disclosure may contain a C-C double bond and, therefore, exist as geometric isomers. Individual geometric isomers of compounds of the present invention can be prepared synthetically from commercially available starting materials that contain a single geometric isomer in high purity and/or through separating a mixture of geometric isomers using chromatographic procedures known in the art. Substituents around a carbon-carbon double bond are designated as being in the "*Z*" or "*E*" configuration wherein the terms "*Z*" and "*E*" are used in accordance with IUPAC standards. Substituents around a carbon-carbon double bond alternatively can be referred to as "cis" or "trans," where "cis" represents substituents on the same side of the double bond and "trans" represents substituents on opposite sides of the double bond.

The compounds may be in amorphic or crystalline form, and the invention encompasses all such amorphic and crystalline forms.

As used herein, the term "pharmaceutical composition" refers to the combination of an active agent with a carrier, inert or active, making the composition especially suitable for therapeutic use *in vivo* or *ex vivo.*

As used herein, the term "pharmaceutically acceptable carrier" refers to any of the standard pharmaceutical carriers, such as a phosphate buffered saline solution, water, emulsions (*e.g.,* such as an oil/water or water/oil emulsions), and various types of wetting agents. The compositions also can include stabilizers and preservatives. For examples of carriers, stabilizers and adjuvants, *see e.g.,* Martin, Remington's Pharmaceutical Sciences, 15th Ed., Mack Publ. Co., Easton, PA [1975].

As used herein, the term "pharmaceutically acceptable salt" refers to any pharmaceutically acceptable salt (*e.g.,* acid or base) of a compound of the present invention which, upon administration to a subject, is capable of providing a compound of this invention. As is known to those of skill in the art, "salts" of the compounds of the present invention may be derived from inorganic or organic acids and bases. Examples of acids include, but are not limited to, hydrochloric, hydrobromic, sulfuric, nitric, perchloric, fumaric, maleic, phosphoric, glycolic, lactic, salicylic, succinic, toluene-p-sulfonic, tartaric, acetic, citric, methanesulfonic, ethanesulfonic, formic, benzoic, malonic, naphthalene-2-sulfonic, benzenesulfonic acid, and the like. Other acids, such as oxalic, while not in themselves pharmaceutically acceptable, may be employed in the preparation of salts useful as intermediates in obtaining the compounds of the invention and their pharmaceutically acceptable acid addition salts.

Examples of bases include, but are not limited to, alkali metal (*e*.*g*., sodium) hydroxides, alkaline earth metal (*e*.*g*., magnesium) hydroxides, ammonia, and compounds of formula NW₃, wherein W is C₁₋₄ alkyl, and the like.

Examples of salts include, but are not limited to: acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, flucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, palmoate, pectinate, persulfate, phenylpropionate, picrate, pivalate, propionate, succinate, tartrate, thiocyanate, tosylate, undecanoate, and the like. Other examples of salts include anions of the compounds of the present invention compounded with a suitable cation such as Na⁺, NH₄⁺, and NW₄⁺ (wherein W is a C₁₋₄ alkyl group), and the like.

For therapeutic use, salts of the compounds of the present invention are contemplated as being pharmaceutically acceptable. However, salts of acids and bases that are non-pharmaceutically acceptable may also find use, for example, in the preparation or purification of a pharmaceutically acceptable compound.

The phrase "therapeutically-effective amount" as used herein means that amount of a compound, material, or composition comprising a compound of the present invention which is effective for producing some desired therapeutic effect in at least a sub-population of cells in an animal at a reasonable benefit/risk ratio applicable to any medical treatment.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

Unless specified otherwise, the term "about" refers to within ±10% of the stated value. The invention encompasses embodiments where the value is within ±9%, ±8%, ±7%, ±6%, ±5%, ±4%, ±3%, ±2%, or ±1% of the stated value.

The term "alkyl" as used herein refers to a saturated straight or branched hydrocarbon, such as a straight or branched group of 1-12, 1-10, or 1-6 carbon atoms, referred to herein as C₁-C₁₂alkyl, C₁-C₁₀alkyl, and C₁-C₆alkyl, respectively. Exemplary alkyl groups include, but are not limited to, methyl, ethyl, propyl, isopropyl, 2-methyl-1-propyl, 2-methyl-2-propyl, 2-methyl-1-butyl, 3-methyl-1-butyl, 2-methyl-3-butyl, 2,2-dimethyl-1-propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, butyl, isobutyl, t-butyl, pentyl, isopentyl, neopentyl, hexyl, heptyl, octyl, etc.

The term "hydroxyalkyl" refers to an alkyl group substituted by 1 or 2 hydroxyl groups. In certain embodiments, the hydroxyalkyl is an alkyl group substituted by only 1 hydroxyl group.

The term "hydroxyalkanoic acid" refers to saturated straight or branched hydrocarbon that is substituted by (i) one -CO₂H group, and (ii) one or two hydroxyl groups.

The term "alkenyl" as used herein refers to an unsaturated straight or branched hydrocarbon having at least one carbon-carbon double bond, such as a straight or branched group of 2-12, 2-10, or 2-6 carbon atoms, referred to herein as C₂₋C₁₂alkenyl, C₂₋C₁₀alkenyl, and C₂₋C₆alkenyl, respectively. Exemplary alkenyl groups include vinyl, allyl, butenyl, pentenyl, hexenyl, butadienyl, pentadienyl, hexadienyl, 2-ethylhexenyl, 2-propyl-2-butenyl, 4-(2-methyl-3-butene)-pentenyl, and the like.

The term "hydroxyalkenyl" refers to an alkenyl group substituted by 1 or 2 hydroxyl groups. In certain embodiments, the hydroxyalkenyl is an alkenyl group substituted by only 1 hydroxyl group.

The term "hydroxyalkenoic acid" refers to an unsaturated straight or branched hydrocarbon having one carbon-carbon double bond, wherein the hydrocarbon is substituted by (i) one -CO₂H group, and (ii) one or two hydroxyl groups.

The term "polyethylene glycolyl" refers to a radical of polyethylene glycol. The polyethylene glycolyl is a chemical fragment that is part of a larger molecule. When the polyethylene glycolyl is bonded at one location to the remainder of the molecule, then the polyethylene glycolyl is a mono-radical, such as "-(CH₂CH₂O)x-H" where x is an integer greater than 1. When the polyethylene glycolyl is used as a component within a molecule connecting two fragments of the molecule, the polyethylene glycolyl is a diradical, having a point of attachment at each terminus of the polyethylene glycolyl, which may illustrated as "-(CH₂CH₂O)x-" where x is an integer greater than 1. In certain embodiments, x is an integer in the range of about 5 to about 100, about 5 to about 50, about 5 to about 25, about 5 to about 15, about 10 to about 50, about 10 to about 30, or about 10 to about 20. In certain embodiments, x is about 10, 11, 12, 13, 14, 15, 16, 17, 18, or 19. In certain preferred embodiments, x is about 15.

As a general matter, compositions specifying a percentage are by weight unless otherwise specified. Further, if a variable is not accompanied by a definition, then the previous definition of the variable controls.

### II. THERAPEUTIC APPLICATIONS FOR KNEE JOINT PAIN

One aspect of the invention provides compositions for use in treating knee joint pain in a patient, such as knee joint pain due to osteoarthritis, while minimizing transient burning sensation experienced by patients due to capsaicin administration, the use comprising sequential dosing of capsaicin. The uses desirably provide relief from joint pain, such as osteoarthritic knee joint pain, for an extended duration, such as at least about 8 months, 10 months, 12 months, 18 months, or 24 months. The uses utilize a cooling article, such as a material wrap cooled via a circulating fluid, to reduce the temperature of tissue to be exposed to capsaicin for certain durations of time, in combination with administering a local anesthetic agent. In a preferred embodiment, the uses are used to ameliorate osteoarthritic knee joint pain in a human patient by sequential administration of capsaicin to the intra-articular space of the joint of the patient's knee via a protocol that applies a cooling article to an exterior surface of the patient's knee presenting with osteoarthritic knee joint pain before and after administration of capsaicin, such as where the cooling article has an exterior surface temperature in the range of from about 5°C to about 15°C, and more preferably from about 5°C to about 10°C, for application to the exterior surface of the patient's knee. Various aspects and embodiments of the uses are described below.

### First Use

One aspect of the invention provides capsaicin for use in ameliorating osteoarthritic knee joint pain in a human patient for a duration of at least 8 months, wherein the use comprises administering by injection into the intra-articular space of the joint of said knee at least a first dose of capsaicin and a second dose of capsaicin to thereby ameliorate osteoarthritic knee joint pain in the human patient for a duration of at least 8 months, wherein the use is characterized by:
(i) the first dose of capsaicin is an amount of 1 mg capsaicin;
(ii) the second dose of capsaicin is an amount of 1 mg capsaicin;
(iii) the second dose of capsaicin is administered no sooner than 5 months after administration of the first dose of capsaicin;
(iv) if any additional dose of capsaicin is administered by injection into the intra-articular space of the joint of said knee, any such additional dose is in an amount of 1 mg of capsaicin and any said additional dose is administered no sooner than 3 months after administration of the prior dose of capsaicin administered by injection into the intra-articular space of the joint of said knee; and
(v) when administering the first dose of capsaicin:
   (a) a cooling article is applied for a duration of 15 minutes to an exterior surface of the human patient's knee presenting with osteoarthritic knee joint pain, wherein the cooling article has an exterior surface temperature in the range of from 3°C to 15°C for application to the exterior surface of said knee; then
   (b) a pharmaceutical composition comprising a single pain-relief agent selected from the group consisting of lidocaine and a pharmaceutically acceptable salt thereof is administered by injection into the intra-articular space of the joint of said knee in order to deliver a dose of lidocaine in an amount ranging from 0.1 g to 0.5 g; then
   (c) a cooling article is applied for a duration of 30 minutes to an exterior surface of said knee, wherein the cooling article has an exterior surface temperature in the range of from 3°C to 15°C for application to the exterior surface of said knee; then
   (d) the first dose of capsaicin is administered by injecting into the intra-articular space of the joint of said knee a pharmaceutical composition comprising capsaicin; and then
   (e) a cooling article is applied to an exterior surface of said knee for a duration of at least 30 minutes, wherein the cooling article has an exterior surface temperature in the range of from 3°C to 15°C for application to the exterior surface of said knee.

### Second Use

One aspect of the invention provides capsaicin for use in ameliorating knee joint pain in a human patient for a duration of at least 8 months, wherein the use comprises administering by injection into the intra-articular space of the joint of said knee at least a first dose of capsaicin and a second dose of capsaicin to thereby ameliorate knee joint pain in the human patient for a duration of at least 8 months, wherein the use is characterized by:
(i) the first dose of capsaicin is an amount of 1 mg capsaicin;
(ii) the second dose of capsaicin is an amount of 1 mg capsaicin;
(iii) the second dose of capsaicin is administered no sooner than 5 months after administration of the first dose of capsaicin; and
(iv) if any additional dose of capsaicin is administered by injection into the intra-articular space of the joint of said knee, any such additional dose is in an amount of 1 mg of capsaicin and any said additional dose is administered no sooner than 3 months after administration of the prior dose of capsaicin administered by injection into the intra-articular space of the joint of said knee.

### Further Exemplary Features of the First Use

The above First Use may be further characterized by additional features, such as a procedure for administering the second dose of capsaicin, a step comprising flexing the knee, duration of cooling an exterior surface of the knee after administration of capsaicin, dose of lidocaine, characterization of the pharmaceutical composition comprising lidocaine, and the like. A more thorough description of such features is provided below. The invention embraces all permutations and combinations of these features.

### Procedure for Administering the Second Dose of Capsaicin

The use may be further characterized according to the procedure for administering the second dose of capsaicin. For example, in certain embodiments, when administering the second dose of capsaicin:
(a) a cooling article is applied for a duration of about 15 minutes to an exterior surface of the human patient's knee presenting with osteoarthritic knee joint pain, wherein the cooling article has an exterior surface temperature in the range of from about 3°C to about 15°C for application to the exterior surface of said knee; then
(b) a pharmaceutical composition comprising a single pain-relief agent selected from the group consisting of lidocaine and a pharmaceutically acceptable salt thereof is administered by injection into the intra-articular space of the joint of said knee in order to deliver a dose of lidocaine in an amount ranging from about 0.1 g to about 0.5 g; then
(c) a cooling article is applied for a duration of about 30 minutes to an exterior surface of said knee, wherein the cooling article has an exterior surface temperature in the range of from about 3°C to about 15°C for application to the exterior surface of said knee; then
(d) the second dose of capsaicin is administered by injecting into the intra-articular space of the joint of said knee a pharmaceutical composition comprising capsaicin; and then
(e) a cooling article is applied to an exterior surface of said knee for a duration of at least about 30 minutes, wherein the cooling article has an exterior surface temperature in the range of from about 3°C to about 15°C for application to the exterior surface of said knee.

### Flexing the Knee

The use may be further characterized according to the presence or absence of a step that involves flexing the knee that received capsaicin. For example, in certain embodiments, after administration of the pharmaceutical composition comprising capsaicin in step (d) but prior to step (e) said knee is flexed. In certain embodiments, after administration of the pharmaceutical composition comprising capsaicin in step (d) but prior to step (e) said knee is flexed about 5 times. In certain embodiments, after administration of the pharmaceutical composition comprising capsaicin in step (d) but prior to step (e) said knee is flexed about 5 times over a period of about 1 minute. In certain embodiments, after administration of the pharmaceutical composition comprising capsaicin in step (d) but prior to step (e) said knee is flexed and extended. In certain embodiments, after administration of the pharmaceutical composition comprising capsaicin in step (d) but prior to step (e) said knee is flexed and extended about 5 times. In certain embodiments, after administration of the pharmaceutical composition comprising capsaicin in step (d) but prior to step (e) said knee is flexed and extended about 5 times over a period of about 1 minute.

### Duration of Cooling an Exterior Surface of the Knee After Administration of Capsaicin

The use may be further characterized according to the duration of time in step (e) that the cooling article is applied to an exterior surface of the knee. For example, in certain embodiments, said duration in step (e) is from about 30 minutes to about 90 minutes. In certain embodiments, said duration in step (e) is from about 30 minutes to about 60 minutes. In certain other embodiments, said duration in step (e) is from about 60 minutes to about 90 minutes.

### Dose of Lidocaine

The use may be further characterized according to the dose of lidocaine administered to the patient. For example, in certain embodiments, the dose of lidocaine is about 0.3 g. In certain embodiments, the dose of lidocaine is 0.3 g. In certain embodiments, the dose of lidocaine is about 0.15 g. In yet other embodiments, the dose of lidocaine is about 0.1 g, about 0.2 g, about 0.4 g, or about 0.5 g.

### Pharmaceutical Composition Comprising Lidocaine

The use may be further characterized according to features of the pharmaceutical composition comprising lidocaine. For example, in certain embodiments, the pharmaceutical composition comprising lidocaine is an aqueous mixture containing lidocaine at a concentration of about 2% w/w. In certain embodiments, the pharmaceutical composition comprising lidocaine is an aqueous mixture containing lidocaine at a concentration of about 1% w/w. In certain embodiments, the pharmaceutical composition comprising lidocaine further comprises sodium chloride. In certain embodiments, the pharmaceutical composition comprising lidocaine further comprises sodium chloride at a concentration ranging from about 4 mg/mL to about 8 mg/mL.

In certain embodiments, the pharmaceutical composition comprising lidocaine has a volume in the range of from about 13 mL to about 17 mL. In certain embodiments, the pharmaceutical composition comprising lidocaine has a volume of about 15 mL. In certain embodiments, the pharmaceutical composition comprising lidocaine has a volume of 15 mL. In yet other embodiments, the pharmaceutical composition comprising a single pain-relief agent has a volume in the range of from about 1 mL to about 3 mL, about 3 mL to about 5 mL, about 5 mL to about 7 mL, about 7 mL to about 9 mL, about 9 mL to about 11 mL, about 11 mL to about 13 mL, about 13 mL to about 15 mL, or about 17 mL to about 19 mL. In yet other embodiments, the pharmaceutical composition comprising a single pain-relief agent has a volume of about 1, 2, 3, 4, 5, 6, 7, 8, 9, 10, 11, 12, 13, 14, 15, 16, 17, 18, or 19 mL.

The use may be further characterized according to the temperature of the pharmaceutical composition comprising lidocaine, which is to be administered to the patient. For example, in certain embodiments, the pharmaceutical composition comprising lidocaine has a temperature in the range of from about 1°C to about 5°C, about 5°C to about 10°C, about 10°C to about 15°C, about 15°C to about 20°C, about 20°C to about 25°C, or about 22°C to about 24°C. In certain embodiments, the pharmaceutical composition comprising lidocaine has a temperature of about 23°C.

### Administration of Other Pain-Relief Medicine

The use may be further characterized according to whether the patient receives any other pain-relief medicine. For example, in certain embodiments, other than administration of (i) the pharmaceutical composition comprising lidocaine and (ii) the first dose of capsaicin, the second dose of capsaicin, and any additional dose of capsaicin, the patient does not receive any other pain-relief medicine for the osteoarthritic knee joint pain. In certain embodiments, other than administration of (i) the pharmaceutical composition comprising lidocaine and (ii) the first dose of capsaicin, the second dose of capsaicin, and any additional dose of capsaicin, the patient does not receive any other pain-relief medicine. In certain embodiments, other than administration of (i) the pharmaceutical composition comprising lidocaine and (ii) the first dose of capsaicin, and the second dose of capsaicin, the patient does not receive any other pain-relief medicine.

### Additional Procedures to Reduce Pain and/or Transient Burning Sensation

The use may be further characterized according to the presence or absence of additional procedures to reduce transient burning sensation caused by capsaicin and/or to reduce osteoarthritic knee joint pain. For example, in certain embodiments, other than the procedures set forth in steps (a), (b), (c), (d), (e), and optionally flexing said knee, the use does not contain any procedure that reduces transient burning sensation experienced by the patient due to administration of capsaicin. In certain embodiments, other than the procedures set forth in steps (a), (b), (c), (d), (e), optionally flexing said knee, administering the first dose of capsaicin, administering the second dose of capsaicin, and administering any additional dose of capsaicin, the use does not contain any procedure that reduces osteoarthritic knee joint pain. In certain embodiments, other than the procedures set forth in steps (a), (b), (c), (d), (e), optionally flexing said knee, administering the first dose of capsaicin, and administering the second dose of capsaicin, the use does not contain any procedure that reduces osteoarthritic knee joint pain.

### Further Exemplary Features of the Second Use

The above Second Use may be further characterized by additional features, such as the type of joint pain, a procedure for administering each dose of capsaicin, application of a cooling article to an exterior surface of the knee, administration of a local anesthetic agent, and the like. A more thorough description of such features is provided below. The invention embraces all permutations and combinations of these features.

### Type of Joint Pain

The uses may be further characterized according to the type of joint pain. For example, in certain embodiments, the joint pain is arthritic joint pain. In certain embodiments, the joint pain is osteoarthritic joint pain.

### Procedure for Administering Each Dose of Capsaicin

The uses may be further characterized according to the procedure for administering each dose of capsaicin. For example, in certain embodiments, when administering the first dose of capsaicin:
(a) optionally a cooling article is applied to an exterior surface of the human patient's knee presenting with joint pain; then
(b) optionally a local anesthetic agent is administered by injection into the intra-articular space of said knee joint; then
(c) a cooling article is applied for a duration of at least about 10 minutes to an exterior surface of said knee, wherein the cooling article has an exterior surface temperature in the range of from about 1°C to about 15°C for application to the exterior surface of said knee; then
(d) the first dose of capsaicin is administered by injecting into the intra-articular space of the joint of said knee a pharmaceutical composition comprising capsaicin; and then
(e) optionally a cooling article is applied for a duration of at least about 10 minutes to an exterior surface of said knee, wherein the cooling article has an exterior surface temperature in the range of from about 1°C to about 15°C for application to the exterior surface of said knee.

In certain embodiments, when administering the second dose of capsaicin:
(a) optionally a cooling article is applied to an exterior surface of the human patient's knee presenting with joint pain; then
(b) optionally a local anesthetic agent is administered by injection into the intra-articular space of said knee joint; then
(c) a cooling article is applied for a duration of at least about 10 minutes to an exterior surface of said knee, wherein the cooling article has an exterior surface temperature in the range of from about 1°C to about 15°C for application to the exterior surface of said knee; then
(d) the second dose of capsaicin is administered by injecting into the intra-articular space of the joint of said knee a pharmaceutical composition comprising capsaicin; and then
(e) optionally a cooling article is applied for a duration of at least about 10 minutes to an exterior surface of said knee, wherein the cooling article has an exterior surface temperature in the range of from about 1°C to about 15°C for application to the exterior surface of said knee.

In certain embodiments, when administering any additional dose of capsaicin:
(a) optionally a cooling article is applied to an exterior surface of the human patient's knee presenting with joint pain; then
(b) optionally a local anesthetic agent is administered by injection into the intra-articular space of said knee joint; then
(c) a cooling article is applied for a duration of at least about 10 minutes to an exterior surface of said knee, wherein the cooling article has an exterior surface temperature in the range of from about 1°C to about 15°C for application to the exterior surface of said knee; then
(d) the additional dose of capsaicin is administered by injecting into the intra-articular space of the joint of said knee a pharmaceutical composition comprising capsaicin; and then
(e) optionally a cooling article is applied for a duration of at least about 10 minutes to an exterior surface of said knee, wherein the cooling article has an exterior surface temperature in the range of from about 1°C to about 15°C for application to the exterior surface of said knee.

Each of the above procedures for administering each dose of capsaicin may be further characterized according to the duration of time in step (c) that the cooling article is applied to an exterior surface of said knee. For example, in certain embodiments, in step (c) the cooling article is applied for a duration of from about 15 minutes to about 45 minutes to the exterior surface of said knee. In certain embodiments, in step (c) the cooling article is applied for a duration of about 45 minutes to the exterior surface of said knee. In certain embodiments, in step (c) the cooling article is applied for a duration of about 30 minutes to the exterior surface of said knee. In certain embodiments, in step (c) the cooling article is applied for a duration of about 20 minutes to the exterior surface of said knee.

### Procedure for Administering Each Dose of Capsaicin

The uses may be further characterized according to use of the following alternative procedure for administering each dose of capsaicin. For example, in certain embodiments, when administering the first dose of capsaicin:
(a) optionally a cooling article is applied to an exterior surface of the human patient's knee presenting with joint pain; then
(b) optionally a local anesthetic agent is administered by injection into the intra-articular space of said knee joint; then
(c) a cooling article is applied to an exterior surface of said knee to achieve a temperature in the range of from about 20°C to about 33°C for tissue or fluid in the interior of said knee joint; then
(d) the first dose of capsaicin is administered by injecting into the intra-articular space of the joint of said knee a pharmaceutical composition comprising capsaicin; and then
(e) optionally a cooling article is applied to an exterior surface of said knee.

In certain embodiments, when administering the second dose of capsaicin:
(a) optionally a cooling article is applied to an exterior surface of the human patient's knee presenting with joint pain; then
(b) optionally a local anesthetic agent is administered by injection into the intra-articular space of said knee joint; then
(c) a cooling article is applied to an exterior surface of said knee to achieve a temperature in the range of from about 20°C to about 33°C for tissue or fluid in the interior of said knee joint; then
(d) the second dose of capsaicin is administered by injecting into the intra-articular space of the joint of said knee a pharmaceutical composition comprising capsaicin; and then
(e) optionally a cooling article is applied to an exterior surface of said knee.

In certain embodiments, when administering any additional dose of capsaicin:
(a) optionally a cooling article is applied to an exterior surface of the human patient's knee presenting with joint pain; then
(b) optionally a local anesthetic agent is administered by injection into the intra-articular space of said knee joint; then
(c) a cooling article is applied to an exterior surface of said knee to achieve a temperature in the range of from about 20°C to about 33°C for tissue or fluid in the interior of said knee joint; then
(d) the additional dose of capsaicin is administered by injecting into the intra-articular space of the joint of said knee a pharmaceutical composition comprising capsaicin; and then
(e) optionally a cooling article is applied to an exterior surface of said knee.

Each of the above procedures for administering each dose of capsaicin may be further characterized according to the temperature of tissue or fluid in the interior of said knee joint that is achieved by application of a cooling article to an exterior surface of said knee. For example, in certain embodiments, in step (c) a cooling article is applied to an exterior surface of said knee to achieve a temperature in the range of from about 20°C to about 22°C for tissue or fluid in the interior of the knee joint. In certain embodiments, in step (c) a cooling article is applied to an exterior surface of said knee to achieve a temperature in the range of from about 22°C to about 24°C for tissue or fluid in the interior of the knee joint. In certain embodiments, in step (c) a cooling article is applied to an exterior surface of said knee to achieve a temperature in the range of from about 24°C to about 26°C for tissue or fluid in the interior of the knee joint. In certain embodiments, in step (c) a cooling article is applied to an exterior surface of said knee to achieve a temperature in the range of from about 26°C to about 28°C for tissue or fluid in the interior of the knee joint. In certain embodiments, in step (c) a cooling article is applied to an exterior surface of said knee to achieve a temperature in the range of from about 27°C to about 29°C for tissue or fluid in the interior of the knee joint. In certain embodiments, in step (c) a cooling article is applied to an exterior surface of said knee to achieve a temperature in the range of from about 28°C to about 30°C for tissue or fluid in the interior of the knee joint. In certain embodiments, in step (c) a cooling article is applied to an exterior surface of said knee to achieve a temperature in the range of from about 30°C to about 32°C for tissue or fluid in the interior of the knee joint.

In certain embodiments, in step (c) a cooling article is applied to an exterior surface of said knee to achieve a temperature of about 20°C for tissue or fluid in the interior of the knee joint. In certain embodiments, in step (c) a cooling article is applied to an exterior surface of said knee to achieve a temperature of about 21°C for tissue or fluid in the interior of the knee joint. In certain embodiments, in step (c) a cooling article is applied to an exterior surface of said knee to achieve a temperature of about 22°C for tissue or fluid in the interior of the knee joint. In certain embodiments, wherein in step (c) a cooling article is applied to an exterior surface of said knee to achieve a temperature of about 23°C for tissue or fluid in the interior of the knee joint. In certain embodiments, in step (c) a cooling article is applied to an exterior surface of said knee to achieve a temperature of about 24°C for tissue or fluid in the interior of the knee joint. In certain embodiments, in step (c) a cooling article is applied to an exterior surface of said knee to achieve a temperature of about 25°C for tissue or fluid in the interior of the knee joint. In certain embodiments, in step (c) a cooling article is applied to an exterior surface of said knee to achieve a temperature of about 26°C for tissue or fluid in the interior of the knee joint. In certain embodiments, in step (c) a cooling article is applied to an exterior surface of said knee to achieve a temperature of about 27°C for tissue or fluid in the interior of the knee joint. In certain embodiments, in step (c) a cooling article is applied to an exterior surface of said knee to achieve a temperature of about 28°C for tissue or fluid in the interior of the knee joint. In certain embodiments, in step (c) a cooling article is applied to an exterior surface of said knee to achieve a temperature of about 29°C for tissue or fluid in the interior of the knee joint. In certain embodiments, in step (c) a cooling article is applied to an exterior surface of said knee to achieve a temperature of about 30°C for tissue or fluid in the interior of the knee joint. In certain embodiments, in step (c) a cooling article is applied to an exterior surface of said knee to achieve a temperature of about 31°C for tissue or fluid in the interior of the knee joint. In certain embodiments, in step (c) a cooling article is applied to an exterior surface of said knee to achieve a temperature of about 32°C for tissue or fluid in the interior of the knee joint. In certain embodiments, in step (c) a cooling article is applied to an exterior surface of said knee to achieve a temperature of about 33°C for tissue or fluid in the interior of the knee joint.

### Application of a Cooling Article Following Administration of Capsaicin

The uses may be further characterized according to the application of a cooling article following administration of a dose of capsaicin to the knee joint. For example, in certain embodiments, the use comprises step (e) in which a cooling article is applied for a duration of at least about 10 minutes to an exterior surface of said knee, wherein the cooling article has an exterior surface temperature in the range of from about 1°C to about 15°C for application to an exterior surface of said knee. In certain embodiments, the use comprises step (e) in which a cooling article is applied to an exterior surface of said knee to achieve a temperature in the range of from about 20°C to about 22°C for tissue or fluid in the interior of the knee joint for a duration of at least 15 minutes. In certain embodiments, the use comprises step (e) in which a cooling article is applied to an exterior surface of said knee to achieve a temperature in the range of from about 22°C to about 24°C for tissue or fluid in the interior of the knee joint for a duration of at least 15 minutes. In certain embodiments, the use comprises step (e) in which a cooling article is applied to an exterior surface of said knee to achieve a temperature in the range of from about 24°C to about 26°C for tissue or fluid in the interior of the knee joint for a duration of at least 15 minutes. In certain embodiments, the use comprises step (e) in which a cooling article is applied to an exterior surface of said knee to achieve a temperature in the range of from about 26°C to about 28°C for tissue or fluid in the interior of the knee joint for a duration of at least 15 minutes. In certain embodiments, the use comprises step (e) in which a cooling article is applied to an exterior surface of said knee to achieve a temperature in the range of from about 27°C to about 29°C for tissue or fluid in the interior of the knee joint for a duration of at least 15 minutes. In certain embodiments, the use comprises step (e) in which a cooling article is applied to an exterior surface of said knee to achieve a temperature in the range of from about 28°C to about 30°C for tissue or fluid in the interior of the knee joint for a duration of at least 15 minutes. In certain embodiments, the use comprises step (e) in which a cooling article is applied to an exterior surface of said knee to achieve a temperature in the range of from about 30°C to about 32°C for tissue or fluid in the interior of the knee joint for a duration of at least 15 minutes.

The uses may be further characterized according to the duration of time that the tissue or fluid in the interior of the knee joint is maintained at the specified temperature due to application of the cooling article. For example, in certain embodiments, the duration in step (e) is at least 30 minutes. In certain embodiments, the duration in step (e) is from about 30 minutes to about 90 minutes. In certain embodiments, the duration in step (e) is from about 30 minutes to about 60 minutes. In certain other embodiments, the duration in step (e) is from about 60 minutes to about 90 minutes. In certain other embodiments, the duration in step (e) is about 30 minutes. In certain other embodiments, the duration in step (e) is about 20 minutes. In yet other embodiments, the duration in step (e) is from about 90 minutes to about 120 minutes, or from about 120 minutes to about 180 minutes.

### Application of a Cooling Article Prior to Administration of Capsaicin

The uses may be further characterized according to the application of a cooling article prior to administration of a dose of capsaicin to the knee joint. For example, in certain embodiments, the use comprises step (a) in which a cooling article is applied to an exterior surface of the human patient's knee presenting with joint pain. In certain embodiments, the use comprises step (a) in which for a duration of from about 5 minutes to about 30 minutes a cooling article is applied to an exterior surface of said knee presenting with joint pain. In certain embodiments, the use comprises step (a) in which for a duration of about 15 minutes a cooling article is applied to an exterior surface of said knee presenting with joint pain.

### Administration of a Local Anesthetic Agent Prior to Administration of Capsaicin

The uses may be further characterized according to the administration of a local anesthetic agent prior to administration of a dose of capsaicin to the knee joint. For example, in certain embodiments, the use comprises step (b) in which a local anesthetic agent is administered by injection into the intra-articular space of said knee joint. In certain embodiments, the use does not contain step (b). In certain embodiments, the use comprises the following additional step that is performed between steps (c) and (d): administering a local anesthetic agent by injection into the intra-articular space of said knee joint.

When a local anesthetic agent is administered, the uses may be further characterized according to additional features, such as the identity, dose, and concentration of the local anesthetic agent. For example, in certain embodiments, the local anesthetic agent is a caine analgesic. In certain embodiments, the local anesthetic agent is lidocaine or a pharmaceutically acceptable salt thereof. In certain embodiments, the local anesthetic agent is lidocaine hydrochloride.

In certain embodiments, the local anesthetic agent is delivered at a dose sufficient to deliver lidocaine in an amount of about 0.1 g to about 0.5 g. In certain embodiments, the local anesthetic agent is delivered at a dose sufficient to deliver lidocaine in an amount of about 0.15 g. In certain embodiments, the local anesthetic agent is delivered at a dose sufficient to deliver lidocaine in an amount of about 0.3 g. In certain embodiments, the local anesthetic agent is delivered at a dose sufficient to deliver lidocaine in an amount of 0.3 g. In yet other embodiments, the local anesthetic agent is delivered at a dose sufficient to deliver lidocaine in an amount of about 0.1 g, about 0.2 g, about 0.4 g, or about 0.5 g.

In certain embodiments, the local anesthetic agent is administered in the form of a pharmaceutical composition. In certain embodiments, the local anesthetic agent is administered in the form of a pharmaceutical composition having a volume in the range of from about 13 mL to about 17 mL. In certain embodiments, the local anesthetic agent is administered in the form of a pharmaceutical composition having a volume of about 15 mL. In certain embodiments, the local anesthetic agent is administered in the form of a pharmaceutical composition having a volume of 15 mL. In yet other embodiments, the local anesthetic agent is administered in the form of a pharmaceutical composition having a volume of in the range of from about 1 mL to about 3 mL, about 3 mL to about 5 mL, about 5 mL to about 7 mL, about 7 mL to about 9 mL, about 9 mL to about 11 mL, about 11 mL to about 13 mL, about 13 mL to about 15 mL, or about 17 mL to about 19 mL. In yet other embodiments, the local anesthetic agent is administered in the form of a pharmaceutical composition having a volume of about 1, 2, 3, 4, 5, 6, 7, 8, 9, 10, 11, 12, 13, 14, 15, 16, 17, 18, or 19 mL.

In certain embodiments, the local anesthetic agent is administered in the form of a pharmaceutical composition that is an aqueous mixture containing lidocaine at a concentration of about 2% w/w. In certain embodiments, the local anesthetic agent is administered in the form of a pharmaceutical composition that is an aqueous mixture containing lidocaine at a concentration of about 1% w/w. In certain embodiments, the aqueous mixture containing lidocaine further comprises sodium chloride. In certain embodiments, the aqueous mixture containing lidocaine further comprises sodium chloride at a concentration ranging from about 4 mg/mL to about 8 mg/mL.

The uses may be further characterized according to the temperature of the pharmaceutical composition comprising the local anesthetic, which is to be administered to the patient. For example, in certain embodiments, the pharmaceutical composition comprising the local anesthetic has a temperature in the range of from about 1°C to about 5°C, about 5°C to about 10°C, about 10°C to about 15°C, about 15°C to about 20°C, about 20°C to about 25°C, or about 22°C to about 24°C. In certain embodiments, the pharmaceutical composition comprising the local anesthetic has a temperature of about 23°C.

### Flexing the Knee

The uses may be further characterized according to the presence or absence of a step that involves flexing the knee that received capsaicin. For example, in certain embodiments, after administration of the pharmaceutical composition comprising capsaicin in step (d) but prior to step (e) said joint is flexed. In certain embodiments, after administration of the pharmaceutical composition comprising capsaicin in step (d) but prior to step (e) said joint is flexed about 5 times. In certain embodiments, after administration of the pharmaceutical composition comprising capsaicin in step (d) but prior to step (e) said joint is flexed about 5 times over a period of about 1 minute. In certain embodiments, after administration of the pharmaceutical composition comprising capsaicin in step (d) but prior to step (e) said joint is flexed and extended. In certain embodiments, after administration of the pharmaceutical composition comprising capsaicin in step (d) but prior to step (e) said joint is flexed and extended about 5 times. In certain embodiments, after administration of the pharmaceutical composition comprising capsaicin in step (d) but prior to step (e) said joint is flexed and extended about 5 times over a period of about 1 minute.

### Additional Procedures or Medicines to Reduce Pain and/or Transient Burning Sensation

The uses may be further characterized according to the presence or absence of additional procedures or medicines to reduce transient burning sensation caused by capsaicin and/or to reduce knee joint pain. For example, in certain embodiments, other than the procedures set forth in steps (a), (b), (c), (d), (e), optionally flexing said knee, and optionally between steps (c) and (d) administering a local anesthetic agent by injection into the intra-articular space of said knee joint, the use does not contain any procedure that reduces transient burning sensation experienced by the patient due to administration of capsaicin. In certain embodiments, other than the procedures set forth in steps (a), (b), (c), (d), (e), optionally flexing said knee, optionally between steps (c) and (d) administering a local anesthetic agent by injection into the intra-articular space of said knee joint, administering the first dose of capsaicin, administering the second dose of capsaicin, and administering any additional dose of capsaicin, the use does not contain any procedure that reduces knee joint pain. In certain embodiments, other than the procedures set forth in steps (a), (b), (c), (d), (e), optionally flexing said knee, optionally between steps (c) and (d) administering a local anesthetic agent by injection into the intra-articular space of said knee joint, administering the first dose of capsaicin, and administering the second dose of capsaicin, the use does not contain any procedure that reduces knee joint pain. In certain embodiments, other than administration of (i) a local anesthetic agent and (ii) the first dose of capsaicin, the second dose of capsaicin, and any additional dose of capsaicin, the human patient does not receive any other pain-relief medicine for the knee joint pain. In certain embodiments, other than administration of (i) a local anesthetic agent and (ii) the first dose of capsaicin, the second dose of capsaicin, and any additional dose of capsaicin, the human patient does not receive any other pain-relief medicine. In certain embodiments, other than administration of (i) a local anesthetic agent and (ii) the first dose of capsaicin, and the second dose of capsaicin, the human patient does not receive any other pain-relief medicine.

### Further Exemplary Features of the First and Second Uses

The above First and Second Uses may be further characterized by additional features, such duration of pain relief, identity of the patient, features of a cooling article used (e.g., the exterior surface temperature of the cooling article), characteristics of a pharmaceutical composition used that comprises capsaicin, the magnitude of transient burning sensation due to capsaicin, the time for administering the second dose of capsaicin, the time for administering any additional dose of capsaicin, and the like. A more thorough description of such features is provided below. The invention embraces all permutations and combinations of these features.

### Temperature of the Cooling Article Surface for Application to Exterior Surface of the Knee

The uses may be further characterized according to the temperature of the exterior surface of a cooling article for application to the exterior surface of the human patient's knee. For example, in certain embodiments, the cooling article has an exterior surface temperature in the range of from about 1°C to about 3°C for application to the exterior surface of the human patient's knee. In certain embodiments, the cooling article has an exterior surface temperature in the range of from about 3°C to about 5°C for application to the exterior surface of the human patient's knee. In certain embodiments, the cooling article has an exterior surface temperature in the range of from about 5°C to about 7°C for application to the exterior surface of the human patient's knee. In certain embodiments, the cooling article has an exterior surface temperature in the range of from about 7°C to about 9°C for application to the exterior surface of the human patient's knee. In certain embodiments, the cooling article has an exterior surface temperature in the range of from about 8°C to about 10°C for application to the exterior surface of the human patient's knee. In certain embodiments, the cooling article has an exterior surface temperature in the range of from about 9°C to about 11°C for application to the exterior surface of the human patient's knee. In certain embodiments, the cooling article has an exterior surface temperature in the range of from about 11°C to about 13°C for application to the exterior surface of the human patient's knee. In certain embodiments, the cooling article has an exterior surface temperature in the range of from about 13°C to about 15°C for application to the exterior surface of the human patient's knee.

In certain embodiments, the cooling article has an exterior surface temperature in the range of from about 5°C to about 15°C for application to the exterior surface of the human patient's knee. In certain embodiments, the cooling article has an exterior surface temperature in the range of from about 6°C to about 13°C for application to the exterior surface of the human patient's knee. In certain embodiments, the cooling article has an exterior surface temperature in the range of from about 7°C to about 13°C for application to the exterior surface of the human patient's knee. In certain embodiments, the cooling article has an exterior surface temperature in the range of from about 7°C to about 10°C for application to the exterior surface of the human patient's knee. In certain other embodiments, the cooling article has an exterior surface temperature in the range of from about 5°C to about 10°C for application to the exterior surface of the human patient's knee. In certain other embodiments, the cooling article has an exterior surface temperature in the range of from about 6°C to about 12°C for application to the exterior surface of the human patient's knee.

In certain embodiments, the cooling article has an exterior surface temperature of about 1°C for application to the exterior surface of the human patient's knee. In certain embodiments, the cooling article has an exterior surface temperature of about 2°C for application to the exterior surface of the human patient's knee. In certain embodiments, the cooling article has an exterior surface temperature of about 3°C for application to the exterior surface of the human patient's knee. In certain embodiments, the cooling article has an exterior surface temperature of about 4°C for application to the exterior surface of the human patient's knee. In certain embodiments, the cooling article has an exterior surface temperature of about 5°C for application to the exterior surface of the human patient's knee.

In certain embodiments, the cooling article has an exterior surface temperature of about 6°C for application to the exterior surface of the human patient's knee. In certain embodiments, the cooling article has an exterior surface temperature of about 7°C for application to the exterior surface of the human patient's knee. In certain embodiments, the cooling article has an exterior surface temperature of about 8°C for application to the exterior surface of the human patient's knee. In certain embodiments, the cooling article has an exterior surface temperature of about 9°C for application to the exterior surface of the human patient's knee. In certain embodiments, the cooling article has an exterior surface temperature of about 10°C for application to the exterior surface of the human patient's knee.

In certain embodiments, the cooling article has an exterior surface temperature of about 11°C for application to the exterior surface of the human patient's knee. In certain embodiments, the cooling article has an exterior surface temperature of about 12°C for application to the exterior surface of the human patient's knee. In certain embodiments, the cooling article has an exterior surface temperature of about 13°C for application to the exterior surface of the human patient's knee. In certain embodiments, the cooling article has an exterior surface temperature of about 14°C for application to the exterior surface of the human patient's knee. In certain embodiments, the cooling article has an exterior surface temperature of about 15°C for application to the exterior surface of the human patient's knee.

### Further Exemplary Characterization of the Cooling Article

The uses may be further characterized according to addtional features of the cooling article. In certain embodiments, the cooling article is a material wrap cooled via a circulating fluid. In certain embodiments, the cooling article is a textile wrap cooled via a circulating fluid. In certain embodiments, the cooling article is an at least partially frozen gel pack.

In certain embodiments, the cooling article covers at least 10%, 20%, 30%, 40%, 50%, 60%, 70%, 80%, or 90% of the external surface of said patient's knee. In certain embodiments, the cooling article covers at least 70% of the external surface of said patient's knee. In certain embodiments, the cooling article covers at least 80% of the external surface of said patient's knee. In certain embodiments, the cooling article covers at least 90% of the external surface of said patient's knee. In certain embodiments, the cooling article covers at least 95% of the external surface of said patient's knee.

In certain embodiments, the cooling article is a wrap-on cooled pad sold by Breg, Inc. Exemplary wrap-on pads sold by Breg, Inc. use circulating ice-water to achieve cooling, and include the Breg Knee WrapOn Polar Pad. Figure 1 herein is an illustration of a cooling article, that is a wrap-on pad, applied to a human knee.

In certain embodiments, the cooling article is an Elasto-Gel All Purpose Therapy Wrap, such as one that measures 6 inches by 24 inches in size. The Elasto-Gel All Purpose Therapy Wrap may be characterized as one that is removed from a freezer (approximately 0°F) just prior to application to a patient.

In certain embodiments, the cooling article is a gel, oil, or other material having a temperature of, for example, from about -20°C to about 5°C when initially placed into contact with the patient.

### Pharmaceutical Composition Comprising Capsaicin

The uses may be further characterized according to features of the pharmaceutical composition comprising capsaicin. For example, in certain embodiments, the pharmaceutical composition comprising capsaicin is an aqueous mixture containing capsaicin. In certain embodiments, the pharmaceutical composition comprising capsaicin has a volume in the range of about 4 mL to about 0.5 mL. In certain embodiments, the pharmaceutical composition comprising capsaicin has a volume of about 4 mL. In certain embodiments, the pharmaceutical composition comprising capsaicin has a volume of about 2 mL. In certain embodiments, the pharmaceutical composition comprising capsaicin has a volume of about 1 mL. In certain embodiments, the pharmaceutical composition comprising capsaicin has a volume of about 0.5 mL.

In certain embodiments, the pharmaceutical composition comprising capsaicin has a volume of about 0.05 mL, 0.1 mL, 0.125 mL, 0.2 mL, 0.5 mL, 0.75 mL, 1.0 mL, 1.25 mL, 1.5 mL, 1.75 mL, 2.0 mL, 2.25 mL, 2.5 mL, 2.75 mL, 3.0 mL, 3.25 mL, 3.5 mL, 3.75 mL, or 4.0 mL. In certain embodiments, the pharmaceutical composition comprising capsaicin has a volume in the range of from about 0.01 mL to about 0.1 mL, about 0.1 mL to about 0.2 mL, about 0.2 mL to about 0.5 mL, about 0.5 mL to about 0.75 mL, about 0.75 mL to about 1.0 mL, about 1.0 mL to about 1.5 mL, about 1.5 mL to about 2.0 mL, about 2.0 mL to about 2.5 mL, about 2.5 mL to about 3.0 mL, about 3.0 mL to about 3.5 mL, about 3.5 mL to about 4.0 mL, about 4.0 mL to about 5.0 mL, about 5.0 mL to about 6.0 mL, about 6.0 mL to about 9 mL, or about 9 mL to about 12 mL.

### Magnitude of Transient Burning Sensation Due to Capsaicin

The uses may be further characterized according to the magnitude of the transient burning sensation due to capsaicin. For example, in certain embodiments, the patient experiences transient burning sensation no greater than level one on a visual analog scale ranging from zero to four (i.e., (0) none, (1) mild, (2) moderate, (3) moderately severe, and (4) severe), due to administering the capsaicin. In certain embodiments, the patient experiences transient burning sensation no greater than level two on a visual analog scale ranging from zero to four (i.e., (0) none, (1) mild, (2) moderate, (3) moderately severe, and (4) severe), due to administering the capsaicin.

In certain embodiments, transient burning sensation is evaluated at about 1 minute after administration of the capsaicin. In certain embodiments, transient burning sensation is evaluated at about 10 minutes after administration of the capsaicin. In certain embodiments, transient burning sensation is evaluated at about 30 minutes after administration of the capsaicin. In certain embodiments, transient burning sensation is evaluated at about 60 minutes after administration of the capsaicin. In certain embodiments, transient burning sensation is evaluated at about 120 minutes after administration of the capsaicin. In certain embodiments, transient burning sensation is evaluated at about 180 minutes after administration of the capsaicin.

### Time for Administering the Second Dose of Capsaicin

The uses may be further characterized according to the time for administering the second dose of capsaicin. For example, in certain embodiments, the second dose of capsaicin is administered no sooner than 4 months after administration of the first dose of capsaicin. In certain embodiments, the second dose of capsaicin is administered no sooner than 5 months after administration of the first dose of capsaicin. In certain embodiments, the second dose of capsaicin is administered no sooner than 6 months after administration of the first dose of capsaicin. In certain embodiments, the second dose of capsaicin is administered no sooner than 7 months after administration of the first dose of capsaicin. In certain embodiments, the second dose of capsaicin is administered no sooner than 8 months after administration of the first dose of capsaicin. In certain embodiments, the second dose of capsaicin is administered no sooner than 9 months after administration of the first dose of capsaicin. In certain embodiments, the second dose of capsaicin is administered no sooner than 10 months after administration of the first dose of capsaicin. In certain embodiments, the second dose of capsaicin is administered no sooner than 11 months after administration of the first dose of capsaicin. In certain embodiments, the second dose of capsaicin is administered no sooner than 12 months after administration of the first dose of capsaicin. In certain embodiments, the second dose of capsaicin is administered no sooner than 13, 14, 15, 16, 17, 18, 19, 20, 21, 22, 23, or 24 months after administration of the first dose of capsaicin.

In certain embodiments, the second dose of capsaicin is administered at a time that is in the range of 3 months to 5 months after administration of the first dose of capsaicin. In certain embodiments, the second dose of capsaicin is administered at a time that is in the range of 4 months to 6 months after administration of the first dose of capsaicin. In certain embodiments, the second dose of capsaicin is administered at a time that is in the range of 5 months to 7 months after administration of the first dose of capsaicin. In certain embodiments, the second dose of capsaicin is administered at a time that is in the range of 6 months to 8 months after administration of the first dose of capsaicin. In certain embodiments, the second dose of capsaicin is administered at a time that is in the range of 7 months to 9 months after administration of the first dose of capsaicin. In certain embodiments, the second dose of capsaicin is administered at a time that is in the range of 8 months to 10 months after administration of the first dose of capsaicin. In certain embodiments, the second dose of capsaicin is administered at a time that is in the range of 9 months to 11 months after administration of the first dose of capsaicin. In certain embodiments, the second dose of capsaicin is administered at a time that is in the range of 10 months to 12 months after administration of the first dose of capsaicin. In certain embodiments, the second dose of capsaicin is administered at a time that is in the range of 12 months to 16 months after administration of the first dose of capsaicin. In certain embodiments, the second dose of capsaicin is administered at a time that is in the range of 16 months to 20 months after administration of the first dose of capsaicin. In certain embodiments, the second dose of capsaicin is administered at a time that is in the range of 20 months to 24 months after administration of the first dose of capsaicin.

In certain embodiments, the second dose of capsaicin is administered at a time that is in the range of 4 months to 9 months after administration of the first dose of capsaicin. In certain embodiments, the second dose of capsaicin is administered at a time that is in the range of 5 months to 9 months after administration of the first dose of capsaicin. In certain embodiments, the second dose of capsaicin is administered at a time that is in the range of 6 months to 9 months after administration of the first dose of capsaicin. In certain embodiments, the second dose of capsaicin is administered at a time that is in the range of 6 months to 12 months after administration of the first dose of capsaicin.

In certain embodiments, the second dose of capsaicin is administered about 4 months after administration of the first dose of capsaicin. In certain embodiments, the second dose of capsaicin is administered about 5 months after administration of the first dose of capsaicin. In certain embodiments, the second dose of capsaicin is administered about 6 months after administration of the first dose of capsaicin. In certain embodiments, the second dose of capsaicin is administered about 26 weeks after administration of the first dose of capsaicin. In certain embodiments, the second dose of capsaicin is administered about 7 months after administration of the first dose of capsaicin. In certain embodiments, the second dose of capsaicin is administered about 8 months after administration of the first dose of capsaicin. In certain embodiments, the second dose of capsaicin is administered about 9 months after administration of the first dose of capsaicin. In certain embodiments, the second dose of capsaicin is administered about 10 months after administration of the first dose of capsaicin. In certain embodiments, the second dose of capsaicin is administered about 11 months after administration of the first dose of capsaicin. In certain embodiments, the second dose of capsaicin is administered about 12 months after administration of the first dose of capsaicin. In certain embodiments, the second dose of capsaicin is administered at about 13, 14, 15, 16, 17, 18, 19, 20, 21, 22, 23, or 24 months after administration of the first dose of capsaicin.

In certain embodiments, the second dose of capsaicin is administered as needed but no sooner than 4 months after administration of the first dose of capsaicin. In certain embodiments, the second dose of capsaicin is administered as needed but no sooner than 6 months after administration of the first dose of capsaicin.

### Time for Administering Any Additional Dose of Capsaicin

The uses may be further characterized according to the time for administering any additional dose of capsaicin. For example, in certain embodiments, any additional dose of capsaicin subsequent to the second dose of capsaicin is administered at a time that is no sooner than about 4 months after administration of the prior dose of capsaicin. In certain embodiments, any additional dose of capsaicin subsequent to the second dose of capsaicin is administered at a time that is no sooner than about 5 months after administration of the prior dose of capsaicin. In certain embodiments, any additional dose of capsaicin subsequent to the second dose of capsaicin is administered at a time that is no sooner than about 6 months after administration of the prior dose of capsaicin. In certain embodiments, any additional dose of capsaicin subsequent to the second dose of capsaicin is administered at a time that is no sooner than about 7 months after administration of the prior dose of capsaicin. In certain embodiments, any additional dose of capsaicin subsequent to the second dose of capsaicin is administered at a time that is no sooner than about 8 months after administration of the prior dose of capsaicin.

In certain embodiments, any additional dose of capsaicin subsequent to the second dose of capsaicin is administered at a time that is no sooner than from about 4 months to about 8 months after administration of the prior dose of capsaicin. In certain embodiments, any additional dose of capsaicin subsequent to the second dose of capsaicin is administered at a time that is no sooner than from about 6 months to about 8 months after administration of the prior dose of capsaicin. In certain embodiments, any additional dose of capsaicin subsequent to the second dose of capsaicin is administered at a time that is no sooner than from about 7 months to about 9 months after administration of the prior dose of capsaicin. In certain embodiments, any additional dose of capsaicin subsequent to the second dose of capsaicin is administered at a time that is no sooner than from about 8 months to about 10 months after administration of the prior dose of capsaicin. In certain embodiments, any additional dose of capsaicin subsequent to the second dose of capsaicin is administered at a time that is no sooner than from about 9 months to about 11 months after administration of the prior dose of capsaicin. In certain embodiments, any additional dose of capsaicin subsequent to the second dose of capsaicin is administered at a time that is no sooner than from about 10 months to about 12 months after administration of the prior dose of capsaicin. In certain embodiments, any additional dose of capsaicin subsequent to the second dose of capsaicin is administered at a time that is no sooner than from about 12 months to about 16 months, about 16 months to about 20 months, or about 20 months to about 24 months after administration of the prior dose of capsaicin.

In certain embodiments, any additional dose of capsaicin is administered as needed but no sooner than 4 months after administration of the prior dose of capsaicin. In certain embodiments, any additional dose of capsaicin is administered as needed but no sooner than 6 months after administration of the prior dose of capsaicin.

### Duration of Reduction in Knee Joint Pain

The uses may be further characterized according to the duration of reduction in knee joint pain (e.g., osteoarthritic knee joint pain). For example, in certain embodiments, the pain is ameliorated for a duration of at least 9 months. In certain embodiments, the pain is ameliorated for a duration of at least 10 months. In certain embodiments, the pain is ameliorated for a duration of at least 11 months. In certain embodiments, the pain is ameliorated for a duration of at least 12 months. In certain embodiments, the pain is ameliorated for a duration of at least 13 months. In certain embodiments, the pain is ameliorated for a duration of at least 14 months. In certain embodiments, the pain is ameliorated for a duration of at least 15 months. In certain embodiments, the pain is ameliorated for a duration of at least 16 months. In certain embodiments, the pain is ameliorated for a duration of at least 18 months. In certain embodiments, the pain is ameliorated for a duration of at least 24 months.

In certain embodiments, the pain is ameliorated for a duration of from about 9 months to about 12 months. In certain embodiments, the pain is ameliorated for a duration of from about 9 months to about 18 months. In certain embodiments, the pain is ameliorated for a duration of from about 9 months to about 24 months. In certain embodiments, the pain is ameliorated for a duration of from about 9 months to about 30 months, about 9 months to about 36 months, about 12 months to about 24 months, about 12 months to about 36 months, about 18 months to about 24 months, about 18 months to about 36 months, or about 24 months to about 36 months.

### Dose of Capsaicin

The uses may be further characterized according to the dose of capsaicin. In certain embodiments, the dose of capsaicin is 1 mg. In a more specific embodiment, the first dose of capsaicin is in an amount of 1 mg capsaicin. In certain embodiments, the second dose of capsaicin is in an amount of 1 mg capsaicin. In certain embodiments, the any additional dose of capsaicin is in an amount of 1 mg capsaicin.

### Exemplary Further Characterization of Reduction in Knee Joint Pain

The uses may be further characterized according to the achieved reduction in knee joint pain. Accordingly, in certain embodiments, the use is characterized by achieving a reduction in average pain in the knee with walking over the previous twenty-four hours by at least 1 on the Numeric Pain Rating Scale of 0-10 (NPRS, where 0 = "no pain" and 10 = "worst possible pain") for a duration of at least 6 months. In certain embodiments, the use is characterized by achieving a reduction in average pain in the knee with walking over the previous twenty-four hours by at least 1 on the Numeric Pain Rating Scale of 0-10 (NPRS) for a duration of at least 7 months. In certain embodiments, the use is characterized by achieving a reduction in average pain in the knee with walking over the previous twenty-four hours by at least 1 on the Numeric Pain Rating Scale of 0-10 (NPRS) for a duration of at least 8 months. In certain embodiments, the use is characterized by achieving a reduction in average pain in the knee with walking over the previous twenty-four hours by at least 1 on the Numeric Pain Rating Scale of 0-10 (NPRS) for a duration of at least 9 months. In certain embodiments, the use is characterized by achieving a reduction in average pain in the knee with walking over the previous twenty-four hours by at least 1 on the Numeric Pain Rating Scale of 0-10 (NPRS) for a duration of at least 10 months. In certain embodiments, the use is characterized by achieving a reduction in average pain in the knee with walking over the previous twenty-four hours by at least 1 on the Numeric Pain Rating Scale of 0-10 (NPRS) for a duration of at least 11 months. In certain embodiments, the use is characterized by achieving a reduction in average pain in the knee with walking over the previous twenty-four hours by at least 1 on the Numeric Pain Rating Scale of 0-10 (NPRS) for a duration of at least 12 months. In certain embodiments, the use is characterized by achieving a reduction in average pain in the knee with walking over the previous twenty-four hours by at least 1 on the Numeric Pain Rating Scale of 0-10 (NPRS) for a duration of at least 13 months. In certain embodiments, the use is characterized by achieving a reduction in average pain in the knee with walking over the previous twenty-four hours by at least 1 on the Numeric Pain Rating Scale of 0-10 (NPRS) for a duration of at least 14 months. In certain embodiments, the use is characterized by achieving a reduction in average pain in the knee with walking over the previous twenty-four hours by at least 1 on the Numeric Pain Rating Scale of 0-10 (NPRS) for a duration of at least 16 months. In certain embodiments, the use is characterized by achieving a reduction in average pain in the knee with walking over the previous twenty-four hours by at least 1 on the Numeric Pain Rating Scale of 0-10 (NPRS) for a duration of at least 18 months. In certain embodiments, the use is characterized by achieving a reduction in average pain in the knee with walking over the previous twenty-four hours by at least 1 on the Numeric Pain Rating Scale of 0-10 (NPRS) for a duration of at least 20 or 22 months. In certain embodiments, the use is characterized by achieving a reduction in average pain in the knee with walking over the previous twenty-four hours by at least 1 on the Numeric Pain Rating Scale of 0-10 (NPRS) for a duration of at least 24 months.

In certain embodiments, the use is characterized by achieving a reduction in average pain in the knee with walking over the previous twenty-four hours by at least 2 on the Numeric Pain Rating Scale of 0-10 (NPRS) for a duration of at least 6 months. In certain embodiments, the use is characterized by achieving a reduction in average pain in the knee with walking over the previous twenty-four hours by at least 2 on the Numeric Pain Rating Scale of 0-10 (NPRS) for a duration of at least 7 months. In certain embodiments, the use is characterized by achieving a reduction in average pain in the knee with walking over the previous twenty-four hours by at least 2 on the Numeric Pain Rating Scale of 0-10 (NPRS) for a duration of at least 8 months. In certain embodiments, the use is characterized by achieving a reduction in average pain in the knee with walking over the previous twenty-four hours by at least 2 on the Numeric Pain Rating Scale of 0-10 (NPRS) for a duration of at least 9 months. In certain embodiments, the use is characterized by achieving a reduction in average pain in the knee with walking over the previous twenty-four hours by at least 2 on the Numeric Pain Rating Scale of 0-10 (NPRS) for a duration of at least 10 months. In certain embodiments, the use is characterized by achieving a reduction in average pain in the knee with walking over the previous twenty-four hours by at least 1 on the Numeric Pain Rating Scale of 0-10 (NPRS) for a duration of at least 11 months. In certain embodiments, the use is characterized by achieving a reduction in average pain in the knee with walking over the previous twenty-four hours by at least 2 on the Numeric Pain Rating Scale of 0-10 (NPRS) for a duration of at least 12 months. In certain embodiments, the use is characterized by achieving a reduction in average pain in the knee with walking over the previous twenty-four hours by at least 2 on the Numeric Pain Rating Scale of 0-10 (NPRS) for a duration of at least 13 months. In certain embodiments, the reduction in average pain in the knee with walking over the previous twenty-four hours by at least 1 on the Numeric Pain Rating Scale of 0-10 (NPRS) for a duration of at least 14 months. In certain embodiments, the use is characterized by achieving a reduction in average pain in the knee with walking over the previous twenty-four hours by at least 2 on the Numeric Pain Rating Scale of 0-10 (NPRS) for a duration of at least 16 months. In certain embodiments, the use is characterized by achieving a reduction in average pain in the knee with walking over the previous twenty-four hours by at least 2 on the Numeric Pain Rating Scale of 0-10 (NPRS) for a duration of at least 18 months. In certain embodiments, the use is characterized by achieving a reduction in average pain in the knee with walking over the previous twenty-four hours by at least 2 on the Numeric Pain Rating Scale of 0-10 (NPRS) for a duration of at least 20 or 22 months. In certain embodiments, the use is characterized by achieving a reduction in average pain in the knee with walking over the previous twenty-four hours by at least 2 on the Numeric Pain Rating Scale of 0-10 (NPRS) for a duration of at least 24 months.

In certain embodiments, the use is characterized by reducing the patient's average pain in the knee with walking so that the patient's average pain in the knee with walking is no greater than 1 on the Numeric Pain Rating Scale of 0-10 (NPRS) for a duration of at least 6 months. In certain embodiments, the use is characterized by reducing the patient's average pain in the knee with walking so that the patient's average pain in the knee with walking is no greater than 1 on the Numeric Pain Rating Scale of 0-10 (NPRS) for a duration of at least 7 months. In certain embodiments, the use is characterized by reducing the patient's average pain in the knee with walking so that the patient's average pain in the knee with walking is no greater than 1 on the Numeric Pain Rating Scale of 0-10 (NPRS) for a duration of at least 8 months. In certain embodiments, the use is characterized by reducing the patient's average pain in the knee with walking so that the patient's average pain in the knee with walking is no greater than 1 on the Numeric Pain Rating Scale of 0-10 (NPRS) for a duration of at least 9 months. In certain embodiments, the use is characterized by reducing the patient's average pain in the knee with walking so that the patient's average pain in the knee with walking is no greater than 1 on the Numeric Pain Rating Scale of 0-10 (NPRS) for a duration of at least 10 months. In certain embodiments, the use is characterized by reducing the patient's average pain in the knee with walking so that the patient's average pain in the knee with walking is no greater than 1 on the Numeric Pain Rating Scale of 0-10 (NPRS) for a duration of at least 11 months. In certain embodiments, the use is characterized by reducing the patient's average pain in the knee with walking so that the patient's average pain in the knee with walking is no greater than 1 on the Numeric Pain Rating Scale of 0-10 (NPRS) for a duration of at least 12 months. In certain embodiments, the use is characterized by reducing the patient's average pain in the knee with walking so that the patient's average pain in the knee with walking is no greater than 1 on the Numeric Pain Rating Scale of 0-10 (NPRS) for a duration of at least 13 months. In certain embodiments, the use is characterized by reducing the patient's average pain in the knee with walking so that the patient's average pain in the knee with walking is no greater than 1 on the Numeric Pain Rating Scale of 0-10 (NPRS) for a duration of at least 14 months. In certain embodiments, the use is characterized by reducing the patient's average pain in the knee with walking so that the patient's average pain in the knee with walking is no greater than 1 on the Numeric Pain Rating Scale of 0-10 (NPRS) for a duration of at least 16 months. In certain embodiments, the use is characterized by reducing the patient's average pain in the knee with walking so that the patient's average pain in the knee with walking is no greater than 1 on the Numeric Pain Rating Scale of 0-10 (NPRS) for a duration of at least 18 months. In certain embodiments, the use is characterized by reducing the patient's average pain in the knee with walking so that the patient's average pain in the knee with walking is no greater than 1 on the Numeric Pain Rating Scale of 0-10 (NPRS) for a duration of at least 20 or 22 months. In certain embodiments, the use is characterized by reducing the patient's average pain in the knee with walking so that the patient's average pain in the knee with walking is no greater than 1 on the Numeric Pain Rating Scale of 0-10 (NPRS) for a duration of at least 24 months.

In certain embodiments, the use is characterized by reducing the patient's average pain in the knee with walking so that the patient's average pain in the knee with walking is no greater than 2 on the Numeric Pain Rating Scale of 0-10 (NPRS) for a duration of at least 6 months. In certain embodiments, the use is characterized by reducing the patient's average pain in the knee with walking so that the patient's average pain in the knee with walking is no greater than 2 on the Numeric Pain Rating Scale of 0-10 (NPRS) for a duration of at least 7 months. In certain embodiments, the use is characterized by reducing the patient's average pain in the knee with walking so that the patient's average pain in the knee with walking is no greater than 2 on the Numeric Pain Rating Scale of 0-10 (NPRS) for a duration of at least 8 months. In certain embodiments, the use is characterized by reducing the patient's average pain in the knee with walking so that the patient's average pain in the knee with walking is no greater than 2 on the Numeric Pain Rating Scale of 0-10 (NPRS) for a duration of at least 9 months. In certain embodiments, the use is characterized by reducing the patient's average pain in the knee with walking so that the patient's average pain in the knee with walking is no greater than 2 on the Numeric Pain Rating Scale of 0-10 (NPRS) for a duration of at least 10 months. In certain embodiments, the use is characterized by reducing the patient's average pain in the knee with walking so that the patient's average pain in the knee with walking is no greater than 2 on the Numeric Pain Rating Scale of 0-10 (NPRS) for a duration of at least 11 months. In certain embodiments, the use is characterized by reducing the patient's average pain in the knee with walking so that the patient's average pain in the knee with walking is no greater than 2 on the Numeric Pain Rating Scale of 0-10 (NPRS) for a duration of at least 12 months. In certain embodiments, the use is characterized by reducing the patient's average pain in the knee with walking so that the patient's average pain in the knee with walking is no greater than 2 on the Numeric Pain Rating Scale of 0-10 (NPRS) for a duration of at least 13 months. In certain embodiments, the use is characterized by reducing the patient's average pain in the knee with walking so that the patient's average pain in the knee with walking is no greater than 2 on the Numeric Pain Rating Scale of 0-10 (NPRS) for a duration of at least 14 months. In certain embodiments, the use is characterized by reducing the patient's average pain in the knee with walking so that the patient's average pain in the knee with walking is no greater than 2 on the Numeric Pain Rating Scale of 0-10 (NPRS) for a duration of at least 16 months. In certain embodiments, the use is characterized by reducing the patient's average pain in the knee with walking so that the patient's average pain in the knee with walking is no greater than 2 on the Numeric Pain Rating Scale of 0-10 (NPRS) for a duration of at least 18 months. In certain embodiments, the use is characterized by reducing the patient's average pain in the knee with walking so that the patient's average pain in the knee with walking is no greater than 2 on the Numeric Pain Rating Scale of 0-10 (NPRS) for a duration of at least 20 or 22 months. In certain embodiments, the use is characterized by reducing the patient's average pain in the knee with walking so that the patient's average pain in the knee with walking is no greater than 2 on the Numeric Pain Rating Scale of 0-10 (NPRS) for a duration of at least 24 months.

### Isomeric Purity of Capsaicin

The uses may be further characterized according to the isomeric purity of capsaicin. For example, in certain embodiments, the capsaicin is a mixture of *cis*-capsaicin and *trans-*capsaicin that contains at least 98% by weight *trans*-capsaicin. In certain embodiments, the capsaicin is a mixture of *cis*-capsaicin and *trans*-capsaicin that contains at least 99% by weight trans-capsaicin. In certain embodiments, the capsaicin consists essentially of the *trans* isomer.

### Chemical Purity of Capsaicin

The uses may be further characterized according to the chemical purity of capsaicin. For example, in certain embodiments, the capsaicin has a chemical purity of at least 98% by weight (which means the presence of a component other than capsaicin is ≤2 % by weight). In certain embodiments, the capsaicin has a chemical purity of at least 99% by weight (which means the presence of a component other than capsaicin is ≤1 % by weight. In certain embodiments, the capsaicin has a chemical purity of at least 99.5% by weight (which means the presence of a component other than capsaicin is ≤0.5 % by weight). In certain embodiments, the capsaicin has a chemical purity of at least 99.8% by weight (which means the presence of a component other than capsaicin is ≤0.2 % by weight).

### Exemplary Further Characterization of Patients for Treatment

The uses may be further characterized according to the patients for treatment. In certain embodiments, the human patient has an age in the range of about 20 to about 30 years old, about 30 to about 40 years old, about 40 to about 50 years old, about 50 to about 60 years old, or about 60 to about 70 years old, or an age greater than 70 years old. In certain embodiments, the human patient is an adult human male or an adult human female. In certain embodiments, the human patient is a pediatric human (e.g., a human that is less than 15, 16, or 18 years old). In certain embodiments, the human patient has a body mass index less than or equal to 45 kg/m². In certain embodiments, the human patient has a body mass index in the range of from about 18 kg/m² to about 32 kg/m².

The human patient may also be characterized by the presence or absence of a co-morbid condition, such as diabetes. In human patients with diabetes, nerve regrowth may occur more slowly, such that a greater amount of time may elapse between administration of the first dose of capsaicin and the second dose of capsaicin while still maintaining good relief from knee joint pain. For instance, in certain embodiments for such patients, the second dose of capsaicin may be administered at a time that is about 8 to about 12 months (or even 10 to 14 months) after administration of the first dose of capsaicin to the patient's knee joint.

The human patient may also be characterized according to how quickly nerve regrowth occurs. Often times, human patients that are older in age exhibit nerve regrowth at a slower rate. As such, patients that are older in age (e.g., patients having an age greater than 45 years old, 50 years old, or 60 years old) may achieve sufficient amelioration of knee joint pain through a procedure in which a greater amount of time may elapses between administration of the first dose of capsaicin and the second dose of capsaicin. For instance, in certain embodiments for such patients, the second dose of capsaicin may be administered at a time that is about 8 to about 12 months (or even 10 to 14 months) after administration of the first dose of capsaicin to the patient's knee joint.

### Avoidance of Heat

The uses may be further characterized according to the presence or absence of a step of avoiding heat for certain durations of time after administration of capsaicin. For example, in certain embodiments, the patient does not expose area receiving a capsaicin dose to heat for a duration of at least 12 hours after administration of capsaicin. In certain embodiments, the patient does not expose area receiving a capsaicin dose to heat for a duration of at least 24 hours after administration of capsaicin.

### Procedures to Evaluate Reduction in Pain

Reduction in pain experienced by the patient can be evaluated using procedures described in the literature, such as Patient Global Impression of Change (PGIC; change vs baseline in index knee on 7-point scale: 1=very much improved; 7=very much worse, with scores of 1 or 2 indicating significant improvement), Patient-specific Functional Scale (PSFS; rate ≤3 important activities difficult to perform due to index knee pain on 0-10 scale: 0=able to perform; 10=unable to perform), and the Western Ontario and McMaster Universities Osteoarthritis Index (WOMAC) B stiffness subscale and WOMAC C function subscale.

### Duration of Time Between Steps

The uses may be further characterized according to the duration of time that elapses between performing individual steps of the use, such as the duration of time between completion of step (a) and start of step (b). In certain embodiments, the use is characterized by one or more of (i) the duration of time between completion of step (a) and start of step (b), (ii) the duration of time between completion of step (b) and start of step (c), (iii) the duration of time between completion of step (c) and start of step (d), and (iv) the duration of time between completion of step (d) and start of step (e). In certain embodiments, the duration of time between sequential steps is as soon as reasonably achievable according to standard medical procedure. In certain embodiments, the duration of time between sequential steps is less than 30 minutes, 20 minutes, 15 minutes, 10 minutes, 5 minutes, 3 minutes, or 1 minute. In a preferred embodiment, the duration of time between sequential steps is less than 20 minutes. In a more preferred embodiment, the duration of time between sequential steps is less than 5 minutes.

The uses can be further characterized according to the duration of time between completion of step (b) and the start of step (d). In certain embodiments, the duration of time between completion of step (b) and the start of step (d) is from about 30 minutes to about 60 minutes. In certain embodiments, the duration of time between completion of step (b) and the start of step (d) is from about 40 minutes to about 60 minutes. In certain embodiments, the duration of time between completion of step (b) and the start of step (d) is from about 50 minutes to about 60 minutes. In certain embodiments, the duration of time between completion of step (b) and the start of step (d) is from about 30 minutes to about 50 minutes. In certain embodiments, the duration of time between completion of step (b) and the start of step (d) is from about 30 minutes to about 45 minutes.

### Reducing Effusion Volume in Knee Joints with Effusion

For patients in which the knee joint to receive capsaicin suffers from effusion, in certain embodiments, the volume of intra-articular fluid in the knee joint presenting with joint effusion is reduced prior to administration of a local anesthetic agent (e.g., the pharmaceutical composition comprising a single pain-relief agent) and/or capsaicin. In certain embodiments, the volume of intra-articular fluid in the joint presenting with joint effusion is reduced prior to administering a local anesthetic agent. In certain embodiments, the volume of intra-articular fluid in the joint presenting with joint effusion is reduced to achieve a volume of intra-articular fluid that is within about 5%, 10% or 20% of that of a healthy patient of similar height, weight, and age.

### Temperature of the Patient's Skin in Proximity to the Joint

The uses may be further characterized according to the temperature of the patient's skin in proximity to the knee joint to receive or which has received capsaicin according to the use. For example, in certain embodiments, in step (c) applying a cooling article to an exterior surface of said knee achieves a temperature in the range of from about 5°C to about 7°C for said skin. In certain embodiments, in step (c) applying a cooling article to an exterior surface of said knee achieves a temperature in the range of from about 7°C to about 9°C for said skin. In certain embodiments, in step (c) applying a cooling article to an exterior surface of said knee achieves a temperature in the range of from about 9°C to about 11°C for said skin. In certain embodiments, in step (c) applying a cooling article to an exterior surface of said knee achieves a temperature in the range of from about 11°C to about 13°C for said skin. In certain embodiments, in step (c) applying a cooling article to an exterior surface of said knee achieves a temperature in the range of from about 13°C to about 15°C for said skin. In certain embodiments, in step (c) applying a cooling article to an exterior surface of said knee achieves a temperature in the range of from about 15°C to about 17°C for said skin. In certain embodiments, in step (c) applying a cooling article to an exterior surface of said knee achieves a temperature in the range of from about 17°C to about 19°C for said skin. In certain embodiments, in step (c) applying a cooling article to an exterior surface of said knee achieves a temperature in the range of from about 19°C to about 21°C for said skin. In certain embodiments, in step (c) applying a cooling article to an exterior surface of said knee achieves a temperature in the range of from about 21°C to about 23°C for said skin. In certain embodiments, in step (c) applying a cooling article to an exterior surface of said knee achieves a temperature in the range of from about 23°C to about 25°C for said skin. In certain embodiments, in step (c) applying a cooling article to an exterior surface of said knee achieves a temperature in the range of from about 25°C to about 27°C for said skin. In certain embodiments, in step (c) applying a cooling article to an exterior surface of said knee achieves a temperature in the range of from about 27°C to about 29°C for said skin. In certain embodiments, in step (c) applying a cooling article to an exterior surface of said knee achieves a temperature in the range of from about 29°C to about 30°C for said skin.

In certain embodiments, in step (c) applying a cooling article to an exterior surface of said knee achieves a temperature of about 7°C for said skin. In certain embodiments, in step (c) applying a cooling article to an exterior surface of said knee achieves a temperature of about 8°C for said skin. In certain embodiments, in step (c) applying a cooling article to an exterior surface of said knee achieves a temperature of about 9°C for said skin. In certain embodiments, in step (c) applying a cooling article to an exterior surface of said knee achieves a temperature of about 10°C for said skin. In certain embodiments, in step (c) applying a cooling article to an exterior surface of said knee achieves a temperature of about 11°C for said skin. In certain embodiments, in step (c) applying a cooling article to an exterior surface of said knee achieves a temperature of about 12°C for said skin. In certain embodiments, in step (c) applying a cooling article to an exterior surface of said knee achieves a temperature of about 13°C for said skin. In certain embodiments, in step (c) applying a cooling article to an exterior surface of said knee achieves a temperature of about 14°C for said skin. In certain embodiments, in step (c) applying a cooling article to an exterior surface of said knee achieves a temperature of about 15°C for said skin. In certain embodiments, in step (c) applying a cooling article to an exterior surface of said knee achieves a temperature of about 16°C for said skin. In certain embodiments, in step (c) applying a cooling article to an exterior surface of said knee achieves a temperature of about 17°C for said skin. In certain embodiments, in step (c) applying a cooling article to an exterior surface of said knee achieves a temperature of about 18°C for said skin. In certain embodiments, in step (c) applying a cooling article to an exterior surface of said knee achieves a temperature of about 19°C for said skin. In certain embodiments, in step (c) applying a cooling article to an exterior surface of said knee achieves a temperature of about 20°C for said skin. In certain embodiments, in step (c) applying a cooling article to an exterior surface of said knee achieves a temperature of about 21°C for said skin. In certain embodiments, in step (c) applying a cooling article to an exterior surface of said knee achieves a temperature of about 22°C for said skin. In certain embodiments, in step (c) applying a cooling article to an exterior surface of said knee achieves a temperature of about 23°C for said skin. In certain embodiments, in step (c) applying a cooling article to an exterior surface of said knee achieves a temperature of about 24°C for said skin. In certain embodiments, in step (c) applying a cooling article to an exterior surface of said knee achieves a temperature of about 25°C for said skin. In certain embodiments, in step (c) applying a cooling article to an exterior surface of said knee achieves a temperature of about 25°C for said skin. In certain embodiments, in step (c) applying a cooling article to an exterior surface of said knee achieves a temperature of about 26°C for said skin. In certain embodiments, in step (c) applying a cooling article to an exterior surface of said knee achieves a temperature of about 28°C for said skin. In certain embodiments, in step (c) applying a cooling article to an exterior surface of said knee achieves a temperature of about 29°C for said skin. In certain embodiments, in step (c) applying a cooling article to an exterior surface of said knee achieves a temperature of about 30°C for said skin.

Further, in certain embodiments, the use comprises step (e) wherein a cooling article is applied to an exterior surface of said knee and achieves a temperature in the range of from about 5°C to about 30°C for said skin for a duration of at least 30 minutes. In certain embodiments, the use comprises step (e) wherein a cooling article is applied to an exterior surface of said knee and achieves a temperature in the range of from about 5°C to about 7°C for said skin for a duration of at least 30 minutes. In certain embodiments, the use comprises step (e) wherein a cooling article is applied to an exterior surface of said knee and achieves a temperature in the range of from about 7°C to about 9°C for said skin for a duration of at least 30 minutes. In certain embodiments, the use comprises step (e) wherein a cooling article is applied to an exterior surface of said knee and achieves a temperature in the range of from about 9°C to about 11°C for said skin for a duration of at least 30 minutes. In certain embodiments, the use comprises step (e) wherein a cooling article is applied to an exterior surface of said knee and achieves a temperature in the range of from about 11°C to about 13°C for said skin for a duration of at least 30 minutes. In certain embodiments, the use comprises step (e) wherein a cooling article is applied to an exterior surface of said knee and achieves a temperature in the range of from about 13°C to about 15°C for said skin for a duration of at least 30 minutes. In certain embodiments, the use comprises step (e) wherein a cooling article is applied to an exterior surface of said knee and achieves a temperature in the range of from about 15°C to about 17°C for said skin for a duration of at least 30 minutes. In certain embodiments, the use comprises step (e) wherein a cooling article is applied to an exterior surface of said knee and achieves a temperature in the range of from about 17°C to about 19°C for said skin for a duration of at least 30 minutes. In certain embodiments, the use comprises step (e) wherein a cooling article is applied to an exterior surface of said knee and achieves a temperature in the range of from about 19°C to about 21°C for said skin for a duration of at least 30 minutes. In certain embodiments, the use comprises step (e) wherein a cooling article is applied to an exterior surface of said knee and achieves a temperature in the range of from about 21°C to about 23°C for said skin for a duration of at least 30 minutes. In certain embodiments, the use comprises step (e) wherein a cooling article is applied to an exterior surface of said knee and achieves a temperature in the range of from about 23°C to about 25°C for said skin for a duration of at least 30 minutes. In certain embodiments, the use comprises step (e) wherein a cooling article is applied to an exterior surface of said knee and achieves a temperature in the range of from about 25°C to about 27°C for said skin for a duration of at least 30 minutes. In certain embodiments, the use comprises step (e) wherein a cooling article is applied to an exterior surface of said knee and achieves a temperature in the range of from about 27°C to about 29°C for said skin for a duration of at least 30 minutes. In certain embodiments, the use comprises step (e) wherein a cooling article is applied to an exterior surface of said knee and achieves a temperature of about 5°C for said skin for a duration of at least 30 minutes. In certain embodiments, the use comprises step (e) wherein a cooling article is applied to an exterior surface of said knee and achieves a temperature of about 6°C for said skin for a duration of at least 30 minutes. In certain embodiments, the use comprises step (e) wherein a cooling article is applied to an exterior surface of said knee and achieves a temperature of about 7°C for said skin for a duration of at least 30 minutes. In certain embodiments, the use comprises step (e) wherein a cooling article is applied to an exterior surface of said knee and achieves a temperature of about 8°C for said skin for a duration of at least 30 minutes. In certain embodiments, the use comprises step (e) wherein a cooling article is applied to an exterior surface of said knee and achieves a temperature of about 9°C for said skin for a duration of at least 30 minutes. In certain embodiments, the use comprises step (e) wherein a cooling article is applied to an exterior surface of said knee and achieves a temperature of about 10°C for said skin for a duration of at least 30 minutes. In certain embodiments, the use comprises step (e) wherein a cooling article is applied to an exterior surface of said knee and achieves a temperature of about 11°C for said skin for a duration of at least 30 minutes. In certain embodiments, the use comprises step (e) wherein a cooling article is applied to an exterior surface of said knee and achieves a temperature of about 12°C for said skin for a duration of at least 30 minutes. In certain embodiments, the use comprises step (e) wherein a cooling article is applied to an exterior surface of said knee and achieves a temperature of about 13°C for said skin for a duration of at least 30 minutes. In certain embodiments, the use comprises step (e) wherein a cooling article is applied to an exterior surface of said knee and achieves a temperature of about 14°C for said skin for a duration of at least 30 minutes. In certain embodiments, the use comprises step (e) wherein a cooling article is applied to an exterior surface of said knee and achieves a temperature of about 15°C for said skin for a duration of at least 30 minutes. In certain embodiments, the use comprises step (e) wherein a cooling article is applied to an exterior surface of said knee and achieves a temperature of about 16°C for said skin for a duration of at least 30 minutes. In certain embodiments, the use comprises step (e) wherein a cooling article is applied to an exterior surface of said knee and achieves a temperature of about 17°C for said skin for a duration of at least 30 minutes. In certain embodiments, the use comprises step (e) wherein a cooling article is applied to an exterior surface of said knee and achieves a temperature of about 18°C for said skin for a duration of at least 30 minutes. In certain embodiments, the use comprises step (e) wherein a cooling article is applied to an exterior surface of said knee and achieves a temperature of about 19°C for said skin for a duration of at least 30 minutes. In certain embodiments, the use comprises step (e) wherein a cooling article is applied to an exterior surface of said knee and achieves a temperature of about 20°C for said skin for a duration of at least 30 minutes. In certain embodiments, the use comprises step (e) wherein a cooling article is applied to an exterior surface of said knee and achieves a temperature of about 21°C for said skin for a duration of at least 30 minutes. In certain embodiments, the use comprises step (e) wherein a cooling article is applied to an exterior surface of said knee and achieves a temperature of about 22°C for said skin for a duration of at least 30 minutes. In certain embodiments, the use comprises step (e) wherein a cooling article is applied to an exterior surface of said knee and achieves a temperature of about 23°C for said skin for a duration of at least 30 minutes. In certain embodiments, the use comprises step (e) wherein a cooling article is applied to an exterior surface of said knee and achieves a temperature of about 24°C for said skin for a duration of at least 30 minutes. In certain embodiments, the use comprises step (e) wherein a cooling article is applied to an exterior surface of said knee and achieves a temperature of about 25°C for said skin for a duration of at least 30 minutes. In certain embodiments, the use comprises step (e) wherein a cooling article is applied to an exterior surface of said knee and achieves a temperature of about 26°C for said skin for a duration of at least 30 minutes. In certain embodiments, the use comprises step (e) wherein a cooling article is applied to an exterior surface of said knee and achieves a temperature of about 27°C for said skin for a duration of at least 30 minutes. In certain embodiments, the use comprises step (e) wherein a cooling article is applied to an exterior surface of said knee and achieves a temperature of about 28°C for said skin for a duration of at least 30 minutes. In certain embodiments, the use comprises step (e) wherein a cooling article is applied to an exterior surface of said knee and achieves a temperature of about 29°C for said skin for a duration of at least 30 minutes.

In certain embodiments, said duration is from about 30 minutes to about 60 minutes. In certain embodiments, said duration is from about 30 minutes to about 90 minutes. In certain embodiments, said duration is from about 60 minutes to about 90 minutes.

### Further Exemplary Characterization of the Uses

The uses may optionally be further characterized according to the presence of one or more of the following features (i) a second or subsequent dose of capsaicin provides a duration of relief from knee joint pain that exceeds the duration of relief from knee joint pain provided by the prior dose of capsaicin, (ii) a second or subsequent dose of capsaicin reduces the level of pain experienced by the patient to a threshold that is lower than the threshold of pain experienced by the patient subsequent to the prior dose of capsaicin (i.e., the patient experiences less knee joint pain after receiving the second or subsequent dose of capsaicin than experienced by the patient after receiving the prior dose of capsaicin), and (iii) the patient experiences less transient burning sensation due to administration of capsaicin when a second or subsequent dose of capsaicin is administered compared to the magnitude of transient burning sensation experienced by the patient upon administration of the prior dose of capsaicin.

In embodiments where a second or subsequent dose of capsaicin provides a duration of relief from knee joint pain that exceeds the duration of relief from knee joint pain provided by the prior dose of capsaicin, the use may be further characterized according to the increase in duration of relief from knee joint pain. For example, in certain embodiments, the increase in duration of relief from knee joint pain is at least a 5%, 10%, 15%, 20%, 25%, 30%, 40%, 50%, 60%, 70%, 80%, 90%, 100%, 125%, 150%, 175%, or 200% increase relative to the duration of pain relief provided by the prior dose of capsaicin. In one illustration, where a first dose of capsaicin provides relief from knee joint pain for a duration of 6 months, and the second dose of capsaicin provides relief from knee joint pain for a duration of 7.5 months, the second dose of capsaicin provides a duration of relief from knee joint pain that 25% longer than the first dose of capsaicin.

An increase in the duration of relief from knee joint pain provided by a second or subsequent dose of capsaicin relative to the duration of relief from knee joint pain provided by the prior dose of capsaicin may be characterized according to the number of days, weeks, or months of additional relief from knee joint pain. To illustrative, in an embodiment where a first dose of capsaicin provides 6 months of relief from knee joint pain and a second dose of capsaicin provides 7 months of relief from knee joint pain, the increase in duration of relief from knee joint pain provided by the second dose of capsaicin (relative to the duration of relief from knee joint pain provided by the first dose of capsaicin) is 1 month. In certain embodiments, the increase in the duration of relief from knee joint pain provided by a second or subsequent dose of capsaicin relative to the duration of relief from knee joint pain provided by the prior dose of capsaicin is at least 2, 3, 4, 5, 6, 7, 8, 9, 10, 11, 12, 13, 14, 15, or 16 weeks. In certain embodiments, the increase in the duration of relief from knee joint pain provided by a second or subsequent dose of capsaicin relative to the duration of relief from knee joint pain provided by the prior dose of capsaicin is about 2, 3, 4, 5, 6, 7, 8, 9, 10, 11, 12, 13, 14, 15, or 16 weeks. In certain embodiments, the increase in the duration of relief from knee joint pain provided by a second or subsequent dose of capsaicin relative to the duration of the relief from knee joint pain provided by the prior dose of capsaicin is at least 3, 4, 5, or 6 weeks. In certain embodiments, the increase in the duration of relief from knee joint pain provided by a second or subsequent dose of capsaicin relative to the duration of relief from knee joint pain provided by the prior dose of capsaicin is about 3, 4, 5, or 6 weeks. In certain embodiments, the increase in the duration of relief from knee joint pain provided by the second dose of capsaicin relative to the duration of relief from knee joint pain provided by the first dose of capsaicin is at least 3, 4, 5, or 6 weeks. In certain embodiments, the increase in the duration of relief from knee joint pain provided by the second dose of capsaicin relative to the duration of relief from knee joint pain provided by the first dose of capsaicin is about 3, 4, 5, or 6 weeks.

The overall duration of relief from knee joint pain provided by a use that entails administering a first dose of capsaicin and then a second and optionally subsequent dose(s) of capsaicin may be characterized according to the number of months or years of relief from knee joint pain. For example, in certain embodiments, the overall duration of relief from knee joint pain is at least 12, 14, 16, 18, 20, 22, 24, 26, 28, 30, 32, 34, 36, 38, 40, 42, 44, 48, or 50 months. In certain embodiments, the overall duration of relief from knee joint pain is about 12, 14, 16, 18, 20, 22, 24, 26, 28, 30, 32, 34, 36, 38, 40, 42, 44, 48, or 50 months. In certain embodiments, the overall duration of relief from knee joint pain is from about 1 year to about 2 years, about 1 year to about 3 years, about 1 year to about 4 years, about 1 year to about 5 years, about 2 years to about 3 years, about 2 years to about 4 years, about 2 years to about 5 years, about 3 years to about 4 years, about 3 years to about 5 years, or about 4 years to about 5 years.

In embodiments where a second or subsequent dose of capsaicin reduces the level of pain experienced by the patient to a threshold that is lower than the threshold of pain experienced by the patient subsequent to the prior dose of capsaicin (i.e., the patient experiences less knee joint pain after receiving the second or subsequent dose of capsaicin than experienced by the patient after receiving the prior dose of capsaicin), the use may be further characterized according to the reduction in threshold of pain experienced by the patient. For example in certain embodiments, the reduction in threshold of pain experienced by the patient subsequent to the second or subsequent dose of capsaicin relative to the prior dose of capsaicin is a reduction of at least 1 on the Numeric Pain Rating Scale of 0-10 (NPRS) characterizing the patient's average pain in the knee with walking. In certain embodiments, the reduction in threshold of pain experienced by the patient subsequent to the second or subsequent dose of capsaicin relative to the prior dose of capsaicin is a reduction of at least 2 on the Numeric Pain Rating Scale of 0-10 (NPRS) characterizing the patient's average pain in the knee with walking. In certain embodiments, the reduction in threshold of pain experienced by the patient subsequent to the second or subsequent dose of capsaicin relative to the prior dose of capsaicin is a reduction in the range of from about 1 to about 3 on the Numeric Pain Rating Scale of 0-10 (NPRS) characterizing the patient's average pain in the knee with walking. In certain embodiments, the reduction in threshold of pain experienced by the patient subsequent to the second or subsequent dose of capsaicin relative to the prior dose of capsaicin is a reduction in the range of from about 1 to about 2 on the Numeric Pain Rating Scale of 0-10 (NPRS) characterizing the patient's average pain in the knee with walking. A more specific illustration is where subsequent to receiving the first dose of capsaicin the patient has an average pain in the knee with walking of 2 on the Numeric Pain Rating Scale of 0-10 (NPRS), and then subsequent to receiving the second dose of capsaicin the patient has an average pain in the knee with walking of 1 on the Numeric Pain Rating Scale of 0-10 (NPRS).

In embodiments where a patient experiences less transient burning sensation due to administration of capsaicin when a second or subsequent dose of capsaicin is administered compared to the magnitude of transient burning sensation experienced by the patient upon administration of the prior dose of capsaicin, administration of the second or subsequent dose of capsaicin may be more comfortable for the patient. In certain embodiments, if the reduction in transient burning sensation is sufficient large, then the second or subsequent dose of capsaicin may be administered to the patient according to a protocol that uses less lidocaine and/or less cooling of the knee. In certain embodiments, if the reduction in transient burning sensation is sufficient large, then the second or subsequent dose of capsaicin may be administered to the patient according to a protocol that does not use lidocaine and/or does not involve cooling the knee. In certain embodiments, if the reduction in transient burning sensation is sufficient large, then the second or subsequent dose of capsaicin may be administered to the patient according to a protocol that does not involve cooling the knee. In certain embodiments, the time at which the second or subsequent dose of capsaicin is administered to the patient is selected so that (i) less lidocaine is required to effectively control any transient burning sensation, (ii) less cooling of the knee is required to effectively control any transient burning sensation, (iii) no lidocaine is required to be administered to the patient to effectively control any transient burning sensation, and/or (iv) no cooling of the knee is required to effectively control any transient burning sensation. In a preferred embodiment, the time at which the second or subsequent dose of capsaicin is administered to the patient is selected so that less cooling (or even no cooling) of the knee is required to effectively control any transient burning sensation. Such a time for administration of the second or subsequent dose of capsaicin would coincide with a time in which the prior dose of capsaicin still provides effect to minimize or effectively control any transient burning sensation due to administration of the dose of capsaicin.

A use that provides two or more of the above features is particularly desirable. This could be, for example, an embodiment where a patient experiences both (i) an increase in the duration of relief from knee joint pain after administration of the second or subsequent dose of capsaicin relative to the duration of relief from knee joint pain provided by the prior dose of capsaicin, and (ii) a reduction in the level of pain experienced by the patient after administration of the second or subsequent dose of capsaicin to a threshold that is lower than the threshold of pain experienced by the patient subsequent to the prior dose of capsaicin (i.e., the patient experiences less knee joint pain after receiving the second or subsequent dose of capsaicin than experienced by the patient after receiving the prior dose of capsaicin).

### III. GENERAL ASPECTS OF INJECTABLE FORMULATIONS

Various injectable formulations are described in the literature and known to those of skill in the art. The injectable formulation may typically contain water and one or more additional components to render the formulation optimally suited for injection into a subject.

When administering capsaicin according to uses described herein, the capsaicin is desirably administered in the form of a pharmaceutical composition formulated for injection. In certain embodiments, the pharmaceutical composition formulated for injection is an aqueous pharmaceutical composition.

The capsaicin may be dissolved in oils, polyethylene glycol (PEG), propylene glycol (PG), and/or other solvents commonly used to prepare injectable or implantable solutions. Suitable pharmaceutically acceptable vehicles include aqueous vehicles, nonaqueous vehicles, antimicrobial agents, isotonic agents, buffers, antioxidants, suspending and dispersing agents, emulsifying agents, sequestering or chelating agents, and combinations or mixtures thereof. It is appreciated that when one or more solvents are used in the formulations of the invention, they may be combined, e.g., with a pharmaceutically acceptable buffer and may be present in the final formulation, e.g., in an amount ranging from about 10% to about 100%, more preferably from about 20% to about 100%.

Exemplary aqueous vehicles include Sodium Chloride Injection, Bacteriostatic Sodium Chloride Injection, Ringers Injection, Isotonic Dextrose Injection, Sterile Water Injection, Bacteriostatic Sterile Water Injection, Dextrose Lactated Ringers Injection and any combinations or mixtures thereof.

Exemplary nonaqueous parenteral vehicles include fixed oils of vegetable origin, cottonseed oil, corn oil, sesame oil, peanut oil, and combinations or mixtures thereof.

Exemplary antimicrobial agents in bacteriostatic or fungistatic concentrations include phenols, cresols, mercurials, benzyl alcohol, chlorobutanol, ethyl and propyl p-hydroxybenzoic acid esters, thimerosal, benzalkonium chloride, benzethonium chloride, and mixtures thereof.

Exemplary isotonic agents include sodium chloride, dextrose, and combinations or mixtures thereof.

Exemplary antioxidants include ascorbic acid, sodium bisulfate, and combinations or mixtures thereof.

Exemplary suspending and dispersing agents include sodium carboxymethylcelluose, hydroxypropyl methylcellulose, polyvinylpyrrolidone, any combinations or mixtures thereof.

Exemplary emulsifying agents include anionic emulsifying agents (*e*.*g*., sodium lauryl sulfate, sodium stearate, calcium oleate, and combinations or mixtures thereof), cationic emulsifying agents (*e.g.,* cetrimide), and non-ionic emulsifying agents (*e.g.,* Polysorbate 80 (Tween 80)).

Exemplary sequestering or chelating agents of metal ions include ethylenediaminetetraacetic acid (EDTA), citric acid, sorbitol, tartaric acid, phosphoric acid, and the like.

Suitable surfactants include, but are not limited to, sodium stearyl fumarate, diethanolamine cetyl sulfate, polyethylene glycol, isostearate, polyethoxylated castor oil, benzalkonium chloride, nonoxyl 10, octoxynol 9, polyoxyethylene sorbitan fatty acids (polysorbate 20, 40, 60 and 80), sodium lauryl sulfate, sorbitan esters (sorbitan monolaurate, sorbitan monooleate, sorbitan monopalmitate, sorbitan monostearate, sorbitan sesquioleate, sorbitan trioleate, sorbitan tristearate, sorbitan laurate, sorbitan oleate, sorbitan palmitate, sorbitan stearate, sorbitan dioleate, sorbitan sesqui-isostearate, sorbitan sesquistearate, sorbitan tri-isostearate), lecithin pharmaceutical acceptable salts thereof and combinations thereof. When one or more surfactants are utilized in the formulations of the invention, they may be combined, *e*.*g*., with a pharmaceutically acceptable vehicle and may be present in the final formulation, *e*.*g*., in an amount ranging from about 0.1% to about 20%, more preferably from about 0.5% to about 10%. In certain other embodiments, a surfactant can preferably be combined with one or more of the pharmaceutically acceptable vehicles previously described herein so that the surfactant or buffering agent prevents the initial stinging or burning discomfort associated with capsaicinoid administration, as a wetting agent, emulsifier, solubilizer and/or antimicrobial.

Buffering agents may also be used to provide drug stability; to control the therapeutic activity of the drug substance (Ansel, Howard C., "Introduction to Pharmaceutical Dosage Forms," 4^{th} Ed., 1985); and/or to prevent the initial stinging or burning discomfort associated with capsaicin administration. Suitable buffers include, but are not limited to, sodium bicarbonate, sodium citrate, citric acid, sodium phosphate, pharmaceutically acceptable salts thereof, and combinations thereof. When one or more buffers are utilized in the formulations of the invention, they may be combined, *e.g.,* with a pharmaceutically acceptable vehicle and may be present in the final formulation, *e.g.,* in an amount ranging from about 0.1% to about 20%, more preferably from about 0.5% to about 10%. In certain embodiments, the buffer is an acetate salt, phosphate salt, citrate salt; corresponding acids of the foregoing; and combinations or mixtures thereof.

In certain embodiments, the pharmaceutical vehicle utilized to deliver the injectable capsaicin may comprise about 20% PEG 300, about 10 mM histidine and about 5% sucrose in water for injection. In certain other embodiments, the pharmaceutical vehicle utilized to deliver the injectable capsaicin may comprise about 30-50% PEG 300. This may be used as such or further diluted in water for injection to achieve a larger volume.

The injectable formulation may be further characterized according to the concentration of capsaicin in the formulation. In certain embodiments, the injectable formulation contains the capsaicin at a concentration ranging from about 0.01 mg/mL to about 4 mg/mL, about 0.05 mg/mL to about 3 mg/mL, about 0.1 mg/mL to about 2 mg/mL, about 0.15 mg/mL to about 2 mg/mL, about 0.2 mg/mL to about 0.8 mg/mL, about 0.25 mg/mL to about 0.6 mg/mL, about 0.25 mg/mL to about 0.5 mg/mL, about 0.3 mg/mL to about 0.5 mg/mL, about 0.3 mg/mL to about 0.4 mg/mL, about 0.35 mg/mL to about 0.45 mg/mL, or about 0.375 mg/mL to about 0.425 mg/mL. In certain preferred embodiments, the injectable formulation contains capsaicin at a concentration ranging from about 0.05 mg/mL to about 0.15 mg/mL, or about 0.3 mg/mL to about 0.4 mg/mL. In certain other preferred embodiments, the injectable formulation contains capsaicin at a concentration of about 0.1 mg/mL.

In certain embodiments, the injectable formulation contains *trans*-capsaicin at a concentration ranging from about 0.01 mg/mL to about 4 mg/mL, about 0.05 mg/mL to about 3 mg/mL, about 0.1 mg/mL to about 2 mg/mL, about 0.15 mg/mL to about 2 mg/mL, about 0.2 mg/mL to about 0.8 mg/mL, about 0.25 mg/mL to about 0.6 mg/mL, about 0.25 mg/mL to about 0.5 mg/mL, about 0.3 mg/mL to about 0.5 mg/mL, about 0.3 mg/mL to about 0.4 mg/mL, about 0.35 mg/mL to about 0.45 mg/mL, or about 0.375 mg/mL to about 0.425 mg/mL. In certain preferred embodiments, the injectable formulation contains *trans*-capsaicin at a concentration ranging from about 0.05 mg/mL to about 0.15 mg/mL, or about 0.3 mg/mL to about 0.4 mg/mL. In certain other preferred embodiments, the injectable formulation contains trans-capsaicin at a concentration of about 0.1 mg/mL.

In certain embodiments, the injectable formulation contains the capsaicin at a concentration of about 0.1 mg/mL, 0.15 mg/mL, 0.2 mg/mL, 0.25 mg/mL, 0.3 mg/mL, 0.325 mg/mL, 0.35 mg/mL, 0.37 mg/mL, 0.38 mg/mL, 0.39 mg/mL, 0.4 mg/mL, 0.41 mg/mL, 0.42 mg/mL, 0.43 mg/mL, 0.44 mg/mL, 0.45 mg/mL, 0.475 mg/mL, 0.5 mg/mL, 0.55 mg/mL, 0.575 mg/mL, 0.6 mg/mL, 0.625 mg/mL, 0.65 mg/mL, 0.675 mg/mL, 0.7 mg/mL, 0.75 mg/mL, 0.8 mg/mL, 0.9 mg/mL, 1.0 mg/mL, 1.5 mg/mL, or 2.0 mg/mL. In certain preferred embodiments, the injectable formulation contains the capsaicin at a concentration of about 0.1 mg/mL.

The injectable formulation may be further characterized according to the solvent present to dissolve the capsaicin. In certain embodiments, the solvent in the injectable formulation is a mixture of water and polyethylene glycol (e.g., polyethylene glycol having a number-average molecular weight of about 300 g/mol). The relative amounts of water and polyethylene glycol in the injectable formulation may be characterized. For example, in certain embodiments, the injectable formulation contains a mixture of water and polyethylene glycol (e.g., polyethylene glycol having a number-average molecular weight of about 300 g/mol) as solvent, wherein upon a volume basis there is 3-6 times more water than polyethylene glycol. In certain embodiments, the injectable formulation contains a mixture of water and polyethylene glycol (e.g., polyethylene glycol having a number-average molecular weight of about 300 g/mol) as solvent, wherein upon a volume basis there is 4-5 times more water than polyethylene glycol. In certain embodiments, the polyethylene glycol has a number-average molecular weight in the range of about 250 g/mol to about 350 g/mol.

The injectable formulation may be further characterized according to the volume of injectable formulation administered to tissue proximal to the intermetatarsal neuroma. In certain embodiments, the volume of injectable formulation administered per unit dose is in the range of about 0.5 mL to about 5 mL, about 0.6 mL to about 4 mL, about 0.7 mL to about 3 mL, about 0.8 mL to about 2.5 mL, or about 1 mL to about 2 mL. In certain other embodiments, the volume of injectable formulation administered per unit dose is in the range of about 1.5 mL to about 2.5 mL. In certain other embodiments, the volume of injectable formulation administered per unit dose is about 2 mL.

The foregoing embodiments, may be combined to describe more specific injectable formulations. For example, in certain embodiments, the injectable formulation comprises trans-capsaicin at a concentration of about 0.1 mg/mL, water, and a polyethylene glycol (e.g., polyethylene glycol having a number-average molecular weight of 300 g/mol). In certain embodiments, the injectable formulation comprises *trans*-capsaicin at a concentration of about 0.1 mg/mL, water, and a polyethylene glycol having a number-average molecular weight of 300 g/mol), wherein upon a volume basis there is 4-5 times more water than polyethylene glycol. In certain embodiments, the injectable formulation consists essentially of *trans-*capsaicin at a concentration of about 0.1 mg/mL, water, and a polyethylene glycol having a number-average molecular weight of 300 g/mol, wherein upon a volume basis there is 4-5 times more water than polyethylene glycol.

### IV. POLYETHYLENE GLYCOL / WATER INJECTABLE FORMULATIONS

In certain embodiments, the uses described herein may administer the capsaicin in the form of a pharmaceutical composition. Such pharmaceutical composition may, in certain embodiments, further comprise water and a poly(ethylene glycol). In certain embodiments, the pharmaceutical composition comprising capsaicin consists essentially of water, capsaicin, and a poly(ethylene glycol). In certain embodiments, the poly(ethylene glycol) has a number-average molecular weight of about 300 g/mol. In certain embodiments, the poly(ethylene glycol) is present in an amount of about 30% by weight of the pharmaceutical formulation.

In certain embodiments, the pharmaceutical composition utilized to deliver capsaicin may comprise about 20% by weight PEG 300, about 10 mM histidine and about 5% sucrose in water for injection.

In certain other embodiments, the pharmaceutical composition utilized to deliver the capsaicin may comprise about 30-50% PEG 300. This may be used as such or further diluted in water for injection to achieve a larger volume.

### V. POLYETHYLENE GLYCOL ESTER / WATER INJECTABLE FORMULATIONS

Capsaisin aqueous injectable formulations containing a polyethylene glycol ester can be used in the uses described herein. A benefit of such capsaicin aqueous injectable formulations containing a polyethylene glycol ester is that they are stable to storage and can be administered directly to a patient via injection. A solubilizing agent containing a polyethylene glycol ester of a long-chain hydroxyalkanoic acid or a polyethylene glycol ester of a long-chain hydroxyalkenoic acid (such as a mixture containing a polyethylene glycol ester of 12-hydroxystearic acid, a polyethylene glycol ester of 12-((12-hydroxyoctadecanoyl)oxy)octadecanoic acid, and polyethylene glycol sold by BASF under the trade name KOLLIPHOR^{®} HS 15) was determined to be able to solubilize greater amounts of capsaicin than other tested solubilizing agents in the aqueous medium at the desired pH range, and yet produced a formulation suitable for injection to a patient and that is sufficiently stable to storage that it may be used in the typical distribution routes for pharmaceutical agents. The above noted solubilizing agent is also superiorly compatible with capsaicin, which improves the stability of the formulation to storage. By contrast, polysorbates, such as Polysorbate 80, can have a greater propensity to form peroxides. Such peroxides can cause undesired oxidation of capsaicin, resulting in loss of capsaicin during storage of the formulation and increase in the amount and identity of impurities. The solubilizing agent specified above overcomes this deficiency of polysorbate. Additionally, the solubilizing agent noted above overcomes the adverse side effect of polysorbates, such as Polysorbate 80, of triggering release of histamine when administered to a patient.

Accordingly, one exemplary aqueous, capsaicin injectable formulation for use in the uses described herein comprises:
a. about 0.03% (w/w) to about 0.3% (w/w) of capsaicin;
b. about 0.1% (w/w) to about 3% (w/w) of a solubilizing agent, wherein the solubilizing agent comprises (i) a polyethylene glycol ester of a (C₁₅-C₂₅) hydroxyalkanoic acid, (ii) a polyethylene glycol ester of a (C₁₅-C₂₅) hydroxyalkenoic acid, or (iii) a polyethylene glycol ester of a (C₁₅-C₂₅) alkanoic acid substituted by a -OC(O)(C₁₄-C₂₄) hydroxyalkyl group;
c. about 0.001% (w/w) to about 2% (w/w) of an antioxidant; and
d. at least 92% (w/w) water.

Another exemplary aqueous, capsaicin injectable formulation for use in the uses described herein comprises:
a. about 0.01% (w/w) to about 0.5% (w/w) of capsaicin;
b. about 0.01% (w/w) to about 5% (w/w) of a solubilizing agent, wherein the solubilizing agent comprises (i) a polyethylene glycol ester of a (C₁₅-C₂₅) hydroxyalkanoic acid, (ii) a polyethylene glycol ester of a (C₁₅-C₂₅) hydroxyalkenoic acid, or (iii) a polyethylene glycol ester of a (C₁₅-C₂₅) alkanoic acid substituted by a -OC(O)(C₁₄-C₂₄) hydroxyalkyl group; and
c. water.

Further description of exemplary components and features of the aqueous injectable formulations are described in more detail below.

### Amount of Solubilizing Agent

The formulation can be further characterized according to the amount of solubilizing agent in the formulation. For example, in certain embodiments, the formulation comprises about 0.5% (w/w) to about 1.5% (w/w) of the solubilizing agent. In certain other embodiments, the formulation comprises about 0.8% (w/w) to about 1.2% (w/w) of the solubilizing agent. In certain other embodiments, the formulation comprises about 1% (w/w) of the solubilizing agent. In certain other embodiments, the formulation comprises about 1.5% (w/w) to about 2.5% (w/w) of the solubilizing agent. In certain other embodiments, the formulation comprises about 2% (w/w) of the solubilizing agent.

### Identity of Solubilizing Agent

The formulation can be further characterized according to the identity of the solubilizing agent in the formulation. For example, in certain embodiments, the solubilizing agent comprises (i) a polyethylene glycol ester of a (C₁₅-C₂₅) hydroxyalkanoic acid, or (ii) a polyethylene glycol ester of a (C₁₅-C₂₅) hydroxyalkenoic acid. In certain embodiments, the solubilizing agent comprises a (C₁₄-C₂₄)hydroxyalkyl-CO₂-(polyethylene glycolyl)-H and (C₁₄-C₂₄)hydroxyalkyl-CO₂-(C₁₄-C₂₄)alkylene-CO₂-(polyethylene glycolyl)-H. In certain embodiments, the solubilizing agent comprises a (C₁₄-C₂₄)hydroxyalkyl-CO₂-(polyethylene glycolyl)-H, (C₁₄-C₂₄)hydroxyalkyl-CO₂-(C₁₄-C₂₄)alkylene-CO₂-(polyethylene glycolyl)-H, and polyethylene glycol. In certain embodiments, the solubilizing agent comprises (a) from about 60% (w/w) to about 80% (w/w) of a mixture of (C₁₄-C₂₄)hydroxyalkyl-CO₂-(polyethylene glycolyl)-H and (C₁₄-C₂₄)hydroxyalkyl-CO₂-(C₁₄-C₂₄)alkylene-CO₂-(polyethylene glycolyl)-H, and (b) from about 20% (w/w) to about 40% (w/w) polyethylene glycol. In certain embodiments, the solubilizing agent comprises (a) about 70% (w/w) of a mixture of (C₁₄-C₂₄)hydroxyalkyl-CO₂-(polyethylene glycolyl)-H and (C₁₄-C₂₄)hydroxyalkyl-CO₂-(C₁₄-C₂₄)alkylene-CO₂-(polyethylene glycolyl)-H, and (b) about 30% (w/w) polyethylene glycol. In certain embodiments, the solubilizing agent is a mixture of (C₁₄-C₂₄)hydroxyalkyl-CO₂-(polyethylene glycolyl)-H, (C₁₄-C₂₄)hydroxyalkyl-CO₂-(C₁₄-C₂₄)alkylene-CO₂-(polyethylene glycolyl)-H, and polyethylene glycol. In certain embodiments, the solubilizing agent is a mixture of (a) from about 60% (w/w) to about 80% (w/w) of a mixture of (C₁₄-C₂₄)hydroxyalkyl-CO₂-(polyethylene glycolyl)-H and (C₁₄-C₂₄)hydroxyalkyl-CO₂-(C₁₄-C₂₄)alkylene-CO₂-(polyethylene glycolyl)-H, and (b) from about 20% (w/w) to about 40% (w/w) polyethylene glycol. In certain embodiments, the solubilizing agent is a mixture of (a) about 70% (w/w) of a mixture of (C₁₄-C₂₄)hydroxyalkyl-CO₂-(polyethylene glycolyl)-H and (C₁₄-C₂₄)hydroxyalkyl-CO₂-(C₁₄-C₂₄)alkylene-CO₂-(polyethylene glycolyl)-H, and (b) about 30% (w/w) polyethylene glycol.

In a more specific embodiment, the solubilizing agent comprises a (C₁₇)hydroxyalkyl-CO₂-(polyethylene glycolyl)-H and (C₁₇)hydroxyalkyl-CO₂-(C₁₇)alkylene-CO₂-(polyethylene glycolyl)-H. In certain embodiments, the solubilizing agent comprises a (C₁₇)hydroxyalkyl-CO₂-(polyethylene glycolyl)-H, (C₁₇)hydroxyalkyl-CO₂-(C₁₇)alkylene-CO₂-(polyethylene glycolyl)-H, and polyethylene glycol. In certain embodiments, the solubilizing agent comprises (a) from about 60% (w/w) to about 80% (w/w) of a mixture of (C₁₇)hydroxyalkyl-CO₂-(polyethylene glycolyl)-H and (C₁₇)hydroxyalkyl-CO₂-(C₁₇)alkylene-CO₂-(polyethylene glycolyl)-H, and (b) from about 20% (w/w) to about 40% (w/w) polyethylene glycol. In certain embodiments, the solubilizing agent comprises (a) about 70% (w/w) of a mixture of (C₁₇)hydroxyalkyl-CO₂-(polyethylene glycolyl)-H and (C₁₇)hydroxyalkyl-CO₂-(C₁₇)alkylene-CO₂-(polyethylene glycolyl)-H, and (b) about 30% (w/w) polyethylene glycol. In certain embodiments, the solubilizing agent is a mixture of (a) from about 60% (w/w) to about 80% (w/w) of a mixture of (C₁₇)hydroxyalkyl-CO₂-(polyethylene glycolyl)-H and (C₁₇)hydroxyalkyl-CO₂-(C₁₇)alkylene-CO₂-(polyethylene glycolyl)-H, and (b) from about 20% (w/w) to about 40% (w/w) polyethylene glycol. In certain embodiments, the solubilizing agent is a mixture of (a) about 70% (w/w) of a mixture of (C₁₇)hydroxyalkyl-CO₂-(polyethylene glycolyl)-H and (C₁₇)hydroxyalkyl-CO₂-(C₁₇)alkylene-CO₂-(polyethylene glycolyl)-H, and (b) about 30% (w/w) polyethylene glycol.

In certain embodiments, the mole ratio of (a) (C₁₄-C₂₄)hydroxyalkyl-CO₂-(polyethylene glycolyl)-H to (b) (C₁₄-C₂₄)hydroxyalkyl-CO₂-(C₁₄-C₂₄)alkylene-CO₂-(polyethylene glycolyl)-H in the formulation is in the range of 10:1 to 1:10, 5:1 to 1:5, 2:1 to 1:2, 10:1 to 5:1, 5:1 to 2:1, 2:1 to 1:1, 1:1 to 1:2, 1:2 to 1:5, 1:5 to 1:10, or is greater than 10:1, or less than 1:1. In certain embodiments, the mole ratio of (a) (C₁₇)hydroxyalkyl-CO₂-(polyethylene glycolyl)-H to (b) (C₁₇)hydroxyalkyl-CO₂-(C₁₇)alkylene-CO₂-(polyethylene glycolyl)-H in the formulation is in the range of 10:1 to 1:10, 5:1 to 1:5, 2:1 to 1:2, 10:1 to 5:1, 5:1 to 2:1, 2:1 to 1:1, 1:1 to 1:2, 1:2 to 1:5, 1:5 to 1:10, or is greater than 10:1, or less than 1:1.

In a more specific embodiment, the solubilizing agent comprises 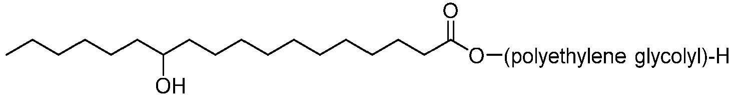 and 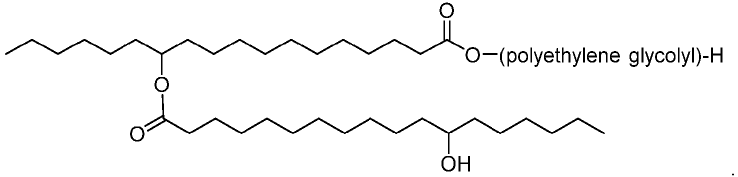 In another more specific embodiment, the solubilizing agent is a mixture of 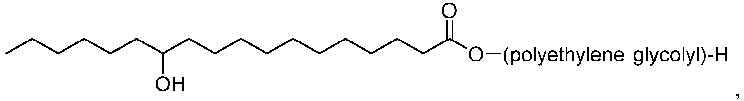 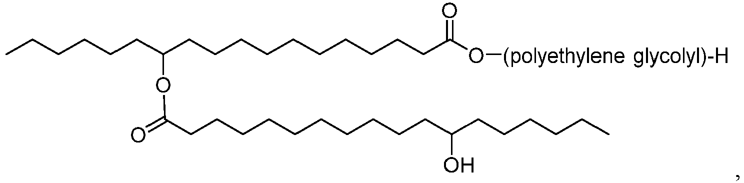 and polyethylene glycol. In certain other embodiments, the solubilizing agent comprises (a) about 70% (w/w) of a mixture of 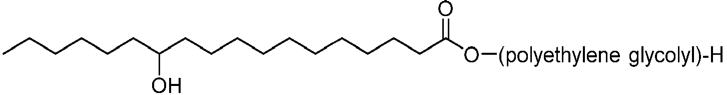 and 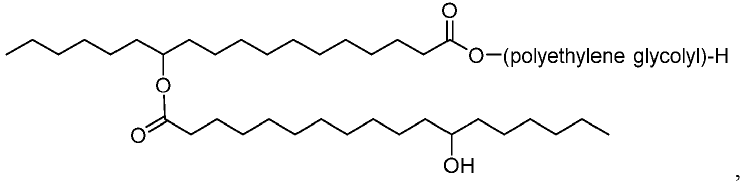 and (b) about 30% (w/w) polyethylene glycol. In certain other embodiments, the solubilizing agent is a mixture of (a) about 70% (w/w) of a mixture of 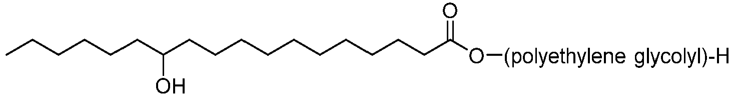 and 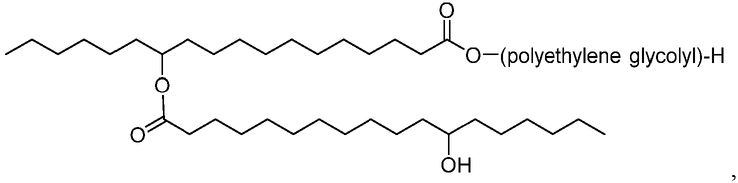 and (b) about 30% (w/w) polyethylene glycol. In certain other embodiments, the solubilizing agent comprises (a) from 68% (w/w) to 72% (w/w) of a mixture of 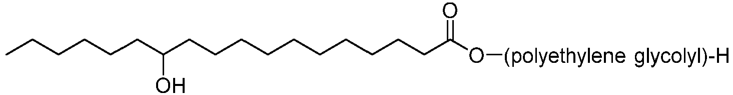 and 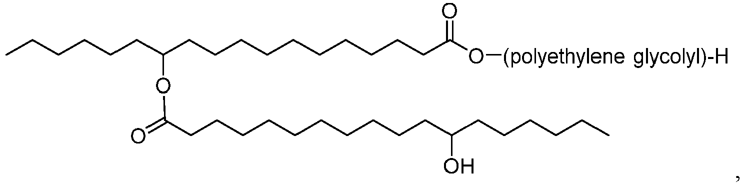 , and (b) from 28% (w/w) to 32% (w/w) polyethylene glycol.

The above solubilizing agent can be further characterized according to the weight-average molecular weight of any polyethylene glycolyl component. For example, in certain embodiments, the polyethylene glycolyl has a weight-average molecular weight in the range of about 100 g/mol to about 3000 g/mol. In certain embodiments, the polyethylene glycolyl has a weight-average molecular weight in the range of about 200 g/mol to about 1500 g/mol. In certain embodiments, the polyethylene glycol has a weight-average molecular weight in the range of about 200 g/mol to about 1000 g/mol. In certain embodiments, the polyethylene glycolyl has a weight-average molecular weight in the range of about 300 g/mol to about 900 g/mol. In certain embodiments, the polyethylene glycolyl has a weight-average molecular weight in the range of about 500 g/mol to about 800 g/mol. In certain embodiments, the polyethylene glycolyl has a weight-average molecular weight in the range of about 600 g/mol to about 750 g/mol. In certain embodiments, the polyethylene glycolyl has a weight-average molecular weight in the range of about 600 g/mol to about 700 g/mol. In certain embodiments, the polyethylene glycolyl has a weight-average molecular weight of about 660 g/mol. In certain embodiments, the polyethylene glycolyl has a weight-average molecular weight in the range of about 100 g/mol to about 300 g/mol, about 300 g/mol to about 500 g/mol, about 500 g/mol to about 1000 g/mol, about 1000 g/mol to about 1500 g/mol, about 1500 g/mol to about 2000 g/mol, or about 2000 g/mol to about 2500 g/mol.

The above solubilizing agent can be further characterized according to the weight-average molecular weight of any polyethylene glycol component. For example, in certain embodiments, the polyethylene glycol has a weight-average molecular weight in the range of about 100 g/mol to about 3000 g/mol. In certain embodiments, the polyethylene glycol has a weight-average molecular weight in the range of about 200 g/mol to about 1500 g/mol. In certain embodiments, the polyethylene glycol has a weight-average molecular weight in the range of about 200 g/mol to about 1000 g/mol. In certain embodiments, the polyethylene glycol has a weight-average molecular weight in the range of about 300 g/mol to about 900 g/mol. In certain embodiments, the polyethylene glycol has a weight-average molecular weight in the range of about 500 g/mol to about 800 g/mol. In certain embodiments, the polyethylene glycol has a weight-average molecular weight in the range of about 600 g/mol to about 750 g/mol. In certain embodiments, the polyethylene glycol has a weight-average molecular weight in the range of about 600 g/mol to about 700 g/mol. In certain embodiments, the polyethylene glycol has a weight-average molecular weight of about 660 g/mol. In certain embodiments, the polyethylene glycol has a weight-average molecular weight in the range of about 100 g/mol to about 300 g/mol, about 300 g/mol to about 500 g/mol, about 500 g/mol to about 1000 g/mol, about 1000 g/mol to about 1500 g/mol, about 1500 g/mol to about 2000 g/mol, or about 2000 g/mol to about 2500 g/mol.

In yet other embodiments, any polyethylene glycol or polyethylene glycolyl each independently have a weight-average molecular weight in the range of about 100 g/mol to about 3000 g/mol. In certain embodiments, any polyethylene glycol or polyethylene glycolyl each independently have a weight-average molecular weight in the range of about 200 g/mol to about 1500 g/mol. In certain embodiments, any polyethylene glycol or polyethylene glycolyl each independently have a weight-average molecular weight in the range of about 200 g/mol to about 1000 g/mol. In certain embodiments, any polyethylene glycol or polyethylene glycolyl each independently have a weight-average molecular weight in the range of about 300 g/mol to about 900 g/mol. In certain embodiments, any polyethylene glycol or polyethylene glycolyl each independently have a weight-average molecular weight in the range of about 500 g/mol to about 800 g/mol. In certain embodiments, any polyethylene glycol or polyethylene glycolyl each independently have a weight-average molecular weight in the range of about 600 g/mol to about 750 g/mol. In certain embodiments, any polyethylene glycol or polyethylene glycolyl each independently have a weight-average molecular weight in the range of about 600 g/mol to about 700 g/mol. In certain embodiments, any polyethylene glycol or polyethylene glycolyl each independently have a weight-average molecular weight of about 660 g/mol. In certain embodiments, any polyethylene glycol or polyethylene glycolyl each independently have a weight-average molecular weight in the range of about 100 g/mol to about 300 g/mol, about 300 g/mol to about 500 g/mol, about 500 g/mol to about 1000 g/mol, about 1000 g/mol to about 1500 g/mol, about 1500 g/mol to about 2000 g/mol, or about 2000 g/mol to about 2500 g/mol.

### Antioxidant

The formulation can be further characterized according to the antioxidant in the formulation. For example, in certain embodiments, the formulation comprises about 0.005% (w/w) to about 0.1% (w/w) of an antioxidant. In certain embodiments, the formulation comprises about 0.01% (w/w) of an antioxidant. In certain embodiments, the antioxidant is an organic compound. In certain embodiments, the antioxidant is a substituted phenol. In certain embodiments, the antioxidant is a phenolic antioxidant. In certain embodiments, the antioxidant is dibutylhydroxytoluene.

### Chelating Agent

The formulation may optionally further comprise a chelating agent. Accordingly, in certain embodiments, the formulation further comprises a chelating agent. In certain embodiments, the formulation comprises about 0.001% (w/w) to about 0.5% (w/w) of a chelating agent. In certain embodiments, the formulation comprises about 0.01% (w/w) to about 0.05% (w/w) of a chelating agent. In certain embodiments, the formulation comprises about 0.025% (w/w) of a chelating agent.

Exemplary chelating agents include, but are not limited to, ethylenediaminetetraacetic acid (EDTA), citric acid, sorbitol, tartaric acid, phosphoric acid, salts of the foregoing, and the like. In certain embodiments, the chelating agent is an aliphatic amine compound containing at least two carboxylic acid groups. In certain embodiments, the chelating agent is ethylenediaminetetraacetic acid or a salt thereof.

In certain embodiments, the chelating agent is a metal ion chelating agent.

In certain embodiments, the combination of an antioxidant and a chelating agent (e.g., ethylenediaminetetraacetic acid or salt thereof) can increase the stability of an aqueous capsaicin formulation.

### Buffer

The formulation may optionally further comprise a buffer. The buffer helps reduce changes in pH of the formulation over time and may provide improved drug stability. Exemplary buffers include, but are not limited to, sodium bicarbonate, sodium citrate, citric acid, sodium phosphate, pharmaceutically acceptable salts thereof, and combinations thereof. In certain embodiments, the buffer is an acetate salt, phosphate salt, citrate salt; corresponding acids of the foregoing; and combinations or mixtures thereof.

Accordingly, in certain embodiments, the formulation further comprises a buffer. In certain embodiments, the buffer comprises a carboxylic acid compound having a molecular weight less than 500 g/mol, a salt thereof, or a mixture thereof. In certain embodiments, the buffer comprises a C₁-C₆ alkanoic acid, a salt thereof, or a mixture thereof. In certain embodiments, the buffer comprises acetic acid, a salt of acetic acid, or a mixture thereof.

### Osmolality

The formulation may be further characterized according to the osmolality of the formulation. Formulations having an osmolality at or near the osmolality of a typical bodily fluid are referred to as isotonic. Formulations having an osmolality greater than the osmolality of a typical bodily fluid are referred to as hypertonic. Formulations having an osmolality less than the osmolality of a typical bodily fluid are referred to as hypotonic.

The osmolality of the formulation may be optionally adjusted by including a tonicity modifier. Accordingly, in certain embodiments, the formulation further comprises a tonicity modifier. In certain embodiments, the formulation comprises about 0.01% (w/w) to about 5% (w/w) of a tonicity modifier. In certain embodiments, the formulation comprises about 0.1% (w/w) to about 2% (w/w) of a tonicity modifier. In certain embodiments, the formulation comprises about 0.3% (w/w) to about 0.9% (w/w) of a tonicity modifier.

In certain embodiments, the tonicity modifier is an alkali metal salt. In certain embodiments, the tonicity modifier is sodium chloride. In certain embodiments, the tonicity modifier is a monosaccharide. In certain embodiments, the tonicity modifier is dextrose.

Formulations may be characterized according to an osmolality threshold or range. For example, in certain embodiments, the formulation may have an osmolality of at least 200 mOsm/kg, 220 mOsm/kg, 240 mOsm/kg, 260 mOsm/kg, 280 mOsm/kg, 300 mOsm/kg, 325 mOsm/kg, 350 mOsm/kg, 375 mOsm/kg, 400 mOsm/kg, 425 mOsm/kg, 450 mOsm/kg, 500 mOsm/kg, 600 mOsm/kg, 700 mOsm/kg, 800 mOsm/kg, 900 mOsm/kg, or 1000 mOsm/kg. In certain embodiments, the formulation has osmolality of at least 240 mOsm/kg. In certain embodiments, the formulation has osmolality of at least 270 mOsm/kg.

In certain embodiments, the formulation has an osmolality in the range of from about 200 mOsm/kg to about 400 mOsm/kg, from about 240 mOsm/kg to about 350 mOsm/kg, from about 240 mOsm/kg to about 340 mOsm/kg, from about 270 mOsm/kg to about 340 mOsm/kg, from about 270 mOsm/kg to about 330 mOsm/kg, from about 270 mOsm/kg to about 310 mOsm/kg, from about 290 mOsm/kg to about 330 mOsm/kg, from about 280 mOsm/kg to about 300 mOsm/kg, or from about 300 mOsm/kg to about 320 mOsm/kg. In certain embodiments, the formulation has an osmolality in the range of from about 240 mOsm/kg to about 340 mOsm/kg. In certain other embodiments, the formulation has an osmolality in the range from about 270 mOsm/kg to about 330 mOsm/kg.

In certain embodiments, the formulation has osmolality of about 200 mOsm/kg, about 220 mOsm/kg, about 240 mOsm/kg, about 250 mOsm/kg, about 260 mOsm/kg, about 270 mOsm/kg, about 280 mOsm/kg, about 290 mOsm/kg, about 300 mOsm/kg, about 310 mOsm/kg, about 320 mOsm/kg, about 330 mOsm/kg, about 340 mOsm/kg, about 350 mOsm/kg, about 360 mOsm/kg, about 370 mOsm/kg, or about 380 mOsm/kg. In a preferred embodiment, the formulation has osmolality of about 290 mOsm/kg. In another preferred embodiment, the formulation has osmolality of about 310 mOsm/kg.

### Amount of Water

The formulation may be further characterized according to the amount of water in the formulation. For example, in certain embodiments, the formulation comprises at least 95% (w/w) water. In certain embodiments, the formulation comprises at least 97% (w/w) water. In certain embodiments, the formulation comprises from about 95% (w/w) to about 99% (w/w) water. In certain embodiments, the formulation comprises from about 97% (w/w) to about 98% (w/w) water. In certain embodiments, the formulation comprises from about 93% (w/w) to about 96% (w/w) water.

### pH of the Formulation

The formulation may be further characterized according to the pH of the formulation. For example, in certain embodiments, the formulation has a pH in the range of about 4 to about 7. In certain embodiments, the formulation has a pH in the range of about 5 to about 6. In certain embodiments, the formulation has a pH of about 5.1, 5.2, 5.3, 5.4, 5.5, 5.6, 5.7, 5.8., or 5.9. In certain embodiments, the formulation has a pH of about 5.5.

### Capsaicin

Capsaicin has the chemical name *N*-[(4-hydroxy-3-methoxyphenyl)methyl]-8-methylnon-6-enamide, and due to the presence of a carbon-carbon double bond can exist as a mixture of *cis* and *trans* isomers. The formulations may be characterized according to the isomeric purity of the capsaicin administered to the patient. For example, in certain embodiments, the capsaicin is a mixture of *cis*-capsaicin and *trans*-capsaicin that contains at least 95% by weight trans-capsaicin. In certain embodiments, the capsaicin is a mixture of *cis-*capsaicin and *trans*-capsaicin that contains at least 97% by weight *trans*-capsaicin. In certain embodiments, the capsaicin is a mixture of *cis*-capsaicin and *trans*-capsaicin that contains at least 98% by weight *trans*-capsaicin. In certain embodiments, the capsaicin is a mixture of *cis-*capsaicin and *trans*-capsaicin that contains at least 99% by weight *trans*-capsaicin. In certain other embodiments, the capsaicin is a mixture of *cis*-capsaicin and *trans*-capsaicin that contains at least 95% by weight *cis*-capsaicin.

The isomeric purity of capsaicin may also be expressed according to the molar ratio of *trans vs. cis* isomer. Accordingly, in certain embodiments, the capsaicin is present as a mixture of *trans* and *cis* isomers, wherein the ratio of *trans:cis* isomers is at least 10:1. In certain embodiments, the ratio of *trans:cis* isomers is at least 15:1. In certain embodiments, the capsaicin consists essentially of the *trans* isomer.

The formulation may be further characterized according to the amount of capsaicin in the formulation. For example, in certain embodiments, the formulation comprises from about 0.03% (w/w) to about 0.15% (w/w) of capsaicin. In certain embodiments, the formulation comprises from about 0.03% (w/w) to about 0.07% (w/w) of capsaicin. In certain embodiments, the formulation comprises from about 0.01% (w/w) to about 0.03% (w/w) of capsaicin, 0.03% (w/w) to about 0.05% (w/w) of capsaicin, 0.05% (w/w) to about 0.07% (w/w) of capsaicin, 0.07% (w/w) to about 0.09% (w/w) of capsaicin, 0.09% (w/w) to about 0.11% (w/w) of capsaicin, or 0.11% (w/w) to about 0.13% (w/w) of capsaicin. In certain embodiments, the formulation comprises about 0.05% (w/w) of capsaicin. In certain embodiments, the formulation comprises from about 0.08% (w/w) to about 0.12% (w/w) of capsaicin. In certain embodiments, the formulation comprises from about 0.12% (w/w) to about 0.15% (w/w) of capsaicin, from about 0.15% (w/w) to about 0.18% (w/w) of capsaicin, from about 0.18% (w/w) to about 0.21% (w/w) of capsaicin, from about 0.21% (w/w) to about 0.25% (w/w) of capsaicin, or from about 0.25% (w/w) to about 0.3% (w/w) of capsaicin. In certain embodiments, the formulation comprises about 0.1% (w/w) of capsaicin.

### Additional Pain-Relief Agent

The formulation may optionally contain a further pain-relief agent. For example, in certain embodiments, the formulation may further comprise a caine alkaloid. Exemplary caine alkaloids include lidocaine, dibucaine, bupivacaine, ropivacaine, etidocaine, tetracaine, procaine, chlorocaine, prilocaine, mepivacaine, xylocaine, 2-chloroprocaine, and pharmaceutically acceptable salts thereof. In certain embodiments, the formulation further comprises lidocaine, such as where the lidocaine is present in an amount of about 0.5% (w/w), 1.0% (w/w), 2.0% (w/w), 3.0% (w/w) or 4.0% (w/w) of the formulation, or in an amount ranging from about 0.5% (w/w) to about 2.0% (w/w), or about 2.0% (w/w) to about 4.0% (w/w) of the formulation.

### Exemplary Formulations

In certain embodiments, the formulation is one of the formulations in Table 1 below.

**TABLE 1.**

| **No.** | **Formulation** | |
|---|---|---|
| 1 | An aqueous, capsaicin injectable formulation, comprising: | |
| | | a. about 0.03% (w/w) to about 0.3% (w/w) of capsaicin; |
| | | b. about 0.1% (w/w) to about 3% (w/w) of a solubilizing agent, wherein the solubilizing agent comprises (i) a polyethylene glycol ester of a (C₁₅-C₂₅) hydroxyalkanoic acid, (ii) a polyethylene glycol ester of a (C₁₅-C₂₅) hydroxyalkenoic acid, or (iii) a polyethylene glycol ester of a (C₁₅-C₂₅) alkanoic acid substituted by a -OC(O)(C₁₄-C₂₄) hydroxyalkyl group; |
| | | c. about 0.001% (w/w) to about 2% (w/w) of an antioxidant; and |
| | | d. at least 92% (w/w) water; and |
| | | having a pH in the range of about 3 to about 8. |
| 2 | An aqueous, capsaicin injectable formulation, comprising: | |
| | | a. about 0.04% (w/w) to about 0.06% (w/w) of capsaicin; |
| | | b. about 0.7% (w/w) to about 1.3% (w/w) of a solubilizing agent, wherein the solubilizing agent comprises (i) a polyethylene glycol ester of a (C₁₅-C₂₅) hydroxyalkanoic acid, or (ii) a polyethylene glycol ester of a (C₁₅-C₂₅) hydroxyalkenoic acid; |
| | | c. about 0.001% (w/w) to about 0.1% (w/w) of an antioxidant; and |
| | | d. at least 92% (w/w) water; and |
| | having a pH in the range of about 4 to about 7. | |
| 3 | An aqueous, capsaicin injectable formulation, comprising: | |
| | | a. about 0.04% (w/w) to about 0.06% (w/w) of trans-capsaicin; |
| | | b. about 0.7% (w/w) to about 1.3% (w/w) of a solubilizing agent, wherein the solubilizing agent comprises (a) from about 60% (w/w) to about 80% (w/w) of a mixture of (C₁₇)hydroxyalkyl-CO₂-(polyethylene glycolyl)-H and (C₁₇)hydroxyalkyl-CO₂-(C₁₇)alkylene-CO₂-(polyethylene glycolyl)-H, and (b) from about 20% (w/w) to about 40% (w/w) polyethylene glycol; |
| | | c. about 0.001% (w/w) to about 0.1% (w/w) of an antioxidant; and |
| | | d. at least 95% (w/w) water; and |
| | having a pH in the range of about 4 to about 7. | |
| 4 | An aqueous, capsaicin injectable formulation, comprising: | |
| | | a. about 0.08% (w/w) to about 0.12% (w/w) of capsaicin; |
| | | b. about 1.8% (w/w) to about 2.2% (w/w) of a solubilizing agent, wherein the solubilizing agent comprises (i) a polyethylene glycol ester of a (C₁₅-C₂₅) hydroxyalkanoic acid, or (ii) a polyethylene glycol ester of a (C₁₅-C₂₅) hydroxyalkenoic acid; |
| | | c. about 0.001% (w/w) to about 0.1% (w/w) of an antioxidant; and |
| | | d. at least 93% (w/w) water; and |
| | having a pH in the range of about 4 to about 7. | |
| 5 | An aqueous, capsaicin injectable formulation, comprising: | |
| | | a. about 0.08% (w/w) to about 0.12% (w/w) of capsaicin; |
| | | b. about 1.8% (w/w) to about 2.2% (w/w) of a solubilizing agent, wherein the solubilizing agent comprises (a) from about 60% (w/w) to about 80% (w/w) of a mixture of (C₁₇)hydroxyalkyl-CO₂-(polyethylene glycolyl)-H and (C₁₇)hydroxyalkyl-CO₂-(C₁₇)alkylene-CO₂-(polyethylene glycolyl)-H, and (b) from about 20% (w/w) to about 40% (w/w) polyethylene glycol; |
| | | c. about 0.001% (w/w) to about 0.1% (w/w) of an antioxidant; and |
| | | d. at least 93% (w/w) water; and |
| | having a pH in the range of about 4 to about 7. | |

Exemplary more specific formulations are provided in Tables 2 and 3 below.

**TABLE 2.**

| **No.** | **Formulation** | | |
|---|---|---|---|
| 1 | An aqueous, capsaicin injectable formulation, comprising: | | |
| | | a. about 0.04% (w/w) to about 0.06% (w/w) of capsaicin; | |
| | | b. about 0.5% (w/w) to about 1.5% (w/w) of a solubilizing agent, wherein the solubilizing agent comprises | |
| | | 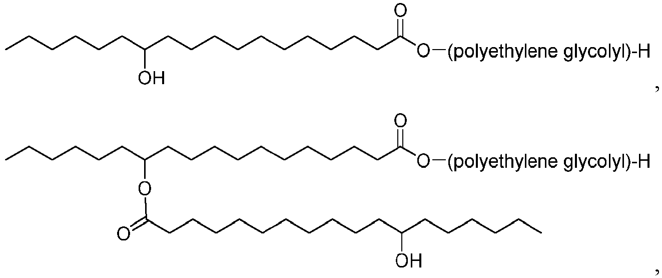 and polyethylene glycol; | |
| | | c. about 0.005% (w/w) to about 0.015% (w/w) of an antioxidant; | |
| | | d. about 0.3% (w/w) to about 1% (w/w) of an alkali metal acetate; | |
| | | e. about 0.01% (w/w) to about 0.05% (w/w) of a chelating agent; | |
| | | f. about 0.3% (w/w) to about 0.9% (w/w) of a tonicity modifier; | |
| | | g. at least 95% (w/w) water; and | |
| | having a pH in the range of about 5 to about 6. | | |
| 2 | An aqueous, capsaicin injectable formulation, comprising: | | |
| | | a. about 0.04% (w/w) to about 0.06% (w/w) of capsaicin; | |
| | | b. about 0.8% (w/w) to about 1.2% (w/w) of a solubilizing agent, wherein the solubilizing agent comprises | |
| | | | 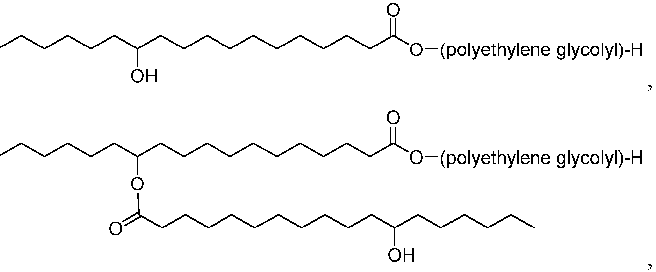 and polyethylene glycol; |
| | | c. about 0.005% (w/w) to about 0.015% (w/w) of dibutylhydroxytoluene; | |
| | | d. about 0.3% (w/w) to about 1% (w/w) of sodium acetate; | |
| | | e. about 0.01% (w/w) to about 0.05% (w/w) of ethylenediaminetetraacetic acid or a salt thereof; | |
| | | f. about 0.3% (w/w) to about 0.9% (w/w) of sodium chloride; | |
| | | g. at least 95% (w/w) water; and | |
| | having a pH in the range of about 5 to about 6. | | |
| 3 | An aqueous, capsaicin injectable formulation, comprising: | | |
| | | a. about 0.05% (w/w) of *trans*-capsaicin; | |
| | | b. about 1% (w/w) of a solubilizing agent, wherein the solubilizing agent comprises | |
| | | | 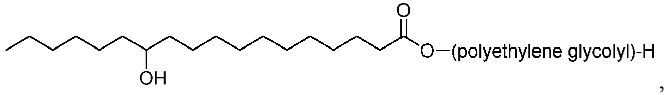 |
| | | | 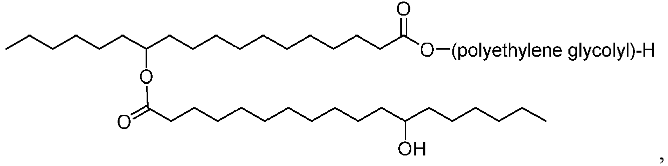 and polyethylene glycol; |
| | | c. about 0.01% (w/w) dibutylhydroxytoluene; | |
| | | d. about 0.5% (w/w) to about 0.8% (w/w) of sodium acetate; | |
| | | e. about 0.01% (w/w) to about 0.05% (w/w) of ethylenediaminetetraacetic acid or a salt thereof; | |
| | | f. about 0.3% (w/w) to about 0.9% (w/w) of sodium chloride; | |
| | | g. at least 95% (w/w) water; and | |
| | having a pH in the range of about 5 to about 6. | | |
| 4 | An aqueous, capsaicin injectable formulation, comprising: | | |
| | | a. about 0.08% (w/w) to about 0.12% (w/w) of capsaicin; | |
| | | b. about 1.5% (w/w) to about 2.5% (w/w) of a solubilizing agent, wherein the solubilizing agent comprises | |
| | | | 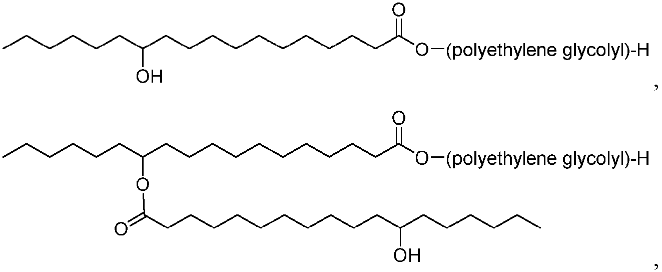 and polyethylene glycol; |
| | | c. about 0.005% (w/w) to about 0.015% (w/w) of an antioxidant; | |
| | | d. about 0.1% (w/w) to about 1% (w/w) of an alkali metal carboxylate compound; | |
| | | e. about 0.01% (w/w) to about 0.5% (w/w) of a chelating agent; | |
| | | f. about 2% (w/w) to about 4% (w/w) of a tonicity modifier; | |
| | | g. at least 93% (w/w) water; and | |
| | having a pH in the range of about 5 to about 6. | | |
| 5 | An aqueous, capsaicin injectable formulation, comprising: | | |
| | | a. about 0.08% (w/w) to about 0.12% (w/w) of capsaicin; | |
| | | b. about 1.8% (w/w) to about 2.2% (w/w) of a solubilizing agent, wherein the solubilizing agent comprises | |
| | | 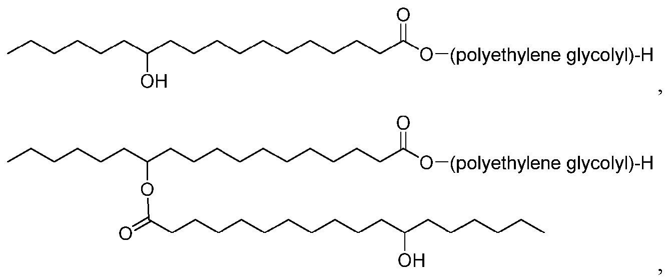 and polyethylene glycol; | |
| | | c. about 0.005% (w/w) to about 0.015% (w/w) of an antioxidant; | |
| | | d. about 0.1% (w/w) to about 1% (w/w) of an alkali metal carboxylate compound; | |
| | | e. about 0.01% (w/w) to about 0.5% (w/w) of a chelating agent; | |
| | | f. about 2% (w/w) to about 4% (w/w) of a tonicity modifier; | |
| | | g. at least 93% (w/w) water; and | |
| | having a pH in the range of about 5 to about 6. | | |
| 6 | An aqueous, capsaicin injectable formulation, comprising: | | |
| | | a. about 0.1% (w/w) of capsaicin; | |
| | | b. about 2% (w/w) of a solubilizing agent, wherein the solubilizing agent comprises | |
| | | 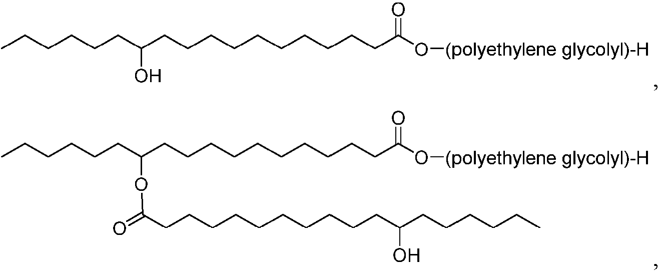 and polyethylene glycol; | |
| | | c. about 0.005% (w/w) to about 0.015% (w/w) of an antioxidant; | |
| | | d. about 0.1% (w/w) to about 1% (w/w) of an alkali metal carboxylate compound; | |
| | | e. about 0.01% (w/w) to about 0.5% (w/w) of a chelating agent; | |
| | | f. about 2.5% (w/w) to about 3.5% (w/w) of a tonicity modifier; | |
| | | g. at least 93% (w/w) water; and | |
| | having a pH in the range of about 5 to about 6. | | |

**TABLE 3.**

| **No.** | **Formulation** | |
|---|---|---|
| 1 | An aqueous, capsaicin injectable formulation, comprising: | |
| | | a. about 0.1% (w/w) of capsaicin; |
| | | b. about 2% (w/w) of a solubilizing agent, wherein the solubilizing agent comprises |
| | | 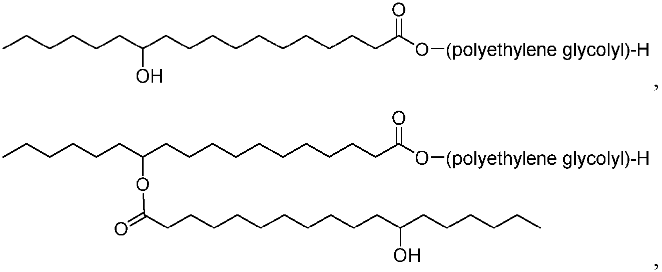 and polyethylene glycol; |
| | | c. about 0.01% (w/w) of an antioxidant; |
| | | d. about 0.1% (w/w) to about 1% (w/w) of an alkali metal citrate salt; |
| | | e. about 0.1% (w/w) of a chelating agent; |
| | | f. about 3% (w/w) of a tonicity modifier; and |
| | | g. at least 93% (w/w) water. |
| 2 | An aqueous, capsaicin injectable formulation, comprising: | |
| | | a. about 0.1% (w/w) of capsaicin; |
| | | b. about 2% (w/w) of a solubilizing agent, wherein the solubilizing agent |
| | | comprises |
| | | 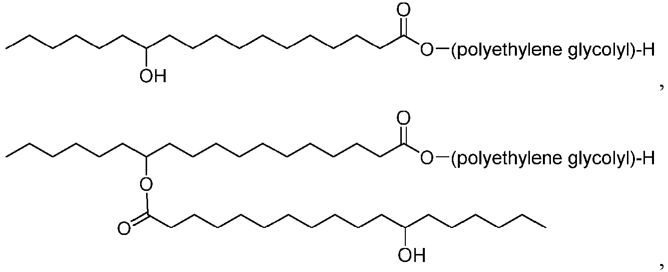 and polyethylene glycol; |
| | | c. about 0.01% (w/w) of dibutylhydroxytoluene; |
| | | d. about 0.1% (w/w) to about 1% (w/w) of a disodium citrate salt; |
| | | e. about 0.1% (w/w) of ethylenediaminetetraacetic acid or a salt thereof; |
| | | f. about 3% (w/w) of dextrose; |
| | | g. at least 93% (w/w) water; and |
| | | having a pH in the range of about 5 to about 6. |
| 3 | An aqueous, capsaicin injectable formulation, comprising: | |
| | | a. about 0.1% (w/w) of *trans*-capsaicin; |
| | | b. about 2% (w/w) of a solubilizing agent, wherein the solubilizing agent that comprises |
| | | 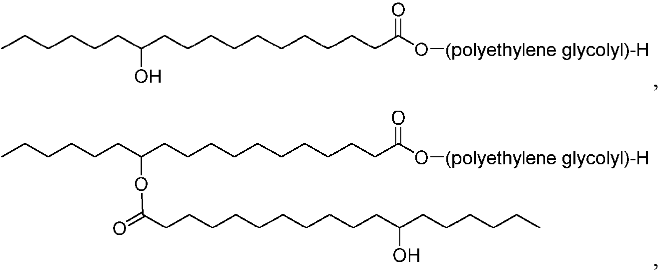 and polyethylene glycol; |
| | | c. about 0.01% (w/w) of dibutylhydroxytoluene; |
| | | d. about 0.1% (w/w) to about 1% (w/w) of a disodium citrate salt; |
| | | e. about 0.1% (w/w) of ethylenediaminetetraacetic acid or a salt thereof; |
| | | f. about 3% (w/w) of dextrose; |
| | | g. at least 93% (w/w) water; and |
| | | having a pH in the range of about 5 to about 6. |

In yet other embodiments, the aqueous, capsaicin injectable formulation comprises (a) about 0.04% (w/w) to about 0.06% (w/w) of capsaicin; (b) about 0.5% (w/w) to about 1.5% (w/w) of a solubilizing agent, wherein the solubilizing agent comprises 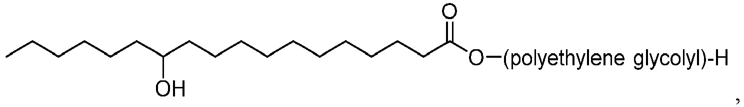 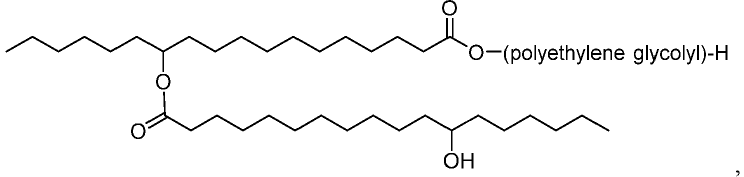 and polyethylene glycol; (c) about 0.005% (w/w) to about 0.015% (w/w) of an antioxidant; (d) about 0.2% (w/w) to about 1% (w/w) of an alkali metal acetate; (e) about 0.01% (w/w) to about 0.05% (w/w) of a chelating agent; (f) about 0.3% (w/w) to about 0.9% (w/w) of a tonicity modifier; and (g) at least 96% (w/w) water; and having a pH in the range of about 5 to about 6.

In other embodiments, the aqueous, capsaicin injectable formulation comprises (a) about 0.04% (w/w) to about 0.06% (w/w) of capsaicin; (b) about 0.8% (w/w) to about 1.2% (w/w) of a solubilizing agent, wherein the solubilizing agent comprises 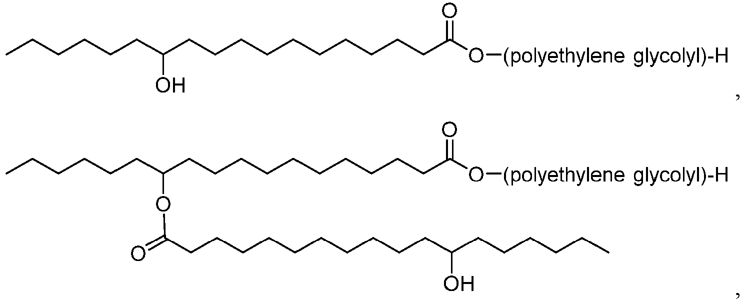 and polyethylene glycol; (c) about 0.005% (w/w) to about 0.015% (w/w) of dibutylhydroxytoluene; (d) about 0.2% (w/w) to about 1% (w/w) of sodium acetate; (e) about 0.01% (w/w) to about 0.05% (w/w) of ethylenediaminetetraacetic acid or a salt thereof; (f) about 0.3% (w/w) to about 0.9% (w/w) of sodium chloride; (g) at least 96% (w/w) water; and has a pH in the range of about 5 to about 6.

In other embodiments, the aqueous, capsaicin injectable formulation comprises
a. about 0.04% (w/w) to about 0.06% (w/w) of capsaicin;
b. about 0.8% (w/w) to about 1.2% (w/w) of a solubilizing agent, wherein the solubilizing agent comprises (a) about 70% (w/w) of a mixture of 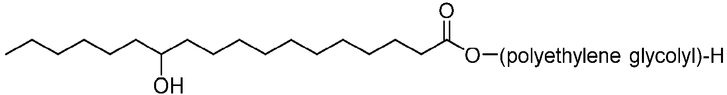 and and (b) 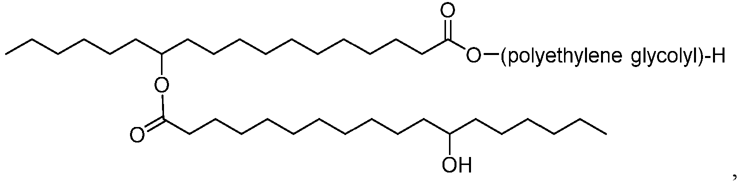 about 30% (w/w) polyethylene glycol;
c. about 0.005% (w/w) to about 0.015% (w/w) of dibutylhydroxytoluene;
d. about 0.2% (w/w) to about 1% (w/w) of buffer, which is a mixture of an alkali metal acetate and acetic acid;
e. about 0.01% (w/w) to about 0.05% (w/w) of ethylenediaminetetraacetic acid or a salt thereof;
f. about 0.3% (w/w) to about 0.9% (w/w) of sodium chloride;
g. at least 96% (w/w) water; and
   having a pH in the range of about 5 to about 6.

In other embodiments, the aqueous, capsaicin injectable formulation comprises
a. about 0.04% (w/w) to about 0.06% (w/w) of capsaicin;
b. about 0.8% (w/w) to about 1.2% (w/w) of a solubilizing agent, wherein the solubilizing agent comprises (a) about 70% (w/w) of a mixture of 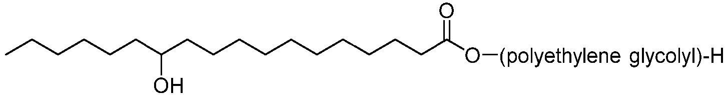 and 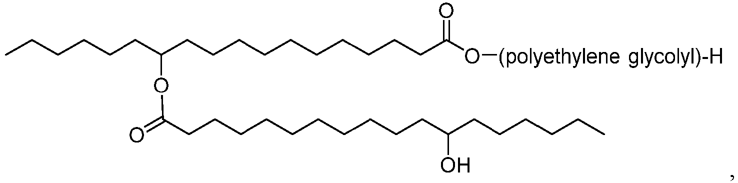 and (b) about 30% (w/w) polyethylene glycol;
c. about 0.005% (w/w) to about 0.015% (w/w) of dibutylhydroxytoluene;
d. about 0.2% (w/w) to about 1% (w/w) of sodium acetate;
e. about 0.01% (w/w) to about 0.05% (w/w) of ethylenediaminetetraacetic acid or a salt thereof;
f. about 0.3% (w/w) to about 0.9% (w/w) of sodium chloride;
g. at least 96% (w/w) water; and
   having a pH in the range of about 5 to about 6.

In other embodiments, the aqueous, capsaicin injectable formulation comprises
a. about 0.04% (w/w) to about 0.06% (w/w) of capsaicin;
b. about 0.8% (w/w) to about 1.2% (w/w) of a solubilizing agent, wherein the solubilizing agent is a mixture of (a) about 70% (w/w) of a mixture of 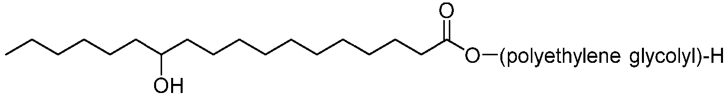 and 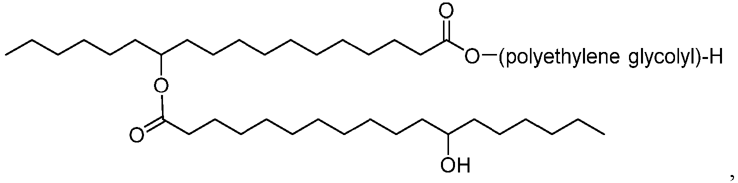 and (b) about 30% (w/w) polyethylene glycol;
c. about 0.005% (w/w) to about 0.015% (w/w) of dibutylhydroxytoluene;
d. about 0.2% (w/w) to about 1% (w/w) of sodium acetate;
e. about 0.01% (w/w) to about 0.05% (w/w) of ethylenediaminetetraacetic acid or a salt thereof;
f. about 0.3% (w/w) to about 0.9% (w/w) of sodium chloride;
g. at least 96% (w/w) water; and
   having a pH in the range of about 5 to about 6.

In other embodiments, the aqueous, capsaicin injectable formulation comprises
a. about 0.05% (w/w) of capsaicin;
b. about 1% (w/w) of a solubilizing agent, wherein the solubilizing agent comprises 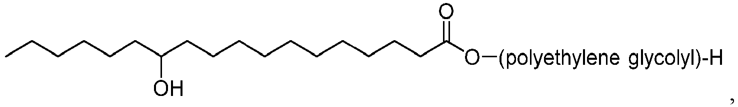 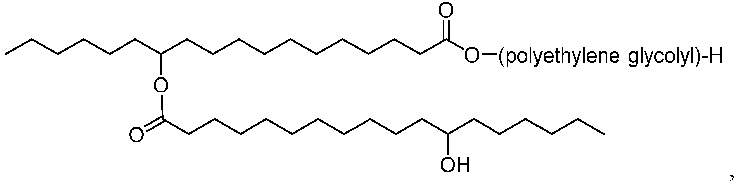 and polyethylene glycol;
c. about 0.005% (w/w) to about 0.015% (w/w) of dibutylhydroxytoluene;
d. about 0.2% (w/w) to about 1% (w/w) of sodium acetate;
e. about 0.01% (w/w) to about 0.05% (w/w) of ethylenediaminetetraacetic acid or a salt thereof;
f. about 0.3% (w/w) to about 0.9% (w/w) of sodium chloride;
g. at least 96% (w/w) water; and
   having a pH of about 5.5.

In other embodiments, the aqueous, capsaicin injectable formulation comprises
a. about 0.05% (w/w) of capsaicin;
b. about 1% (w/w) of a solubilizing agent, wherein the solubilizing agent is a mixture of 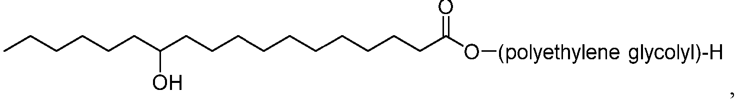 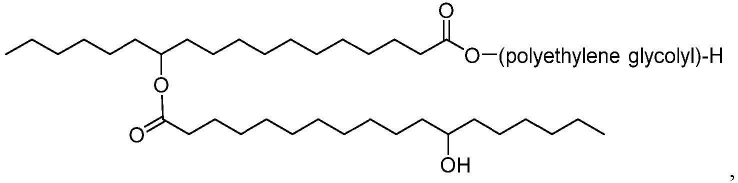 and polyethylene glycol;
c. about 0.005% (w/w) to about 0.015% (w/w) of dibutylhydroxytoluene;
d. about 0.2% (w/w) to about 1% (w/w) of sodium acetate;
e. about 0.01% (w/w) to about 0.05% (w/w) of ethylenediaminetetraacetic acid or a salt thereof;
f. about 0.3% (w/w) to about 0.9% (w/w) of sodium chloride;
g. at least 96% (w/w) water; and
having a pH of about 5.5.

In other embodiments, the aqueous, capsaicin injectable formulation comprises
a. about 0.05% (w/w) of capsaicin;
b. about 1% (w/w) of a solubilizing agent, wherein the solubilizing agent is a mixture of 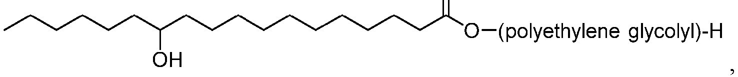 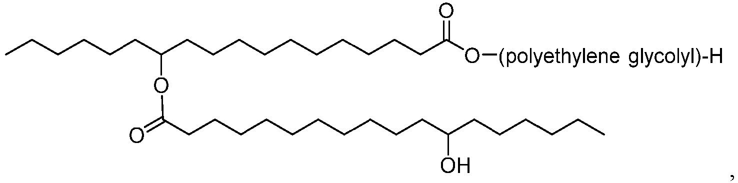 and polyethylene glycol;
c. about 0.005% (w/w) to about 0.015% (w/w) of dibutylhydroxytoluene;
d. about 0.2% (w/w) to about 1% (w/w) of buffer, which is a mixture of sodium acetate and acetic acid;
e. about 0.01% (w/w) to about 0.05% (w/w) of ethylenediaminetetraacetic acid or a salt thereof;
f. about 0.3% (w/w) to about 0.9% (w/w) of sodium chloride;
g. at least 96% (w/w) water; and
having a pH of about 5.5.

Each of the foregoing formulations may be further characterized according to the weight-average molecular weight of the polyethylene glycol component(s). Accordingly, in certain embodiments, the polyethylene glycol has a weight-average molecular weight in the range of about 200 g/mol to about 1500 g/mol. In certain embodiments, the polyethylene glycol has a weight-average molecular weight in the range of about 200 g/mol to about 1000 g/mol. In certain embodiments, the polyethylene glycol has a weight-average molecular weight in the range of about 300 g/mol to about 900 g/mol. In certain embodiments, the polyethylene glycol has a weight-average molecular weight in the range of about 500 g/mol to about 800 g/mol. In certain embodiments, the polyethylene glycol has a weight-average molecular weight in the range of about 600 g/mol to about 700 g/mol. In certain embodiments, the polyethylene glycol has a weight-average molecular weight in the range of about 100 g/mol to about 300 g/mol, about 300 g/mol to about 500 g/mol, about 500 g/mol to about 1000 g/mol, about 1000 g/mol to about 1500 g/mol, about 1500 g/mol to about 2000 g/mol, or about 2000 g/mol to about 2500 g/mol.

Exemplary more specific formulations are provided in Tables 4 and 5 below.

**TABLE 4.**

| **No.** | **Formulation** | |
|---|---|---|
| 1 | An aqueous, capsaicin injectable formulation, comprising: | |
| | | a. about 0.05% (w/w) of *trans*-capsaicin; |
| | | b. about 1% (w/w) of a solubilizing agent, wherein the solubilizing agent is a mixture of |
| | | 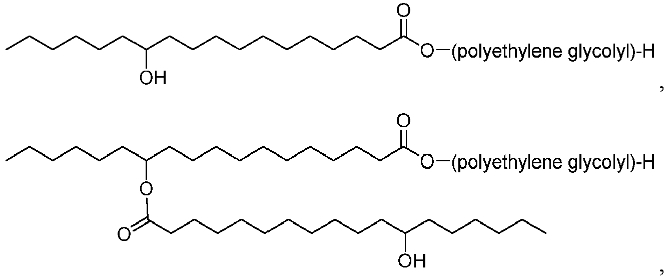 |
| | | and polyethylene glycol; wherein the polyethylene glycolyl has a weight average molecular weight of about 660 g/mol; |
| | | c. about 0.01% (w/w) dibutylhydroxytoluene; |
| | | d. about 0.68% (w/w) of sodium acetate or a mixture of sodium acetate and acetic acid; |
| | | e. about 0.025% (w/w) of ethylenediaminetetraacetic acid or a salt thereof; |
| | | f. about 0.6% (w/w) of sodium chloride; |
| | | g. at least 97% (w/w) water; and |
| | having a pH of about 5.5. | |
| 2 | An aqueous, capsaicin injectable formulation, comprising: | |
| | | a. 0.05% (w/w) of *trans*-capsaicin; |
| | | b. 1% (w/w) of a solubilizing agent, wherein the solubilizing agent is a mixture of |
| | | 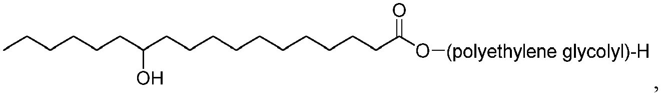 |
| | | 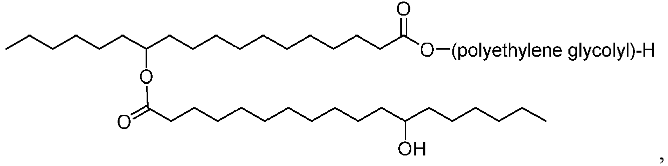 |
| | | and polyethylene glycol; wherein the polyethylene glycolyl has a weight average molecular weight of about 660 g/mol; |
| | | c. 0.01% (w/w) dibutylhydroxytoluene; |
| | | d. 0.68% (w/w) of sodium acetate or a mixture of sodium acetate and acetic acid; |
| | | e. 0.025% (w/w) of ethylenediaminetetraacetic acid or a salt thereof; |
| | | f. 0.6% (w/w) of sodium chloride; |
| | | g. at least 97% (w/w) water; and |
| | having a pH of 5.5. | |
| 3 | An aqueous, capsaicin injectable formulation, comprising: | |
| | | a. about 0.05% (w/w) of *trans*-capsaicin; |
| | | b. about 1% (w/w) of a solubilizing agent, wherein the solubilizing agent is a mixture of |
| | | 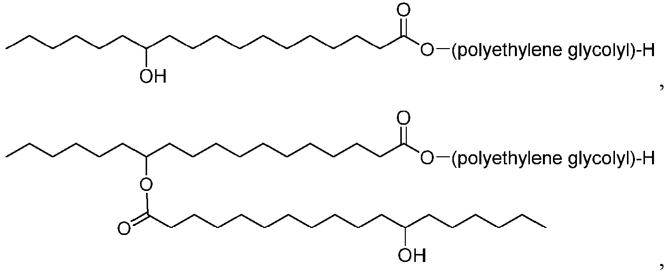 |
| | | and polyethylene glycol; wherein the polyethylene glycolyl has a weight average molecular weight of about 660 g/mol; |
| | | c. about 0.01% (w/w) dibutylhydroxytoluene; |
| | | d. about 0.34% (w/w) of sodium acetate or a mixture of sodium acetate and acetic acid; |
| | | e. about 0.025% (w/w) of ethylenediaminetetraacetic acid or a salt thereof; |
| | | f. about 0.75% (w/w) of sodium chloride; |
| | | g. at least 97% (w/w) water; and |
| | having a pH of about 5.5. | |
| 4 | An aqueous, capsaicin injectable formulation, comprising: | |
| | | a. 0.05% (w/w) of *trans*-capsaicin; |
| | | b. 1% (w/w) of a solubilizing agent, wherein the solubilizing agent is a mixture of |
| | | 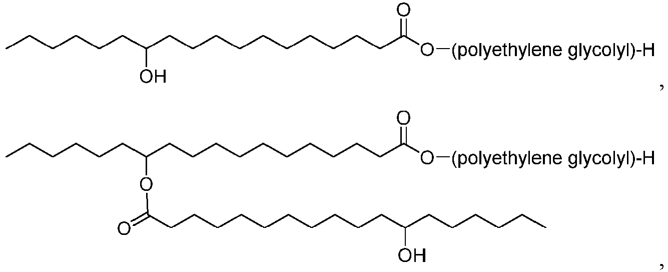 |
| | | and polyethylene glycol; wherein the polyethylene glycolyl has a weight average molecular weight of about 660 g/mol; |
| | | c. 0.01% (w/w) dibutylhydroxytoluene; |
| | | d. 0.34% (w/w) of sodium acetate or a mixture of sodium acetate and acetic acid; |
| | | e. 0.025% (w/w) of ethylenediaminetetraacetic acid or a salt thereof; |
| | | f. 0.75% (w/w) of sodium chloride; |
| | | g. at least 97% (w/w) water; and |
| | having a pH of 5.5. | |

**TABLE 5.**

| **No.** | **Formulation** | |
|---|---|---|
| 1 | An aqueous, capsaicin injectable formulation, comprising: | |
| | | a. about 0.05% (w/w) of *trans*-capsaicin; |
| | | b. about 1% (w/w) of a solubilizing agent, wherein the solubilizing agent is a mixture of |
| | | 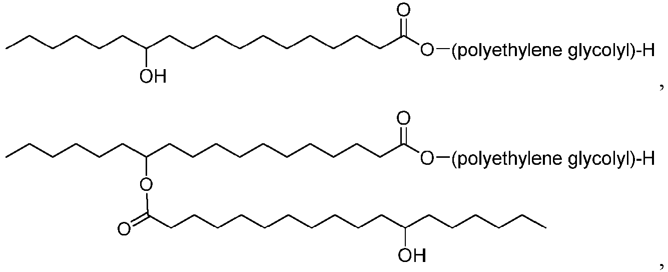 |
| | | and polyethylene glycol; wherein the polyethylene glycolyl has a weight average molecular weight of about 660 g/mol; |
| | | c. about 0.01% (w/w) dibutylhydroxytoluene; |
| | | d. about 0.22% (w/w) of sodium citrate or a mixture of sodium citrate and citric acid; |
| | | e. about 0.025% (w/w) of ethylenediaminetetraacetic acid or a salt thereof; |
| | | f. about 0.8% (w/w) of sodium chloride; |
| | | g. at least 97% (w/w) water; and |
| | having a pH of about 5.5. | |
| 2 | An aqueous, capsaicin injectable formulation, comprising: | |
| | | a. 0.05% (w/w) of *trans*-capsaicin; |
| | | b. 1% (w/w) of a solubilizing agent, wherein the solubilizing agent is a mixture of |
| | | 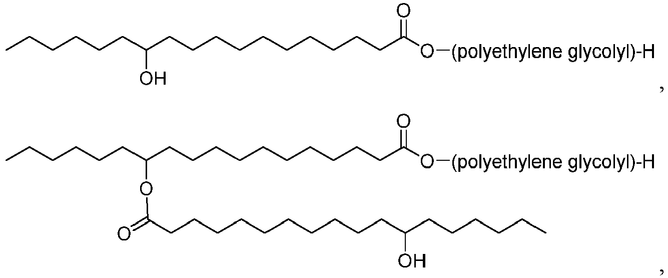 |
| | | and polyethylene glycol; wherein the polyethylene glycolyl has a |
| | | weight average molecular weight of about 660 g/mol; |
| | | c. 0.01% (w/w) dibutylhydroxytoluene; |
| | | d. 0.22% (w/w) of sodium citrate or a mixture of sodium citrate and citric acid; |
| | | e. 0.025% (w/w) of ethylenediaminetetraacetic acid or a salt thereof; |
| | | f. 0.8% (w/w) of sodium chloride; |
| | | g. at least 97% (w/w) water; and |
| | having a pH of 5.5. | |
| 3 | An aqueous, capsaicin injectable formulation, comprising: | |
| | | a. about 1% (w/w) of *trans*-capsaicin; |
| | | b. about 2% (w/w) of a solubilizing agent, wherein the solubilizing agent is a mixture of |
| | | 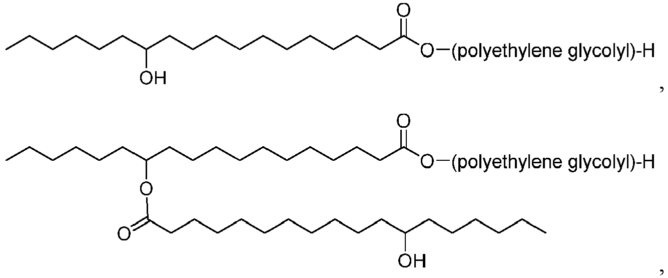 |
| | | and polyethylene glycol; wherein the polyethylene glycolyl has a weight average molecular weight of about 660 g/mol; |
| | | c. about 0.01% (w/w) dibutylhydroxytoluene; |
| | | d. about 20 mM of sodium citrate or a mixture of sodium citrate and citric acid; |
| | | e. about 0.1% (w/w) of ethylenediaminetetraacetic acid or a salt thereof; |
| | | f. about 3.15% (w/w) of dextrose; |
| | | g. at least 93% (w/w) water; and |
| | having a pH of about 5 to about 6. | |
| 4 | An aqueous, capsaicin injectable formulation, comprising: | |
| | | a. 1% (w/w) of *trans*-capsaicin; |
| | | b. 2% (w/w) of a solubilizing agent, wherein the solubilizing agent is a mixture of |
| | | 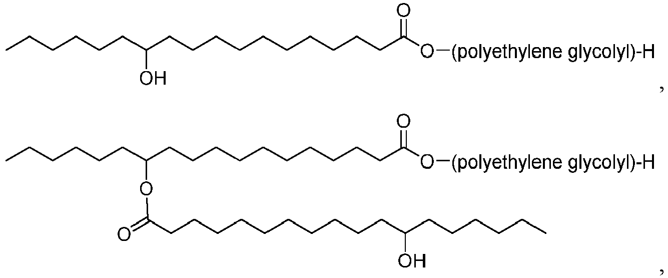 |
| | | and polyethylene glycol; wherein the polyethylene glycolyl has a weight average molecular weight of about 660 g/mol; |
| | | c. 0.01% (w/w) dibutylhydroxytoluene; |
| | | d. 20 mM of sodium citrate or a mixture of sodium citrate and citric acid; |
| | | e. 0.1% (w/w) of ethylenediaminetetraacetic acid or a salt thereof; |
| | | f. 3.15% (w/w) of dextrose; |
| | | g. at least 93% (w/w) water; and |
| | having a pH of about 5 to about 6. | |

In certain embodiments, the formulation is one of the formulations described in Tables 1-5 above, wherein the formulation has an osmolality in the range of from about 240 mOsm/kg to about 340 mOsm/kg. In certain embodiments, the formulation is one of the formulations described in Tables 1-5 above, wherein the formulation has an osmolality in the range from about 270 mOsm/kg to about 330 mOsm/kg.

### Stability of the Aqueous Capsaicin Injectable Formulations

A formulation containing capsaicin can be further characterized according to the stability of the formulation upon storage. For example, in certain embodiments, the formulation is characterized by the feature that less than 1% of the capsaicin degrades upon storage at 25°C for 24 weeks. In certain other embodiments, less than 0.5% of the capsaicin degrades upon storage at 25°C for 24 weeks. In certain other embodiments, less than 0.1% of the capsaicin degrades upon storage at 25°C for 24 weeks. In certain other embodiments, less than 1% of the capsaicin degrades upon storage at 40°C for 24 weeks. In certain other embodiments, less than 0.5% of the capsaicin degrades upon storage at 40°C for 24 weeks.

### Amount of Capsaicin-dimer in an Aqueous Capsaicin Injectable Formulations

A formulation containing capsaicin can be further characterized according to the amount of any impurities in the formulation, such as the amount of capsaicin-dimer having the following formula:

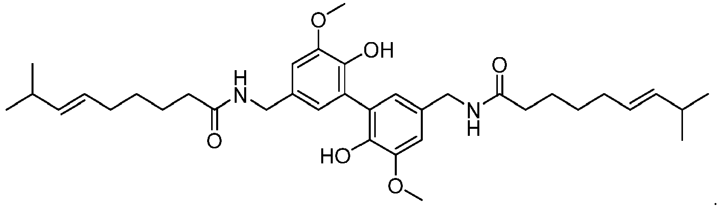

Accordingly, in certain embodiments, the formulation is characterized by the feature that it contains less than 3% (w/w) of capsaicin-dimer having the following structure: 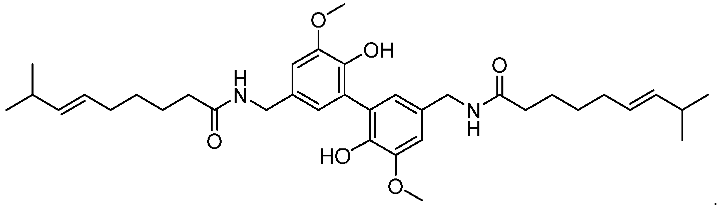 In certain other embodiments, the formulation contains less than 2% (w/w) of the capsaicin-dimer. In certain other embodiments, the formulation contains less than 1% (w/w) of the capsaicin-dimer. In certain other embodiments, the formulation contains less than 0.6% (w/w) of the capsaicin-dimer.

In certain other embodiments, upon storage at 25°C for 12 weeks, the formulation contains less than 3% (w/w) of capsaicin-dimer having the following structure: 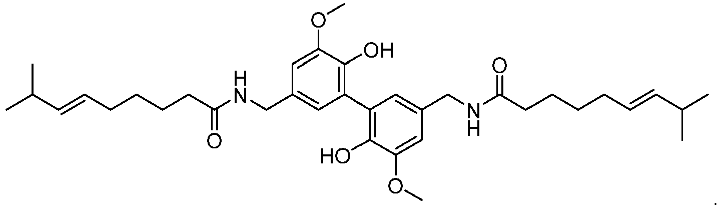 In certain other embodiments, upon storage at 25°C for 12 weeks, the formulation contains less than 2% (w/w) of capsaicin-dimer. In certain other embodiments, upon storage at 25°C for 24 weeks, the formulation contains less than 1% (w/w) of the capsaicin-dimer. In certain other embodiments, upon storage at 25°C for 24 weeks, the formulation contains less than 0.6% (w/w) of the capsaicin-dimer.

### Amount of Substituted 1,1'-Biphenyl Compound in an Aqueous Capsaicin Injectable Formulations

A formulation containing capsaicin can be further characterized according to the amount of substituted 1,1'-biphenyl compound having the following structure: 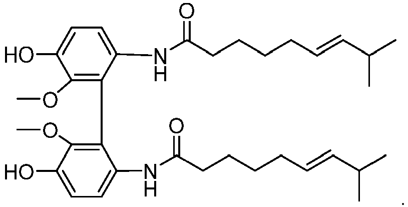 In certain embodiments, the formulation contains less than 2% (w/w) of the substituted 1,1'-biphenyl compound. In certain embodiments, the formulation contains less than 1% (w/w) of the substituted 1,1'-biphenyl compound.

In certain other embodiments, upon storage at 25°C for 12 weeks, the formulation contains less than 3% (w/w) of the aforementioned substituted 1,1'-biphenyl compound. In certain other embodiments, upon storage at 25°C for 12 weeks, the formulation contains less than 2% (w/w) of the substituted 1,1'-biphenyl compound. In certain other embodiments, upon storage at 25°C for 24 weeks, the formulation contains less than 1% (w/w) of the substituted 1,1'-biphenyl compound. In certain other embodiments, upon storage at 25°C for 24 weeks, the formulation contains less than 0.6% (w/w) of substituted 1,1'-biphenyl compound.

### Amount of Optional Other Components in the Injectable Formulations

Formulations herein can be further characterized according to the amount of optional other components. For example, in certain embodiments, the formulation contains less than 0.1% (w/w) of any polysorbate (e.g., polysorable 20 or polysorbate 80). In certain embodiments, the formulation does not contain any polysorbate. In certain embodiments, the formulation contains less than 0.1% (w/w) of any polysorbate, cyclodextrin, or alcohol. In certain embodiments, the formulation does not contain any polysorbate, cyclodextrin, or alcohol.

In yet other embodiments, other than said solubilizing agent, the formulation contains less than 0.1% (w/w) of any polymer, oligomer-containing agent, or agent that improves the solubility of capsaicin. In yet other embodiments, other than said solubilizing agent, the formulation does not contain any polymer, oligomer-containing agent, or agent that improves the solubility of capsaicin. In yet other embodiments, the formulation contains less than 0.1% (w/w) of any cyclodextrin, cellulose, alcohol (e.g., menthol), or hyaluronic acid. In yet other embodiments, the formulation does not contain any cyclodextrin, cellulose, alcohol (e.g., menthol), or hyaluronic acid.

In certain embodiments, the formulation contains less than 0.1% (w/w) of any phospholipid, polysaccharide, protein polymer, cellulose, sorbitan ester, or histidine. In certain embodiments, the formulation does not contain of any phospholipid, polysaccharide, protein polymer, cellulose, sorbitan ester, or histidine. In certain embodiments, the formulation contains less than 0.1% (w/w) of any polyvinylpyrrolidone polymer. In certain embodiments, the formulation does not contain any polyvinylpyrrolidone polymer.

In certain embodiments, the formulation contains less than 0.5% (w/w) of any polyalkylene glycol (e.g., polyethylene glycol) polymer. In certain embodiments, the formulation contains less than 0.3% (w/w), 0.25% (w/w), 0.2% (w/w), 0.15% (w/w), 0.1% (w/w), 0.05% (w/w) 0.01% (w/w) of any polyalkylene glycol (e.g., polyethylene glycol) polymer.

In certain embodiments, the formulation contains less than 0.5% (w/w) of any surfactant. In certain embodiments, the formulation contains less than 0.3% (w/w), 0.25% (w/w), 0.2% (w/w), 0.15% (w/w), 0.1% (w/w), 0.05% (w/w) 0.01% (w/w) of any surfactant. In certain embodiments, but for any component of the formulation named in the description of the formulation that would qualify as a surfactant, the formulation does not contain any other agent that is a surfactant.

Additional formulations are described in international patent application WO 2018/085476.

### EXAMPLES

The invention now being generally described, will be more readily understood by reference to the following examples, which are included merely for purposes of illustration of certain aspects and embodiments of the present invention, and is not intended to limit the invention.

### EXAMPLE 1 - SEQUENTIAL INTRA-ARTICULAR INJECTION OF CAPSAICIN TO TREAT OSTEOARTHRITIC KNEE JOINT PAIN IN HUMAN PATIENTS

Human patients experiencing chronic, moderate to severe osteoarthritic knee joint pain may be treated for such pain by receiving a first intra-articular injection of a 1.0 mg dose of trans-capsaicin into the osteoarthritic knee joint and then twenty-six weeks after the first intra-articular injection of *trans*-capsaicin a second intra-articular injection of a 1.0 mg dose of trans-capsaicin into the osteoarthritic knee joint is performed, all according to the clinical protocol described below. The investigational medicinal product (IMP) in this protocol is a 1.0 mg dose of *trans*-capsaicin provided as a pre-filled syringe containing 2.0 mL of solution containing *trans*-capsaicin (concentration of *trans*-capsaicin in the solution is 0.5 mg/mL). Patients are selected for treatment according to the patient selection criteria described below, and then treated according to the treatment procedure described below. Controlled cooling may be performed using a Breg cooling wrap cooled by circulating ice-cold water.

### Patients Selection

Patients must satisfy the inclusion criteria set forth the below, and also not have any of the exclusion criteria set forth below.

### Inclusion Criteria:

1. Male or female patients between 40 and 95 years of age (inclusive) at the time of the Screening Visit with the ability to comply with answering the electronic diary using the study-provided tablet computers.
2. Confirmation of osteoarthritis of the knee: radiography of both knees with a posterior-anterior, fixed-flexion view taken during the Pre-screening Period. The index knee must show evidence of chronic osteoarthritis with a K-L grade of 2, 3 and 4. The IRT system will limit the number of K-L grade 4 patients to approximately 55 patients (approximately 20% of enrollment).
3. Confirmation of osteoarthritis of the index knee: American College of Rheumatology (ACR) diagnostic criteria.
4. Moderate to severe pain in the index knee associated with osteoarthritis must be stable for a minimum of 6 months prior to Screening, as assessed by the investigator.
5. For qualifying Baseline knee pain with walking, the Baseline score will be derived from the 14 days of pre-dose pain scores collected immediately preceding patient randomization on Day 1. The Baseline pain score will be calculated by the randomization algorithm as the mean of the scores from the 14 days immediately preceding Day 1. Patients must use a numeric pain rating scale (NPRS) (0-10; 0 = no pain, 10 = worst pain ever) to rate their index knee pain with walking, reported using the electronic patient-reported outcomes (ePRO) system. Pain in the index knee must be rated daily at bedtime (9:00 PM ± 3 hours) to determine the average knee pain with walking during the previous 24 hours. The site and patient training will target at least an 85% compliance rate, using ongoing assessment of compliance data, and site communication with the patient if compliance drops below 85%.
6. At Baseline only, pain with walking will also be collected and assessed for the non-index knee, as noted in the assessment above (5).
7. Compliance with diary entry to meet minimal acceptable criteria during Screening.
8. Understanding of the outcome measures, and the relationship among them, after patient training and testing.
9. Body mass index (BMI) ≤45 kg/m².
10. Patients must have failed 2 or more prior therapies. Failure is deemed to be inadequate relief in the opinion of the investigator. A therapy may be deemed to have been inadequate because of one or more of the following:
   a) unacceptable adverse events (AEs);
   b) inadequate response, or loss of response for chronic osteoarthritis knee pain or
      ∘ pain in the index knee was minimally improved, not improved, or was worse and/or
      ∘ improvement in function, and/or stiffness in the index knee was minimally improved, not improved, or was worse
   c) medical condition resulting in contraindication to the standard of care appropriate to the severity of the index knee osteoarthritis pain.
   "Therapies" include, but are not limited to, each of the following:
   ∘ nonsteroidal anti-inflammatory drugs (NSAIDs) (including topical), opioids, duloxetine, other systemic therapy, intra-articular (IA) corticosteroids, IA viscosupplements
   ∘ physical therapy, bracing, and orthotics.
11. Females not of childbearing potential, defined as post-menopausal for at least 1 year, or surgically sterile (bilateral tubal ligation, bilateral oophorectomy, or hysterectomy), or practicing one of the following medically acceptable methods of birth control throughout the study period:
   - Hormonal methods such as oral, implantable, injectable, or transdermal contraceptives for a minimum of 1 full cycle (based on the patient's usual menstrual cycle period) before IMP administration
   - Total abstinence from sexual intercourse since the last menses before IMP administration
   - Intrauterine device
   - Double barrier method (condoms, sponge, diaphragm, with spermicidal jellies or cream)
12. Able to speak, read, and understand the language of the study used for the informed consent and ePRO.
13. Willing and able to:
   a) understand the study requirements,
   b) abide by the study restrictions and requirements,
   c) complete the study procedures,
   d) be compliant and independently (i.e., without assistance) record responses on the pain scales and make daily entries using ePRO (e.g., have the ability to comply with answering the electronic diary using the study provided tablet computers),
   e) independently communicate meaningfully with the study personnel.
14. Signed informed consent form approved by the institutional review board (IRB).
15. Patient agrees to stay on their current pain medication (including over the counter (OTC) medications) from the time of Pre-screening through Week 12. The current pain medication must be taken only for pain in the index knee, and not for another pain indication. Their current pain medication must be one of the allowed rescue medications and dosages, or hydrocodone at a dose up to 15 mg daily (or another opioid equivalent). The rescue medication is not allowed to be in the same drug class as the ongoing medication. For example, patients will not be allowed to take tramadol as a rescue medication if they are taking an opioid as concomitant medication, or an NSAID as rescue if they are using an NSAID as concomitant medication. From Week 12 to completion of the study, patients may stay on current pain medication as prescribed or OTC medication, but this is not required. If reducing or stopping background medications for the index knee osteoarthritis pain, that information must be recorded by the site/patient.
16. Patient agrees to take only the allowed rescue medications for osteoarthritis knee pain of the index knee from the time of the first Treatment Visit through study completion and agrees to use no topical medications for osteoarthritis knee pain during the trial.

### Exclusion criteria:

1. Joint replacement surgery of the index knee at any time (full or partial), or open surgery of the index knee in the past 24 months.
2. Prior arthroscopic surgery of the index knee within 6 months of Screening.
3. Any painful conditions of the index knee due to joint disease other than osteoarthritis. For example, radicular or referred pain involving the index knee or from joint disease other than osteoarthritis involving the index knee, such as, but not restricted to, inflammatory diseases, e.g., rheumatoid arthritis, psoriatic arthritis, chondromalacia patella, metabolic diseases, gout/pseudogout, hemochromatosis, acromegaly, etc.
4. Periarticular pain from any cause, including referred pain, bursitis, tendonitis, soft tissue tenderness, or subacute/acute pain from injury.
5. Pain in the non-index knee that is >3 (NPRS 0-10) when walking or at rest.
6. Other chronic pain anywhere in the body that requires the use of analgesic medications, including, but not limited to, local painful areas, myofascial pain syndromes, fibromyalgia, genetic, metabolic abnormalities, hematologic or neuropathic pain, and any acute or chronic pain that may interfere with the study pain assessments by the patient.
7. Instability of the index knee (e.g., cruciate ligament tear or rupture, significant protruding meniscus, substantial ligamentous laxity).
8. Misalignment (>10 degrees varus or valgus) of the index knee on standing.
9. Documented history of neuropathic arthropathy or finding of bony fragmentation in the index knee with imaging (radiographic, computed tomography, or magnetic resonance imaging).
10. Physical/occupational/chiropractic therapy for the lower extremities or acupuncture for the lower extremities within 30 days of Screening, or need for such therapy during the study.
11. Plans to have surgery, other invasive procedures, or intra-articular (IA) injections (other than the IMP) while participating in the study.
12. Has used topical capsaicin on the index knee within 90 days of Screening.
13. Current use of opioids for any condition other than for osteoarthritis of the index knee (maximum dose of 15 mg of hydrocodone [or equivalent] per day).
14. Corticosteroid injection into the index knee within 90 days of Screening.
15. Received IA viscosupplementation (e.g., SYNVISC^{®}, HYALGAN^{®}) within 90 days of Screening.
16. History of allergic reaction to the planned local anesthesia/analgesic regimens, ethylenediaminetetraacetic acid (EDTA), Kolliphor HS 15, butylated hydroxytoluene (BHT), or capsaicin.
17. Presence of any medical condition or unstable health status that, in the judgment of the investigator, might adversely impact the safety of the patient, or the conduct of the study, or negatively affect the resulting data, including chronic conditions that are likely to alter the rate of healing or are likely to result in safety complications unrelated to the study medication, or significantly compromise key organ systems. For any question regarding eligibility, it is strongly recommended that the investigator discuss the patient with the medical monitor.
18. Is pregnant or is breast feeding.
19. Has a malignancy, a history of malignancy, or has received treatment for malignancy at any time, with exception of resected and cured basal cell carcinoma and squamous cell carcinoma of the skin.
20. Regular use of anticoagulant blood thinners (except low-dose aspirin, Dabigatran 150 mg once daily [qd], Enoxaparin 40 mg qd, Rivaroxaban 10 mg qd, Apixaban 2.5 mg twice daily [bid], or clopidogrel 75 mg qd, which are allowed).
21. Active cutaneous disease at the anticipated site of IMP injection that would prevent the safe administration of IMP.
22. Ulcer or open wound anywhere on the index knee.
23. Specific laboratory abnormalities:
   - Hemoglobin <11.0 g/dL
   - White blood cells (WBC) <2.5 X 10⁹/L
   - Neutrophils <1.5 X 10⁹/L
   - Platelets <100 X 10⁹/L
   - Aspartate transaminase (AST) or alanine transaminase (ALT) >2 X upper limit of normal
   - Creatinine >1.6 mg/dL
   - Glucose (fasting) >250 mg/dL
   - HgbA1c >9
24. Clinically significant abnormal laboratory result at the Screening Visit (in the opinion of the investigator), or significant organ disease that would put the patient at undue risk or affect the ability of the patient to participate in the trial. For any question regarding eligibility, it is strongly recommended that the investigator discusses the patient with the medical monitor.
25. Use of an investigational medication within 30 days of Screening or 5 pharmacokinetic or pharmacodynamic half-lives (whichever is longer), or scheduled to receive such an agent while participating in the current study.
26. Prior participation in a capsaicin intra-articular knee joint injection study.
27. Has any of the following characteristics:
   - Active or historic substance use disorder within the previous year as defined by the Diagnostic and Statistical Manual for Mental Health Disorders, fifth edition
   - Test is positive upon urine drug screen for a substance of abuse (prescribed opioids acceptable)
   - Has a history, at any time, or currently, of suicidal ideation, suicide attempt, or increased risk of suicide
   - Has unacceptable level of depression or anxiety as measured by the Hospital Anxiety and Depression Scale (HADS)
   - Has unacceptable chronic pain as measured by the Fibromyalgia Symptom Scale Score (FSS)
   - Has a positive pregnancy test at the Screening or Treatment Visit
   - Has ongoing litigation for workers' compensation
   - Has any condition, or is taking any medication, that would be contraindicated for study participation

### Treatment Procedure

Patients meeting the selection criteria described above are to be treated for their chronic, moderate to severe osteoarthritic knee joint pain according to the procedure set forth below. The procedure is based on a clinical study protocol, where the overall maximum duration of the study is expected to be approximately 58 weeks. Approximately 325 patients will be enrolled with a 2:3 allocation (placebo to active) (approximately 125 patients assigned to placebo and 200 patients assigned to 1.0 mg of *trans*-capsaicin). The sequence and maximum duration of the study periods will be as follows:
- Pre-Screening (patients should return for Screening as soon as feasible)
- Screening Period: up to 30 days +3-day window
- Treatment Period (double-blind, placebo-controlled): first dose at Day 1 (1 day); second dose at Week 26 (1 day)
- Post-Treatment Double-Blind period: 52 weeks

The maximum IMP treatment period for each patient is 2 days. The maximum study duration for each patient is approximately 58 weeks.

Patients will be stratified to balance across treatment groups for:
- K-L grades 2, 3 and 4, with a limit of approximately 20% of patients having K-L grade 4,
- Body mass index (BMI) <30 or ≥30 kg/m², and
- Sex of patient.

Patients will be randomized to receive single IA injections of 1.0 mg of *trans-*capsaicin, or matching placebo, injected into the index knee at Baseline and at Week 26. The second injection will consist of the original randomized IMP or placebo. Patients will continue to be followed for a further 26 weeks after the second injection (52 weeks total from the Baseline injection).

The investigator, at his or her discretion, may pre-medicate patients with an oral dose of an opioid or nonsteroidal anti-inflammatory drug (NSAID). The skin may, also at the discretion of the investigator, be infiltrated with 1-2 mL of lidocaine solution at the point of the subsequent injections.

Controlled joint cooling will be applied 15 minutes prior to the intra-articular injection of the required full 15 mL 2% lidocaine (without epinephrine) into the joint. Controlled cooling will be resumed for a further 30 minutes after the intra-articular 2% lidocaine (without epinephrine) injection. The cooling device will then be removed and IMP injection will be administered. The use of ultrasound for injections is recommended, but not required. The knee joint will be passively flexed and extended 5 times over 1 minute to facilitate distribution of the IMP within the index knee. Then controlled cooling will be reapplied for a minimum of 30 minutes, and up to 90 minutes, after IMP injection, depending on the patient's comfort. The cooling may be discontinued after a minimum of 30 minutes following IMP IA injection, if the patient has a pain level that is acceptable for the patient and investigator (0-4 scale: none, mild, moderate, moderately severe, and severe).

Patients should not take a hot bath or shower, or expose the injected knee to external heat, within 12 hours after the injection.

A provided tablet computer will be used to record the electronic patient-reported outcomes (ePRO). Entries will be made daily to evaluate the index knee pain (the primary endpoint) and use of rescue medication. All other assessments will be evaluated by telephone and/or clinic visits. Study staff will call patients to assess osteoarthritis pain, overall satisfaction with the treatment procedure, adverse events, and the use of rescue medication, on Day 3 after each injection. Patients will return to the clinic at Weeks 4, 8, 12, 18, 26, 30, 38, 46, and 52 for study assessments.

Efficacy will be assessed by osteoarthritis index knee pain with walking, including durability of treatment response and cumulative responder analyses (cumulative and Outcome Measures in Rheumatology [OMERACT]-Osteoarthritis Research Society International [OARSI]; Western Ontario and McMaster Universities Osteoarthritis Index [WOMAC] knee pain, stiffness, and function [WOMAC A, B and C, respectively]; Patient Global Impression of Change [PGIC] for osteoarthritis index knee pain; Knee Injury and Osteoarthritis Outcome Score [KOOS]; Medical Outcomes Study [MOS] sleep scale; SF-36 health questionnaire; EQ-5D-5L quality of life; Work Productivity and Activity Impairment Scale [WPAI]; and rescue medication use).

Safety will be assessed by injection site assessment (erythema and edema), assessment of treatment procedure pain, adverse events (AEs), physical examination findings, sensory testing, vital sign measurements, 12-lead electrocardiograms (ECG), clinical laboratory test results, and the stability of knee radiographs from Baseline to Week 52.

From Pre-screening through Week 12, patients are to stay on their current pain medication (prescription or OTC). The current pain medication must be taken only for pain in the index knee, and not for another pain indication. The current pain medication must be either one of the allowed medications listed in Table 1 (maximum dose as noted in Table 1) or hydrocodone at a dose up to 15 mg daily (or another opioid equivalent). From Week 12 through completion of the study, patients are permitted to change or discontinue their doses of these background pain medications to treat the index knee osteoarthritis pain.

Starting with the first Treatment Visit, one of the rescue medications shown in Table 1 may be added for the index knee osteoarthritis pain. If the patient's background medication is one of the allowed medications in Table 1, they may continue that therapy and use one of the other classes of rescue medication along with their background medication. The rescue medication is not allowed to be in the same drug class as the ongoing medication. For example, patients will not be allowed to take tramadol as a rescue medication if they are taking an opioid as concomitant medication, or an NSAID as rescue if they are using an NSAID as a concomitant medication. Details of all rescue medication will be recorded daily by the patient in the ePRO system.

Patients are not to take rescue medication within 24 hours of any post-treatment planned study visit.

Rescue medications may include one of the following:
- acetaminophen,
- a single NSAID, or
- Tramadol, up to 200 mg/day.

**Table 1: Rescue Medication Ladder for Pain Relief.**

| **Rescue Tier** | **Rescue Medication** | **Maximum daily dosage** | **Recommended Dose for Rescue Pain Relief** |
|---|---|---|---|
| 1 | Acetaminophen | 650 mg × 4 = 2.6 gm | 325 mg, 2 tablets PO, QID, PRN |
| 2 | Ibuprofen | 600 mg × 4 = 2400 mg | 600 mg PO, QID, PRN |
| 2 | Naproxen | 500 mg × 2 = 1000 mg | 500 mg PO, BID, PRN |
| 2 | Celecoxib | 200 mg | 100 mg PO, BID, PRN |
| 2 | Meloxicam | 15 mg | 7.5 mg, PO, BID or 15 mg QD, PRN |
| 3 | Tramadol | 200 mg | 25 - 50 mg PO, QID, PRN |
| | | | Caution patients of possible adverse events of dizziness, nausea, and somnolence. |

| | | | |
|---|---|---|---|
| BID = twice daily; PO = by mouth (orally); PRN = as needed; QD = once daily; QID = four times daily. | | | |

The following therapies are prohibited both prior to and during the study:
- Injection of corticosteroids in the index knee from 90 days prior to Screening through study completion.
- Topical medications applied to the index knee for osteoarthritis pain (including capsaicin, lidocaine, prescription, or OTC medications) from 90 days prior to Screening through study completion.
- Current use of opioids for any condition other than for osteoarthritis of the index knee (maximum dose of 15 mg of hydrocodone [or equivalent] per day as background medication allowed at entry). Permitted analgesics, taken occasionally for conditions other than index knee osteoarthritis (not rescue analgesics) should be avoided within 24 h of any post-IMP treatment planned study visit, and are to be recorded as concomitant medications.
- Regular use of anticoagulant blood thinners (except low-dose aspirin, Dabigatran 150 mg once daily [qd], Enoxaparin 40 mg qd, Rivaroxaban 10 mg qd, Apixaban 2.5 mg twice daily [bid], or clopidogrel 75 mg qd, which are allowed).
- Use of an investigational medication within 30 days prior to Screening, or 5 phamacokinetic or pharmacodynamic half-lives (whichever is longer), or scheduled to receive such an agent while participating in the study.
- Physical/occupational/chiropractic therapy for the lower extremities or acupuncture for the lower extremities within 30 days of Screening, or need for such therapy during the course of the study.
- Joint replacement surgery of the index knee at any time (full or partial), or open surgery of the index knee in the past 24 months prior to Screening, or prior arthroscopic surgery of the index knee within 6 months of Screening.
- Surgery, or other invasive procedures, or IA injections (other than the IMP) while participating in the study.

The patient should be excluded from study participation if they have taken any medication prior to randomization that would indicate that the patient has a serious or unstable illness, is not in good general health, or has a condition that would contraindicate study participation.

Patients receiving excluded therapies will be ineligible for study enrollment. If patients receive excluded therapies after enrollment, continuation in the study will be at the discretion of the sponsor/investigator/medical monitor.

Procedure pain will be assessed by asking patients to rate their index knee for pain (1) at rest prior to pre-medication; (2) prior to intra-articular 2% lidocaine (without epinephrine); (3) at rest 10 minutes (± 2 minutes) after intra-articular 2% lidocaine (without epinephrine); (4) at 30 minutes (± 5 minutes) after intra-articular injection of the IMP and (5) at rest at the 1 h and 2 h time points after intra-articular injection of the IMP (± 10 minutes). Categorical scoring will be used: none, mild, moderate, moderately severe, or severe.

An adverse event (AE) is defined as any untoward medical occurrence in a patient or clinical investigation patient administered a pharmaceutical product that does not necessarily have a causal relationship with the product. An AE can therefore be any unfavorable and unintended sign (including a new, clinically important abnormal laboratory finding), symptom, or disease, temporally associated with the product, whether or not related to the product.

Pre-existing diseases or conditions will not be considered AEs unless there is an increase in the frequency or severity, or a change in the quality of the disease or condition (worsening of a pre-existing condition is considered an AE).

Events occurring in patients treated with placebo or active comparator or during treatment-free periods of the study are also considered AEs.

The numbers of patients randomized, completing, and withdrawing, along with reasons for withdrawal, will be tabulated overall and by treatment group. The number of patients in each analysis population will be reported.

Protocol deviations will be identified and classified as minor or major for statistical analysis purposes before unblinding, and will be summarized or listed as appropriate. Critical protocol deviations will be used to exclude patients from the per protocol analysis.

### Evaluation Criteria

Evaluation criteria include a primary efficacy endpoint, second efficacy endpoints, and exploratory efficacy endpoints.

### Primary Efficacy Endpoint

The primary efficacy endpoint is the change from Baseline (from Day -14 through Day -1) to Week 12 (mean of Day 78 through Day 84) in the average pain in the index knee with walking over the previous 24 hours, using the NPRS (0-10, where 0 = "no pain" and 10 = "worst possible pain"), in patients treated with 1.0 mg of *trans*-capsaicin, compared with placebo.

### Secondary Efficacy Endpoints

The secondary efficacy endpoints include the following:
- Area under the curve (AUC) calculated on change from Baseline through Week 12 (Day 84) in the average pain in the index knee with walking over the previous 24 hours, using the NPRS (0-10), in patients treated with 1.0 mg of *trans*-capsaicin, compared with placebo.
- Change from Baseline (pre-dose Day 1) to each study visit through Week 12 in the average function in the index knee, using the WOMAC C function subscale.
- AUC calculated on change from Baseline through Week 26 (Day 182) in the average pain in the index knee with walking over the previous 24 hours, using the NPRS (0-10), in patients treated with 1.0 mg of *trans*-capsaicin, compared with placebo.
- Change from Baseline through Week 26 in the average pain in the index knee with walking over the previous 24 hours, using the NPRS (0-10), in patients treated with 1.0 mg of *trans*-capsaicin, compared with placebo.
- Change from Baseline (pre-dose Day 1) to each study visit through Week 38 and Week 52 in the average pain in the index knee, using the WOMAC A pain subscale.
- Change from Baseline (pre-dose Day 1) to each study visit through Week 52 in the average stiffness in the index knee, using the WOMAC B stiffness subscale.
- PGIC for the index knee for each study visit through Week 52.
- Change from Baseline (pre-dose Day 1) to each study visit through Week 52 in the quality of life (QOL), as measured by the SF-36 Health Survey.
- Change from Baseline (pre-dose Day 1) to each study visit through Week 52 in QOL, as measured by the EQ-5D-5L.
- Change from Baseline through Weeks 38 and 52 in the average pain in the index knee with walking over the previous 24 hours, using the NPRS (0-10), in patients treated with 1.0 mg of trans-capsaicin, compared with placebo.
- Change from Baseline (pre-dose Day 1) to each study visit through Week 52 in the average function in the index knee, using the WOMAC C function subscale.
- Durability of efficacy of a first (up to Week 26) and a second IA injection (up to Week 52) of trans-capsaicin, as measured by the time from Day 1 (Baseline) to the return of Baseline (NPRS pain with walking) pain.
- Cumulative responder analysis for all patients at each study visit. Responders will be defined using 1) percent change from Baseline in the average pain, 2) the absolute change in the average pain from Baseline, 3) the absolute value in NPRS achieved, and 4) the OMERACT-OARSI response at each time point.

The Western Ontario and McMaster Universities developed the WOMAC score in use among patients with knee and/or hip osteoarthritis. The WOMAC is a validated, multidimensional measure of pain, stiffness, and physical functional disability that is sensitive to the effects of drugs or other intervention. It is available in 5-point Likert, 100 mm Visual Analogue and 11-box Numerical Rating Scale formats. This study will use the 11-box Numerical Pain Rating Scale (NPRS) format. The NPRS uses end points of 0="None" (or "No pain/stiffness/difficulty") and 10="Extreme."

The WOMAC is a self-administered index and contains 24 items asked in relation to the index knee joint. The WOMAC has 3 dimensions: Pain (Section A), Stiffness (Section B), and Physical Function (Section C).
- The pain section includes 5 items about the amount of pain experienced doing various activities (walking, stair climbing, nocturnal, at rest, weight bearing).
- The stiffness section has 2 items: joint stiffness upon wakening and joint stiffness later in the day.
- The function section asks about the degree of difficulty in doing 17 activities due to the reference joint (descending stairs, ascending stairs, rising from sitting position, standing, bending to the floor, walking on a flat surface, getting into or out of a car, going shopping, putting on socks, lying in bed, taking off socks, rising from bed, getting into or out of the bath, sitting, getting onto or off the toilet, heavy domestic duties, and light domestic duties).

The Patient Global Impression of Change (PGIC) in Knee Pain is a 7-point categorical scale, where the patient rates their change in overall status compared to Baseline as follows: 1 = Very much improved; 2 = Much improved; 3 = Minimally improved; 4 = No change; 5 = Minimally worse; 6 = Much worse; and 7 = Very much worse.

The KOOS was developed as an extension of the WOMAC Osteoarthritis Index with the purpose of evaluating short-term and long-term symptoms and function in patients with knee injury and osteoarthritis. The KOOS holds 5 separately scored subscales: Pain, other symptoms, Function in Daily Living (ADL), Function in Sport and Recreation (Sport/Rec), and knee-related Quality of Life (QOL). The KOOS has been validated for several orthopedic interventions such as anterior cruciate ligament reconstruction, meniscectomy, and total knee replacement. In addition, the instrument has been used to evaluate physical therapy, nutritional supplementation, and glucosamine supplementation. The effect size is generally largest for the subscale QOL followed by the subscale Pain. The KOOS is a valid, reliable, and responsive self-administered instrument that can be used for short-term and long-term follow-up of several types of knee injury, including osteoarthritis.

Intended to assess the extent of sleep problems, the Medical Outcomes Study (MOS) Sleep Scale measures 6 dimensions of sleep, including initiation, maintenance (i.e., staying asleep), quantity, adequacy, somnolence (i.e., drowsiness), and respiratory impairments (e.g., shortness of breath, snoring). Disturbed sleep has a major impact on quality of life and is often a common symptom of many other chronic conditions, such as chronic pain and mood disorders.

### Exploratory Efficacy Endpoints

Exploratory efficacy endpoints in patients treated with 1.0 mg of trans-capsaicin, compared with placebo, include:
- OMERACT-OARSI score responders at each study visit through Week 52
- Change from Baseline (pre-dose Day 1) to each study visit through Week 52 in quality of sleep, as measured by the MOS sleep scale
- KOOS at each study visit through Week 52
- Change from Baseline (pre-dose Day 1) through Week 52 in weight and BMI
- Change from Baseline (pre-dose Day 1) through Week 52 in abdominal girth
- Likelihood of patient need for joint replacement surgery, based on pain, function, and other patient-reported outcomes from Baseline through Week 52
- Effect on the Work Productivity and Activity Impairment Scale (WPAI)
- The effect of K-L grade, sex, BMI and age on the analgesic efficacy of trans-capsaicin, measured as the average pain in the index knee with walking, using the NPRS (0-10), (mean of daily pain scores for each week) through Week 52. Additionally, patients with radiographic evidence of osteoarthritis in the non-index (opposite) knee (K-L=2, 3, or 4) vs patients with no radiographic evidence of osteoarthritis in the non-index knee (K-L=0 or 1) will be analyzed
- Frequency of use of rescue and background analgesic medication for the index knee pain throughout the study period
- Patient's guess as to which treatment they think they received

### Safety Endpoints

Safety endpoints in patients treated with trans-capsaicin, include:
- AEs
- Vital signs
- Clinical laboratory evaluations (hematology, chemistry, and urinalysis)
- 12-lead electrocardiograms (ECG)
- Physical examination (including the presence or absence of an effusion in the index knee, periarticular pain/tenderness)
- Sensory testing using a 5-point Likert scale
- Concomitant medications and therapies
- Degree of procedure pain (not recorded as AEs)
- Local physical findings after injection of the index knee
- Injection site assessment of erythema and edema.
- Stability of the radiographic findings, using OARSI score, of knee radiographs from Baseline to Week 52.

### Statistical Analysis

Statistical analysis of the results may be performed according to the following procedure. For the primary analysis of the change from Baseline in average pain in the index knee with walking at Week 12, and for secondary analyses at time points through Week 52, a mixed-model repeated measures (MMRM) analysis will be conducted as the primary analysis using the Intent-to-Treat (ITT) population. Results from the MMRM primary analysis will be used to determine study success.

The MMRM model will include terms for pooled site, K-L grade, BMI, sex, treatment, visit, treatment by visit interaction, and baseline average daily osteoarthritis knee pain score with walking as a covariate. Least squares estimates of the treatment differences at Week 12 (and secondary time points), along with 95% confidence intervals, will be derived from this model and reported.

The analysis for the first secondary endpoint is the AUC calculated on change from Baseline through Week 12 (Day 84) in the average pain in the index knee with walking over the previous 24 hours, using the NPRS (0-10), in patients treated with 1.0 mg of *trans-*capsaicin, compared with placebo, analyzed using an analysis of covariance based on the ITT population.

To control for the overall type I error rate, hierarchical testing, based on a fixed sequence procedure, will be used for the primary endpoint and continuing for the secondary endpoints. The MMRM primary analysis will start the hierarchal testing. If statistical significance is declared for the primary MMRM analysis, formal hypothesis testing will be done for the second primary endpoint (ANCOVA analysis on the AUC), and all secondary endpoints in sequence until a non-significant result is reached. The sequence for remaining secondary endpoints will be specified in the statistical analysis plan (SAP). All other p values from secondary and exploratory endpoints, after a non-significant p value is reached, will be considered nominal. The primary and first secondary analysis are powered to detect a statistical significant difference. The primary and secondary endpoints follow a hierarchal sequence to meet the regulatory requirements.

A sensitivity analysis of the primary endpoint will be performed on the per-protocol (PP) population. Further sensitivity analyses on the primary endpoint to explore the impact of missing data will be performed and will be described in a detailed statistical analysis plan (SAP).

Descriptive summaries (such as mean, standard error, median, minimum, and maximum) will also be provided for the primary endpoint.

All other continuous secondary and exploratory efficacy endpoint will be summarized using descriptive statistics by treatment group and week/visit, as appropriate, and analyzed using an MMRM analysis or by analysis of covariance, as appropriate. Categorical endpoints will be compared between treatments using Pearson's chi-square or Fisher's exact test, as appropriate.

Safety analyses will be conducted using data from the safety population. No formal inferential analyses will be conducted for safety variables unless otherwise noted.

### EXAMPLE 2 - INTRA-ARTICULAR INJECTION OF CAPSAICIN TO TREAT OSTEOARTHRITIC KNEE JOINT PAIN IN HUMAN PATIENTS

Human patients experiencing osteoarthritic knee joint pain received either placebo, a 0.5 mg intra-articular injection of capsaicin into the osteoarthritic knee joint, or a 1.0 mg intra-articular injection of capsaicin into the osteoarthritic knee joint. Transient burning sensation due to administration of capsaicin was attenuated by administering capsaicin according to the following procedure: (i) applying for a duration of about 15 minutes a cooling article to an exterior surface of a human patient's knee presenting with osteoarthritic knee joint pain, wherein the cooling article was a Breg cooling wrap cooled by circulating ice-cold water, (ii) administering by injection into the intra-articular space of the joint of the knee a 15 mL aliquot of a 2% w/w lidocaine solution in saline, (iii) applying for a duration of about 30 minutes a cooling article to an exterior surface of the knee, wherein the cooling article was a Breg cooling wrap cooled by circulating ice-cold water, (iv) administering by injection into the intra-articular space of the joint of the knee a 4 mL aliquot of a solution containing water, polyethylene glycol having a number-average molecular weight of about 300 g/mol, and for patients receiving capsaicin a dose of capsaicin in an amount of 0.5 mg or 1.0 mg, and (v) applying a cooling article to an exterior surface of the knee for a duration of at least about 30 minutes (and up to 60 minutes upon patient request), wherein the cooling article was a Breg cooling wrap cooled by circulating ice-cold water. Further description of experimental procedures and results are provided below.

### Experimental Procedures

Human patients aged 45-80 years with chronic knee osteoarthritis, stable moderate to severe pain, and intolerability to analgesics were randomized to receive a single intra-articular injection of placebo into the osteoarthritic knee, a single intra-articular injection of a 0.5 mg dose of capsaicin into the osteoarthritic knee, or a single intra-articular injection of a 1.0 mg dose of capsaicin into the osteoarthritic knee.

The test article (i.e., placebo or capsaicin) was administered according to the following procedure: (i) applying for a duration of about 15 minutes a cooling article to an exterior surface of a human patient's knee presenting with osteoarthritic knee joint pain, wherein the cooling article was a Breg cooling wrap cooled by circulating ice-cold water, (ii) administering by injection into the intra-articular space of the joint of the knee a 15 mL aliquot of a 2% w/w lidocaine solution in saline, (iii) applying for a duration of about 30 minutes a cooling article to an exterior surface of the knee, wherein the cooling article was a Breg cooling wrap cooled by circulating ice-cold water, (iv) administering by injection into the intra-articular space of the joint of the knee a 4 mL aliquot of a solution containing water, polyethylene glycol having a number-average molecular weight of about 300 g/mol, and for patients receiving capsaicin a dose of capsaicin in an amount of 0.5 mg or 1.0 mg, and (v) applying a cooling article to an exterior surface of the knee for a duration of at least about 30 minutes (and up to 60 minutes upon patient request), wherein the cooling article was a Breg cooling wrap cooled by circulating ice-cold water.

Safety assessments included treatment-emergent adverse events (TEAEs), serious AEs (SAEs), laboratory abnormalities, and procedural pain ratings (none, mild, moderate, moderately severe, severe). The procedure pain ratings characterize the extent of transient burning sensation experienced by patients due to administration of capsaicin.

Patients completed Patient Global Impression of Change (PGIC; change vs baseline in index knee on 7-point scale: 1=very much improved; 7=very much worse, with scores of 1 or 2 indicating significant improvement) and Patient-specific Functional Scale (PSFS; rate ≤3 important activities difficult to perform due to index knee pain on 0-10 scale: 0=able to perform; 10=unable to perform) at scheduled visits. Odds ratios for PGIC scores were calculated with chi-square tests. Changes in Western Ontario and McMaster Universities Osteoarthritis Index (WOMAC) B stiffness subscale and WOMAC C function subscale through week 24 were also assessed. PSFS, WOMAC B, and WOMAC C scores were evaluated using a mixed model for repeated measures. Statistical tests were 2-sided (alpha, P=0.10).

### Results - Safety

The safety population included 175 patients (placebo, n=70; capsaicin at 0.5 mg dose, n=34; and capsaicin at 1.0 mg dose, n=71). TEAEs were reported by 21 (30%), 16 (47%), and 21 (30%) patients in the placebo, capsaicin 0.5 mg dose, and capsaicin 1.0 mg dose groups, respectively, and were mild (19%, 29%, 20%) or moderate (11%, 18%, 9.9%) in severity. The most common TEAEs with capsaicin, regardless of dose, were arthralgia (7.6% vs 5.7% placebo) and upper respiratory tract infection (4.8% vs 4.3%). One SAE (shoulder pain from osteoarthritis) in the capsaicin 0.5 mg dose group was not considered treatment related. Few laboratory abnormalities were observed, with most being mild and associated with comorbidities.

Most patients had moderate pain at rest before injection of capsaicin (or placebo). At 2 hours after injection of capsaicin (or placebo), most patients in all groups reported no (50%) or mild (39%) pain.

Incidence of TEAEs was 30% for placebo, 47% for capsaicin at a 0.5 mg dose, and 30% for capsaicin at a 1.0 mg dose, with most mild or moderate in severity and unrelated to treatment. The most common TEAE with the capsaicin 1.0 mg dose was arthralgia (placebo, 5.7%; capsaicin 1.0 mg dose, 7.0%). The following table lists the most commonly reported TEAE through 24 weeks of treatment.

**Table. Most Commonly Reported TEAEs Through 24 Weeks of Treatment**

| **TEAE, n (%)** | **Placebo (n=70)** | **Capsaicin 0.5 mg Dose (n=34)** | **Capsaicin 1.0 mg Dose (n=71)** | **Capsaicin at Either 0.5 mg or 1.0 Dose (n=105)** |
|---|---|---|---|---|
| **All TEAEs** | 21 (30) | 16 (47) | 21 (30) | 37 (35) |
| Arthralgia | 4 (5.7) | 3 (8.8) | 5 (7.0) | 8 (7.6) |
| Upper respiratory tract infection | 3 (4.3) | 2 (5.9) | 3 (4.2) | 5 (4.8) |
| Influenza | 3 (4.3) | 1 (2.9) | 2 (2.8) | 3 (2.9) |
| Nasopharyngitis | 2 (2.9) | 1 (2.9) | 2 (2.8) | 3 (2.9) |
| Joint effusion | 0 | 3 (8.8) | 0 | 3 (2.9) |
| Hepatic enzyme increased | 0 | 2 (5.9) | 1 (1.4) | 3 (2.9) |

The results demonstrate that a single injection of capsaicin at a 1.0 mg dose was well tolerated with a safety profile generally comparable to placebo for up to 24 weeks.

### Results - Efficacy in Treating Osteoarthritic Knee Joint Pain

Efficacy was evaluated in 172 patients (placebo, n=69; capsaicin at 0.5 mg dose, n=33; and capsaicin at 1.0 mg dose, n=70). Based on PGIC, patients receiving a 1.0 mg dose of capsaicin had a greater than two-fold likelihood of having "significant improvement" (ratings of "very much improved" or "much improved") at weeks 4, 8, and 12 compared with placebo. At week 4, greater proportions of patients reported "significant improvement" in the index knee with the 0.5 mg dose capsaicin (58%; *P=*0.18) or 1.0 mg dose of capsaicin (64%; P=0.01) versus placebo (44%); similar findings were observed at weeks 12 and 24.

On PSFS, a 1.0 mg dose of capsaicin significantly improved patients' ability to perform activities versus placebo at weeks 4-16. A 1.0 mg dose of capsaicin significantly improved WOMAC B score (least squares mean difference [LSMD]: -2.1; P=0.007) and WOMAC C score (LSMD: -14.8; P=0.02) versus placebo through week 16. Tabulated results are provided in the following Table.

**Table. Effect of Capsaicin Injection vs Placebo on Patient Global Impression of Change (PGIC) and Patient-specific Functional Scale (PSFS) Through 24 Weeks of Treatment**

| **Week No.** | **PGIC: Odds Ratio vs Placebo (*P* value)^{a}** | | **PSFS: LSMD versus Placebo (P value)** | |
|---|---|---|---|---|
| | **Capsaicin at 0.5 mg Dose** | **Capsaicin at 1.0 mg Dose** | **Capsaicin at 0.5 mg Dose** | **Capsaicin at 1.0 mg Dose** |
| 4 | 1.76 (*P*=0.18) | 2.34 *(P=*0.01) | 0.01 (*P*=0.98) | -1.4 (*P*=0.0006) |
| 8 | 1.89 *(P=*0.14) | 2.50 (*P*=0.008) | -0.03 (*P*=0.96) | -0.9 *(P*=0.03*)* |
| 12 | 2.75 *(P=*0.03) | 2.40 *(P=*0.01) | -0.4 (*P*=0.40) | -1.1 *(P=0.007)* |
| 16 | 2.37 *(P=*0.05) | 1.85 *(P=*0.07) | *-0.5 (P=*0.35*)* | -0.9 (*P*=0.04) |
| 24 | 1.68 *(P=*0.23) | 1.64 *(P=*0.15) | -0.05 (*P*=0.93) | -0.5 *(P=*0.27) |

| | | | | |
|---|---|---|---|---|
| ^{a} Odds Ratio (OR) for significant improvement (scores of 1 [very much improved] or 2 [much improved] on PGIC). LSMD, least squares mean difference; OR, odds ratio. Prespecified alpha for significance set at 0.1. | | | | |

The results demonstrate that a single injection of capsaicin at a 1.0 mg dose produced statistically significant improvements in physical function for 12-16 weeks and numerically greater improvements at 24 weeks versus placebo in patients with moderate to severe osteoarthritis knee pain.

### EXAMPLE 3 - CAPSAICIN AQUEOUS FORMULATIONS CONTAINING A SOLUBILIZING AGENT

Multiple aqueous formulations were prepared and analyzed to determine the amount of dissolved capsaicin. The formulations contained different solubilizing agents to increase the amount of capsaicin dissolved in the aqueous medium. The experimental procedures and results are described below.

### Part I - Analysis of Capsaicin Solubility in Multiple Aqueous Formulations

Aqueous formulations were prepared containing capsaicin and a solubilizing agent selected from Tween 20, Tween 80, Kolliphor ELP, Kolliphor HS 15, Kollidon 12 PF, and Kollidon 17 PF as further defined below. Experimental procedures and results are described below.

### Experimental Procedures

The equilibrium solubility of capsaicin was determined in a series of aqueous solutions. Six different types of vehicles were prepared at three different concentrations each. Tween 20 solutions were prepared at a range of 0.2% to 10% (w/v). Tween 80 solutions were prepared at a range of 0.2% to 1.0% (w/v). Kolliphor ELP and Kolliphor HS 15 solutions were both prepared at a range of 5% to 20% (w/v). Kollidon 12 PF solutions were prepared at a range of 2.5% to 10% (w/v). Kollidon 17 PF solutions were prepared at a range of 0.5% to *2.0% (w*/*v).*

For each test solution, quantities of 20-30 mg of capsaicin were added to a micro centrifuge tubes. A volume of 1.5 mL of the appropriate test vehicle was added to each to create a suspension. The capped tubes were mixed on a laboratory rotator at ambient temperature. At approximately 48 hours after sample preparation, the tubes were removed from the rotator and centrifuged to separate the solid phase from the solution. An aliquot of the supernatant was withdrawn from each sample and diluted as necessary for HPLC analysis to determine the solution concentration of the capsaicin. The pH of the supernatant was measured 48 hours after preparation and the appearance of solid and supernatant were noted.

As reported in the literature, Tween 20 is also known as Polysorbate 20, which has the chemical name polyoxyethylene (20) sorbitan monolaurate. Tween 80 is also known as Polysorbate 80, which has the chemical name polyoxyethylene (20) sorbitan monooleate. Kolliphor ELP has CAS Registry No. 61791-12-6, and is a composition sold by BASF under the chemical name polyoxyl-35-castor oil and marketed by BASF as Kolliphor^{™} ELP; the composition is made by reacting castor oil with ethylene oxide in a molar ratio of 1:35. The Kolliphor HS 15 has CAS Registry No. 70142-34-6, and is a mixture containing about (a) about 70% (w/w) of a mixture of 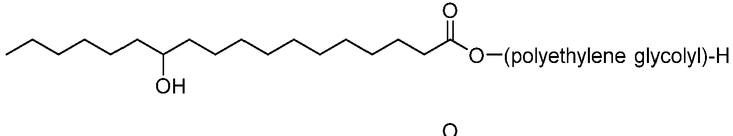 and 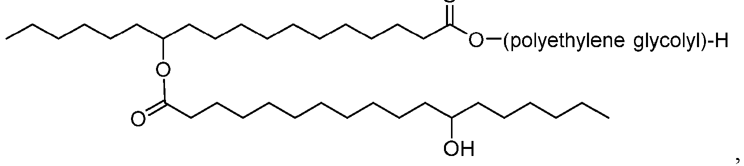 and (b) about 30% (w/w) polyethylene glycol; where the polyethylene glycolyl has a weight-average molecular weight of about 660 g/mol; which is sold and marketed by BASF as Kolliphor^{®} HS 15. Kollidon 12 PF is a polyvinylpyrrolidone having a weight-average molecular weight in the range of 2,000 to 3,000 g/mol, sold by BASF under the name Kollidon^{®} 12 PF. Kollidon 17 PF is a polyvinylpyrrolidone having a weight-average molecular weight in the range of 7,000 to 11,000 g/mol, sold by BASF under the name Kollidon^{®} 17 PF.

### Results

Results from the above analysis are presented in Table 1. For all test solutions except those containing Kollidon12 PF or Kollidon17 PF, the observed concentration of capsaicin increased concordant with increasing surfactant concentration. With the exception of Kollidon 12 PF and Kollidon 17 PF, at least one test solution from each of different solubilizing agents reached the minimum target concentration of capsaicin of 1 mg/mL capsaicin. Both Kollidon 12 PF and Kollidon 17 PF solutions, at all strengths, failed to reach the minimum target concentration of 1 mg/mL capsaicin. The highest concentrations of capsaicin were observed in the 20% strength Kolliphor ELP {c(Capsaicin) = 13.0 mg/mL} and 20% Kolliphor HS 15 {c(Capsaicin) = 12.2 mg/mL} solutions.

The observed pH-values in the supernatants of the test solutions ranged from pH = 3.88 to pH = 7.27. Appearances of both the liquid supernatant and the remaining solid were observed to be clear and as at initial solution preparation. For all samples that had remaining solid, the solid appeared white and had the no notable difference from its starting consistency.

After centrifugation of the sample containing 20% Kolliphor ELP no solid residue could be detected, which signifies that the equilibrium solubility for Capsaicin in this vehicle was not reached and is greater than the observed c(Capsaicin) = 13.0 mg/mL. For the 20% Kolliphor HS vehicle, the amount of pelleted solid from centrifugation was at the limit of detection.

**TABLE 1.**

| **Sample (amount in weight percent)** | **Observed [Capsaicin] (mg/mL)** | **pH (at 48 hr)** | **appearance pellet** | **appearance supernatant** |
|---|---|---|---|---|
| Tween 20 (0.2%) | 0.146 | 6.78 | white | clear |
| Tween 20 (2%) | 1.11 | 6.16 | white | clear |
| Tween 20 (10%) | 5.39 | 6.03 | white | clear |
| Tween 80 (0.2%) | 0.233 | 6.45 | white | clear |
| Tween 80 (0.5%) | 0.245 | 7.27 | white | clear |
| Tween 80 (1.0%) | 1.00 | 7.03 | white | clear |
| Kolliphor ELP (5%) | 4.20 | 5.61 | white | clear |
| Kolliphor ELP (10%) | 8.14 | 5.21 | white | clear |
| Kolliphor ELP (20%) | 13.0 | 4.70 | none | clear |
| Kolliphor HS 15 (5%) | 3.81 | 6.65 | white | clear |
| Kolliphor HS 15 (10%) | 7.18 | 6.97 | white | clear |
| Kolliphor HS 15 (20%) | 12.2 | 7.01 | white | clear |
| Kollidon 12 (2.5%) | 0.276 | 4.22 | white | clear |
| Kollidon 12 (5%) | 0.624 | 4.00 | white | clear |
| Kollidon 12 (10%) | 0.378 | 3.88 | white | clear |
| Kollidon 17 (0.5%) | 0.150 | 5.75 | white | clear |
| Kollidon 17 (1.0%) | 0.247 | 4.66 | white | clear |
| Kollidon 17 (2.0%) | 0.199 | 4.20 | white | clear |

### Part II - Capsaicin Solubility in Cyclodextrin Solutions

Aqueous formulations were prepared containing capsaicin and a solubilizing agent selected from hydroxypropyl-β-cyclodextrin and captisol (i.e., sodium sulfobutyl ethers β-cyclodextrin). Experimental procedures and results are described below.

### Experimental Procedures

For each cyclodextrin solution, quantities of about 20-30 mg of capsaicin were suspended in 1.5 mL of the respective cyclodextrin solution. The capped tubes were mixed on a laboratory rotator at ambient temperature. At approximately 48 hours after sample preparation, the tubes were removed from the rotator and centrifuged to separate the solid phase from the solution. An aliquot of the supernatant was withdrawn from each sample and diluted as necessary for HPLC analysis to determine the solution concentration of the capsaicin, which was quantitated relative to the reference standard. The pH of the supernatant was measured and the appearance of both the supernatant and the solid were noted at 48 hours.

### Results

Results from the above analysis are presented in Table 2. For both cyclodextrins tested, at all solution strengths, at least 2 mg/mL capsaicin was observed. Hydroxypropyl-β-cyclodextrin had slightly higher concentrations of capsaicin than captisol for all solution strengths. The pH of the solutions ranged from 7.00 to 7.94. The liquid portion of each sample was clear and appeared unchanged from its original state. The solid portion of each sample was white, granular, and appeared as it did prior to the addition of the cyclodextrin solution. The 25% solutions of both cyclodextrins had very little remaining solid.

**TABLE 2.**

| **Sample** | **Peak area (mAU)** | **Observed [Capsaicin], mg/mL** | **appearance pellet** | **appearance supernatant** | **pH (at 48 hr)** |
|---|---|---|---|---|---|
| 5% Hydroxypropyl-β-cyclodextrin | 4247905 | 2.39 | White | clear | 7.32 |
| 10% Hydroxypropyl-β-cyclodextrin | 7891725 | 4.45 | White | clear | 7.44 |
| 25% Hydroxypropyl-β-cyclodextrin | 20541037 | 11.6 | White | clear | 7.00 |
| 5% Captisol | 3734548 | 2.10 | White | clear | 7.94 |
| 10% Captisol | 6561988 | 3.70 | White | clear | 7.65 |
| 25% Captisol | 14660216 | 8.26 | White | clear | 7.23 |

### Part III - Capsaicin Solubility in Additional Aqueous Solutions

Aqueous formulations were prepared containing capsaicin and an additive. The solubility of capsaicin was also analyzed in deionized water. Experimental procedures and results are described below.

### Experimental Procedures

For each of the six solutions, quantities of about 20-30 mg of capsaicin were added to each of six micro centrifuge tubes. A volume of 1.5 mL of the appropriate solution was added to each to create a suspension. The capped tubes were mixed on a laboratory rotator at ambient temperature. At approximately 7 days after sample preparation, the tubes were removed from the rotator and centrifuged to separate the solid phase from the solution. An aliquot of the supernatant was withdrawn from each sample and diluted as necessary for HPLC analysis to determine the solution concentration of the capsaicin, which was quantitated relative to the reference standard. The pH-values of the supernatant were measured and the appearance of both the supernatant and the pelleted solid were noted.

### Results

Results from the above analysis are presented in Table 3. The lowest concentration of the capsaicin was observed in deionized water with c(Capsaicin) = 7.6 µg/mL while solubilization of capsaicin in aqueous 2.5% glycerol resulted in the highest observed concentration of capsaicin with c(Capsaicin) = 38 µg/mL.

**TABLE 3.**

| **Sample** | **Peak area (mAU)** | **Observed [Capsaicin], mg/mL** | **appearance pellet** | **appearance supernatant** | **pH (at 7 days)** |
|---|---|---|---|---|---|
| Water | 135565 | 0.008 | White | clear | 4.53 |
| 5% mannitol | 381253 | 0.021 | White | clear | 5.53 |
| 5% mannitol, 0.1M pH 5 Citrate | 513817 | 0.020 | White | clear | 4.73 |
| 5% mannitol, 0.1M pH 6 Citrate | 378148 | 0.021 | White | clear | 5.86 |
| 5% mannitol, 0.1M pH 5 Acetate | 484164 | 0.027 | White | clear | 5.25 |
| 2.5% glycerol in water | 682320 | 0.038 | White | clear | 6.47 |

### EXAMPLE 4 - PREPARATION OF ADDITIONAL EXEMPLARY CAPSAICIN AQUEOUS FORMULATIONS

Three additional exemplary stable aqueous capsaicin injectable formulations were prepared. Experimental procedures and results are provided below.

### Part I - Preparation of First Exemplary Additional Formulation

The formulation listed in the table below was prepared by the following procedure:
(a) Place 900 mL of water in a vessel;
(b) Add 6.80 grams of sodium acetate to the vessel containing water;
(c) Adjust solution pH to 5.5 by adding 1N HCl;
(d) Add 10.0 grams of Kolliphor HS 15 to the solution [the Kolliphor HS 15 has CAS Registry No 70142-34-6, and is a mixture containing (a) about 70% (w/w) of a mixture of 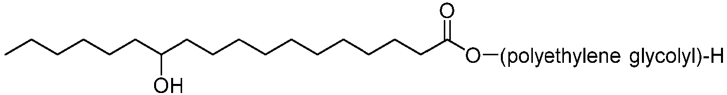 and 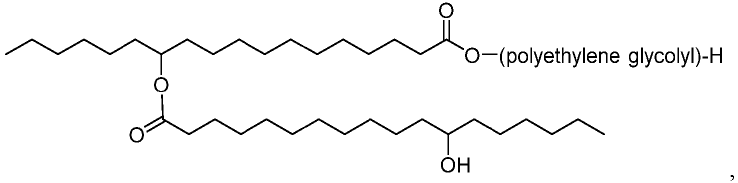 and (b) about 30% (w/w) polyethylene glycol; where the polyethylene glycolyl has a weight-average molecular weight of about 660 g/mol; which is sold and marketed by BASF as Kolliphor^{®} HS 15];
(e) Add 0.10 grams of dibutylhydroxytoluene to the solution, and let the solution age for at least 2 hours;
(f) Add 0.25 grams of ethylenediaminetetraacetic acid tetrasodium salt to the solution;
(g) Add 0.50 grams of capsaicin to the solution, and age the solution until capsaicin dissolves;
(h) Add 6.0 grams of NaCl to the solution;
(i) Adjust pH of the solution to pH = 5.5 by adding 1N HCl or 1N NaOH as needed;
(j) qs. with water so the volume of the solution reaches 1 liter; and
(k) Sterile filter the solution.

| **Formulation** | |
|---|---|
| An aqueous, capsaicin injectable formulation, comprising: | |
| | a. 0.05% (w/w) of trans-capsaicin; |
| | b. 1% (w/w) of a solubilizing agent, wherein the solubilizing agent is a mixture of |
| | 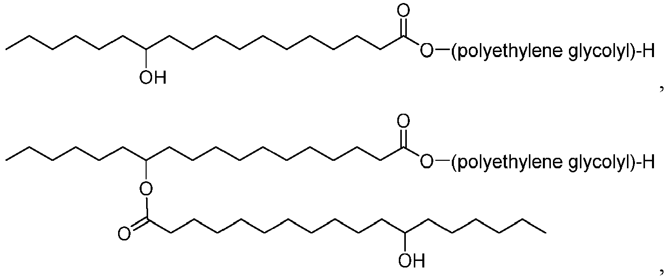 |
| | and polyethylene glycol; wherein the polyethylene glycolyl has a weight average molecular weight of about 660 g/mol; |
| | c. 0.01% (w/w) dibutylhydroxytoluene; |
| | d. 0.68% (w/w) of sodium acetate or a mixture of sodium acetate and acetic acid; |
| | e. 0.025% (w/w) of ethylenediaminetetraacetic acid or a salt thereof; |
| | f. 0.6% (w/w) of sodium chloride; |
| | g. qs. with water (i.e., least 97.6% (w/w)); and |
| having a pH of 5.5. | |

### Part II - Preparation of Second Exemplary Additional Formulation

The formulation listed in the table below was prepared by the following procedure:
(a) Place 900 mL of water in a vessel;
(b) Add 3.40 grams of sodium acetate to the vessel containing water;
(c) Adjust solution pH to 5.5 by adding 1N HCl;
(d) Add 10.0 grams of Kolliphor HS 15 to the solution [the Kolliphor HS 15 has CAS Registry No 70142-34-6, and is a mixture containing (a) about 70% (w/w) of a mixture of 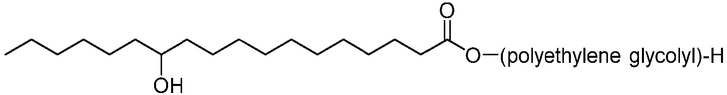 and 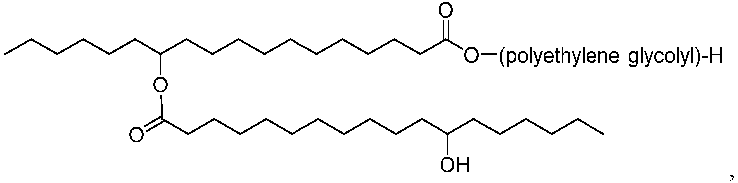 and (b) about 30% (w/w) polyethylene glycol; where the polyethylene glycolyl has a weight-average molecular weight of about 660 g/mol; which is sold and marketed by BASF as Kolliphor^{®} HS 15];
(e) Add 0.10 grams of dibutylhydroxytoluene to the solution, and let the solution age for at least 2 hours;
(f) Add 0.25 grams of ethylenediaminetetraacetic acid tetrasodium salt to the solution;
(g) Add 0.50 grams of capsaicin to the solution, and age the solution until capsaicin dissolves;
(h) Add 7.5 grams of NaCl to the solution;
(i) Adjust pH of the solution to pH = 5.5 by adding 1N HCl or 1N NaOH as needed;
(j) qs. with water so the volume of the solution reaches 1 liter; and
(k) Sterile filter the solution.

| **Formulation** | |
|---|---|
| An aqueous, capsaicin injectable formulation, comprising: | |
| | a. 0.05% (w/w) of *trans*-capsaicin; |
| | b. 1% (w/w) of a solubilizing agent, wherein the solubilizing agent is a mixture of |
| | 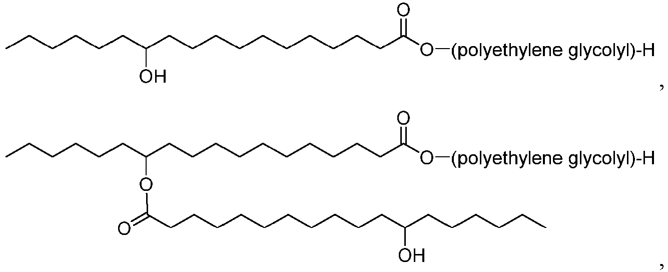 |
| | and polyethylene glycol; wherein the polyethylene glycolyl has a weight average molecular weight of about 660 g/mol; |
| | c. 0.01% (w/w) dibutylhydroxytoluene; |
| | d. 0.34% (w/w) of sodium acetate or a mixture of sodium acetate and acetic acid; |
| | e. 0.025% (w/w) of ethylenediaminetetraacetic acid or a salt thereof; |
| | f. 0.75% (w/w) of sodium chloride; |
| | g. qs. with water (i.e., least 97.8% (w/w)); and |
| having a pH of 5.5. | |

### Part III - Preparation of Third Exemplary Additional Formulation

The formulation listed in the table below was prepared by the following procedure:
(a) Place 900 mL of water in a vessel;
(b) Add 2.2 grams of trisodium citrate dihydrate to the vessel containing water;
(c) Adjust solution pH to 5.5 by adding 1N HCl;
(d) Add 10.0 grams of Kolliphor HS 15 to the solution [the Kolliphor HS 15 has CAS Registry No 70142-34-6, and is a mixture containing (a) about 70% (w/w) of a mixture of 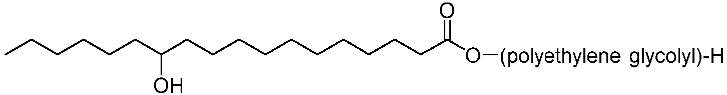 and 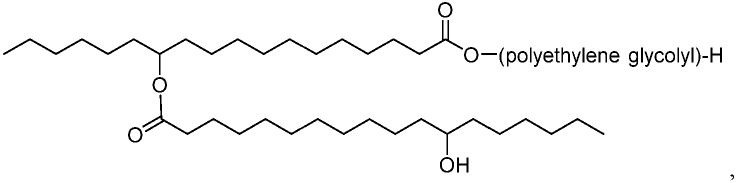 and (b) about 30% (w/w) polyethylene glycol; where the polyethylene glycolyl has a weight-average molecular weight of about 660 g/mol; which is sold and marketed by BASF as Kolliphor^{®} HS 15];
(e) Add 0.10 grams of dibutylhydroxytoluene to the solution, and let the solution age for at least 2 hours;
(f) Add 0.25 grams of ethylenediaminetetraacetic acid tetrasodium salt to the solution;
(g) Add 0.50 grams of capsaicin to the solution, and age the solution until capsaicin dissolves;
(h) Add 8.0 grams of NaCl to the solution;
(i) Adjust pH of the solution to pH = 5.5 by adding 1N HCl or 1N NaOH as needed;
(j) qs. with water so the volume of the solution reaches 1 liter; and
(k) Sterile filter the solution.

| **Formulation** | |
|---|---|
| An aqueous, capsaicin injectable formulation, comprising: | |
| | a. 0.05% (w/w) of *trans*-capsaicin; |
| | b. 1% (w/w) of a solubilizing agent, wherein the solubilizing agent is a mixture of |
| | 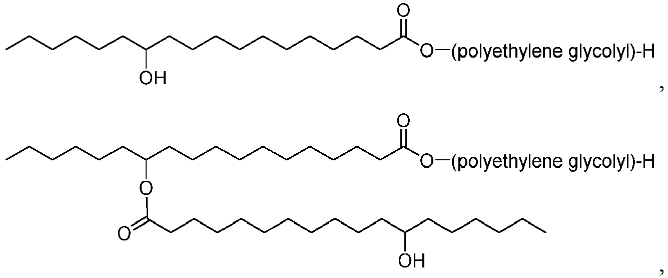 |
| | and polyethylene glycol; wherein the polyethylene glycolyl has a weight average molecular weight of about 660 g/mol; |
| | c. 0.01% (w/w) dibutylhydroxytoluene; |
| | d. 0.22% (w/w) of sodium citrate or a mixture of sodium citrate and citric acid; |
| | e. 0.025% (w/w) of ethylenediaminetetraacetic acid or a salt thereof; |
| | f. 0.8% (w/w) of sodium chloride; |
| | g. qs. with water (i.e., 97.9% (w/w) water); and |
| having a pH of 5.5. | |

### EXAMPLE 5 - PREPARATION OF ADDITIONAL EXEMPLARY CAPSAICIN AQUEOUS FORMULATIONS

The exemplary aqueous capsaicin formulations listed in Table 1 below were prepared. The abbreviation BHT refers to dibutylhydroxytoluene. The abbreviation "EDTA" refers to ethylenediaminetetraacetic acid. The Kolliphor HS-15 has CAS Registry No 70142-34-6, and is a mixture containing (a) about 70% (w/w) of a mixture of 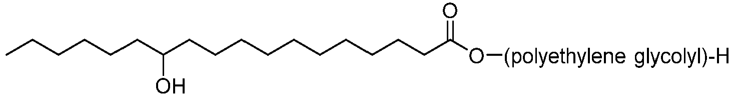 and 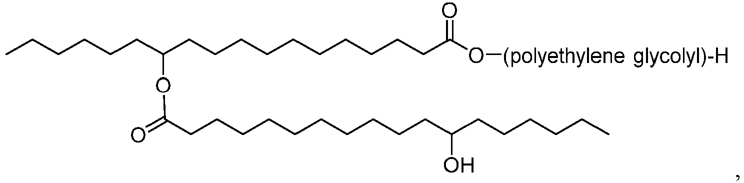 and (b) about 30% (w/w) polyethylene glycol; where the polyethylene glycolyl has a weight-average molecular weight of about 660 g/mol; which is sold and marketed by BASF as Kolliphor^{®} HS 15.

**TABLE 1.**

| **Solution 1A:** | **Solution 1P:** |
|---|---|
| 1 mg/ml Capsaicin | 2% Kolliphor HS-15 |
| 2% Kolliphor HS-15 | |
| 20 mM citrate buffer | 20 mM citrate buffer |
| 0.1% disodium EDTA | 0.1% disodium EDTA |
| 0.01% BHT | 0.01% BHT |
| 0.625% NaCl | 0.625% NaCl |

| **Solution 2A:** | **Solution 3A:** |
|---|---|
| 2 mg/ml Capsaicin | 1 mg/ml Capsaicin |
| 4% Kolliphor HS-15 | |
| 20 mM citrate buffer | 2% Kolliphor HS-15 |
| 0.1% disodium EDTA | 0.1% disodium EDTA |
| 0.01% BHT | 0.01% BHT |
| 0.625% NaCl | 3.15% Dextrose |

| **Solution 3P:** | **Solution 4A:** |
|---|---|
| 2% Kolliphor HS-15 | 2 mg/ml Capsaicin |
| 20 mM citrate buffer | 4% Kolliphor HS-15 |
| 0.1% disodium EDTA | 20 mM citrate buffer |
| 0.01% BHT | 0.1% disodium EDTA |
| 3.15% Dextrose | 0.01% BHT |
| | 3.15% Dextrose |

### EXAMPLE 6 - ANALYSIS OF HUMAN KNEE TEMPERATURE DURING COOLING

Human patients were subjected to cooling of the knee joint using two different cooling methodologies. A temperature probe was placed into the intraarticular space of the patient's knee joint to measure intraarticular temperature of the knee joint. A temperature probe was also placed on the skin in the area to be cooled in order to measure skin temperature in the area to be cooled. The first cooling methodology tested was a Breg Knee WrapOn Polar Pad (as illustrated in Figure 1) that utilizes circulating ice-water to achieve cooling. The second cooling methodology tested was ice-pack cooling, in which the patient's knee was wrapped with a stockinette and then the ice pack was positioned on top of the stockinette so that the ice pack is positioned over the patient's patella; the ice pack is then secured in place using an elasticated bandage. The ice pack was a LEADSTAR pain relief reusable cold therapy ice pack (size = 6 inches). Temperature measurements were recorded over time. Experimental procedures and results are provided below.

### Part I - Experimental Procedures

Five healthy human patients were recruited for this study evaluating cooling of the knee joint using two different cooling methodologies. The first cooling methodology tested was a Breg Knee WrapOn Polar Pad (as illustrated in Figure 1) that utilizes circulating ice-water to achieve cooling, where the pad is placed on skin surrounding the knee. The second cooling methodology tested was ice-pack cooling, in which the patient's knee was wrapped with a stockinette and then the ice pack was positioned on top of the stockinette so that the ice pack (having a surface for application to the patient, wherein said surface has a diameter of approximately six inches) is positioned over the patient's patella; the ice pack is then secured in place using an elasticated bandage. To reduce any potential bias from order effects, three of the patients received the Breg Knee WrapOn Polar Pad on their left knee and ice-pack cooling on their right knee, while the other two patients received ice-pack cooling on their left knee and the Breg Knee WrapOn Polar Pad on their right knee. Temperatures within the knee joint were obtained from the recording device at no less than 5 minute intervals (+/- 2 min) from the time of placement of the probes until the probes are removed.

Prior to cooling of the knee joint, under sterile conditions, an intraarticular temperature probe was positioned in each knee and an additional temperature probe was placed on the surface of the knee near to the site of injection. At the discretion of the physician performing the procedure, in order to reduce discomfort for the patient, the protocol authorized the physician to instill a volume of 1-2 cc of 2% w/w lidocaine (without epinephrine) into the skin and subcutaneous tissue of the knee at the site of intra-articular probe insertion. Temperature measurements were carried out on the left knee first for three patients, while temperature measurements were carried out on the right knee first for two patients. After obtaining baseline knee intraarticular and skin temperature, the cooling regimen was applied for 15 minutes, followed by intraarticular injection of 2% w/w lidocaine (without epinephrine). Cooling was continued for up to a maximum total of 120 minutes. The probe was removed within approximately 30 minutes after removal of the cooling and the patient was allowed some rest and then the analogous procedure was performed on the patient's right knee for three patients, and the left knee for the other two patients. There was a maximum of four hours between the end of cooling on the left knee and start of cooling on the right knee.

Patients that participated in the study passed the following screening criteria and meet the patient inclusion and exclusion criteria are set forth below. As part of patient screening, patients received an intradermal injection of 100 µL (100 µg) capsaicin into the non-dominant volar forearm. To be eligible to enter the study, patients were able to tolerate the capsaicin injection. Screening patients rated the pain from capsaicin injection at 5, 10, 20, and 30 minutes after injection using a 0-10 Numerical Pain Rating Scale, where "0" equals no pain and "10" equals worst possible pain.

Inclusion Criteria
1. Patient is male or female.
2. Patient is aged between 18 and 45 years, inclusive.
3. Patient has signed and dated an ethics approved informed consent form (ICF).
4. Patient's Body Mass Index (BMI) is between 18 and 32 kg/m2, inclusive and patient's weight is greater than or equal to 50 kg.
5. Patients must be in good health, in the opinion of the Investigator, as determined by a medical history, physical examination, clinical laboratory tests, vital signs and 12 lead electrocardiogram (ECG).
6. Patients must be able to communicate well with the Investigator, understand and comply with the requirements of the study.
7. Patients must be able to tolerate the capsaicin injection given at screening.

### Exclusion Criteria

1. Patient has had a clinically significant illness that has not completely resolved in the four weeks before screening.
2. Patient has a history of neurological disorder which may impact the perception of pain or impairs the patient's ability to fully participate in the trial.
3. Patient has used analgesic medications in the 2 days prior to dosing for cohorts 2 to 4, except for paracetamol, as needed.
4. Patient has used topical medications applied to the knee for osteoarthritis pain (including capsaicin, lidocaine, prescription or OTC medications) from 90 days prior to screening through to dosing.
5. Patient has been injected with corticosteroids in the knee 90 days prior to screening through to dosing.
6. Patient uses any prescription or non-prescription medications, including herbal and dietary supplements (including St. John's wort) within 14 days prior to the first dose of study medication. By exception, the patient may take acetaminophen (≤2 grams/day) for up to 48 hours prior to any dose of study medication.
7. Patient has a significant history of drug/solvent abuse or a positive drugs of abuse (DOA) test at screening.
8. Patient has a history of alcohol abuse or currently drinks more than 28 units per week.
9. Patient is, in the opinion of the Investigator, not suitable to participate in the study.
10. Patient has participated in any clinical study with an investigational drug/device within 3 months (or five half-lives if this longer than 3 months) prior to the first day of dosing.
11. Patient has a positive Human Immunodeficiency Virus (HIV), Hepatitis B or Hepatitis C screen.
12. Patient has lost or donated 500 mL or more of blood within the 3 months prior to screen, or intends to donate blood during the study.
13. Patient with known intolerance to capsaicin, hot peppers or any excipient in the investigational medicinal product (i.e., capsaicin formulation for injection) or lidocaine.
14. Pregnant or breastfeeding females.
15. History of allergic reaction to the planned local anesthesia/ analgesic regimens, ethylenediaminetetraacetic acid (EDTA), Kolliphor HS 15, butylated hydroxytoluene (BHT), or capsaicin.
16. Patient has any active skin disorders, skin trauma, significant scarring or skin disease on either forearm, or a significant history of trauma or skin disease in either arm.
17. Any other severe acute or chronic medical or psychiatric condition, or laboratory abnormality, that may increase the risk associated with a) study participation b) Investigational product administration c) may interfere with the interpretation of study results and, in the judgment of the investigator, in discussion with the Sponsor, would make the patient inappropriate for entry into this study.

Patients rated pain from the procedure using a Numerical Pain Rating Scale (NPRS) (0-10) at the following time points:
- At pre-cooling after placement of the temperature probes.
- At rest, 5 minutes prior to intra-articular lidocaine (without epinephrine) injection.
- At rest, 10 minutes after intra-articular injection of 2% w/w lidocaine (without epinephrine).
- Ratings will continue at 10-minute intervals until removal of the temperature probes. The 10 minute intervals will allow for a +/- 2-minute variance in timing.

### Part II - Results

The experimental procedure was completed successfully for four healthy human patients. For the fifth patient subjected to the experimental procedure, there was a deviation from protocol due to early removal of a temperature probe. Therefore, experimental results are provided below for the four healthy human patients upon which the experimental procedure was completed successfully.

Mean temperature values for intraarticular temperature in the knee joint recorded over time are presented in Figure 2, along with skin temperature values recorded over time. Data show that the Breg Knee WrapOn Polar Pad resulted in lower intraarticular temperature for the patient's knee joint after about 30 minutes of cooling, compared to ice-pack cooling.

Figure 3 provides mean temperature values for intraarticular temperature in the knee joint recorded over time, along with NPRS pain values recorded over time.

Tabulated mean temperature values along with mean NPRS Pain scores recorded in the study are provided in Table 1 below.

**TABLE 1.**

| Tabulated mean temperature values along with mean NPRS Pain scores recorded in the study are provided in Table 1 below. | | | | | |
|---|---|---|---|---|---|
| **Cooling Method** | **Baseline** | **5 min** | **10 min** | **15 min** | **20 min** |
| **IA Temperature** | | | | | |
| Ice Pack | 33.2 (+/- 1.1) | 32.6 (+/- 1.2) | 32.4 (+/- 1.2) | 32.4 (+/- 1.0) | 32.6 (+/- 0.9) |
| Standard Cooling Device | 33.6 (+/- 0.8) | 33.1 (+/- 0.6) | 32.9 (+/- 0.8) | 32.8 (+/- 1.1) | 32.8 (+/- 1.1) |

| **Skin Temperature** | | | | | |
|---|---|---|---|---|---|
| Ice Pack | 29.0 (+/- 1.0) | 28.1 (+/- 1.2) | 26.3 (+/- 1.3) | 23.9 (+/- 1.3) | 23.6 (+/- 1.0) |
| Standard Cooling Device | 28.9 (+/- 1.1) | 28.1 (+/- 0.6) | 26.7 (+/- 0.4) | 24.6 (+/- 0.4) | 23.5 (+/- 1.2) |

| **Knee Pain (NPRS)** | | | | | |
|---|---|---|---|---|---|
| Ice Pack | 0.3 (+/- 0.5) | | | | 0.5 (+/- 1.0) |
| Standard Cooling Device | 0.0 (+/- 0.0) | | | | 0.0 (+/- 0.0) |

| **Cooling Method** | **25 min** | **30 min** | **35 min** | **40 min** | **45 min** |
|---|---|---|---|---|---|
| **IA Temperature** | | | | | |
| Ice Pack | 32.4 (+/- 0.8) | 32.3 (+/- 0.6) | 32.7 (+/- 0.5) | 32.9 (+/- 0.8) | 33.2 (+/- 0.7) |
| Standard Cooling Device | 32.3 (+/- 0.8) | 31.9 (+/- 1.1) | 31.7 (+/- 1.0) | 31.4 (+/- 0.9) | 31.2 (+/- 0.9) |

| **Skin Temperature** | | | | | |
|---|---|---|---|---|---|
| Ice Pack | 25.3 (+/- 0.7) | 25.6 (+/- 1.1) | 23.7 (+/- 2.4) | 22.1 (+/- 2.1) | 21.2 (+/- 1.9) |
| Standard Cooling Device | 24.0 (+/- 1.8) | 24.2 (+/- 1.2) | 23.1 (+/- 2.3) | 22.1 (+/- 2.7) | 21.2 (+/- 2.7) |

| **Knee Pain (NPRS)** | | | | | |
|---|---|---|---|---|---|
| **Cooling Method** | **25 min** | **30 min** | **35 min** | **40 min** | **45 min** |
| Ice Pack | | 0.0 (+/- 00) | | 0.8 (+/- 1.5) | |
| Standard Cooling Device | | 0.3 (+/- 0.6) | | 0.0 (+/- 0.0) | |

| **Cooling Method** | **50 min** | **55 min** | **60 min** | **65 min** | **70 min** |
|---|---|---|---|---|---|
| **IA Temperature** | | | | | |
| Ice Pack | 33.4 (+/- 0.6) | 33.6 (+/- 0.5) | 33.7 (+/- 0.4) | 33.7 (+/- 0.5) | 33.7 (+/- 0.6) |
| Standard Cooling Device | 31.0 (+/- 0.8) | 30.7 (+/- 0.8) | 30.4 (+/- 0.9) | 30.1 (+/- 1.0) | 29.7 (+/- 1.2) |

| **Skin Temperature** | | | | | |
|---|---|---|---|---|---|
| Ice Pack | 20.9 (+/- 2.3) | 20.7 (+/- 2.7) | 20.6 (+/- 3.1) | 20.7 (+/- 33) | 20.8 (+/- 3.6) |
| Standard Cooling Device | 20.6 (+/- 2.8) | 20.0 (+/- 2.8) | 19.5 (+/- 2.6) | 19.2 (+/- 2.7) | 18.7 (+/- 2.7) |

| **Knee Pain (NPRS)** | | | | | |
|---|---|---|---|---|---|
| Ice Pack | 0.5 (+/- 1.0) | | 0.5 (+/- 1.0) | | 0.3 (+/- 0.5) |
| Standard Cooling Device | 0.3 (+/- 0.5) | | 0.3 (+/- 0.5) | | 0.3 (+/- 0.5) |

| **Cooling Method** | **75 min** | **80 min** | **85 min** | **90 min** | **95 min** |
|---|---|---|---|---|---|
| **IA Temperature** | | | | | |
| Ice Pack | 33.6 (+/- 0.7) | 33.6 (+/- 0.9) | 33.5 (+/- 1.0) | 33.5 (+/- 1.1) | 33.4 (+/- 1.3) |
| Standard Cooling Device | 292 (+/- 1.4) | 28.8 (+/- 1.4) | 28.3 (+/- 1.5) | 27.8 (+/- 1.5) | 27.1 (+/- 1.2) |

| **Skin Temperature** | | | | | |
|---|---|---|---|---|---|
| Ice Pack | 20.8 (+/- 3.9) | 20.8 (+/- 4.1) | 20.8 (+/- 4.1) | 20.8 (+/- 4.1) | 20.8 (+/- 4.1) |
| Standard Cooling Device | 18.3 (+/- 2.5) | 17.9 (+/- 2.4) | 17.5 (+/- 2.3) | 17.2 (+/- 2.2) | 16.8 (+/- 2.1) |

| **Knee Pain (NPRS)** | | | | | |
|---|---|---|---|---|---|
| Ice Pack | | 0.3 (+/- 0.5) | | 0.3 (+/- 0.5) | |
| Standard Cooling Device | | 0.3 (+/- 0.5) | | 0.3 (+/- 0.5) | |

| **Cooling Method** | **100 min** | **105 min** | **110 min** | **115 min** | **120 min** |
|---|---|---|---|---|---|
| **IA Temperature** | | | | | |
| Ice Pack | 33.2 (+/- 1.4) | 33.2 (+/- 1.4) | 33.1 (+/- 1.4) | 33.0 (+/- 1.5) | 33.0 (+/- 1.5) |
| Standard Cooling Device | 26.5 (+/- 1.0) | 26.1 (+/- 1.2) | 25.7 (+/- 1.6) | 25.3 (+/- 1.5) | 25.1 (+/- 1.6) |

| **Skin Temperature** | | | | | |
|---|---|---|---|---|---|
| Ice Pack | 20.7 (+/- 4.2) | 20.6 (+/- 4.3) | 20.4 (+/- 4.4) | 20.4 (+/- 4.6) | 20.3 (+/- 4.7) |
| Standard Cooling Device | 16.5 (+/- 2.1) | 16.2 (+/- 2.0) | 15.8 (+/- 1.9) | 15.5 (+/- 1.9) | 15.3 (+/- 1.8) |

| **Knee Pain (NPRS)** | | | | | |
|---|---|---|---|---|---|
| Ice Pack | 0.3 (+/- 0.5) | | 0.3 (+/- 0.5) | | 0.3 (+/- 0.5) |
| Standard Cooling Device | 0.3 (+/- 0.5) | | 0.3 (+/- 0.5) | | 0.0 (+/- 0.0) |

| **Cooling Method** | **125 min** | **130 min** | **135 min** | **140 min** | **145 min** |
|---|---|---|---|---|---|
| **IA Temperature** | | | | | |
| Ice Pack | 32.9 (+/- 1.4) | 32.8 (+/- 1.4) | 327 (+/- 1.3) | 32.5 (+/- 1.3) | 32.4 (+/- 1.2) |
| Standard Cooling Device | 24.7 (+/- 1.7) | 24.4 (+/- 1.8) | 24.1 (+/- 1.8) | 23.7 (+/- 1.8) | 23.3 (+/- 1.7) |

| **Skin Temperature** | | | | | |
|---|---|---|---|---|---|
| Ice Pack | 20.1 (+/- 4.5) | 20.0 (+/- 4.4) | 20.1 (+/- 4.6) | 19.9 (+/- 4.4) | 19.6 (+/- 4.0) |
| Standard Cooling Device | 15.2 (+/- 1.7) | 15.0 (+/- 1.5) | 14.8 (+/- 1.4) | 14.7 (+/- 1.3) | 14.6 (+/- 1.3) |

| **Knee Pain (NPRS)** | | | | | |
|---|---|---|---|---|---|
| Ice Pack | | 0.5 (+/- 1.0) | | 0.3 (+/- 0.5) | |
| Standard Cooling Device | | 0.0 (+/- 0.0) | | 0.0 (+/- 0.0) | |

| **Cooling Method** | **150 min** | **155 min** | **160 min** | **165 min** | **170 min** |
|---|---|---|---|---|---|
| **IA Temperature** | | | | | |
| Ice Pack | 32.4 (+/- 1.1) | 32.3 (+/- 1.1) | 32.2 (+/- 1.2) | 32.3 (+/- 1.2) | 32.3 (+/- 1.3) |
| Standard Cooling Device | 23.2 (+/- 1.4) | 23.0 (+/- 1.1) | 22.8 (+/- 1.1) | 22.5 (+/- 1.1) | 22.3 (+/- 1.3) |

| **Skin Temperature** | | | | | |
|---|---|---|---|---|---|
| Ice Pack | 20.7 (+/- 4.4) | 22.5 (+/- 4.7) | 24.2 (+/- 4.2) | 25.4 (+/- 3.7) | 26.3 (+/- 3.5) |
| Standard Cooling Device | 15.1 (+/- 1.2) | 16.2 (+/- 1.5) | 17.3 (+/- 1.5) | 18.0 (+/- 1.4) | 18.7 (+/- 1.2) |

| **Knee Pain (NPRS)** | | | | | |
|---|---|---|---|---|---|
| Ice Pack | 0.5 (+/- 1.0) | | 0.3 (+/- 0.5) | | 0.3 (+/- 0.5) |
| Standard Cooling Device | 0.3 (+/- 0.5) | | 0.3 (+/- 0.5) | | 0.3 (+/- 0.5) |

| **Cooling Method** | **175 min** | **180 min** |
|---|---|---|
| **IA Temperature** | | |
| Ice Pack | 32.1 | |
| Standard Cooling Device | | |

| **Skin Temperature** | | |
|---|---|---|
| Ice Pack | 27.1 | |
| Standard Cooling Device | | |

| **Knee Pain (NPRS)** | | |
|---|---|---|
| Ice Pack | | 1.0 |
| Standard Cooling Device | | 0.0 |

### EXAMPLE 7 - ANALYSIS OF PROCEDURE PAIN DUE TO INTRAARTICULAR ADMINISTRATION OF CAPSAICIN

Human patients experiencing osteoarthritic knee joint pain were subjected to two different protocols for intraarticular administration of trans-capsaicin. Temporary pain expected due to administration of capsaicin was analyzed, along with the intraarticular temperature of the knee joint and the temperature of the patient's skin in the area to be cooled.

One of the protocols utilized a Breg Knee WrapOn Polar Pad (as illustrated in Figure 1) that utilizes circulating ice-water to achieve cooling, where the pad is placed on skin surrounding the knee in order to cool the knee joint. The other protocol utilized ice-pack cooling, in which the patient's knee was wrapped with a stockinette and then the ice pack was positioned on top of the stockinette so that the ice pack is positioned over the patient's patella to thereby cool the knee joint. The ice pack was a LEADSTAR pain relief reusable cold therapy ice pack (size = 6 inches). A temperature probe was placed into the intraarticular space of the patient's knee joint to measure intraarticular temperature of the knee joint. A temperature probe was also placed on the skin in the area to be cooled in order to measure skin temperature in the area to be cooled.

### Experimental procedures and results are provided below

### Part I - Experimental Procedures

Five human patients suffering from moderate to severe painful bilateral knee osteoarthritis were recruited for this study evaluating the impact that cooling protocol has on the magnitude of temporary pain experienced by the patient due to administration of capsaicin. Patients had an average "pain with walking over the past 24 hours" for each knee in the range of 4-9 (inclusive) on a 0-10 numerical pain rating scale where "0" equals no pain and "10" equals worst possible pain (NPRS). Patients that participated in the study passed the following screening criteria and meet the patient inclusion and exclusion criteria are set forth below. As part of patient screening, patients received an intradermal injection of 100 µL (100 µg) capsaicin into the non-dominant volar forearm. To be eligible to enter the study, patients were able to tolerate the capsaicin injection. Screening patients rated the pain from capsaicin injection at 5, 10, 20, and 30 minutes after injection using a 0-10 Numerical Pain Rating Scale, where "0" equals no pain and "10" equals worst possible pain.

### Inclusion Criteria

1. Patient is male or female.
2. Patient is aged between 45 and 75 years, inclusive.
3. Patient has signed and dated an ethics approved informed consent form (ICF).
4. Patient's Body Mass Index (BMI) is between 18 and 32 kg/m2, inclusive and patient's weight is greater than or equal to 50 kg.
5. Patient has a diagnosis of bilateral moderate to severe painful knee osteoarthritis (patients will be required to have a score on Pain with walking in the previous 24 hours, of 4 to 9, inclusive (Numeric Pain Rating Scale 0-10). The condition must be chronic with a history of painful arthritis for at least 3 months prior to entry into the study.
6. All patients must otherwise be in good health, in the opinion of the Investigator, as determined by a medical history, physical examination, clinical laboratory tests, vital signs and 12 lead electrocardiogram (ECG).
7. Patients must be able to communicate well with the Investigator, understand and comply with the requirements of the study.
8. Patients have had previous bilateral AP radiographs (or CT/MRI scan) of the knees which demonstrate osteoarthritis in both knee joints within the prior 36 months.
9. Patient must be able to tolerate the capsaicin injection given at screening.

### Exclusion Criteria

1. Patient has had a clinically significant illness, other than osteoarthritis, that has not completely resolved in the four weeks before screening.
2. Patient has a history of neurological disorder which may impact the perception of pain or impairs the patient's ability to fully participate in the trial.
3. Patient has used analgesic medications in the 2 days prior to dosing, except for paracetamol, as needed.
4. Patient has used topical medications applied to the knee for osteoarthritis pain (including capsaicin, lidocaine, prescription or OTC medications) from 90 days prior to screening through to dosing.
5. Patient has been injected with corticosteroids in the knee 90 days prior to screening through to dosing.
6. Patient currently uses opioids for any condition other than osteoarthritis knee pain (maximum dose 15 mg hydrocodone, or equivalent, per day prescribed by a physician).
7. Patient has physical/occupational/chiropractic therapy for the lower extremities or acupuncture for the lower extremities 30 days prior to screening or during the period to dosing.
8. Patient has had joint replacement surgery at any time, or open surgery of the knee in the past 12 months prior to screening, or prior arthroscopic surgery of the knee within 6 months prior to screening.
9. Patient has a history of a bleeding diathesis, or is using anti-coagulant drugs, excluding low dose aspirin.
10. Patient has a significant history of drug/solvent abuse or a positive drugs of abuse (DOA) test at screening. Prescribed opioids, as noted in exclusion, 6 are permitted.
11. Patient has a history of alcohol abuse or currently drinks more than 28 units per week.
12. Patient is, in the opinion of the Investigator, not suitable to participate in the study.
13. Patient has participated in any clinical study with an investigational drug/device within 3 months (or five half-lives if this longer than 3 months) prior to the first day of dosing.
14. Patient has a positive Human Immunodeficiency Virus (HIV), Hepatitis B or Hepatitis C screen.
15. Patient has lost or donated 500 mL or more of blood within the 3 months prior to screen, or intends to donate blood during the study.
16. Patient with active chronic pain conditions other than knee osteoarthritis, including periarticular pain about the knee.
17. Patient with known intolerance to capsaicin, hot peppers or any excipient in the investigational medicinal product or lidocaine.
18. Pregnant or breastfeeding females.
19. History of allergic reaction to the planned local anesthesia/ analgesic regimens, ethylenediaminetetraacetic acid (EDTA), Kolliphor HS 15, butylated hydroxytoluene (BHT), or capsaicin.
20. Patient has any active skin disorders, skin trauma, significant scarring or skin disease on either forearm, or a significant history of trauma or skin disease in either arm.
21. Any other severe acute or chronic medical or psychiatric condition, or laboratory abnormality, that may increase the risk associated with a) study participation b) Investigational product administration c) may interfere with the interpretation of study results and, in the judgment of the investigator, in discussion with the Sponsor, would make the patient inappropriate for entry into this study.

The following therapies were prohibited both prior to and during the study (i.e., prohibiting therapies):
- Injection of corticosteroids in the index knee from 90 days prior to Screening through study completion.
- Topical medications applied to the index knee for osteoarthritis pain (including capsaicin, lidocaine, prescription, or OTC medications) from 90 days prior to Screening through study completion.
- Current use of opioids for any condition other than for osteoarthritis of the index knee (maximum dose of 15 mg of hydrocodone [or equivalent] per day as background medication is allowed at entry if prescribed by a physician).
- Regular use of anticoagulant blood thinners.
- Use of an investigational medication within 30 days prior to Screening, or 5 PK or PD half- lives (whichever is longer), or scheduled to receive such an agent while participating in the study.
- Physical/ occupational/ chiropractic or acupuncture therapy for the lower extremities within 30 days of Screening, or need for such therapy during the course of the study.
- Joint replacement surgery of the index knee at any time, or open surgery of the index knee in the past 12 months prior to Screening, or prior arthroscopic surgery of the index knee within 6 months of Screening.
- Surgery, or other invasive procedures, or intraarticular injections (other than the study drug) while participating in the study.

The patient was excluded from study participation if they had taken any medication prior to randomization that would indicate that the patient has a serious or unstable illness, is not in good general health, or has a condition that would contraindicate study participation. If a patient received an excluded therapy after enrolment, continuation in the study was at the discretion of the sponsor/investigator/medical monitor. Patients were not to take a hot bath or shower, or expose the injected knee to external heat within 12 hours after the injection.

One of the protocols utilized a Breg Knee WrapOn Polar Pad (as illustrated in Figure 1) that utilizes circulating ice-water to achieve cooling, where the pad is placed on skin surrounding the knee in order to cool the knee joint. The other protocol utilized ice-pack cooling, in which the patient's knee was wrapped with a stockinette and then the ice pack was positioned on top of the stockinette so that the ice pack (having a surface for application to the patient, wherein said surface has a diameter of approximately six inches) is positioned over the patient's patella to thereby cool the knee joint. A temperature probe was placed into the intraarticular space of the patient's knee joint to measure intraarticular temperature of the knee joint. At the discretion of the physician performing the procedure, in order to reduce discomfort for the patient, the protocol authorized the physician to instill a volume of 1-2 cc of 2% w/w lidocaine (without epinephrine) into the skin and subcutaneous tissue of the knee at the site of intra-articular probe insertion. A temperature probe was also placed on the skin in the area to be cooled in order to measure skin temperature in the area to be cooled.

One the first day of the study, patients were randomized so that (i) three patients receiving cooling of the knee joint using the Breg Knee WrapOn Polar Pad and (ii) two patients will receive ice-pack cooling over the patella as set forth above. Patients were subjected to the following procedure:
∘ Placement of intraarticular temperature probe and skin temperature probe. At the discretion of the physician performing the procedure, in order to reduce discomfort for the patient, the protocol authorized the physician to instill a volume of 1-2 cc of 2% w/w lidocaine (without epinephrine) into the skin and subcutaneous tissue of the knee at the site of intra-articular probe insertion.
∘ Cooling using the designated technique was performed for 15 minutes (+/- 2 minutes).
∘ Cooling apparatus was removed from the patient's knee.
∘ Intraarticular injection of lidocaine 2% w/w (without epinephrine) 15 mL solution into the patient's knee joint.
∘ Cooling using the designated technique was performed for 30 minutes (+/- 2 minutes).
∘ Intraarticular injection of the investigational medicinal product (IMP) was performed to deliver *trans*-capsaicin in the amount of 1 mg. The IMP was provided as a prefilled syringe containing 2 mL of fluid containing trans-capsaicin at a concentration of 0.5 mg/mL.
∘ Knee joint was flexed and extended five times over 1 minute to ensure appropriate distribution of IMP.
∘ Cooling using the designated technique was performed for 60 minutes (up to a maximum total of 120 minutes).
∘ The temperature probe was removed approximately 30 minutes after removal of the cooling apparatus.

Temperatures within the knee and on the skin in the region undergoing cooling were obtained from the recording device at no less than 5 minute intervals (+/- 2 min) from the time of placement of the probes until the probes were removed.

Pain due to injection of trans-capsaicin was assessed on a Numerical Pain Rating scale (0-10) for 75 minutes after injection of trans-capsaicin. A verbal NPRS was used to assess procedure pain during the study. Patients were asked to indicate the severity of any pain experienced on a scale of 0 to 10 (NPRS; 0 corresponds to no pain and 10 corresponds to the worst pain imaginable). Patients were instructed to consider procedure pain separately from their baseline osteoarthritis pain. Pain was assessed on a Numerical Pain Rating scale (0-10) according to the following schedule:
∘ At pre-cooling after placement of the temperature probes.
∘ At rest, prior to intra-articular lidocaine injection.
∘ At rest, 10 minutes after intra-articular lidocaine injection.
∘ At rest prior to injection of *trans*-capsaicin.
∘ Ratings will continue at 10 minute intervals beginning 10 minutes after the injection of trans-capsaicin until removal of the temperature probe. The 10 minute intervals allow for a +/- 2-minute variance in timing.

Patients were permitted to leave the clinic when they could ambulate independently, but no sooner than 1 hour after removal of the temperature probe.

Patients returned to the clinic 7±2 days later to have the procedure performed on their right knee.

### Part II - Results

The experimental procedure was completed successfully for four human patients. For the fifth patient subjected to the experimental procedure, there was a deviation from protocol. For this reason, experimental results are provided below separately for (i) the four human patients for which the experimental procedure was completed successfully, and (ii) all five human patients.

Mean values for intraarticular temperature in the knee joint recorded over time are presented in Figure 4, along with mean values for skin temperature recorded over time, for the four human patients for which the experimental procedure was completed successfully. Mean values for intraarticular temperature in the knee joint recorded over time are presented in Figure 5, along with mean values for skin temperature recorded over time, for all five human patients.

Mean NPRS Pain scores recorded over time are presented in Figure 6, along with mean values for intraarticular temperature in the knee joint, for the four human patients for which the experimental procedure was completed successfully. Mean NPRS Pain scores recorded over time are presented in Figure 7, along with mean values for intraarticular temperature in the knee joint, for all five four human patients.

For the four human patients for which the experimental procedure was completed successfully, tabulated mean temperature values along with mean NPRS Pain scores recorded in the study are provided in Table 1 below. Table 2 below provides tabulated mean temperature values along with mean NPRS Pain scores recorded in the study for all five human patients.

The data show that the Breg Knee WrapOn Polar Pad resulted in lower intraarticular temperature for the patient's knee joint after about 30 minutes of cooling, compared to ice-pack cooling. Additionally, pain due to capsaicin injection was less when Breg Knee WrapOn Polar Pad cooling was used, compared to ice-pack cooling.

Tabulated mean temperature values along with mean NPRS Pain scores recorded in the study are provided in Table 1 below.

**TABLE 1.**

| **Cooling Method** | **Baseline** | **5 min** | **10 min** | **15 min** | **20 min** |
|---|---|---|---|---|---|
| **IA Temperature** | | | | | |
| Ice Pack | 33.4 (+/- 1.6) | 33.2 (+/- 1.3) | 33.2 (+/- 1.2) | 33.3 (+/- 1.2) | 33.4 (+/- 1.0) |
| Standard Cooling Device | 33.2 (+/- 0.8) | 32.8 (+/- 0.8) | 32.6 (+/- 0.9) | 32.4 (+/- 1.2) | 32.4 (+/- 1.4) |

| **Skin Temperature** | | | | | |
|---|---|---|---|---|---|
| Ice Pack | 28.1 (+/- 1.2) | 27.4 (+/- 1.2) | 26.2 (+/- 1.3) | 24.5 (+/- 1.7) | 24.0 (+/- 1.7) |
| Standard Cooling Device | 27.6 (+/- 0.4) | 26.2 (+/- 0.6) | 23.7 (+/- 0.5) | 20.4 (+/- 0.8) | 19.1 (+/- 1.0) |

| **Knee Pain (NPRS)** | | | | | |
|---|---|---|---|---|---|
| Ice Pack | 1.3 (+/- 2.5) | | | | 0.5 (+/- 0.6) |
| Standard Cooling Device | 2.0 (+/- 2.7) | | | | 1.3 (+/- 1.9) |

| **Cooling Method** | **25 min** | **30 min** | **35 min** | **40 min** | **45 min** |
|---|---|---|---|---|---|
| **IA Temperature** | | | | | |
| Ice Pack | 32.5 (+/- 0.8) | 31.4 (+/- 0.8) | 31.7 (+/- 1.1) | 31.9 (+/- 1.2) | 32.0 (+/- 1.2) |
| Standard Cooling Device | 31.7 (+/- 1.3) | 30.8 (+/- 1.5) | 30.6 (+/- 1.9) | 30.4 (+/- 1.9) | 30.1 (+/- 1.9) |

| **Skin Temperature** | | | | | |
|---|---|---|---|---|---|
| Ice Pack | 25.1 (+/- 1.5) | 25.4 (+/- 0.9) | 23.9 (+/- 1.0) | 23.0 (+/- 1.6) | 22.5 (+/- 2.0) |
| Standard Cooling Device | 20.3 (+/- 0.9) | 20.1 (+/- 1.0) | 17.9 (+/- 1.4) | 16.6 (+/- 1.6) | 15.7 (+/- 1.7) |

| **Knee Pain (NPRS)** | | | | | |
|---|---|---|---|---|---|
| Ice Pack | | 0.3 (+/- 0.6) | | 0.0 | |
| Standard Cooling Device | | 1.3 (+/- 1.3) | | | |

| **Cooling Method** | **50 min** | **55 min** | **60 min** | **65 min** | **70 min** |
|---|---|---|---|---|---|
| **IA Temperature** | | | | | |
| Ice Pack | 32.0 (+/- 1.2) | 32.1 (+/- 1.2) | 32.0 (+/- 0.5) | 31.6 (+/- 0.8) | 31.3 (+/- 2.2) |
| Standard Cooling Device | 29.8 (+/- 2.0) | 29.7 (+/- 2.0) | 29.5 (+/- 1.6) | 28.4 (+/- 1.0) | 27.2 (+/- 0.9) |

| **Cooling Method** | **50 min** | **55 min** | **60 min** | **65 min** | **70 min** |
|---|---|---|---|---|---|
| **Skin Temperature** | | | | | |
| Ice Pack | 22.2 (+/- 2.3) | 22.1 (+/- 2.6) | 23.3 (+/- 2.4) | 23.7 (+/- 2.3) | 22.1 (+/- 2.8) |
| Standard Cooling Device | 15.1 (+/- 1.7) | 14.8 (+/- 1.6) | 16.3 (+/- 1.2) | 17.4 (+/- 1.0) | 16.2 (+/- 1.1) |

| **Knee Pain (NPRS)** | | | | | |
|---|---|---|---|---|---|
| Ice Pack | 0.3 (+/- 0.5) | | | | 7.3 (+/- 1.9) |
| Standard Cooling Device | 1.0 (+/- 0.8) | | | | 4.0 (+/- 2.2) |

| **Cooling Method** | **75 min** | **80 min** | **85 min** | **90 min** | **95 min** |
|---|---|---|---|---|---|
| **IA Temperature** | | | | | |
| Ice Pack | 31.0 (+/- 2.7) | 30.9 (+/- 3.1) | 30.9 (+/- 3.1) | 30.8 (+/- 3.3) | 30.8 (+/- 3.3) |
| Standard Cooling Device | 26.8 (+/- 0.9) | 26.4 (+/- 0.8) | 26.1 (+/- 1.0) | 25.9 (+/- 1.3) | 25.7 (+/- 1.5) |

| **Skin Temperature** | | | | | |
|---|---|---|---|---|---|
| Ice Pack | 20.9 (+/- 3.2) | 20.4 (+/- 3.6) | 19.9 (+/- 4.0) | 19.1 (+/- 4.5) | 19.0 (+/- 4.5) |
| Standard Cooling Device | 15.1 (+/- 1.2) | 14.5 (+/- 1.2) | 14.1 (+/- 1.2) | 13.8 (+/- 1.1) | 13.5 (+/- 1.1) |

| **Knee Pain (NPRS)** | | | | | |
|---|---|---|---|---|---|
| Ice Pack | | 6.8 (+/- 2.5) | | 4.3 (+/- 1.7) | |
| Standard Cooling Device | | 3.8 (+/- 1.5) | | 2.5 (+/- 1.7) | |

| **Cooling Method** | **100 min** | **105 min** | **110 min** | **115 min** | **120 min** |
|---|---|---|---|---|---|
| **IA Temperature** | | | | | |
| Ice Pack | 30.8 (+/- 3.3) | 30.8 (+/- 3.4) | 30.7 (+/- 3.6) | 30.6 (+/- 3.7) | 30.5 (+/- 3.8) |
| Standard Cooling Device | 25.4 (+/- 1.6) | 25.1 (+/- 1.7) | 24.8 (+/- 1.8) | 24.4 (+/- 1.7) | 23.9 (+/- 1.7) |

| **Skin Temperature** | | | | | |
|---|---|---|---|---|---|
| Ice Pack | 18.8 (+/- 4.5) | 18.6 (+/- 4.6) | 18.4 (+/- 4.6) | 18.2 (+/- 4.4) | 18.0 (+/- 4.3) |
| Standard Cooling Device | 13.3 (+/- 1.0) | 13.1 (+/- 1.0) | 13.0 (+/- 0.9) | 12.9 (+/- 0.9) | 12.8 (+/- 0.9) |

| **Knee Pain (NPRS)** | | | | | |
|---|---|---|---|---|---|
| Ice Pack | 3.5 (+/- 1.7) | | 2.8 (+/- 2.2) | | 2.0 (+/- 1.4) |
| Standard Cooling Device | 1.8 (+/- 1.5) | | 1.3 (+/- 1.0) | | 1.0 (+/- 0.8) |

| **Cooling Method** | **125 min** | **130 min** | **135 min** | **140 min** | **145 min** |
|---|---|---|---|---|---|
| **IA Temperature** | | | | | |
| Ice Pack | 30.5 (+/- 3.8) | 30.6 (+/- 3.4) | 30.8 (+/- 3.0) | 30.9 (+/- 2.7) | 30.6 (+/- 3.1) |
| Standard Cooling Device | 23.7 (+/- 1.7) | 23.6 (+/- 1.9) | 23.7 (+/- 2.0) | 23.8 (+/- 2.2) | 23.7 (+/- 2.5) |

| **Skin Temperature** | | | | | |
|---|---|---|---|---|---|
| Ice Pack | 18.1 (+/- 4.1) | 19.1 (+/- 3.4) | 20.5 (+/- 2.7) | 21.4 (+/- 2.5) | ¹22.1 (+/- 2.6) |
| Standard Cooling Device | 13.1 (+/- 1.2) | 14.3 (+/- 1.3) | 16.1 (+/- 1.2) | 17.4 (+/- 1.0) | 18.4 (+/- 1.0) |

| **Knee Pain (NPRS)** | | | | | |
|---|---|---|---|---|---|
| **Cooling Method** | **125 min** | **130 min** | **135 min** | **140 min** | **145 min** |
| Ice Pack | | 2.3 (+/- 1.0) | | 1.8 (+/- 1.3) | |
| Standard Cooling Device | | 0.8 (+/- 0.5) | | 1.0 (+/- 0.8) | |

| **Cooling Method** | **150 min** | **155 min** | **160 min** | **165 min** | **170 min** |
|---|---|---|---|---|---|
| **IA Temperature** | | | | | |
| Ice Pack | 30.2 (+/- 3.9) | 31.7 | 31.6 | 31.7 | 31.7 |
| Standard Cooling Device | 23.7 (+/- 2.6) | | | | |

| **Skin Temperature** | | | | | |
|---|---|---|---|---|---|
| Ice Pack | 22.7 (+/- 2.7) | 19.8 | 19.7 | 20.4 | 22.0 |
| Standard Cooling Device | 19.1 (+/- 1.0) | | | | |

| **Knee Pain (NPRS)** | | | | | |
|---|---|---|---|---|---|
| Ice Pack | 1.3 (+/- 1.0) | | 2.0 | | 1.0 |
| Standard Cooling Device | 1.3 (+/- 1.3) | | | | |

| **Cooling Method** | **175 min** | **180 min** | **190 min** |
|---|---|---|---|
| **IA Temperature** | | | |
| Ice Pack | 31.7 | 31.6 | |
| Standard Cooling Device | | | |

| **Skin Temperature** | | | |
|---|---|---|---|
| **Ice Pack** | 23.3 | 24.2 | |
| **Standard Cooling Device** | | | |

| **Knee Pain (NPRS)** | | | |
|---|---|---|---|
| Ice Pack | | 7.0 | 2.0 |
| Standard Cooling Device | | | |

**TABLE 2.**

| **Cooling Method** | **Baseline** | **5 min** | **10 min** | **15 min** | **20 min** |
|---|---|---|---|---|---|
| **IA Temperature** | | | | | |
| Ice Pack | 33.5 (+/- 1.4) | 33.4 (+/- 1.2) | 33.3 (+/- 1.1) | 33.4 (+/- 1.1) | 33.3 (+/- 0.9) |
| Standard Cooling Device | 33.6 (+/- 1.2) | 33.3 (+/- 1.3) | 33.1 (+/- 1.5) | 33.0 (+/- 1.7) | 32.9 (+/- 1.7) |

| **Skin Temperature** | | | | | |
|---|---|---|---|---|---|
| Ice Pack | 28.3 (+/- 1.2) | 27.7 (+/- 1.3) | 26.5 (+/- 1.3) | 24.8 (+/- 1.5) | 24.2 (+/- 1.6) |
| Standard Cooling Device | 28.0 (+/-1.0) | 26.7 (+/-1.1) | 24.2 (+/-1.2) | 20.9 (+/-1.4) | 19.5 (+/-1.4) |

| **Knee Pain (NPRS)** | | | | | |
|---|---|---|---|---|---|
| Ice Pack | 1.0 (+/- 2.2) | | | | 0.4 (+/- 0.5) |
| Standard Cooling Device | 1.8 (+/- 2.4) | | | | 1.0 (+/- 1.7) |

| **Cooling Method** | **25 min** | **30 min** | **35 min** | **40 min** | **45 min** |
|---|---|---|---|---|---|
| **IA Temperature** | | | | | |
| Ice Pack | 33.5 (+/- 2.4) | 31.4 (+/- 0.8) | 31.7 (+/- 1.1) | 31.9 (+/- 1.2) | 32.0 (+/- 1.2) |
| Standard Cooling Device | 32.2 (+/- 1.7) | 31.5 (+/- 2.1) | 31.5 (+/- 2.5) | 31.3 (+/- 2.6) | 31.1 (+/- 2.7) |

| Skin Temperature | | | | | |
|---|---|---|---|---|---|
| Ice Pack | 25.3 (+/- 1.3) | 25.4 (+/- 0.9) | 23.9 (+/- 1.0) | 23.0 (+/- 1.6) | 22.5 (+/- 2.0) |
| Standard Cooling Device | 20.7 (+/- 1.2) | 20.6 (+/- 1.4) | 18.5 (+/- 1.8) | 17.1 (+/- 1.9) | 16.2 (+/- 1.9) |

| **Knee Pain (NPRS)** | | | | | |
|---|---|---|---|---|---|
| Ice Pack | | 0.3 (+/- 0.5) | | 0.0 | |
| Standard Cooling Device | | 1.2 (+/- 1.1) | | | |

| **Cooling Method** | **50 min** | **55 min** | **60 min** | **65 min** | **70 min** |
|---|---|---|---|---|---|
| **IA Temperature** | | | | | |
| Ice Pack | 32.4 (+/- 1.3) | 32.4 (+/- 1.2) | 32.3 (+/- 0.7) | 32.0 (+/- 1.0) | 31.7 (+/- 2.1) |
| Standard Cooling Device | 30.8 (+/- 2.8) | 30.6 (+/- 2.7) | 30.1 (+/- 2.0) | 29.2 (+/- 2.2) | 28.6 (+/- 3.1) |

| **Skin Temperature** | | | | | |
|---|---|---|---|---|---|
| Ice Pack | 23.3 (+/- 3.1) | 22.6 (+/- 2.5) | 23.3 (+/- 2.1) | 23.4 (+/- 2.1) | 21.9 (+/- 2.4) |
| Standard Cooling Device | 15.6 (+/- 1.9) | 15.3 (+/- 1.7) | 16.8 (+/- 1.4) | 17.8 (+/- 1.3) | 16.7 (+/- 1.4) |

| **Knee Pain (NPRS)** | | | | | |
|---|---|---|---|---|---|
| Ice Pack | 0.3 (+/- 0.5) | | | | 7.3 (+/- 1.9) |
| Standard Cooling Device | 0.8 (+/- 0.8) | | | | 5.0 (+/- 2.9) |

| **Cooling Method** | **75 min** | **80 min** | **85 min** | **90 min** | **95 min** |
|---|---|---|---|---|---|
| **IA Temperature** | | | | | |
| Ice Pack | 31.4 (+/- 2.5) | 31.3 (+/- 2.8) | 31.3 (+/- 2.8) | 31.3 (+/- 3.0) | 31.3 (+/- 3.1) |
| Standard Cooling Device | 28.3 (+/- 3.3) | 27.9 (+/- 3.5) | 27.7 (+/- 3.7) | 27.5 (+/- 3.9) | 27.4 (+/- 4.0) |

| **Skin Temperature** | | | | | |
|---|---|---|---|---|---|
| Ice Pack | 20.9 (+/- 2.8) | 20.4 (+/- 3.1) | 20.3 (+/- 3.5) | 19.8 (+/- 4.2) | 19.4 (+/- 4.1) |
| Standard Cooling Device | 15.7 (+/- 1.6) | 15.1 (+/- 1.6) | 14.6 (+/- 1.6) | 14.3 (+/- 1.6) | 14.1 (+/- 1.6) |

| **Knee Pain (NPRS)** | | | | | |
|---|---|---|---|---|---|
| Ice Pack | | 5.4 (+/- 3.7) | | 4.0 (+/- 1.6) | |
| Standard Cooling Device | | 4.0 (+/- 1.4) | | 2.8 (+/- 1.6) | |

| **Cooling Method** | **100 min** | **105 min** | **110 min** | **115 min** | **120 min** |
|---|---|---|---|---|---|
| **IA Temperature** | | | | | |
| Ice Pack | 31.4 (+/- 3.1) | 31.3 (+/- 3.2) | 31.3 (+/- 3.4) | 31.2 (+/- 3.5) | 31.1 (+/- 3.6) |
| Standard Cooling Device | 27.2 (+/- 4.2) | 26.9 (+/- 4.3) | 26.7 (+/- 4.5) | 26.3 (+/- 4.6) | 25.9 (+/- 4.8) |

| **Skin Temperature** | | | | | |
|---|---|---|---|---|---|
| Ice Pack | 19.1 (+/- 4.0) | 18.9 (+/- 4.0) | 18.7 (+/- 4.0) | 18.5 (+/- 3.9) | 18.3 (+/- 3.8) |
| Standard Cooling Device | 139 (+/- 1.6) | 13.7 (+/- 1.6) | 13.6 (+/- 1.6) | 13.5 (+/- 1.6) | 13.4 (+/- 1.6) |

| **Knee Pain (NPRS)** | | | | | |
|---|---|---|---|---|---|
| Ice Pack | 3.2 (+/- 1.6) | | 2.4 (+-2.1) | | 1.8 (+/- 1.3) |
| Standard Cooling Device | 2.0 (+/- 1.4) | | 1.2 (+/- 0.8) | | 1.0 (+/- 0.7) |

| **Cooling Method** | **125 min** | **130 min** | **135 min** | **140 min** | **145 min** |
|---|---|---|---|---|---|
| **IA Temperature** | | | | | |
| Ice Pack | 31.1 (+/- 3.6) | 31.2 (+/- 3.3) | 31.3 (+/- 2.9) | 31.4 (+/- 2.7) | 31.3 (+/- 3.1) |
| Standard Cooling Device | 25.7 (+/- 4.9) | 25.7 (+/- 5.0) | 25.7 (+/- 4.9) | 25.8 (+/- 4.8) | 25.7 (+/- 4.9) |

| **Skin Temperature** | | | | | |
|---|---|---|---|---|---|
| Ice Pack | 18.3 (+/- 3.6) | 19.1 (+/- 2.9) | 20.2 (+/- 2.4) | 21.0 (+/- 2.4) | 21.5 (+/- 2.6) |
| Standard Cooling Device | 13.6 (+/- 1.6) | 14.7 (+/- 1.4) | 16.3 (+/- 1.2) | 17.6 (+/- 1.0) | 18.6 (+/- 1.0) |

| **Knee Pain (NPRS)** | | | | | |
|---|---|---|---|---|---|
| Ice Pack | | 2.0 (+/- 1.0) | | 1.8 (+/- 1.1) | |
| Standard Cooling Device | | 0.6 (+/- 0.5) | | 1.2 (+/- 0.8) | |

| **Cooling Method** | **150 min** | **155 min** | **160 min** | **165 min** | **170 min** |
|---|---|---|---|---|---|
| **IA Temperature** | | | | | |
| Ice Pack | 30.9 (+/- 3.8) | 32.7 (+/- 1.4) | 32.7 (+/- 1.6) | 32.9 (+/- 1.7) | 33.0 (+/- 1.8) |
| Standard Cooling Device | 25.6 (+/- 49) | | | | |

| **Skin Temperature** | | | | | |
|---|---|---|---|---|---|
| Ice Pack | 22.1 (+/- 2.6) | 20.8 (+/- 1.5) | 21.6 (+/- 2.7) | 22.4 (+/- 2.9) | 23.6 (+/- 2.3) |
| Standard Cooling Device | 19.3 (+/- 1.0) | | | | |

| **Knee Pain (NPRS)** | | | | | |
|---|---|---|---|---|---|
| Ice Pack | 1.4 (+/- 0.9) | | 1.0 (+/- 1.4) | | *0.5 (+*/*- 0.7)* |
| Standard Cooling Device | 1.2 (+/- 1.1) | | | | |

| **Cooling Method** | **175 min** | **180 min** | **190 min** |
|---|---|---|---|
| **IA Temperature** | | | |
| Ice Pack | 33.0 (+/- 1.9) | 31.6 | |
| Standard Cooling Device | | | |

| **Skin Temperature** | | | |
|---|---|---|---|
| Ice Pack | 24.6 (+/- 1.8) | 24.2 | |
| Standard Cooling Device | | | |

| **Knee Pain (NPRS)** | | | |
|---|---|---|---|
| Ice Pack | | 7.0 | 2.0 |
| Standard Cooling Device | | | |

### EXAMPLE 8 - ANALYSIS OF PROCEDURE PAIN DUE TO INTRAARTICULAR ADMINISTRATION OF CAPSAICIN

Human patients experiencing osteoarthritic knee joint pain were subjected to two different protocols for intraarticular administration of trans-capsaicin. Temporary pain expected due to administration of capsaicin was analyzed, along with the intraarticular temperature of the knee joint and the temperature of the patient's skin in the area to be cooled.

One of the protocols utilized a Breg Knee WrapOn Polar Pad (as illustrated in Figure 1) that utilizes circulating ice-water to achieve cooling, where the pad is placed on skin surrounding the knee in order to cool the knee joint. The other protocol utilized an Elasto-Gel All Purpose Therapy Wrap measuring 6 inches by 24 inches in size, in order to cool the knee joint. A temperature probe was placed into the intraarticular space of the patient's knee joint to measure intraarticular temperature of the knee joint. A temperature probe was also placed on the skin in the area to be cooled in order to measure skin temperature in the area to be cooled.

### Experimental procedures and results are provided below

### Part I - Experimental Procedures

Experimental procedure used was the same as described in Example 8 herein, except that an Elasto-Gel All Purpose Therapy Wrap measuring 6 inches by 24 inches in size was used to cool the knee joint, in lieu of the ice pack. The Elasto-Gel All Purpose Therapy Wrap was removed from a freezer (approximately 0°F) just prior to use.

### Part II - Results

The experimental procedure was completed successfully for four human patients. For the fifth patient subjected to the experimental procedure, there was a deviation from protocol. For this reason, experimental results are provided below separately for (i) the four human patients for which the experimental procedure was completed successfully and (ii) all five human patients.

Mean values for intraarticular temperature in the knee joint recorded over time are presented in Figure 8, along with mean values for skin temperature recorded over time, for the four human patients for which the experimental procedure was completed successfully. Mean values for intraarticular temperature in the knee joint recorded over time are presented in Figure 9, along with mean values for skin temperature recorded over time, for all five human patients.

Mean NPRS Pain scores recorded over time are presented in Figure 10, along with mean values for intraarticular temperature in the knee joint, for the four human patients for which the experimental procedure was completed successfully. Mean NPRS Pain scores recorded over time are presented in Figure 11, along with mean values for intraarticular temperature in the knee joint, for all five four human patients.

For the four human patients for which the experimental procedure was completed successfully, tabulated mean temperature values along with mean NPRS Pain scores recorded in the study are provided in Table 1 below. Table 2 below provides tabulated mean temperature values along with mean NPRS Pain scores recorded in the study for all five human patients.

**TABLE 1.**

| **Cooling Method** | **Baseline** | **5 min** | **10 min** | **15 min** | **20 min** |
|---|---|---|---|---|---|
| **IA Temperature** | | | | | |
| Ice-Gel Pack | 33.6 (+/- 0.9) | 33.3 (+/- 1.0) | 33.1 (+/- 1.1) | 32.7 (+/- 1.2) | 32.2 (+/- 1.4) |
| Standard Cooling Device | 32.6 (+/- 0.4) | 32.1 (+/- 0.5) | 31.9 (+/- 0.6) | 31.7 (+/- 0.6) | 31.3 (+/- 0.9) |

| **Skin Temperature** | | | | | |
|---|---|---|---|---|---|
| Ice-Gel Pack | 29.0 (+/- 0.9) | 26.2 (+/- 2.3) | 22.9 (+/- 2.5) | 19.1 (+/- 2.9) | 18.6 (+/- 2.9) |
| Standard Cooling Device | 28.9 (+/- 1.5) | 26.5 (+/- 2.2) | 24.2 (+/- 2.7) | 21.6 (+/- 3.4) | 20.6 (+/- 3.6) |

| **Knee Pain (NPRS)** | | | | | |
|---|---|---|---|---|---|
| Ice-Gel Pack | 1.0 (+/- 0.8) | | | | 0.8 (+/- 0.5) |
| Standard Cooling Device | 0.5 (+/- 0.6) | | | | 0.5 (+/- 0.6) |

| **Cooling Method** | **25 min** | **30 min** | **35 min** | **40 min** | **45 min** |
|---|---|---|---|---|---|
| **IA Temperature** | | | | | |
| Ice-Gel Pack | 31.3 (+/- 1.4) | 30.5 (+/- 1.4) | 30.4 (+/- 1.6) | 30.4 (+/- 1.7) | 30.3 (+/- 2.1) |
| Standard Cooling Device | 29.8 (+/- 1.7) | 28.6 (+/- 2.1) | 29.1 (+/- 1.6) | 29.5 (+/- 1.6) | 29.7 (+/- 1.7) |

| **Skin Temperature** | | | | | |
|---|---|---|---|---|---|
| Ice-Gel Pack | 20.7 (+/- 2.5) | 21.0 (+/- 1.9) | 19.1 (+/- 2.1) | 18.7 (+/- 2.3) | 18.8 (+/- 2.4) |
| Standard Cooling Device | 21.9 (+/- 3.1) | 22.1 (+/- 2.7) | 20.3 (+/- 3.1) | 19.4 (+/- 3.3) | 18.9 (+/- 3.3) |

| **Knee Pain (NPRS)** | | | | | |
|---|---|---|---|---|---|
| Ice-Gel Pack | | 0.5 (+/- 0.6) | | | |
| Standard Cooling Device | | 0.5 (+/- 0.6) | | | |

| **Cooling Method** | **50 min** | **55 min** | **60 min** | **65 min** | **70 min** |
|---|---|---|---|---|---|
| **IA Temperature** | | | | | |
| Ice-Gel Pack | 30.1 (+/- 2.5) | 29.8 (+/- 2.7) | 29.6 (+/- 2.4) | 29.6 (+/- 1.9) | 29.8 (+/- 1.7) |
| Standard Cooling Device | 29.7 (+/- 1.9) | 29.7 (+/- 2.1) | 29.5 (+/- 2.0) | 28.7 (+/- 2.1) | 28.0 (+/- 2.6) |

| **Skin Temperature** | | | | | |
|---|---|---|---|---|---|
| Ice-Gel Pack | 19.2 (+/- 2.6) | 19.6 (+/- 2.7) | 21.2 (+/- 2.7) | 20.6 (+/- 2.5) | 16.9 (+/- 2.3) |
| Standard Cooling Device | 18.5 (+/- 3.3) | 18.2 (+/- 3.4) | 19.3 (+/- 3.4) | 19.9 (+/- 3.6) | 18.7 (+/- 3.5) |

| **Knee Pain (NPRS)** | | | | | |
|---|---|---|---|---|---|
| Ice-Gel Pack | 0.8 (+/- 1.0) | | | | 4.8 (+/- 3.2) |
| Standard Cooling Device | 0.8 (+/- 1.0) | | | | 2.5 (+/- 3.7) |

| **Cooling Method** | **75 min** | **80 min** | **85 min** | **90 min** | **95 min** |
|---|---|---|---|---|---|
| **IA Temperature** | | | | | |
| Ice-Gel Pack | 29.7 (+/- 1.5) | 29.3 (+/- 1.6) | 28.7 (+/- 2.0) | 28.0 (+/- 2.5) | 27.1 (+/- 3.3) |
| Standard Cooling Device | 27.8 (+/- 2.7) | 27.5 (+/- 2.8) | 27.2 (+/- 3.1) | 27.0 (+/- 3.5) | 26.8 (+/- 4.0) |

| **Skin Temperature** | | | | | |
|---|---|---|---|---|---|
| Ice-Gel Pack | 14.8 (+/- 2.3) | 14.2 (+/- 2.5) | 140 (+/- 2.8) | 14.1 (+/- 3.0) | 14.5 (+/- 3.4) |
| Standard Cooling Device | 17.6 (+/- 3.0) | 16.9 (+/- 2.7) | 16.4 (+/- 2.4) | 16.0 (+/- 2.1) | 15.7 (+/- 2.0) |

| **Knee Pain (NPRS)** | | | | | |
|---|---|---|---|---|---|
| Ice-Gel Pack | | 4.3 (+/- 3.0) | | 3.0 (+/- 1.8) | |
| Standard Cooling Device | | 2.5 (+/- 3.0) | | 2.5 (+/- 2.4) | |

| **Cooling Method** | **100 min** | **105 min** | **110 min** | **115 min** | **120 min** |
|---|---|---|---|---|---|
| **IA Temperature** | | | | | |
| Ice-Gel Pack | 26.7 (+/- 3.3) | 26.6 (+/- 3.4) | 26.6 (+/- 3.3) | 26.6 (+/- 3.2) | 26.5 (+/- 3.0) |
| Standard Cooling Device | 26.9 (+/- 3.9) | 27.0 (+/- 3.9) | 27.2 (+/- 3.8) | 27.6 (+/- 3.3) | 27.9 (+/- 3.1) |

| **Skin Temperature** | | | | | |
|---|---|---|---|---|---|
| Ice-Gel Pack | 14.7 (+/- 3.5) | 14.9 (+/- 3.3) | 15.9 (+/- 3.3) | 17.4 (+/- 3.5) | 18.7 (+/- 4.0) |
| Standard Cooling Device | 15.6 (+/- 2.1) | 15.4 (+/- 2.1) | 15.9 (+/- 2.0) | 17.0 (+/- 2.1) | 18.1 (+/- 2.4) |

| **Knee Pain (NPRS)** | | | | | |
|---|---|---|---|---|---|
| Ice-Gel Pack | 2.8 (+/- 1.5) | | 2.8 (+/- 2.1) | | 4.5 (+/- 3.3) |
| Standard Cooling Device | 1.8 (+/- 1.5) | | 1.8 (+/- 1.5) | | 1.3 (+/- 1.3) |

| **Cooling Method** | **125 min** | **130 min** | **135 min** |
|---|---|---|---|
| **IA Temperature** | | | |
| Ice-Gel Pack | 26.5 (+/- 2.7) | 26.6 (+/- 2.7) | 28.1 (+/- 2.0) |
| Standard Cooling Device | 28.3 (+/- 3.0) | 28.6 (+/- 2.8) | 30.2 |

| **Skin Temperature** | | | |
|---|---|---|---|
| Ice-Gel Pack | 19.9 (+/- 4.6) | 20.9 (+/- 4.9) | 22.0 (+/- 5.9) |
| Standard Cooling Device | 19.1 (+/- 2.7) | 19.8 (+/- 2.8) | 18.4 |

| **Knee Pain (NPRS)** | | | |
|---|---|---|---|
| Ice-Gel Pack | | 3.0 (+/- 1.4) | |
| Standard Cooling Device | | 1.5 (+/- 1.0) | |

**TABLE 2.**

| **Cooling Method** | **Baseline** | **5 min** | **10 min** | **15 min** | **20 min** |
|---|---|---|---|---|---|
| **IA Temperature** | | | | | |
| Ice-Gel Pack | 33.7 (+/- 0.8) | 33.5 (+/- 0.9) | 33.3 (+/- 1.0) | 33.0 (+/- 1.3) | 32.6 (+/- 1.5) |
| Standard Cooling Device | 32.5 (+/- 0.4) | 32.1 (+/- 0.5) | 31.9 (+/- 0.5) | 31.6 (+/- 0.6) | 31.3 (+/- 0.8) |

| **Skin Temperature** | | | | | |
|---|---|---|---|---|---|
| Ice-Gel Pack | 28.8 (+/- 0.9) | 26.1 (+/- 2.0) | 22.7 (+/- 2.2) | 18.9 (+/- 2.5) | 18.3 (+/- 2.6) |
| Standard Cooling Device | 28.7 (+/- 1.3) | 26.6 (+/- 1.9) | 24.5 (+/- 2.4) | 22.1 (+/- 3.1) | 21.1 (+/- 3.4) |

| **Knee Pain (NPRS)** | | | | | |
|---|---|---|---|---|---|
| Ice-Gel Pack | 0.8 (+/- 0.8) | | | | 0.6 (+/- 0.5) |
| Standard Cooling Device | 0.4 (+/- 0.5) | | | | 0.4 (+/- 0.5) |

| **Cooling Method** | **25 min** | **30 min** | **35 min** | **40 min** | **45 min** |
|---|---|---|---|---|---|
| **IA Temperature** | | | | | |
| Ice-Gel Pack | 31.7 (+/- 1.5) | 30.9 (+/- 1.5) | 30.9 (+/- 1.8) | 31.0 (+/- 2.0) | 30.8 (+/- 2.2) |
| Standard Cooling Device | 30.0 (+/- 1.5) | 29.1 (+/- 2.1) | 29.5 (+/- 1.6) | 29.7 (+/- 1.5) | 29.8 (+/- 1.5) |

| **Skin Temperature** | | | | | |
|---|---|---|---|---|---|
| Ice-Gel Pack | 20.4 (+/- 2.2) | 20.8 (+/- 1.7) | 19.1 (+/- 1.8) | 18.7 (+/- 2.0) | 18.8 (+/- 2.1) |
| Standard Cooling Device | 22.4 (+/- 2.9) | 22.4 (+/- 2.4) | 20.4 (+/- 2.7) | 19.3 (+/- 2.9) | 18.7 (+/- 2.9) |

| **Knee Pain (NPRS)** | | | | | |
|---|---|---|---|---|---|
| Ice-Gel Pack | | 0.4 (+/- 0.5) | | | |
| Standard Cooling Device | | 0.4 (+/- 0.5) | | | |

| **Cooling Method** | **50 min** | **55 min** | **60 min** | **65 min** | **70 min** |
|---|---|---|---|---|---|
| **IA Temperature** | | | | | |
| Ice-Gel Pack | 30.6 (+/- 2.5) | 30.3 (+/- 2.6) | 29.9 (+/- 2.2) | 29.6 (+/- 1.7) | 29.7 (+/- 1.5) |
| Standard Cooling Device | 29.8 (+/- 1.6) | 29.6 (+/- 1.8) | 29.6 (+/- 1.8) | 29.6 (+/- 2.7) | 29.0 (+/- 3.3) |

| **Skin Temperature** | | | | | |
|---|---|---|---|---|---|
| Ice-Gel Pack | 19.2 (+/- 2.2) | 19.6 (+/- 2.3) | 21.0 (+/- 2.3) | 20.1 (+/- 2.4) | 16.2 (+/- 2.5) |
| Standard Cooling Device | 18.2 (+/- 2.9) | 17.9 (+/- 3.1) | 19.0 (+/- 3.0) | 19.6 (+/- 3.2) | 18.5 (+/- 3.1) |

| **Knee Pain (NPRS)** | | | | | |
|---|---|---|---|---|---|
| Ice-Gel Pack | 0.6 (+/- 0.9) | | | | 4.2 (+/- 3.0) |
| Standard Cooling Device | 0.6 (+/- 0.9) | | | | 2.2 (+/- 3.3) |

| **Cooling Method** | **75 min** | **80 min** | **85 min** | **90 min** | **95 min** |
|---|---|---|---|---|---|
| **IA Temperature** | | | | | |
| Ice-Gel Pack | 29.7 (+/- 1.3) | 29.3 (+/- 1.4) | 28.8 (+/- 1.8) | 28.2 (+/- 2.3) | 27.2 (+/- 2.9) |
| Standard Cooling Device | 28.9 (+/- 3.5) | 28.7 (+/- 3.6) | 28.5 (+/- 3.8) | 28.3 (+/- 4.2) | 28.1 (+/- 4.5) |

| **Skin Temperature** | | | | | |
|---|---|---|---|---|---|
| Ice-Gel Pack | 14.1 (+/- 2.5) | 13.5 (+/- 2.7) | 13.3 (+/- 2.9) | 13.4 (+/- 3.0) | 13.9 (+/- 3.3) |
| Standard Cooling Device | 17.5 (+/- 2.6) | 16.7 (+/- 2.3) | 16.3 (+/- 2.1) | 15.9 (+/- 1.9) | 15.6 (+/- 1.8) |

| **Knee Pain (NPRS)** | | | | | |
|---|---|---|---|---|---|
| Ice-Gel Pack | | 3.6 (+/- 3.0) | | 2.6 (+/- 1.8) | |
| Standard Cooling Device | | 2.2 (+/- 2.7) | | 2.2 (+/- 2.2) | |

| **Cooling Method** | **100 min** | **105 min** | **110 min** | **115 min** | **120 min** |
|---|---|---|---|---|---|
| **IA Temperature** | | | | | |
| Ice-Gel Pack | 26.8 (+/- 2.9) | 26.7 (+/- 2.9) | 26.6 (+/- 2.9) | 26.6 (+/- 2.8) | 26.4 (+/- 2.6) |
| Standard Cooling Device | 28.1 (+/- 4.4) | 28.2 (+/- 4.3) | 28.3 (+/- 4.2) | 28.6 (+/- 3.7) | 28.8 (+/- 3.4) |

| **Skin Temperature** | | | | | |
|---|---|---|---|---|---|
| Ice-Gel Pack | 14.1 (+/- 3.4) | 14.3 (+/- 3.2) | 15.4 (+/- 3.1) | 17.0 (+/- 3.1) | 18.4 (+/- 3.6) |
| Standard Cooling Device | 15.4 (+/- 1.8) | 15.2 (+/- 1.9) | 15.6 (+/- 1.8) | 16.6 (+/- 2.1) | 17.7 (+/- 2.3) |

| **Knee Pain (NPRS)** | | | | | |
|---|---|---|---|---|---|
| Ice-Gel Pack | 2.2 (+/- 1.8) | | 2.2 (+/- 2.2) | | 3.6 (+/- 3.5) |
| Standard Cooling Device | 1.6 (+/- 1.3) | | 1.6 (+/- 1.3) | | 1.2 (+/- 1.1) |

| **Cooling Method** | **125 min** | **130 min** | **135 min** |
|---|---|---|---|
| **IA Temperature** | | | |
| Ice-Gel Pack | 26.4 (+/- 2.4) | 26.6 (+/- 2.4) | 28.1 (+/- 2.0) |
| Standard Cooling Device | 29.2 (+/- 3.3) | 29.5 (+/- 3.2) | 31.7 (+/- 2.1) |

| **Skin Temperature** | | | |
|---|---|---|---|
| Ice-Gel Pack | 19.6 (+/- 4.0) | 20.6 (+/- 4.3) | 22.0 (+/- 5.9) |
| Standard Cooling Device | 18.7 (+/- 2.5) | 19.5 (+/- 2.6) | 18.6 (+/- 0.3) |

| **Knee Pain (NPRS)** | | | |
|---|---|---|---|
| Ice-Gel Pack | | 2.4 (+/- 1.8) | |
| Standard Cooling Device | | 1.4 (+/- 0.9) | |

### EXAMPLE 9 - ANALYSIS OF PROCEDURE PAIN DUE TO INTRAARTICULAR ADMINISTRATION OF CAPSAICIN

Human patients experiencing osteoarthritic knee joint pain were subjected to two different protocols for intraarticular administration of trans-capsaicin. Temporary pain expected due to administration of capsaicin was analyzed, along with the intraarticular temperature of the knee joint and the temperature of the patient's skin in the area to be cooled.

One of the protocols utilized a Breg Knee WrapOn Polar Pad (as illustrated in Figure 1) that utilizes circulating ice-water to achieve cooling, where the pad is placed on skin surrounding the knee in order to cool the knee joint. The other protocol utilized an Elasto-Gel All Purpose Therapy Wrap measuring 6 inches by 24 inches in size to cool the knee joint. The Elasto-Gel All Purpose Therapy Wrap was removed from a freezer (approximately 0°F) just prior to use. A temperature probe was placed into the intraarticular space of the patient's knee joint to measure intraarticular temperature of the knee joint. A temperature probe was also placed on the skin in the area to be cooled in order to measure skin temperature in the area to be cooled.

### Experimental procedures and results are provided below

### Part I - Experimental Procedures

Five human patients suffering from moderate to severe painful bilateral knee osteoarthritis were recruited for this study evaluating the impact that cooling protocol has on the magnitude of temporary pain experienced by the patient due to administration of capsaicin. Patients had an average "pain with walking over the past 24 hours" for each knee in the range of 4-9 (inclusive) on a 0-10 numerical pain rating scale where "0" equals no pain and " 10" equals worst possible pain (NPRS). Patients that participated in the study passed the following screening criteria and meet the patient inclusion and exclusion criteria are set forth below. As part of patient screening, patients received an intradermal injection of 100 µL (100 µg) capsaicin into the non-dominant volar forearm. To be eligible to enter the study, patients were able to tolerate the capsaicin injection. Screening patients rated the pain from capsaicin injection at 5, 10, 20, and 30 minutes after injection using a 0-10 Numerical Pain Rating Scale, where "0" equals no pain and "10" equals worst possible pain.

Inclusion Criteria
1. Patient is male or female.
2. Patient is aged between 45 and 75 years, inclusive.
3. Patient has signed and dated an ethics approved informed consent form (ICF).
4. Patient's Body Mass Index (BMI) is between 18 and 32 kg/m2, inclusive and patient's weight is greater than or equal to 50 kg.
5. Patient has a diagnosis of bilateral moderate to severe painful knee osteoarthritis (patients will be required to have a score on Pain with walking in the previous 24 hours, of 4 to 9, inclusive (Numeric Pain Rating Scale 0-10). The condition must be chronic with a history of painful arthritis for at least 3 months prior to entry into the study.
6. All patients must otherwise be in good health, in the opinion of the Investigator, as determined by a medical history, physical examination, clinical laboratory tests, vital signs and 12 lead electrocardiogram (ECG).
7. Patients must be able to communicate well with the Investigator, understand and comply with the requirements of the study.
8. Patients have had previous bilateral AP radiographs (or CT/MRI scan) of the knees which demonstrate osteoarthritis in both knee joints within the prior 36 months.
9. Patient must be able to tolerate the capsaicin injection given at screening.

### Exclusion Criteria

1. Patient has had a clinically significant illness, other than osteoarthritis, that has not completely resolved in the four weeks before screening.
2. Patient has a history of neurological disorder which may impact the perception of pain or impairs the patient's ability to fully participate in the trial.
3. Patient has used analgesic medications in the 2 days prior to dosing, except for paracetamol, as needed.
4. Patient has used topical medications applied to the knee for osteoarthritis pain (including capsaicin, lidocaine, prescription or OTC medications) from 90 days prior to screening through to dosing.
5. Patient has been injected with corticosteroids in the knee 90 days prior to screening through to dosing.
6. Patient currently uses opioids for any condition other than osteoarthritis knee pain (maximum dose 15mg hydrocodone, or equivalent, per day prescribed by a physician).
7. Patient has physical/occupational/chiropractic therapy for the lower extremities or acupuncture for the lower extremities 30 days prior to screening or during the period to dosing.
8. Patient has had joint replacement surgery at any time, or open surgery of the knee in the past 12 months prior to screening, or prior arthroscopic surgery of the knee within 6 months prior to screening.
9. Patient has a history of a bleeding diathesis, or is using anti-coagulant drugs, excluding low dose aspirin.
10. Patient has a significant history of drug/solvent abuse or a positive drugs of abuse (DOA) test at screening. Prescribed opioids, as noted in exclusion, 6 are permitted.
11. Patient has a history of alcohol abuse or currently drinks more than 28 units per week.
12. Patient is, in the opinion of the Investigator, not suitable to participate in the study.
13. Patient has participated in any clinical study with an investigational drug/device within 3 months (or five half-lives if this longer than 3 months) prior to the first day of dosing.
14. Patient has a positive Human Immunodeficiency Virus (HIV), Hepatitis B or Hepatitis C screen.
15. Patient has lost or donated 500 mL or more of blood within the 3 months prior to screen, or intends to donate blood during the study.
16. Patient with active chronic pain conditions other than knee osteoarthritis, including periarticular pain about the knee.
17. Patient with known intolerance to capsaicin, hot peppers or any excipient in the investigational medicinal product or lidocaine.
18. Pregnant or breastfeeding females.
19. History of allergic reaction to the planned local anesthesia/ analgesic regimens, ethylenediaminetetraacetic acid (EDTA), Kolliphor HS 15, butylated hydroxytoluene (BHT), or capsaicin.
20. Patient has any active skin disorders, skin trauma, significant scarring or skin disease on either forearm, or a significant history of trauma or skin disease in either arm.
21. Any other severe acute or chronic medical or psychiatric condition, or laboratory abnormality, that may increase the risk associated with a) study participation b) Investigational product administration c) may interfere with the interpretation of study results and, in the judgment of the investigator, in discussion with the Sponsor, would make the patient inappropriate for entry into this study.

The following therapies were prohibited both prior to and during the study (i.e., prohibiting therapies):
- Injection of corticosteroids in the index knee from 90 days prior to Screening through study completion.
- Topical medications applied to the index knee for osteoarthritis pain (including capsaicin, lidocaine, prescription, or OTC medications) from 90 days prior to Screening through study completion.
- Current use of opioids for any condition other than for osteoarthritis of the index knee (maximum dose of 15 mg of hydrocodone [or equivalent] per day as background medication is allowed at entry if prescribed by a physician).
- Regular use of anticoagulant blood thinners.
- Use of an investigational medication within 30 days prior to Screening, or 5 PK or PD half- lives (whichever is longer), or scheduled to receive such an agent while participating in the study.
- Physical/ occupational/ chiropractic or acupuncture therapy for the lower extremities within 30 days of Screening, or need for such therapy during the course of the study.
- Joint replacement surgery of the index knee at any time, or open surgery of the index knee in the past 12 months prior to Screening, or prior arthroscopic surgery of the index knee within 6 months of Screening.
- Surgery, or other invasive procedures, or intraarticular injections (other than the study drug) while participating in the study.

The patient was excluded from study participation if they had taken any medication prior to randomization that would indicate that the patient has a serious or unstable illness, is not in good general health, or has a condition that would contraindicate study participation. If a patient received an excluded therapy after enrolment, continuation in the study was at the discretion of the sponsor/investigator/medical monitor. Patients were not to take a hot bath or shower, or expose the injected knee to external heat within 12 hours after the injection.

One of the protocols utilized a Breg Knee WrapOn Polar Pad (as illustrated in Figure 1) that utilizes circulating ice-water to achieve cooling, where the pad is placed on skin surrounding the knee in order to cool the knee joint. The other protocol utilized an Elasto-Gel All Purpose Therapy Wrap measuring 6 inches by 24 inches, whereby the wrap is placed around the knee joint in order to cool the knee joint. The Elasto-Gel All Purpose Therapy Wrap was removed from a freezer (approximately 0°F) just prior to use. A temperature probe was placed into the intraarticular space of the patient's knee joint to measure intraarticular temperature of the knee j oint. At the discretion of the physician performing the procedure, in order to reduce discomfort for the patient, the protocol authorized the physician to instill a volume of 1-2 cc of 2% w/w lidocaine (without epinephrine) into the skin and subcutaneous tissue of the knee at the site of intra-articular probe insertion. A temperature probe was also placed on the skin in the area to be cooled in order to measure skin temperature in the area to be cooled.

One the first day of the study, patients were randomized so that (i) three patients received cooling of the knee joint using the Breg Knee WrapOn Polar Pad and (ii) two patients received Elasto-Gel All Purpose Therapy Wrap as set forth above. Patients receiving cooling of the knee joint using the Breg Knee WrapOn Polar Pad were subjected to the following procedure:
∘ Placement of intraarticular temperature probe and skin temperature probe. At the discretion of the physician performing the procedure, in order to reduce discomfort for the patient, the protocol authorized the physician to instill a volume of 1-2 cc of 2% w/w lidocaine (without epinephrine) into the skin and subcutaneous tissue of the knee at the site of intra-articular probe insertion.
∘ Cooling using the designated technique was performed for 30 minutes (+/- 2 minutes).
∘ Cooling apparatus was removed from the patient's knee.
∘ Intraarticular injection of lidocaine 2% w/w (without epinephrine) 15 mL solution into the patient's knee joint.
∘ Intraarticular injection of the investigational medicinal product (IMP) was performed to deliver *trans*-capsaicin in the amount of 1 mg. The trans-capsaicin was injected, using the same needle as the lidocaine 2% w/w (without epinephrine), 3 minutes after the intra-articular injection of lidocaine 2% w/w (without epinephrine). The IMP was provided as a pre-filled syringe containing 2 mL of fluid containing *trans*-capsaicin at a concentration of 0.5 mg/mL.
∘ Knee joint was flexed and extended five times over 1 minute to ensure appropriate distribution of IMP.
∘ Cooling using the designated technique was performed for 10 minutes.
∘ The temperature probe was removed approximately 20 minutes after removal of the cooling apparatus.

Patients receiving cooling of the knee joint using the Elasto-Gel All Purpose Therapy Wrap were subjected to the following procedure:
∘ Placement of intraarticular temperature probe and skin temperature probe. At the discretion of the physician performing the procedure, in order to reduce discomfort for the patient, the protocol authorized the physician to instill a volume of 1-2 cc of 2% w/w lidocaine (without epinephrine) into the skin and subcutaneous tissue of the knee at the site of intra-articular probe insertion.
∘ Cooling using the designated technique was performed for 30 minutes (+/- 2 minutes).
∘ Cooling apparatus was removed from the patient's knee.
∘ Intraarticular injection of lidocaine 2% w/w (without epinephrine) 15 mL solution into the patient's knee joint.
∘ Intraarticular injection of the investigational medicinal product (IMP) was performed to deliver *trans*-capsaicin in the amount of 1 mg. The trans-capsaicin was injected, using the same needle as the lidocaine 2% w/w (without epinephrine), 3 minutes after the intra-articular injection of lidocaine 2% w/w (without epinephrine). The IMP was provided as a pre-filled syringe containing 2 mL of fluid containing *trans*-capsaicin at a concentration of 0.5 mg/mL.
∘ Knee joint was flexed and extended five times over 1 minute to ensure appropriate distribution of IMP.
∘ The temperature probe was removed approximately 30 minutes after removal of the cooling apparatus.

Temperatures within the knee and on the skin in the region undergoing cooling were obtained from the recording device at no less than 5 minute intervals (+/- 2 min) from the time of placement of the probes until the probes were removed.

Pain due to injection of *trans*-capsaicin was assessed on a Numerical Pain Rating scale (0-10) for 75 minutes after injection of *trans*-capsaicin. A verbal NPRS was used to assess procedure pain during the study. Patients were asked to indicate the severity of any pain experienced on a scale of 0 to 10 (NPRS; 0 corresponds to no pain and 10 corresponds to the worst pain imaginable). Patients were instructed to consider procedure pain separately from their baseline osteoarthritis pain. Pain was assessed on a Numerical Pain Rating scale (0-10) according to the following schedule:
∘ At pre-cooling after placement of the temperature probes.
∘ At rest, prior to intra-articular lidocaine injection.
∘ At rest, 10 minutes after intra-articular lidocaine injection.
∘ At rest prior to injection of *trans*-capsaicin.
∘ Ratings will continue at 10 minute intervals beginning 10 minutes after the injection of *trans*-capsaicin until removal of the temperature probe. The 10 minute intervals will allow for a +/- 2-minute variance in timing.

Patients were permitted to leave the clinic when they could ambulate independently, but no sooner than 1 hour after removal of the temperature probe.

Patients returned to the clinic 7±2 days later to have the procedure performed on their right knee.

### Part II - Results

The experimental procedure was completed successfully for four human patients. For the fifth patient subjected to the experimental procedure, there was a deviation from protocol. Experimental results are provided below for the four human patients for which the experimental procedure was completed successfully.

Mean values for intraarticular temperature in the knee joint recorded over time are presented in Figure 12, along with mean values for skin temperature recorded over time. Mean NPRS Pain scores recorded over time are presented in Figure 13, along with mean values for intraarticular temperature in the knee joint. Tabulated mean temperature values along with mean NPRS Pain scores recorded in the study are provided in Table 1 below.

**TABLE 1.**

| **Cooling Method** | **Baseline** | **5 min** | **10 min** | **15 min** | **20 min** |
|---|---|---|---|---|---|
| **IA Temperature** | | | | | |
| Ice-Gel Pack | 33.8 (+/- 1.7) | 33.5 (+/- 2.0) | 33.4 (+/- 2.2) | 33.2 (+/- 2.4) | 33.0 (+/- 2.7) |
| Standard Cooling Device | 32.5 (+/- 2.6) | 31.9 (+/- 2.8) | 31.5 (+/- 2.9) | 31.2 (+/- 3.1) | 30.8 (+/- 3.4) |

| **Skin Temperature** | | | | | |
|---|---|---|---|---|---|
| Ice-Gel Pack | 28.4 (+/- 0.9) | 26.5 (+/- 1.0) | 23.2 (+/- 1.1) | 19.2 (+/- 1.1) | 17.7 (+/- 1.4) |
| Standard Cooling Device | 27.7 (+/- 0.9) | 26.7 (+/- 1.0) | 25.0 (+/- 1.2) | 22.9 (+/- 1.4) | 21.7 (+/- 1.3) |

| **Knee Pain (NPRS)** | | | | | |
|---|---|---|---|---|---|
| Ice-Gel Pack | 0.3 (+/- 0.5) | | | | |
| Standard Cooling Device | 0.0 (+/- 0.0) | | | | |

| **Cooling Method** | **25 min** | **30 min** | **35 min** | **40 min** | **45 min** |
|---|---|---|---|---|---|
| **IA Temperature** | | | | | |
| Ice-Gel Pack | 32.7 (+/- 3.0) | 32.3 (+/- 3.3) | 31.8 (+/- 3.1) | 30.4 (+/- 2.5) | 28.7 (+/- 2.7) |
| Standard Cooling Device | 30.4 (+/- 3.6) | 29.9 (+/- 3.7) | 29.3 (+/- 3.8) | 28.5 (+/- 3.6) | 27.5 (+/- 3.5) |

| **Skin Temperature** | | | | | |
|---|---|---|---|---|---|
| Ice-Gel Pack | 17.1 (+/- 1.8) | 16.9 (+/- 2.1) | 17.4 (+/- 2.2) | 19.5 (+/- 1.9) | 21.4 (+/- 1.5) |
| Standard Cooling Device | 20.6 (+/- 1.3) | 19.7 (+/- 1.3) | 19.4 (+/- 1.1) | 20.7 (+/- 0.8) | 21.2 (+/- 0.9) |

| **Knee Pain (NPRS)** | | | | | |
|---|---|---|---|---|---|
| Ice-Gel Pack | | 0.0 (+/- 0.0) | | 00 (+/- 00) | |
| Standard Cooling Device | | 0.0 (+/- 0.0) | | 0.5 (+/- 1.0) | |

| **Cooling Method** | **50 min** | **55 min** | **60 min** | **65 min** | **70 min** |
|---|---|---|---|---|---|
| **IA Temperature** | | | | | |
| Ice-Gel Pack | 28.4 (+/- 3.3) | 28.5 (+/- 3.5) | 28.4 (+/- 3.5) | | |
| Standard Cooling Device | 27.1 (+/- 4.3) | 27.0 (+/- 4.8) | 27.1 (+/- 4.5) | 27.3 (+/- 3.8) | 28.0 (+/- 3.2) |

| **Skin Temperature** | | | | | |
|---|---|---|---|---|---|
| Ice-Gel Pack | 22.0 (+/- 1.4) | 22.6 (+/- 1.5) | 23.3 (+/- 1.6) | | |
| Standard Cooling Device | 19.5 (+/- 1.2) | 18.7 (+/- 1.2) | 19.6 (+/- 1.0) | 21.2 (+/- 1.2) | 22.5 (+/- 1.6) |

| **Knee Pain (NPRS)** | | | | | |
|---|---|---|---|---|---|
| Ice-Gel Pack | 1.5 (+/- 2.4) | | 2.8 (+/- 3.1) | | 1.0 (+/- 0.8) |
| Standard Cooling Device | 3.8 (+/- 2.9) | | 1.8 (+/- 2.4) | | 1.0 (+/- 2.0) |

| **Cooling Method** | **75 min** | **80 min** | **90 min** | **100 min** | **110 min** |
|---|---|---|---|---|---|
| **IA Temperature** | | | | | |
| Ice-Gel Pack | | | | | |
| Standard Cooling Device | 28.3 (+/- 3.3) | 28.5 (+/- 3.3) | | | |

| Skin Temperature | | | | | |
|---|---|---|---|---|---|
| Ice-Gel Pack | 23.2 (+/- 2.2) | 23.9 (+/- 2.7) | | | |
| Standard Cooling Device | | | | | |

| **Knee Pain (NPRS)** | | | | | |
|---|---|---|---|---|---|
| Ice-Gel Pack | | 1.8 (+/- 2.9) | 2.5 (+/- 2.9) | 1.8 (+/- 2.9) | 0.8 (+/- 1.5) |
| Standard Cooling Device | | 2.0 (+/- 4.0) | 1.5 (+/- 2.4) | 0.5 (+/- 1.0) | 2.0 (+/- 4.0) |

### EXAMPLE 10 - TEMPERATURE PROFILE ANALYSIS OF COOLING APPARATUS

The temperature of the cooling surface of the following cooling devices was measured over time: (i) Breg Knee WrapOn Polar Pad (as illustrated in Figure 1) that utilizes circulating ice-water to achieve cooling, (ii) an Elasto-Gel All Purpose Therapy Wrap measuring 6 inches by 24 inches in size, which was removed from a freezer immediately prior to use, and (iii) an ice-pack wrapped in a stockinet of thickness analogous to that used when an ice pack is used to provide cooling a human patient's knee (in which the patient's knee is wrapped in a stockinet to avoid direct contact between the ice pack and the patient's skin). Temperature values recorded as a function of time are presented in Table 1 below. Temperature values are also displayed graphically in Figure 14. The first temperature value in Table 1 was taken 5 minutes after the start of the experiment in order to allow equilibration of the cooling surface temperature from initiation of the experiment. Temperature values provided for the Breg Knee WrapOn Polar Pad are the average temperature observed for the first cooling pad and the second cooling pad of the device. The Elasto-Gel All Purpose Therapy Wrap and the ice-pack were removed from a freezer (approximately 0°F) just prior to use.

**TABLE 1.**

| **Time (min)** | **Temperature of Cooling Surface (°F)** | | |
|---|---|---|---|
| | **Breg Knee WrapOn Polar Pad** | **Elasto-Gel** | **Ice Pack Wrapped in Stockinet** |
| 5 | 53.6 | 42.9 | 40.6 |
| 6 | 53.4 | 43.2 | 40.1 |
| 7 | 53.25 | 43.7 | 39.9 |
| 8 | 53 | 43.9 | 39.5 |
| 9 | 53.25 | 44.2 | 39.2 |
| 10 | 52.6 | 44.9 | 39.1 |
| 11 | 52.25 | 45.1 | 38.8 |
| 12 | 52.05 | 45.5 | 38.8 |
| 13 | 51.85 | 45.9 | 38.8 |
| 14 | 52.75 | 46.1 | 38.6 |
| 15 | 52.45 | 46.4 | 38.6 |
| 16 | 50.4 | 46.6 | 38.4 |
| 17 | 49.95 | 47 | 38.4 |
| 18 | 49.1 | 47.2 | 38.4 |
| 19 | 48.4 | 47.4 | 38.4 |
| 20 | 48.15 | 47.6 | 38.4 |
| 21 | 48.1 | 48 | 38.4 |
| 22 | 47.8 | 48.1 | 38.3 |
| 23 | 47.75 | 48.4 | 38.3 |
| 24 | 47.9 | 49.1 | 38.3 |
| 25 | 47.6 | 49.2 | 37.9 |
| 26 | 47.6 | 49.4 | 37.5 |
| 27 | 47.6 | 49.8 | 36.8 |
| 28 | 47.55 | 50.1 | 36.8 |
| 29 | 47.45 | 50.3 | 36.8 |
| 30 | 47.45 | 50.7 | 37 |
| 31 | 47.35 | 50.9 | 37.2 |
| 32 | 47.35 | 51.2 | 37.2 |
| 33 | 47.25 | 51.6 | 37.4 |
| 34 | 47.15 | 51.9 | 37.5 |
| 35 | 47.2 | 52.5 | 37.6 |
| 36 | 47.15 | 52.5 | 37.8 |
| 37 | 47.05 | 52.8 | 37.7 |
| 38 | 47 | 53.2 | 37.7 |
| 39 | 47 | 53.4 | 37.9 |
| 40 | 47.05 | 53.7 | 38.1 |
| 41 | 47.05 | 54.1 | 38.1 |
| 42 | 47.05 | 54.3 | 38.3 |
| 43 | 47.05 | 54.6 | 38.4 |
| 44 | 46.9 | 54.8 | 38.3 |
| 45 | 47.05 | 55 | 38.3 |
| 46 | 47.05 | 55.2 | 38.4 |
| 47 | 47.05 | 55.5 | 38.5 |
| 48 | 47 | 55.7 | 38.6 |
| 49 | 47.05 | 56.1 | 38.6 |
| 50 | 47.05 | 56.3 | 38.8 |
| 51 | 47.05 | 56.4 | 38.8 |
| 52 | 47.05 | 56.8 | 38.8 |
| 53 | 47.15 | 57.1 | 39 |
| 54 | 47.15 | 57.2 | 39 |
| 55 | 47.25 | 57.5 | 39.2 |
| 56 | 47.1 | 57.7 | 39 |
| 57 | 47.05 | 57.9 | 39 |
| 58 | 47.1 | 58.1 | 39.2 |
| 59 | 47.05 | 58.6 | 39.2 |
| 60 | 47.15 | 59 | 39.3 |
| 61 | 47.05 | 59 | 39.3 |
| 62 | 47.05 | 59.3 | 39.3 |
| 63 | 47.05 | 59.5 | 39.5 |
| 64 | 46.95 | 59.7 | 39.5 |
| 65 | 47.05 | 59.9 | 39.7 |
| 66 | 47.05 | 60.2 | 39.7 |
| 67 | 47.05 | 60.2 | 39.7 |
| 68 | 46.85 | 60.2 | 39.5 |
| 69 | 46.85 | 60.4 | 39.7 |
| 70 | 46.9 | 60.6 | 39.7 |
| 71 | 46.95 | 60.8 | 39.7 |
| 72 | 46.95 | 60.9 | 39.9 |
| 73 | 46.95 | 61.1 | 39.9 |
| 74 | 46.95 | 61.3 | 39.9 |
| 75 | 47.05 | 61.5 | 39.9 |
| 76 | 47.05 | 61.7 | 39.9 |
| 77 | 47.25 | 61.8 | 40.1 |
| 78 | 47.15 | 62 | 40.1 |
| 79 | 47.15 | 62.2 | 40.1 |
| 80 | 47.15 | 62.4 | 40.2 |
| 81 | 47.2 | 62.4 | 40.2 |
| 82 | 47.25 | 62.7 | 40.2 |
| 83 | 47.15 | 62.9 | 40.2 |
| 84 | 47.25 | 63.1 | 40.2 |
| 85 | 47.25 | 63.3 | 40.2 |
| 86 | 47.2 | 63.5 | 40.2 |
| 87 | 47.1 | 63.6 | 40.2 |
| 88 | 47.05 | 63.6 | 40.4 |
| 89 | 47.05 | 63.8 | 40.4 |
| 90 | 47.1 | 64 | 40.4 |
| 91 | 47.1 | 64 | 40.6 |
| 92 | 47 | 64.2 | 40.6 |
| 93 | 47 | 64.4 | 40.6 |
| 94 | 47 | 64.5 | 40.6 |
| 95 | 47.05 | 64.5 | 40.6 |
| 96 | 47 | 64.7 | 40.6 |
| 97 | 46.95 | 64.7 | 40.6 |
| 98 | 47.05 | 65.1 | 40.6 |
| 99 | 47.15 | 65.1 | 40.8 |
| 100 | 47.15 | 65.3 | 40.8 |
| 101 | 47.15 | 65.4 | 40.8 |
| 102 | 47.15 | 65.4 | 40.8 |
| 103 | 47.15 | 65.6 | 41 |
| 104 | 47.15 | 65.6 | 41 |
| 105 | 47.25 | 65.7 | 41 |
| 106 | 47.15 | 66 | 41 |
| 107 | 47.25 | 66 | 41 |
| 108 | 47.25 | 66.2 | 41 |
| 109 | 47.2 | 66.3 | 41 |
| 110 | 47.25 | 66.3 | 41 |
| 111 | 47.05 | 66.5 | 41 |
| 112 | 47.05 | 66.7 | 41 |
| 113 | 47.15 | 66.7 | 41 |
| 114 | 47.05 | 66.8 | 41 |
| 115 | 47.05 | 66.9 | 41 |
| 116 | 47.05 | 67.1 | 41 |
| 117 | 46.95 | 67.1 | 41 |
| 118 | 47.15 | 67.1 | 41 |
| 119 | 47.15 | 67.2 | 41 |
| 120 | 47.15 | 67.4 | 41.1 |
| 121 | 47.15 | 67.4 | 41.1 |

The invention may be embodied in other specific forms without departing from the essential characteristics thereof. The foregoing embodiments are therefore to be considered in all respects illustrative rather than limiting the invention described herein. Scope of the invention is thus indicated by the appended claims rather than by the foregoing description.

## Claims

1. Capsaicin for use in ameliorating osteoarthritic knee joint pain in a human patient for a duration of at least 8 months, wherein the use comprises administering by injection into the intra-articular space of the joint of said knee at least a first dose of capsaicin and a second dose of capsaicin to thereby ameliorate osteoarthritic knee joint pain in the human patient for a duration of at least 8 months, wherein the use is **characterized by**:
(i) the first dose of capsaicin is an amount of 1 mg capsaicin;
(ii) the second dose of capsaicin is an amount of 1 mg capsaicin;
(iii) the second dose of capsaicin is administered no sooner than 5 months after administration of the first dose of capsaicin;
(iv) if any additional dose of capsaicin is administered by injection into the intra-articular space of the joint of said knee, any such additional dose is in an amount of 1 mg of capsaicin and any said additional dose is administered no sooner than 3 months after administration of the prior dose of capsaicin administered by injection into the intra-articular space of the joint of said knee; and
(v) when administering the first dose of capsaicin:
(a) a cooling article is applied for a duration of 15 minutes to an exterior surface of the human patient's knee presenting with osteoarthritic knee joint pain, wherein the cooling article has an exterior surface temperature in the range of from 3°C to 15°C for application to the exterior surface of said knee; then
(b) a pharmaceutical composition comprising a single pain-relief agent selected from the group consisting of lidocaine and a pharmaceutically acceptable salt thereof is administered by injection into the intra-articular space of the joint of said knee in order to deliver a dose of lidocaine in an amount ranging from 0.1 g to 0.5 g; then
(c) a cooling article is applied for a duration of 30 minutes to an exterior surface of said knee, wherein the cooling article has an exterior surface temperature in the range of from 3°C to 15°C for application to the exterior surface of said knee; then
(d) the first dose of capsaicin is administered by injecting into the intra-articular space of the joint of said knee a pharmaceutical composition comprising capsaicin; and then
(e) a cooling article is applied to an exterior surface of said knee for a duration of at least 30 minutes, wherein the cooling article has an exterior surface temperature in the range of from 3°C to 15°C for application to the exterior surface of said knee.

2. Capsaicin for use in ameliorating knee joint pain in a human patient for a duration of at least 8 months, wherein the use comprises administering by injection into the intra-articular space of the joint of said knee at least a first dose of capsaicin and a second dose of capsaicin to thereby ameliorate knee joint pain in the human patient for a duration of at least 8 months, wherein the use is **characterized by**:
(i) the first dose of capsaicin is an amount of 1 mg capsaicin;
(ii) the second dose of capsaicin is an amount of 1 mg capsaicin;
(iii) the second dose of capsaicin is administered no sooner than 5 months after administration of the first dose of capsaicin; and
(iv) if any additional dose of capsaicin is administered by injection into the intra-articular space of the joint of said knee, any such additional dose is in an amount of 1 mg of capsaicin and any said additional dose is administered no sooner than 3 months after administration of the prior dose of capsaicin administered by injection into the intra-articular space of the joint of said knee.

3. The capsaicin for use according to claim 2, wherein the joint pain is osteoarthritic joint pain.

4. The capsaicin for use according to any one of claims 2-3, wherein when administering the first dose of capsaicin:
(a) optionally a cooling article is applied to an exterior surface of the human patient's knee presenting with joint pain; then
(b) optionally a local anesthetic agent is administered by injection into the intra-articular space of said knee joint; then
(c) a cooling article is applied for a duration of at least 10 minutes to an exterior surface of said knee, wherein the cooling article has an exterior surface temperature in the range of from 1°C to 15°C for application to the exterior surface of said knee; then
(d) the first dose of capsaicin is administered by injecting into the intra-articular space of the joint of said knee a pharmaceutical composition comprising capsaicin; and then
(e) optionally a cooling article is applied for a duration of at least 10 minutes to an exterior surface of said knee, wherein the cooling article has an exterior surface temperature in the range of from 1°C to 15°C for application to the exterior surface of said knee.

5. The capsaicin for use according to any one of claims 2-4, wherein when administering the second dose of capsaicin:
(a) optionally a cooling article is applied to an exterior surface of the human patient's knee presenting with joint pain; then
(b) optionally a local anesthetic agent is administered by injection into the intra-articular space of said knee joint; then
(c) a cooling article is applied for a duration of at least 10 minutes to an exterior surface of said knee, wherein the cooling article has an exterior surface temperature in the range of from 1°C to 15°C for application to the exterior surface of said knee; then
(d) the second dose of capsaicin is administered by injecting into the intra-articular space of the joint of said knee a pharmaceutical composition comprising capsaicin; and then
(e) optionally a cooling article is applied for a duration of at least 10 minutes to an exterior surface of said knee, wherein the cooling article has an exterior surface temperature in the range of from 1°C to 15°C for application to the exterior surface of said knee.

6. The capsaicin for use according to any one of claims 2-5, wherein when administering any additional dose of capsaicin:
(a) optionally a cooling article is applied to an exterior surface of the human patient's knee presenting with joint pain; then
(b) optionally a local anesthetic agent is administered by injection into the intra-articular space of said knee joint; then
(c) a cooling article is applied for a duration of at least 10 minutes to an exterior surface of said knee, wherein the cooling article has an exterior surface temperature in the range of from 1°C to 15°C for application to the exterior surface of said knee; then
(d) the additional dose of capsaicin is administered by injecting into the intra-articular space of the joint of said knee a pharmaceutical composition comprising capsaicin; and then
(e) optionally a cooling article is applied for a duration of at least 10 minutes to an exterior surface of said knee, wherein the cooling article has an exterior surface temperature in the range of from 1°C to 15°C for application to the exterior surface of said knee.

7. The capsaicin for use according to any one of claims 4-6, wherein:
(i) in step (c) the cooling article is applied for a duration of from 15 minutes to 45 minutes to the exterior surface of said knee,
(ii) in step (c) the cooling article is applied for a duration of 45 minutes to the exterior surface of said knee,
(iii) in step (c) the cooling article is applied for a duration of 30 minutes to the exterior surface of said knee, or
(iv) in step (c) the cooling article is applied for a duration of 20 minutes to the exterior surface of said knee.

8. The capsaicin for use according to any one of claims 2-3, wherein when administering the first dose of capsaicin:
(a) optionally a cooling article is applied to an exterior surface of the human patient's knee presenting with joint pain; then
(b) optionally a local anesthetic agent is administered by injection into the intra-articular space of said knee joint; then
(c) a cooling article is applied to an exterior surface of said knee to achieve a temperature in the range of from 28°C to 30°C for tissue or fluid in the interior of said knee joint; then
(d) the first dose of capsaicin is administered by injecting into the intra-articular space of the joint of said knee a pharmaceutical composition comprising capsaicin; and then
(e) optionally a cooling article is applied to an exterior surface of said knee.

9. The capsaicin for use according to any one of claims 2, 3, or 8, wherein when administering the second dose of capsaicin:
(a) optionally a cooling article is applied to an exterior surface of the human patient's knee presenting with joint pain; then
(b) optionally a local anesthetic agent is administered by injection into the intra-articular space of said knee joint; then
(c) a cooling article is applied to an exterior surface of said knee to achieve a temperature in the range of from 28°C to 30°C for tissue or fluid in the interior of said knee joint; then
(d) the second dose of capsaicin is administered by injecting into the intra-articular space of the joint of said knee a pharmaceutical composition comprising capsaicin;
and then
optionally a cooling article is applied to an exterior surface of said knee.

10. The capsaicin for use according to any one of claims 2, 3, 8, or 9, wherein when administering any additional dose of capsaicin:
(a) optionally a cooling article is applied to an exterior surface of the human patient's knee presenting with joint pain; then
(b) optionally a local anesthetic agent is administered by injection into the intra-articular space of said knee joint; then
(c) a cooling article is applied to an exterior surface of said knee to achieve a temperature in the range of from 28°C to 30°C for tissue or fluid in the interior of said knee joint; then
(d) the additional dose of capsaicin is administered by injecting into the intra-articular space of the joint of said knee a pharmaceutical composition comprising capsaicin; and then
(e) optionally a cooling article is applied to an exterior surface of said knee.

11. The capsaicin for use according to any one of claims 4-10, wherein the use is further **characterized by**:
(i) the use comprises the following additional step that is performed between steps (c) and (d): administering a local anesthetic agent by injection into the intra-articular space of said knee joint,
(ii) the local anesthetic agent is a caine analgesic,
(iii) the local anesthetic agent is lidocaine or a pharmaceutically acceptable salt thereof,
(iv) the local anesthetic agent is lidocaine hydrochloride,
(v) the local anesthetic agent is delivered at a dose sufficient to deliver lidocaine in an amount of 0.1 g to 0.5 g,
(vi) the local anesthetic agent is delivered at a dose sufficient to deliver lidocaine in an amount of 0.15 g,
(vii) the local anesthetic agent is delivered at a dose sufficient to deliver lidocaine in an amount of 0.3 g,
(viii) the local anesthetic agent is administered in the form of a pharmaceutical composition having a volume in the range of from 13 mL to 17 mL,
(ix) the local anesthetic agent is administered in the form of a pharmaceutical composition having a volume of 15 mL,
(x) the local anesthetic agent is administered in the form of a pharmaceutical composition that is an aqueous mixture containing lidocaine at a concentration of 2% w/w, or
(xi) the local anesthetic agent is administered in the form of a pharmaceutical composition that is an aqueous mixture containing lidocaine at a concentration of 1% w/w.

12. The capsaicin for use according to any one of claims 4-11, wherein other than administration of (i) a local anesthetic agent and (ii) the first dose of capsaicin, the second dose of capsaicin, and any additional dose of capsaicin, the human patient does not receive any other pain-relief medicine.

13. The capsaicin for use according to any one of claims 1 or 4-12, wherein:
(i) the cooling article has an exterior surface temperature in the range of from 5°C to 15°C for application to the exterior surface of the human patient's knee,
(ii) the cooling article has an exterior surface temperature in the range of from 6°C to 13°C for application to the exterior surface of the human patient's knee,
(iii) the cooling article has an exterior surface temperature in the range of from 7°C to 13°C for application to the exterior surface of the human patient's knee,
(iv) the cooling article has an exterior surface temperature in the range of from 7°C to 10°C for application to the exterior surface of the human patient's knee,
(v) the cooling article has an exterior surface temperature in the range of from 5°C to 10°C for application to the exterior surface of the human patient's knee,
(vi) the cooling article has an exterior surface temperature in the range of from 6°C to 12°C for application to the exterior surface of the human patient's knee,
(vii) the cooling article has an exterior surface temperature of 9°C for application to the exterior surface of the human patient's knee,
(viii) the cooling article has an exterior surface temperature of 10°C for application to the exterior surface of the human patient's knee, or
(ix) cooling article has an exterior surface temperature of 11°C for application to the exterior surface of the human patient's knee.

14. The capsaicin for use according to any one of claims 1 or 4-13, wherein the pharmaceutical composition comprising capsaicin is an aqueous injectable formulation that comprises:
a. 0.03% (w/w) to 0.3% (w/w) of capsaicin;
b. 0.1% (w/w) to 3% (w/w) of a solubilizing agent, wherein the solubilizing agent comprises (i) a polyethylene glycol ester of a (C₁₅-C₂₅) hydroxyalkanoic acid, (ii) a polyethylene glycol ester of a (C₁₅-C₂₅) hydroxyalkenoic acid, or (iii) a polyethylene glycol ester of a (C₁₅-C₂₅) alkanoic acid substituted by a -OC(O)(C₁₄-C₂₄) hydroxyalkyl group;
c. 0.001% (w/w) to 2% (w/w) of an antioxidant; and
d. at least 92% (w/w) water; and wherein optionally the solubilizing agent comprises (a) 70% (w/w) of a mixture of 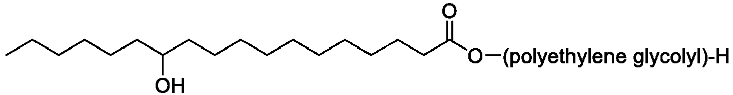 and 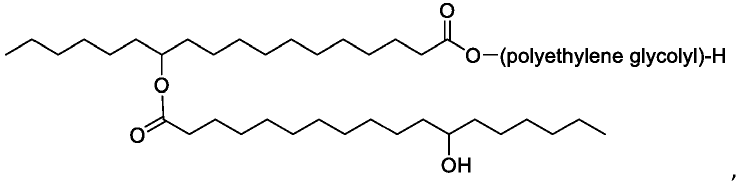 and (b) 30% (w/w) polyethylene glycol.

15. The capsaicin for use according to any one of claims 1-14, wherein the second dose of capsaicin is administered:
(i) no sooner than 6 months after administration of the first dose of capsaicin,
(ii) no sooner than 7 months after administration of the first dose of capsaicin,
(iii) no sooner than 8 months after administration of the first dose of capsaicin, or
(iv) no sooner than 9 months after administration of the first dose of capsaicin.

16. The capsaicin for use according to any one of claims 1-14, wherein the second dose of capsaicin is administered:
(i) at a time that is in the range of 6 months to 8 months after administration of the first dose of capsaicin,
(ii) at a time that is in the range of 7 months to 9 months after administration of the first dose of capsaicin,
(iii) at a time that is in the range of 6 months to 9 months after administration of the first dose of capsaicin,
(iv) 6 months after administration of the first dose of capsaicin,
(v) 26 weeks after administration of the first dose of capsaicin,
(vi) 7 months after administration of the first dose of capsaicin, or
(vii) 8 months after administration of the first dose of capsaicin.

17. The capsaicin for use according to any one of claims 1-16, wherein the pain is ameliorated for:
(i) a duration of at least 10 months,
(ii) a duration of at least 11 months,
(iii) a duration of at least 12 months,
(iv) a duration of at least 13 months, or
(v) a duration of at least 14 months.

18. The capsaicin for use according to any one of claims 1-17, wherein the use is **characterized by**:
(i) achieving a reduction in average pain in the knee with walking over the previous twenty-four hours by at least 1 on the Numeric Pain Rating Scale of 0-10 (NPRS) for a duration of at least 8 months, or
(ii) achieving a reduction in average pain in the knee with walking over the previous twenty-four hours by at least 1 on the Numeric Pain Rating Scale of 0-10 (NPRS) for a duration of at least 9 months,
(iii) achieving a reduction in average pain in the knee with walking over the previous twenty-four hours by at least 1 on the Numeric Pain Rating Scale of 0-10 (NPRS) for a duration of at least 10 months,
(iv) achieving a reduction in average pain in the knee with walking over the previous twenty-four hours by at least 1 on the Numeric Pain Rating Scale of 0-10 (NPRS) for a duration of at least 11 months, or
(v) achieving a reduction in average pain in the knee with walking over the previous twenty-four hours by at least 1 on the Numeric Pain Rating Scale of 0-10 (NPRS) for a duration of at least 12 months.

19. The capsaicin for use according to any one of claims 1-17, wherein the use is **characterized by**:
(i) achieving a reduction in average pain in the knee with walking over the previous twenty-four hours by at least 2 on the Numeric Pain Rating Scale of 0-10 (NPRS) for a duration of at least 8 months,
(ii) achieving a reduction in average pain in the knee with walking over the previous twenty-four hours by at least 2 on the Numeric Pain Rating Scale of 0-10 (NPRS) for a duration of at least 9 months,
(iii) achieving a reduction in average pain in the knee with walking over the previous twenty-four hours by at least 2 on the Numeric Pain Rating Scale of 0-10 (NPRS) for a duration of at least 10 months,
(iv) achieving a reduction in average pain in the knee with walking over the previous twenty-four hours by at least 1 on the Numeric Pain Rating Scale of 0-10 (NPRS) for a duration of at least 11 months,
(v) achieving a reduction in average pain in the knee with walking over the previous twenty-four hours by at least 2 on the Numeric Pain Rating Scale of 0-10 (NPRS) for a duration of at least 12 months.

20. The capsaicin for use according to any one of claims 1-19, wherein the use is **characterized by**:
(i) reducing the patient's average pain in the knee with walking so that the patient's average pain in the knee with walking is no greater than 1 on the Numeric Pain Rating Scale of 0-10 (NPRS) for a duration of at least 8 months,
(ii) reducing the patient's average pain in the knee with walking so that the patient's average pain in the knee with walking is no greater than 1 on the Numeric Pain Rating Scale of 0-10 (NPRS) for a duration of at least 9 months,
(iii) reducing the patient's average pain in the knee with walking so that the patient's average pain in the knee with walking is no greater than 1 on the Numeric Pain Rating Scale of 0-10 (NPRS) for a duration of at least 10 months,
(iv) reducing the patient's average pain in the knee with walking so that the patient's average pain in the knee with walking is no greater than 1 on the Numeric Pain Rating Scale of 0-10 (NPRS) for a duration of at least 11 months, or
(v) reducing the patient's average pain in the knee with walking so that the patient's average pain in the knee with walking is no greater than 1 on the Numeric Pain Rating Scale of 0-10 (NPRS) for a duration of at least 12 months.

21. The capsaicin for use according to any one of claims 1-19, wherein the use is **characterized by**:
(i) reducing the patient's average pain in the knee with walking so that the patient's average pain in the knee with walking is no greater than 2 on the Numeric Pain Rating Scale of 0-10 (NPRS) for a duration of at least 8 months,
(ii) reducing the patient's average pain in the knee with walking so that the patient's average pain in the knee with walking is no greater than 2 on the Numeric Pain Rating Scale of 0-10 (NPRS) for a duration of at least 9 months,
(iii) reducing the patient's average pain in the knee with walking so that the patient's average pain in the knee with walking is no greater than 2 on the Numeric Pain Rating Scale of 0-10 (NPRS) for a duration of at least 10 months,
(iv) reducing the patient's average pain in the knee with walking so that the patient's average pain in the knee with walking is no greater than 2 on the Numeric Pain Rating Scale of 0-10 (NPRS) for a duration of at least 11 months, or
(v) reducing the patient's average pain in the knee with walking so that the patient's average pain in the knee with walking is no greater than 2 on the Numeric Pain Rating Scale of 0-10 (NPRS) for a duration of at least 12 months.

## Patentansprüche

1. Capsaicin zur Verwendung bei der Linderung von osteoarthritischen Kniegelenkschmerzen bei einem menschlichen Patienten für eine Dauer von mindestens 8 Monaten, wobei die Verwendung die Verabreichung von mindestens einer ersten Capsaicin-Dosis und einer zweiten Capsaicin-Dosis durch Injektion in den intraartikulären Raum des Gelenks des Knies umfasst, um dadurch osteoarthritische Kniegelenkschmerzen bei dem menschlichen Patienten für eine Dauer von mindestens 8 Monaten zu lindern, wobei die Verwendung **dadurch gekennzeichnet ist, dass**:
(i) die erste Capsaicin-Dosis einer Menge von 1 mg Capsaicin entspricht;
(ii) die zweite Capsaicin-Dosis einer Menge von 1 mg Capsaicin entspricht;
(iii) die zweite Capsaicin-Dosis nicht früher als 5 Monate nach Verabreichung der ersten Capsaicin-Dosis verabreicht wird;
(iv) wenn eine zusätzliche Capsaicin-Dosis durch Injektion in den intraartikulären Raum des Gelenks des Knies verabreicht wird, jede solche zusätzliche Dosis in einer Menge von 1 mg Capsaicin liegt und jede solche zusätzliche Dosis nicht früher als 3 Monate nach der Verabreichung der vorherigen Capsaicin-Dosis, die durch Injektion in den intraartikulären Raum des Gelenks des Knies verabreicht wurde, verabreicht wird; und
(v) bei der Verabreichung der ersten Capsaicin-Dosis:
(a) ein Kühlartikel für eine Dauer von 15 Minuten auf die äußere Oberfläche des Knies eines menschlichen Patienten mit osteoarthritischen Kniegelenkschmerzen aufgetragen wird, wobei der Kühlartikel eine äußere Oberflächentemperatur im Bereich von 3 °C bis 15 °C zum Aufbringen auf die äußere Oberfläche des Knies aufweist; dann
(b) eine pharmazeutische Zusammensetzung, die ein einzelnes schmerzlinderndes Mittel enthält, das aus der Gruppe ausgewählt ist, die aus Lidocain und einem pharmazeutisch annehmbaren Salz davon besteht, durch Injektion in den intraartikulären Raum des Gelenks des Knies verabreicht wird, um eine Dosis Lidocain in einer Menge von 0,1 g bis 0,5 g abzugeben; dann
(c) ein Kühlartikel für eine Dauer von 30 Minuten auf eine äußere Oberfläche des Knies aufgebracht wird, wobei der Kühlartikel eine äußere Oberflächentemperatur im Bereich von 3 °C bis 15 °C zum Aufbringen auf die äußere Oberfläche des Knies aufweist; dann
(d) die erste Capsaicin-Dosis durch Injektion einer Capsaicin umfassenden pharmazeutischen Zusammensetzung in den intraartikulären Raum des Gelenks des Knies verabreicht wird; und dann
(e) ein Kühlartikel für eine Dauer von mindestens 30 Minuten auf eine äußere Oberfläche des Knies aufgebracht wird, wobei der Kühlartikel eine äußere Oberflächentemperatur im Bereich von 3 °C bis 15 °C zum Aufbringen auf die äußere Oberfläche des Knies aufweist.

2. Capsaicin zur Verwendung bei der Linderung von Kniegelenkschmerzen bei einem menschlichen Patienten für eine Dauer von mindestens 8 Monaten, wobei die Verwendung die Verabreichung von mindestens einer ersten Capsaicin-Dosis und einer zweiten Capsaicin-Dosis durch Injektion in den intraartikulären Raum des Gelenks des Knies umfasst, um dadurch Kniegelenkschmerzen bei dem menschlichen Patienten für eine Dauer von mindestens 8 Monaten zu lindern, wobei die Verwendung **dadurch gekennzeichnet ist, dass**:
(i) die erste Capsaicin-Dosis einer Menge von 1 mg Capsaicin entspricht;
(ii) die zweite Capsaicin-Dosis einer Menge von 1 mg Capsaicin entspricht;
(iii) die zweite Capsaicin-Dosis nicht früher als 5 Monate nach Verabreichung der ersten Capsaicin-Dosis verabreicht wird; und
(iv) wenn eine zusätzliche Capsaicin-Dosis durch Injektion in den intraartikulären Raum des Gelenks des Knies verabreicht wird, jede solche zusätzliche Dosis in einer Menge von 1 mg Capsaicin liegt und jede solche zusätzliche Dosis nicht früher als 3 Monate nach der Verabreichung der vorherigen Capsaicin- Dosis, die durch Injektion in den intraartikulären Raum des Gelenks des Knies verabreicht wurde, verabreicht wird.

3. Capsaicin zur Verwendung nach Anspruch 2, wobei der Gelenkschmerz ein osteoarthritischer Gelenkschmerz ist.

4. Capsaicin zur Verwendung nach einem der Ansprüche 2-3, wobei bei der Verabreichung der ersten Capsaicin-Dosis:
(a) optional ein Kühlartikel auf eine äußere Oberfläche des Knies des menschlichen Patienten mit Gelenkschmerzen aufgebracht wird; dann
(b) optional ein Lokalanästhetikum durch Injektion in den intraartikulären Raum des Kniegelenks verabreicht wird; dann
(c) ein Kühlartikel für eine Dauer von mindestens 10 Minuten auf eine äußere Oberfläche des Knies aufgebracht wird, wobei der Kühlartikel eine äußere Oberflächentemperatur im Bereich von 1 °C bis 15 °C zum Aufbringen auf die äußere Oberfläche des Knies aufweist; dann
(d) die erste Capsaicin-Dosis durch Injektion einer Capsaicin umfassenden pharmazeutischen Zusammensetzung in den intraartikulären Raum des Gelenks des Knies verabreicht wird; und dann
(e) optional ein Kühlartikel für eine Dauer von mindestens 10 Minuten auf eine äußere Oberfläche des Knies aufgebracht wird, wobei der Kühlartikel eine äußere Oberflächentemperatur im Bereich von 1 °C bis 15 °C zum Aufbringen auf die äußere Oberfläche des Knies aufweist.

5. Capsaicin zur Verwendung nach einem der Ansprüche 2-4, wobei bei der Verabreichung der zweiten Capsaicin-Dosis:
(a) optional ein Kühlartikel auf eine äußere Oberfläche des Knies des menschlichen Patienten mit Gelenkschmerzen aufgebracht wird; dann
(b) gegebenenfalls ein Lokalanästhetikum durch Injektion in den intraartikulären Raum des Kniegelenks verabreicht wird; dann
(c) ein Kühlartikel für eine Dauer von mindestens 10 Minuten auf eine äußere Oberfläche des Knies aufgebracht wird, wobei der Kühlartikel eine äußere Oberflächentemperatur im Bereich von 1 °C bis 15 °C zum Aufbringen auf die äußere Oberfläche des Knies aufweist; dann
(d) die zweite Capsaicin-Dosis durch Injektion einer Capsaicin umfassenden pharmazeutischen Zusammensetzung in den intraartikulären Raum des Gelenks des Knies verabreicht wird; und dann
(e) optional ein Kühlartikel für eine Dauer von mindestens 10 Minuten auf eine äußere Oberfläche des Knies aufgebracht wird, wobei der Kühlartikel eine äußere Oberflächentemperatur im Bereich von 1 °C bis 15 °C zum Aufbringen auf die äußere Oberfläche des Knies aufweist.

6. Capsaicin zur Verwendung nach einem der Ansprüche 2-5, wobei bei der Verabreichung der zusätzlichen Capsaicin-Dosis:
(a) optional ein Kühlartikel auf eine äußere Oberfläche des Knies des menschlichen Patienten mit Gelenkschmerzen aufgebracht wird; dann
(b) optional ein Lokalanästhetikum durch Injektion in den intraartikulären Raum des Kniegelenks verabreicht wird; dann
(c) ein Kühlartikel für eine Dauer von mindestens 10 Minuten auf eine äußere Oberfläche des Knies aufgebracht wird, wobei der Kühlartikel eine äußere Oberflächentemperatur im Bereich von 1 °C bis 15 °C zum Aufbringen auf die äußere Oberfläche des Knies aufweist; dann
(d) die zusätzliche Capsaicin-Dosis durch Injektion einer Capsaicin umfassenden pharmazeutischen Zusammensetzung in den intraartikulären Raum des Gelenks des Knies verabreicht wird; und dann
(e) optional ein Kühlartikel für eine Dauer von mindestens 10 Minuten auf eine äußere Oberfläche des Knies aufgebracht wird, wobei der Kühlartikel eine äußere Oberflächentemperatur im Bereich von 1 °C bis 15 °C zum Aufbringen auf die äußere Oberfläche des Knies aufweist.

7. Capsaicin zur Verwendung nach einem der Ansprüche 4-6, wobei
(i) in Schritt (c) der Kühlartikel für eine Dauer von 15 Minuten bis 45 Minuten auf die äußere Oberfläche des Knies aufgebracht wird,
(ii) in Schritt (c) der Kühlartikel für eine Dauer von 45 Minuten auf die äußere Oberfläche des Knies aufgebracht wird,
(iii) in Schritt (c) der Kühlartikel für eine Dauer von 30 Minuten auf die äußere Oberfläche des Knies aufgebracht wird, oder
(iv) in Schritt (c) der Kühlartikel für eine Dauer von 20 Minuten auf die äußere Oberfläche des Knies aufgebracht wird.

8. Capsaicin zur Verwendung nach einem der Ansprüche 2-3, wobei bei der Verabreichung der ersten Capsaicin-Dosis:
(a) optional ein Kühlartikel auf eine äußere Oberfläche des Knies des menschlichen Patienten mit Gelenkschmerzen aufgebracht wird; dann
(b) optional ein Lokalanästhetikum durch Injektion in den intraartikulären Raum des Kniegelenks verabreicht wird; dann
(c) ein Kühlartikel auf eine äußere Oberfläche des Knies aufgebracht wird, um eine Temperatur im Bereich von 28 °C bis 30 °C für Gewebe oder Flüssigkeit im Inneren des Kniegelenks zu erreichen; dann
(d) die erste Capsaicin-Dosis durch Injektion einer Capsaicin umfassenden pharmazeutischen Zusammensetzung in den intraartikulären Raum des Gelenks des Knies verabreicht wird; und dann
(e) optional ein Kühlartikel auf eine äußere Oberfläche des Knies aufgebracht wird.

9. Capsaicin zur Verwendung nach einem der Ansprüche 2, 3 oder 8, wobei bei der Verabreichung der zweiten Capsaicin-Dosis:
(a) optional ein Kühlartikel auf eine äußere Oberfläche des Knies des menschlichen Patienten mit Gelenkschmerzen aufgebracht wird; dann
(b) optional ein Lokalanästhetikum durch Injektion in den intraartikulären Raum des Kniegelenks verabreicht wird; dann
(c) ein Kühlartikel auf eine äußere Oberfläche des Knies aufgebracht wird, um eine Temperatur im Bereich von 28 °C bis 30 °C für Gewebe oder Flüssigkeit im Inneren des Kniegelenks zu erreichen; dann
(d) die zweite Capsaicin-Dosis durch Injektion einer Capsaicin umfassenden pharmazeutischen Zusammensetzung in den intraartikulären Raum des Gelenks des Knies verabreicht wird; und dann
optional ein Kühlartikel auf eine äußere Oberfläche des Knies aufgebracht wird.

10. Capsaicin zur Verwendung nach einem der Ansprüche 2, 3, 8 oder 9, wobei bei der Verabreichung einer zusätzlichen Capsaicin-Dosis:
(a) optional ein Kühlartikel auf eine äußere Oberfläche des Knies des menschlichen Patienten mit Gelenkschmerzen aufgebracht wird; dann
(b) optional ein Lokalanästhetikum durch Injektion in den intraartikulären Raum des Kniegelenks verabreicht wird; dann
(c) ein Kühlartikel auf eine äußere Oberfläche des Knies aufgebracht wird, um eine Temperatur im Bereich von 28 °C bis 30 °C für Gewebe oder Flüssigkeit im Inneren des Kniegelenks zu erreichen; dann
(d) die zusätzliche Capsaicin-Dosis durch Injektion einer Capsaicin umfassenden pharmazeutischen Zusammensetzung in den intraartikulären Raum des Gelenks des Knies verabreicht wird; und dann
(e) optional ein Kühlartikel auf eine äußere Oberfläche des Knies aufgebracht wird.

11. Capsaicin zur Verwendung nach einem der Ansprüche 4-10, wobei die Verwendung ferner **dadurch gekennzeichnet ist, dass**:
(i) die Verwendung den folgenden zusätzlichen Schritt, der zwischen den Schritten (c) und (d) durchgeführt wird, umfasst: Verabreichen eines Lokalanästhetikums durch Injektion in den intraartikulären Raum des Kniegelenks,
(ii) das Lokalanästhetikum ein Cain-Analgetikum ist,
(iii) das Lokalanästhetikum Lidocain oder ein pharmazeutisch annehmbares Salz davon ist,
(iv) das Lokalanästhetikum Lidocainhydrochlorid ist,
(v) das Lokalanästhetikum in einer Dosis verabreicht wird, die ausreicht, um Lidocain in einer Menge von 0,1 g bis 0,5 g abzugeben,
(vi) das Lokalanästhetikum in einer Dosis verabreicht wird, die ausreicht, um Lidocain in einer Menge von 0,15 g abzugeben,
(vii) das Lokalanästhetikum in einer Dosis verabreicht wird, die ausreicht, um Lidocain in einer Menge von 0,3 g abzugeben,
(viii) das Lokalanästhetikum in Form einer pharmazeutischen Zusammensetzung mit einem Volumen im Bereich von 13 ml bis 17 ml verabreicht wird,
(ix) das Lokalanästhetikum in Form einer pharmazeutischen Zusammensetzung mit einem Volumen von 15 ml verabreicht wird,
(x) das Lokalanästhetikum in Form einer pharmazeutischen Zusammensetzung verabreicht wird, bei der es sich um eine wässrige Mischung handelt, die Lidocain in einer Konzentration von 2 % w/w enthält, oder
(xi) das Lokalanästhetikum in Form einer pharmazeutischen Zusammensetzung verabreicht wird, bei der es sich um eine wässrige Mischung handelt, die Lidocain in einer Konzentration von 1 % w/w enthält.

12. Capsaicin zur Verwendung nach einem der Ansprüche 4 bis 11, wobei der menschliche Patient außer der Verabreichung (i) eines Lokalanästhetikums und (ii) der ersten Capsaicin-Dosis, der zweiten Capsaicin-Dosis und jeder zusätzlichen Capsaicin-Dosis kein weiteres schmerzlinderndes Arzneimittel erhält.

13. Capsaicin zur Verwendung nach einem der Ansprüche 1 oder 4-12, wobei:
(i) der Kühlartikel eine äußere Oberflächentemperatur im Bereich von 5 °C bis 15 °C zum Aufbringen auf der äußeren Oberfläche des Knies des menschlichen Patienten hat,
(ii) der Kühlartikel eine äußere Oberflächentemperatur im Bereich von 6 °C bis 13 °C zum Aufbringen auf der äußeren Oberfläche des Knies des menschlichen Patienten hat,
(iii) der Kühlartikel eine äußere Oberflächentemperatur im Bereich von 7 °C bis 13 °C zum Aufbringen auf der äußeren Oberfläche des Knies des menschlichen Patienten hat,
(iv) der Kühlartikel eine äußere Oberflächentemperatur im Bereich von 7 °C bis 10 °C zum Aufbringen auf der äußeren Oberfläche des Knies des menschlichen Patienten hat,
(v) der Kühlartikel eine äußere Oberflächentemperatur im Bereich von 5 °C bis 10 °C zum Aufbringen auf der äußeren Oberfläche des Knies des menschlichen Patienten hat,
(vi) der Kühlartikel eine äußere Oberflächentemperatur im Bereich von 6 °C bis 12 °C zum Aufbringen auf der äußeren Oberfläche des Knies des menschlichen Patienten hat,
(vii) der Kühlartikel eine äußere Oberflächentemperatur 9 °C zum Aufbringen auf der äußeren Oberfläche des Knies des menschlichen Patienten hat,
(viii) der Kühlartikel eine äußere Oberflächentemperatur 10 °C zum Aufbringen auf der äußeren Oberfläche des Knies des menschlichen Patienten hat oder
(ix) der Kühlartikel eine äußere Oberflächentemperatur 11 °C zum Aufbringen auf der äußeren Oberfläche des Knies des menschlichen Patienten hat.

14. Capsaicin zur Verwendung nach einem der Ansprüche 1 oder 4-13, wobei die Capsaicin umfassende pharmazeutische Zusammensetzung eine wässrige injizierbare Formulierung ist, die Folgendes umfasst:
a. zu 0,03 % (w/w) bis 0,3 % (w/w) Capsaicin;
b. zu 0,1 % (w/w) bis 3 % (w/w) ein Solubilisierungsmittel, wobei das Solubilisierungsmittel (i) einen Polyethylenglykolester einer (C₁₅-C₂₅)-Hydroxyalkansäure, (ii) einen Polyethylenglykolester einer (C₁₅-C₂₅)-Hydroxyalkensäure oder (iii) einen Polyethylenglykolester einer (C₁₅-C₂₅)-Alkansäure, die mit einer - OC(O)(C₁₄-C₂₄)-Hydroxyalkylgruppe substituiert ist, umfasst;
c. zu 0,001 % (w/w) bis 2 % (w/w) ein Antioxidationsmittel; und
d. zu mindestens 92 % (w/w) Wasser; und wobei optional
das Solubilisierungsmittel zu (a) 70 % (w/w) eine Mischung aus 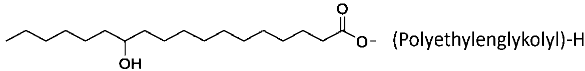 und 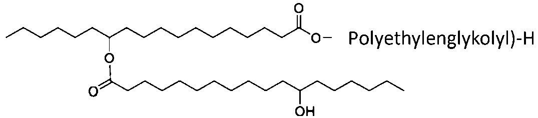 und zu (b) 30 % (w/w) Polyethylen umfasst.

15. Capsaicin zur Verwendung nach einem der Ansprüche 1-14, wobei die zweite Capsaicin-Dosis:
(i) nicht früher als 6 Monate nach Verabreichung der ersten Capsaicin-Dosis,
(ii) nicht früher als 7 Monate nach Verabreichung der ersten Capsaicin-Dosis,
(iii) nicht früher als 8 Monate nach Verabreichung der ersten Capsaicin-Dosis oder
(iv) nicht früher als 9 Monate nach Verabreichung der ersten Capsaicin-Dosis verabreicht wird.

16. Capsaicin zur Verwendung nach einem der Ansprüche 1-14, wobei die zweite Capsaicin-Dosis:
(i) zu einem Zeitpunkt, der im Bereich von 6 bis 8 Monaten nach Verabreichung der ersten Capsaicin-Dosis liegt,
(ii) zu einem Zeitpunkt, der im Bereich von 7 bis 9 Monaten nach Verabreichung der ersten Capsaicin-Dosis liegt,
(iii) zu einem Zeitpunkt, der im Bereich von 6 bis 9 Monaten nach Verabreichung der ersten Capsaicin-Dosis liegt,
(iv) 6 Monate nach Verabreichung der ersten Capsaicin-Dosis,
(v) 26 Wochen nach Verabreichung der ersten Capsaicin-Dosis,
(vi) 7 Monate nach Verabreichung der ersten Capsaicin-Dosis, oder
(vii) 8 Monate nach Verabreichung der ersten Capsaicin-Dosis verabreicht wird.

17. Capsaicin zur Verwendung nach einem der Ansprüche 1-16, wobei der Schmerz für:
(i) eine Dauer von mindestens 10 Monaten,
(ii) eine Dauer von mindestens 11 Monaten,
(iii) eine Dauer von mindestens 12 Monaten,
(iv) eine Dauer von mindestens 13 Monaten oder
(v) eine Dauer von mindestens 14 Monaten gelindert wird.

18. Capsaicin zur Verwendung nach einem der Ansprüche 1-17, wobei die Verwendung **gekennzeichnet ist durch**:
(i) Erreichen einer Verringerung der durchschnittlichen Schmerzen im Knie beim Gehen in den letzten vierundzwanzig Stunden um mindestens 1 auf der numerischen Schmerzbewertungsskala von 0-10 (NPRS) über eine Dauer von mindestens 8 Monaten oder
(ii) Erreichen einer Verringerung der durchschnittlichen Schmerzen im Knie beim Gehen in den letzten vierundzwanzig Stunden um mindestens 1 auf der numerischen Schmerzbewertungsskala von 0-10 (NPRS) über eine Dauer von mindestens 9 Monaten,
(iii) Erreichen einer Verringerung der durchschnittlichen Schmerzen im Knie beim Gehen in den letzten vierundzwanzig Stunden um mindestens 1 auf der numerischen Schmerzbewertungsskala von 0-10 (NPRS) über eine Dauer von mindestens 10 Monaten,
(iv) Erreichen einer Verringerung der durchschnittlichen Schmerzen im Knie beim Gehen in den letzten vierundzwanzig Stunden um mindestens 1 auf der numerischen Schmerzbewertungsskala von 0-10 (NPRS) über eine Dauer von mindestens 11 Monaten oder
(v) Erreichen einer Verringerung der durchschnittlichen Schmerzen im Knie beim Gehen in den letzten vierundzwanzig Stunden um mindestens 1 auf der numerischen Schmerzbewertungsskala von 0-10 (NPRS) über eine Dauer von mindestens 12 Monaten.

19. Capsaicin zur Verwendung nach einem der Ansprüche 1-17, wobei die Verwendung **gekennzeichnet ist durch**:
(i) Erreichen einer Verringerung der durchschnittlichen Schmerzen im Knie beim Gehen in den letzten vierundzwanzig Stunden um mindestens 2 auf der numerischen Schmerzbewertungsskala von 0-10 (NPRS) über eine Dauer von mindestens 8 Monaten,
(ii) Erreichen einer Verringerung der durchschnittlichen Schmerzen im Knie beim Gehen in den letzten vierundzwanzig Stunden um mindestens 2 auf der numerischen Schmerzbewertungsskala von 0-10 (NPRS) über eine Dauer von mindestens 9 Monaten,
(iii) Erreichen einer Verringerung der durchschnittlichen Schmerzen im Knie beim Gehen in den letzten vierundzwanzig Stunden um mindestens 2 auf der numerischen Schmerzbewertungsskala von 0-10 (NPRS) über eine Dauer von mindestens 10 Monaten,
(iv) Erreichen einer Verringerung der durchschnittlichen Schmerzen im Knie beim Gehen in den letzten vierundzwanzig Stunden um mindestens 1 auf der numerischen Schmerzbewertungsskala von 0-10 (NPRS) über eine Dauer von mindestens 11 Monaten,
(v) Erreichen einer Verringerung der durchschnittlichen Schmerzen im Knie beim Gehen in den letzten vierundzwanzig Stunden um mindestens 2 auf der numerischen Schmerzbewertungsskala von 0-10 (NPRS) über eine Dauer von mindestens 12 Monaten.

20. Capsaicin zur Verwendung nach einem der Ansprüche 1-19, wobei die Verwendung **gekennzeichnet ist durch**:
(i) Verringern der durchschnittlichen Knieschmerzen des Patienten beim Gehen, so dass die durchschnittlichen Knieschmerzen des Patienten beim Gehen auf der numerischen Schmerzbewertungsskala von 0-10 (NPRS) über eine Dauer von mindestens 8 Monaten nicht mehr als 1 betragen,
(ii) Verringern der durchschnittlichen Knieschmerzen des Patienten beim Gehen, so dass die durchschnittlichen Knieschmerzen des Patienten beim Gehen auf der numerischen Schmerzbewertungsskala von 0-10 (NPRS) über eine Dauer von mindestens 9 Monaten nicht mehr als 1 betragen,
(iii) Verringern der durchschnittlichen Knieschmerzen des Patienten beim Gehen, so dass die durchschnittlichen Knieschmerzen des Patienten beim Gehen auf der numerischen Schmerzbewertungsskala von 0-10 (NPRS) für eine Dauer von mindestens 10 Monaten nicht mehr als 1 betragen,
(iv) Verringern der durchschnittlichen Knieschmerzen des Patienten beim Gehen, so dass die durchschnittlichen Knieschmerzen des Patienten beim Gehen auf der numerischen Schmerzbewertungsskala von 0-10 (NPRS) über eine Dauer von mindestens 11 Monaten nicht mehr als 1 betragen, oder
(v) Verringern der durchschnittlichen Knieschmerzen des Patienten beim Gehen, so dass die durchschnittlichen Knieschmerzen des Patienten beim Gehen auf der numerischen Schmerzbewertungsskala von 0-10 (NPRS) über eine Dauer von mindestens 12 Monaten nicht mehr als 1 betragen.

21. Capsaicin zur Verwendung nach einem der Ansprüche 1-19, wobei die Verwendung **gekennzeichnet ist durch**:
(i) Verringern der durchschnittlichen Knieschmerzen des Patienten beim Gehen, so dass die durchschnittlichen Knieschmerzen des Patienten beim Gehen auf der numerischen Schmerzbewertungsskala von 0-10 (NPRS) über eine Dauer von mindestens 8 Monaten nicht mehr als 2 betragen,
(ii) Verringern der durchschnittlichen Knieschmerzen des Patienten beim Gehen, so dass die durchschnittlichen Knieschmerzen des Patienten beim Gehen auf der numerischen Schmerzbewertungsskala von 0-10 (NPRS) über eine Dauer von mindestens 9 Monaten nicht mehr als 2 betragen,
(iii) Verringern der durchschnittlichen Knieschmerzen des Patienten beim Gehen, so dass die durchschnittlichen Knieschmerzen des Patienten beim Gehen auf der numerischen Schmerzbewertungsskala von 0-10 (NPRS) über eine Dauer von mindestens 10 Monaten nicht mehr als 2 betragen,
(iv) Verringern der durchschnittlichen Knieschmerzen des Patienten beim Gehen, so dass die durchschnittlichen Knieschmerzen des Patienten beim Gehen auf der numerischen Schmerzbewertungsskala von 0-10 (NPRS) über eine Dauer von mindestens 11 Monaten nicht mehr als 2 betragen, oder
(v) Verringern der durchschnittlichen Knieschmerzen des Patienten beim Gehen, so dass die durchschnittlichen Knieschmerzen des Patienten beim Gehen auf der numerischen Schmerzbewertungsskala von 0-10 (NPRS) über eine Dauer von mindestens 12 Monaten nicht mehr als 2 betragen.

## Revendications

1. Capsaïcine destinée à être utilisée pour soulager la douleur articulaire arthrosique du genou chez un patient humain pendant une durée d'au moins 8 mois, dans laquelle l'utilisation comprend l'administration par injection dans l'espace intra-articulaire de l'articulation dudit genou d'au moins une première dose de capsaïcine et d'une seconde dose de capsaïcine pour ainsi soulager la douleur articulaire arthrosique du genou chez le patient humain pendant une durée d'au moins 8 mois, dans laquelle l'utilisation est **caractérisée en ce que** :
(i) la première dose de capsaïcine est une quantité de 1 mg de capsaïcine ;
(ii) la seconde dose de capsaïcine est une quantité de 1 mg de capsaïcine ;
(iii) la seconde dose de capsaïcine est administrée au plus tôt 5 mois après l'administration de la première dose de capsaïcine ;
(iv) si une dose supplémentaire de capsaïcine est administrée par injection dans l'espace intra-articulaire de l'articulation dudit genou, une telle dose supplémentaire est d'une quantité de 1 mg de capsaïcine et ladite dose supplémentaire est administrée au plus tôt 3 mois après l'administration de la dose précédente de capsaïcine administrée par injection dans l'espace intra-articulaire de l'articulation dudit genou ; et
(v) lors de l'administration de la première dose de capsaïcine :
(a) un article de refroidissement est appliqué pendant une durée de 15 minutes sur une surface extérieure du genou du patient humain présentant une douleur articulaire arthrosique du genou, dans laquelle l'article de refroidissement a une température de surface extérieure dans la plage de 3 °C à 15 °C pour une application sur la surface extérieure dudit genou ; puis
(b) une composition pharmaceutique comprenant un agent analgésique unique choisi dans le groupe constitué de la lidocaïne et d'un sel pharmaceutiquement acceptable de celle-ci est administrée par injection dans l'espace intra-articulaire de l'articulation dudit genou afin de délivrer une dose de lidocaïne en une quantité allant de 0,1 g à 0,5 g ; puis
(c) un article de refroidissement est appliqué pendant une durée de 30 minutes sur une surface extérieure dudit genou, dans laquelle l'article de refroidissement a une température de surface extérieure dans la plage de 3 °C à 15 °C pour une application sur la surface extérieure dudit genou ; puis
(d) la première dose de capsaïcine est administrée en injectant dans l'espace intra-articulaire de l'articulation dudit genou une composition pharmaceutique comprenant de la capsaïcine ; puis
(e) un article de refroidissement est appliqué sur une surface extérieure dudit genou pendant une durée d'au moins 30 minutes, dans laquelle l'article de refroidissement a une température de surface extérieure dans la plage de 3 °C à 15 °C pour une application sur la surface extérieure dudit genou.

2. Capsaïcine destinée à être utilisée pour soulager la douleur articulaire arthrosique du genou chez un patient humain pendant une durée d'au moins 8 mois, dans laquelle l'utilisation comprend l'administration par injection dans l'espace intra-articulaire de l'articulation dudit genou d'au moins une première dose de capsaïcine et d'une seconde dose de capsaïcine pour ainsi soulager la douleur articulaire arthrosique du genou chez le patient humain pendant une durée d'au moins 8 mois, dans laquelle l'utilisation est **caractérisée en ce que** :
(i) la première dose de capsaïcine est une quantité de 1 mg de capsaïcine ;
(ii) la seconde dose de capsaïcine est une quantité de 1 mg de capsaïcine ;
(iii) la seconde dose de capsaïcine est administrée au plus tôt 5 mois après l'administration de la première dose de capsaïcine ; et
(iv) si une dose supplémentaire de capsaïcine est administrée par injection dans l'espace intra-articulaire de l'articulation dudit genou, une telle dose supplémentaire est d'une quantité de 1 mg de capsaïcine et toute dose supplémentaire est administrée au plus tôt 3 mois après l'administration de la dose précédente de capsaïcine administrée par injection dans l'espace intra-articulaire de l'articulation dudit genou.

3. Capsaïcine destinée à être utilisée selon la revendication 2, dans laquelle la douleur articulaire est une douleur articulaire arthrosique.

4. Capsaïcine destinée à être utilisée selon l'une quelconque des revendications 2 à 3, dans laquelle, lors de l'administration de la première dose de capsaïcine :
(a) éventuellement, un article de refroidissement est appliqué sur une surface extérieure du genou du patient humain présentant une douleur articulaire ; puis
(b) éventuellement, un agent anesthésique local est administré par injection dans l'espace intra-articulaire de ladite articulation du genou ; puis
(c) un article de refroidissement est appliqué pendant une durée d'au moins 10 minutes sur une surface extérieure dudit genou, dans laquelle l'article de refroidissement a une température de surface extérieure dans la plage de 1 °C à 15 °C pour une application sur la surface extérieure dudit genou ; puis
(d) la première dose de capsaïcine est administrée en injectant dans l'espace intra-articulaire de l'articulation dudit genou une composition pharmaceutique comprenant de la capsaïcine ; puis
(e) éventuellement, un article de refroidissement est appliqué pendant une durée d'au moins 10 minutes sur une surface extérieure dudit genou, dans laquelle l'article de refroidissement a une température de surface extérieure dans la plage de 1 °C à 15 °C pour une application sur la surface extérieure dudit genou.

5. Capsaïcine destinée à être utilisée selon l'une quelconque des revendications 2 à 4, dans laquelle, lors de l'administration de la seconde dose de capsaïcine :
(a) éventuellement, un article de refroidissement est appliqué sur une surface extérieure du genou du patient humain présentant une douleur articulaire ; puis
(b) éventuellement, un agent anesthésique local est administré par injection dans l'espace intra-articulaire de ladite articulation du genou ; puis
(c) un article de refroidissement est appliqué pendant une durée d'au moins 10 minutes sur une surface extérieure dudit genou, dans laquelle l'article de refroidissement a une température de surface extérieure dans la plage de 1 °C à 15 °C pour une application sur la surface extérieure dudit genou ; puis
(d) la seconde dose de capsaïcine est administrée en injectant dans l'espace intra-articulaire de l'articulation dudit genou une composition pharmaceutique comprenant de la capsaïcine ; puis
(e) éventuellement, un article de refroidissement est appliqué pendant une durée d'au moins 10 minutes sur une surface extérieure dudit genou, dans laquelle l'article de refroidissement a une température de surface extérieure dans la plage de 1 °C à 15 °C pour une application sur la surface extérieure dudit genou.

6. Capsaïcine destinée à être utilisée selon l'une quelconque des revendications 2 à 5, dans laquelle, lors de l'administration d'une dose supplémentaire de capsaïcine :
(a) éventuellement, un article de refroidissement est appliqué sur une surface extérieure du genou du patient humain présentant une douleur articulaire ; puis
(b) éventuellement, un agent anesthésique local est administré par injection dans l'espace intra-articulaire de ladite articulation du genou ; puis
(c) un article de refroidissement est appliqué pendant une durée d'au moins 10 minutes sur une surface extérieure dudit genou, dans laquelle l'article de refroidissement a une température de surface extérieure dans la plage de 1 °C à 15 °C pour une application sur la surface extérieure dudit genou ; puis
(d) la dose supplémentaire de capsaïcine est administrée en injectant dans l'espace intra-articulaire de l'articulation dudit genou une composition pharmaceutique comprenant de la capsaïcine ; puis
(e) éventuellement, un article de refroidissement est appliqué pendant une durée d'au moins 10 minutes sur une surface extérieure dudit genou, dans laquelle l'article de refroidissement a une température de surface extérieure dans la plage de 1 °C à 15 °C pour une application sur la surface extérieure dudit genou.

7. Capsaïcine destinée à être utilisée selon l'une quelconque des revendications 4 à 6, dans laquelle :
(i) à l'étape (c), l'article de refroidissement est appliqué pendant une durée de 15 minutes à 45 minutes sur la surface extérieure dudit genou,
(ii) à l'étape (c), l'article de refroidissement est appliqué pendant une durée de 45 minutes sur la surface extérieure dudit genou,
(iii) à l'étape (c), l'article de refroidissement est appliqué pendant une durée de 30 minutes sur la surface extérieure dudit genou, ou
(iv) à l'étape (c), l'article de refroidissement est appliqué pendant une durée de 20 minutes sur la surface extérieure dudit genou.

8. Capsaïcine destinée à être utilisée selon l'une quelconque des revendications 2 à 3, dans laquelle, lors de l'administration de la première dose de capsaïcine :
(a) éventuellement, un article de refroidissement est appliqué sur une surface extérieure du genou du patient humain présentant une douleur articulaire ; puis
(b) éventuellement, un agent anesthésique local est administré par injection dans l'espace intra-articulaire de ladite articulation du genou ; puis
(c) un article de refroidissement est appliqué sur une surface extérieure dudit genou pour atteindre une température dans la plage de 28 °C à 30 °C pour les tissus ou les fluides à l'intérieur de ladite articulation du genou ; puis
(d) la première dose de capsaïcine est administrée en injectant dans l'espace intra-articulaire de l'articulation dudit genou une composition pharmaceutique comprenant de la capsaïcine ; puis
(e) éventuellement, un article de refroidissement est appliqué sur une surface extérieure dudit genou.

9. Capsaïcine destinée à être utilisée selon l'une quelconque des revendications 2, 3 ou 8, dans laquelle, lors de l'administration de la seconde dose de capsaïcine :
(a) éventuellement, un article de refroidissement est appliqué sur une surface extérieure du genou du patient humain présentant une douleur articulaire ; puis
(b) éventuellement, un agent anesthésique local est administré par injection dans l'espace intra-articulaire de ladite articulation du genou ; puis
(c) un article de refroidissement est appliqué sur une surface extérieure dudit genou pour atteindre une température dans la plage de 28 °C à 30 °C pour les tissus ou les fluides à l'intérieur de ladite articulation du genou ; puis
(d) la seconde dose de capsaïcine est administrée en injectant dans l'espace intra-articulaire de l'articulation dudit genou une composition pharmaceutique comprenant de la capsaïcine ; puis
éventuellement, un article de refroidissement est appliqué sur une surface extérieure dudit genou.

10. Capsaïcine destinée à être utilisée selon l'une quelconque des revendications 2, 3, 8 ou 9, dans laquelle, lors de l'administration d'une dose supplémentaire de capsaïcine :
(a) éventuellement, un article de refroidissement est appliqué sur une surface extérieure du genou du patient humain présentant une douleur articulaire ; puis
(b) éventuellement, un agent anesthésique local est administré par injection dans l'espace intra-articulaire de ladite articulation du genou ; puis
(c) un article de refroidissement est appliqué sur une surface extérieure dudit genou pour atteindre une température dans la plage de 28 °C à 30 °C pour les tissus ou les fluides à l'intérieur de ladite articulation du genou ; puis
(d) la dose supplémentaire de capsaïcine est administrée en injectant dans l'espace intra-articulaire de l'articulation dudit genou une composition pharmaceutique comprenant de la capsaïcine ; puis
(e) éventuellement, un article de refroidissement est appliqué sur une surface extérieure dudit genou.

11. Capsaïcine destinée à être utilisée selon l'une quelconque des revendications 4 à 10, dans laquelle l'utilisation est en outre **caractérisée en ce que** :
(i) l'utilisation comprend l'étape supplémentaire suivante qui est réalisée entre les étapes (c) et (d) : l'administration d'un agent anesthésique local par injection dans l'espace intra-articulaire de ladite articulation du genou,
(ii) l'agent anesthésique local est un analgésique caïnique,
(iii) l'agent anesthésique local est la lidocaïne ou un sel pharmaceutiquement acceptable de celle-ci,
(iv) l'agent anesthésique local est le chlorhydrate de lidocaïne,
(v) l'agent anesthésique local est délivré à une dose suffisante pour délivrer de la lidocaïne en une quantité de 0,1 g à 0,5 g,
(vi) l'agent anesthésique local est délivré à une dose suffisante pour délivrer de la lidocaïne en une quantité de 0,15 g,
(vii) l'agent anesthésique local est délivré à une dose suffisante pour délivrer de la lidocaïne en une quantité de 0,3 g,
(viii) l'agent anesthésique local est administré sous la forme d'une composition pharmaceutique ayant un volume dans la plage de 13 ml à 17 ml,
(ix) l'agent anesthésique local est administré sous la forme d'une composition pharmaceutique ayant un volume de 15 ml,
(x) l'agent anesthésique local est administré sous la forme d'une composition pharmaceutique qui est un mélange aqueux contenant de la lidocaïne à une concentration de 2 % p/p, ou
(xi) l'agent anesthésique local est administré sous la forme d'une composition pharmaceutique qui est un mélange aqueux contenant de la lidocaïne à une concentration de 1 % p/p.

12. Capsaïcine destinée à être utilisée selon l'une quelconque des revendications 4 à 11, dans laquelle, hormis l'administration (i) d'un agent anesthésique local et (ii) de la première dose de capsaïcine, de la seconde dose de capsaïcine et de toute dose supplémentaire de capsaïcine, le patient humain ne reçoit aucun autre médicament analgésique.

13. Capsaïcine destinée à être utilisée selon l'une quelconque des revendications 1 ou 4 à 12, dans laquelle :
(i) l'article de refroidissement a une température de surface extérieure dans la plage de 5 °C à 15 °C pour une application sur la surface extérieure du genou du patient humain,
(ii) l'article de refroidissement a une température de surface extérieure dans la plage de 6 °C à 13 °C pour une application sur la surface extérieure du genou du patient humain,
(iii) l'article de refroidissement a une température de surface extérieure dans la plage de 7 °C à 13 °C pour une application sur la surface extérieure du genou du patient humain,
(iv) l'article de refroidissement a une température de surface extérieure dans la plage de 7 °C à 10 °C pour une application sur la surface extérieure du genou du patient humain,
(v) l'article de refroidissement a une température de surface extérieure dans la plage de 5 °C à 10 °C pour une application sur la surface extérieure du genou du patient humain,
(vi) l'article de refroidissement a une température de surface extérieure dans la plage de 6 °C à 12 °C pour une application sur la surface extérieure du genou du patient humain,
(vii) l'article de refroidissement a une température de surface extérieure de 9 °C pour une application sur la surface extérieure du genou du patient humain,
(viii) l'article de refroidissement a une température de surface extérieure de 10 °C pour une application sur la surface extérieure du genou du patient humain, ou
(ix) l'article de refroidissement a une température de surface extérieure de 11 °C pour une application sur la surface extérieure du genou du patient humain.

14. Capsaïcine destinée à être utilisée selon l'une quelconque des revendications 1 ou 4 à 13, dans laquelle la composition pharmaceutique comprenant de la capsaïcine est une formulation aqueuse injectable qui comprend :
a. 0,03 % (p/p) à 0,3 % (p/p) de capsaïcine ;
b. 0,1 % (p/p) à 3 % (p/p) d'un agent solubilisant, dans laquelle l'agent solubilisant comprend (i) un ester de polyéthylène glycol d'un acide hydroxyalcanoïque en C₁₅-C₂₅, (ii) un ester de polyéthylène glycol d'un acide hydroxyalcénoïque en C₁₅-C₂₅, ou (iii) un ester de polyéthylène glycol d'un acide alcanoïque en C₁₅-C₂₅ substitué par un groupe hydroxyalkyle -OC(O) en C₁₄-C₂₄ ;
c. 0,001 % (p/p) à 2 % (p/p) d'un antioxydant ; et
d. au moins 92 % (p/p) d'eau ; et dans laquelle éventuellement
l'agent solubilisant comprend (a) 70 % (p/p) d'un mélange de 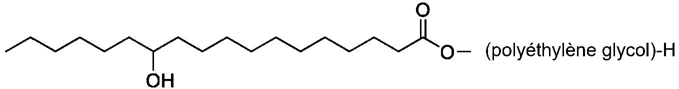 et 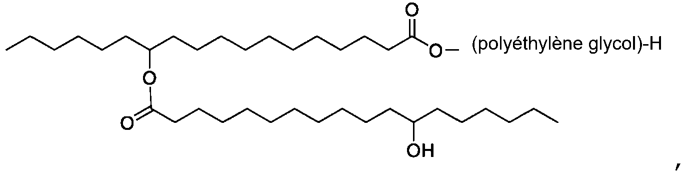 et (b) 30 % (p/p) de polyéthylène glycol.

15. Capsaïcine destinée à être utilisée selon l'une quelconque des revendications 1 à 14, dans laquelle la seconde dose de capsaïcine est administrée :
(i) au plus tôt 6 mois après l'administration de la première dose de capsaïcine,
(ii) au plus tôt 7 mois après l'administration de la première dose de capsaïcine,
(iii) au plus tôt 8 mois après l'administration de la première dose de capsaïcine, ou
(iv) au plus tôt 9 mois après l'administration de la première dose de capsaïcine.

16. Capsaïcine destinée à être utilisée selon l'une quelconque des revendications 1 à 14, dans laquelle la seconde dose de capsaïcine est administrée :
(i) à un moment situé dans la plage de 6 mois à 8 mois après l'administration de la première dose de capsaïcine,
(ii) à un moment situé dans la plage de 7 mois à 9 mois après l'administration de la première dose de capsaïcine,
(iii) à un moment situé dans la plage de 6 mois à 9 mois après l'administration de la première dose de capsaïcine,
(iv) 6 mois après l'administration de la première dose de capsaïcine,
(v) 26 semaines après l'administration de la première dose de capsaïcine,
(vi) 7 mois après l'administration de la première dose de capsaïcine, ou
(vii) 8 mois après l'administration de la première dose de capsaïcine.

17. Capsaïcine destinée à être utilisée selon l'une quelconque des revendications 1 à 16, dans laquelle la douleur est atténuée pendant :
(i) une durée d'au moins 10 mois,
(ii) une durée d'au moins 11 mois,
(iii) une durée d'au moins 12 mois,
(iv) une durée d'au moins 13 mois, ou
(v) une durée d'au moins 14 mois.

18. Capsaïcine destinée à être utilisée selon l'une quelconque des revendications 1 à 17, dans laquelle l'utilisation est **caractérisée par** :
(i) l'obtention d'une réduction de la douleur moyenne au genou lors de la marche au cours des vingt-quatre heures précédentes d'au moins 1 sur l'échelle numérique d'évaluation de la douleur de 0 à 10 (NPRS) pendant une durée d'au moins 8 mois, ou
(ii) l'obtention d'une réduction de la douleur moyenne au genou lors de la marche au cours des vingt-quatre heures précédentes d'au moins 1 sur l'échelle numérique d'évaluation de la douleur de 0 à 10 (NPRS) pendant une durée d'au moins 9 mois,
(iii) l'obtention d'une réduction de la douleur moyenne au genou lors de la marche au cours des vingt-quatre heures précédentes d'au moins 1 sur l'échelle numérique d'évaluation de la douleur de 0 à 10 (NPRS) pendant une durée d'au moins 10 mois,
(iv) l'obtention d'une réduction de la douleur moyenne au genou lors de la marche au cours des vingt-quatre heures précédentes d'au moins 1 sur l'échelle numérique d'évaluation de la douleur de 0 à 10 (NPRS) pendant une durée d'au moins 11 mois, ou
(v) l'obtention d'une réduction de la douleur moyenne au genou lors de la marche au cours des vingt-quatre heures précédentes d'au moins 1 sur l'échelle numérique d'évaluation de la douleur de 0 à 10 (NPRS) pendant une durée d'au moins 12 mois.

19. Capsaïcine destinée à être utilisée selon l'une quelconque des revendications 1 à 17, dans laquelle l'utilisation est **caractérisée par** :
(i) l'obtention d'une réduction de la douleur moyenne au genou lors de la marche au cours des vingt-quatre heures précédentes d'au moins 2 sur l'échelle numérique d'évaluation de la douleur de 0 à 10 (NPRS) pendant une durée d'au moins 8 mois,
(ii) l'obtention d'une réduction de la douleur moyenne au genou lors de la marche au cours des vingt-quatre heures précédentes d'au moins 2 sur l'échelle numérique d'évaluation de la douleur de 0 à 10 (NPRS) pendant une durée d'au moins 9 mois,
(iii) l'obtention d'une réduction de la douleur moyenne au genou lors de la marche au cours des vingt-quatre heures précédentes d'au moins 2 sur l'échelle numérique d'évaluation de la douleur de 0 à 10 (NPRS) pendant une durée d'au moins 10 mois,
(iv) l'obtention d'une réduction de la douleur moyenne au genou lors de la marche au cours des vingt-quatre heures précédentes d'au moins 1 sur l'échelle numérique d'évaluation de la douleur de 0 à 10 (NPRS) pendant une durée d'au moins 11 mois,
(v) l'obtention d'une réduction de la douleur moyenne au genou lors de la marche au cours des vingt-quatre heures précédentes d'au moins 2 sur l'échelle numérique d'évaluation de la douleur de 0 à 10 (NPRS) pendant une durée d'au moins 12 mois.

20. Capsaïcine destinée à être utilisée selon l'une quelconque des revendications 1 à 19, dans laquelle l'utilisation est **caractérisée par** :
(i) la réduction de la douleur moyenne du patient au genou lors de la marche de sorte que la douleur moyenne du patient au genou lors de la marche n'est pas supérieure à 1 sur l'échelle numérique d'évaluation de la douleur de 0 à 10 (NPRS) pendant une durée d'au moins 8 mois,
(ii) la réduction de la douleur moyenne du patient au genou lors de la marche de sorte que la douleur moyenne du patient au genou lors de la marche n'est pas supérieure à 1 sur l'échelle numérique d'évaluation de la douleur de 0 à 10 (NPRS) pendant une durée d'au moins 9 mois,
(iii) la réduction de la douleur moyenne du patient au genou lors de la marche de sorte que la douleur moyenne du patient au genou lors de la marche n'est pas supérieure à 1 sur l'échelle numérique d'évaluation de la douleur de 0 à 10 (NPRS) pendant une durée d'au moins 10 mois,
(iv) la réduction de la douleur moyenne du patient au genou lors de la marche de sorte que la douleur moyenne du patient au genou lors de la marche n'est pas supérieure à 1 sur l'échelle numérique d'évaluation de la douleur de 0 à 10 (NPRS) pendant une durée d'au moins 11 mois, ou
(v) la réduction de la douleur moyenne du patient au genou lors de la marche de sorte que la douleur moyenne du patient au genou lors de la marche n'est pas supérieure à 1 sur l'échelle numérique d'évaluation de la douleur de 0 à 10 (NPRS) pendant une durée d'au moins 12 mois.

21. Capsaïcine destinée à être utilisée selon l'une quelconque des revendications 1 à 19, dans laquelle l'utilisation est **caractérisée par** :
(i) la réduction de la douleur moyenne du patient au genou lors de la marche de sorte que la douleur moyenne du patient au genou lors de la marche n'est pas supérieure à 2 sur l'échelle numérique d'évaluation de la douleur de 0 à 10 (NPRS) pendant une durée d'au moins 8 mois,
(ii) la réduction de la douleur moyenne du patient au genou lors de la marche de sorte que la douleur moyenne du patient au genou lors de la marche n'est pas supérieure à 2 sur l'échelle numérique d'évaluation de la douleur de 0 à 10 (NPRS) pendant une durée d'au moins 9 mois,
(iii) la réduction de la douleur moyenne du patient au genou lors de la marche de sorte que la douleur moyenne du patient au genou lors de la marche n'est pas supérieure à 2 sur l'échelle numérique d'évaluation de la douleur de 0 à 10 (NPRS) pendant une durée d'au moins 10 mois,
(iv) la réduction de la douleur moyenne du patient au genou lors de la marche de sorte que la douleur moyenne du patient au genou lors de la marche n'est pas supérieure à 2 sur l'échelle numérique d'évaluation de la douleur de 0 à 10 (NPRS) pendant une durée d'au moins 11 mois, ou
(v) la réduction de la douleur moyenne du patient au genou lors de la marche de sorte que la douleur moyenne du patient au genou lors de la marche n'est pas supérieure à 2 sur l'échelle numérique d'évaluation de la douleur de 0 à 10 (NPRS) pendant une durée d'au moins 12 mois.
